# EUROPEAN PATENT APPLICATION

(11) **EP 4 074 818 A2**
(43) Date of publication of application: **19.10.2022**
(21) Application number: 22161080.1
(22) Date of filing: 08.12.2017
(51) Int. Cl.: C12N 5/071, G01N 33/50, A61P 25/32, A61P 25/36, A61P 25/30

(54) **TISSUE ORGANOIDS**

(30) Priority: 09.12.2016 US 201662432346 P; 09.12.2016 US 201662432350 P; 05.09.2017 US 201762554560 P
(62) Divisional of application: 17835745.5
(71) Applicant: The University of Chicago, Chicago, IL 60637 (US)
(72) Inventor: WU, Xiaoyang, Chicago, IL 60615 (US); XU, Ming, Chicago, IL 60615 (US); YUE, Jiping, Chicago, IL 60615 (US)
(74) Representative: Thomann, William John

(57) **Abstract**

Physiologically-tailored tissue organoids are disclosed for monitoring and treating diseases and improving an individual's health.

SEQUENCE LISTING

<110>  The University of Chicago

<120>  Tissue Organoids

<130>  150-406 PCTEPT1

<140>
<141>  2017-12-08

<150>  US 62/432,346
<151>  2016-12-09

<150>  US 62/432,350
<151>  2016-12-09

<150>  US 62/554,560
<151>  2017-09-05

<160>  67

<170>  PatentIn version 3.5

<210>  1
<211>  1071
<212>  DNA
<213>  Unknown

<220>
<223>  Luciferase

<400>  1
atgccagagc cagcgaagtc tgctcccgcc ccgaaaaagg gctccaagaa ggcggtgact      60

aaggcgcaga agaaaggcgg caagaagcgc aagcgcagcc gcaaggagag ctattccatc     120

tatgtgtaca aggttctgaa gcaggtccac cctgacaccg gcatttcgtc caaggccatg     180

ggcatcatga actcgtttgt gaacgacatt ttcgagcgca tcgcaggtga ggcttcccgc     240

ctggcgcatt acaacaagcg ctcgaccatc acctccaggg agatccagac ggccgtgcgc     300

ctgctgctgc ctggggagtt ggccaagcac gccgtgtccg agggtactaa ggccatcacc     360

aagtacacca gcgctaagga tccaccggtc gccaccatgg cctcctccga ggacgtcatc     420

aaggagttca tgcgcttcaa ggtgcgcatg gagggctccg tgaacggcca cgagttcgag     480

atcgagggcg agggcgaggg ccgcccctac gagggcaccc agaccgccaa gctgaaggtg     540

accaagggcg gccccctgcc cttcgcctgg gacatcctgt cccctcagtt ccagtacggc     600

tccaaggcct acgtgaagca ccccgccgac atccccgact acttgaagct gtccttcccc     660

gagggcttca agtgggagcg cgtgatgaac ttcgaggacg gcggcgtggt gaccgtgacc     720

caggactcct ccctgcagga cggcgagttc atctacaagg tgaagctgcg cggcaccaac     780

ttcccctccg acggccccgt aatgcagaag aagaccatgg gctgggaggc ctccaccgag     840

cggatgtacc ccgaggacgg cgccctgaag ggcgagatca agatgaggct gaagctgaag     900

gacggcggcc actacgacgc cgaggtcaag accacctaca tggccaagaa gcccgtgcag      960

ctgcccggcg cctacaagac cgacatcaag ctggacatca cctcccacaa cgaggactac     1020

accatcgtgg aacagtacga gcgcgccgag ggccgccact ccaccggcgc c              1071


<210>  2
<211>  1648
<212>  DNA
<213>  Unknown

<220>
<223>  H2B-RFP

<400>  2
atggaagacg ccaaaaacat aaagaaaggc ccggcgccat tctatccgct ggaagatgga       60

accgctggag agcaactgca taaggctatg aagagatacg ccctggttcc tggaacaatt      120

gcttttacag atgcacatat cgaggtggac atcacttacg ctgagtactt cgaaatgtcc      180

gttcggttgg cagaagctat gaaacgatat gggctgaata caaatcacag aatcgtcgta      240

tgcagtgaaa actctcttca attctttatg ccggtgttgg gcgcgttatt tatcggagtt      300

gcagttgcgc ccgcgaacga catttataat gaacgtgaat tgctcaacag tatgggcatt      360

tcgcagccta ccgtggtgtt cgtttccaaa aaggggttgc aaaaaatttt gaacgtgcaa      420

aaaaagctcc caatcatcca aaaaattatt atcatggatt ctaaaacgga ttaccaggga      480

tttcagtcga tgtacacgtt cgtcacatct catctacctc ccggttttaa tgaatacgat      540

tttgtgccag agtccttcga tagggacaag acaattgcac tgatcatgaa ctcctctgga      600

tctactggtc tgcctaaagg tgtcgctctg cctcatagaa ctgcctgcgt gagattctcg      660

catgccagag atcctatttt tggcaatcaa atcattccgg atactgcgat tttaagtgtt      720

gttccattcc atcacggttt tggaatgttt actacactcg gatatttgat atgtggattt      780

cgagtcgtct taatgtatag atttgaagaa gagctgtttc tgaggagcct tcaggattac      840

aagattcaaa gtgcgctgct ggtgccaacc ctattctcct tcttcgccaa aagcactctg      900

attgacaaat acgatttatc taatttacac gaaattgctt ctggtggcgc tcccctctct      960

aaggaagtcg gggaagcggt tgccaagagg ttccatctgc caggtatcag gcaaggatat     1020

gggctcactg agactacatc agctattctg attacacccg agggggatga taaaccgggc     1080

gcggtcggta aagttgttcc attttttgaa gcgaaggttg tggatctgga taccgggaaa     1140

acgctgggcg ttaatcaaag aggcgaactg tgtgtgagag gtcctatgat tatgtccggt     1200

tatgtaaaca atccggaagc gaccaacgcc ttgattgaca aggatggatg gctacattct     1260

ggagacatag cttactggga cgaagacgaa cacttcttca tcgttgaccg cctgaagtct     1320

ctgattaagt acaaaggcta tcaggtggct cccgctgaat tggaatccat cttgctccaa     1380

caccccaaca tcttcgacgc aggtgtcgca ggtcttcccg acgatgacgc cggtgaactt     1440

cccgccgccg ttgttgtttt ggagcacgga aagacgatga cggaaaaaga gatcgtggat 1500

tacgtcgcca gtcaagtaac aaccgcgaaa aagttgcgcg gaggagttgt gtttgtggac 1560

gaagtaccga aaggtcttac cggaaaactc gacgcaagaa aaatcagaga gatcctcata 1620

aaggccaaga agggcggaaa gatcgccg 1648

<210> 3
<211> 34
<212> DNA
<213> Unknown

<220>
<223> gRNA expression cassette forward primer

<400> 3
aaggaaaaaa gcggccgctg tacaaaaaag cagg 34

<210> 4
<211> 24
<212> DNA
<213> Unknown

<220>
<223> gRNA expression cassette reverse primer

<400> 4
ggaattctaa tgccaacttt gtac 24

<210> 5
<211> 26
<212> DNA
<213> Unknown

<220>
<223> Rosa26-targeting gRNA forward primer 1

<400> 5
acaccggcag gcttaaaggc taaccg 26

<210> 6
<211> 26
<212> DNA
<213> Unknown

<220>
<223> Rosa26-targeting gRNA reverse primer 1

<400> 6
aaaacggtta gcctttaagc ctgccg 26

<210> 7
<211> 26
<212> DNA
<213> Unknown

<220>
<223>  Rosa26-targeting gRNA forward primer 2

<400>  7
acaccgagga caacgcccac acaccg                                          26


<210>  8
<211>  26
<212>  DNA
<213>  Unknown

<220>
<223>  Rosa26-targeting gRNA reverse primer 2

<400>  8
aaaacggtgt gtgggcgttg tcctcg                                          26


<210>  9
<211>  26
<212>  DNA
<213>  Unknown

<220>
<223>  AAVS1-targeting gRNA forward primer 1

<400>  9
acaccgtcac caatcctgtc cctagg                                          26


<210>  10
<211>  26
<212>  DNA
<213>  Unknown

<220>
<223>  AAVS1-targeting gRNA reverse primer 1

<400>  10
aaaacctagg gacaggattg gtgacg                                          26


<210>  11
<211>  26
<212>  DNA
<213>  Unknown

<220>
<223>  AAVS1-targeting gRNA forward primer 2

<400>  11
acaccgcccc acagtggggc cactag                                          26


<210>  12
<211>  26
<212>  DNA
<213>  Unknown

<220>
<223>  AAVS1-targeting gRNA reverse primer 2

<400>  12
aaaactagtg gccccactgt ggggcg                                          26


<210>  13
<211>  50
<212>  DNA
<213>  Unknown

<220>
<223>  Rosa26 targeting vector forward primer 1

<400>  13
gactagtgaa ttcggatcct taattaaggc ctccgcgccg ggttttggcg                50


<210>  14
<211>  47
<212>  DNA
<213>  Unknown

<220>
<223>  Rosa26 targeting vector reverse primer 1

<400>  14
gactagtccc gggggatcca ccggtcagga acaggtggtg gcggccc                   47


<210>  15
<211>  37
<212>  DNA
<213>  Unknown

<220>
<223>  Rosa26 targeting vector forward primer 2

<400>  15
cgggatccac cggtgagggc agaggaagcc ttctaac                              37


<210>  16
<211>  33
<212>  DNA
<213>  Unknown

<220>
<223>  Rosa26 targeting vector reverse primer 2

<400>  16
tcccccgggt acaaaatcag aaggacaggg aag                                  33


<210>  17
<211>  35
<212>  DNA
<213>  Unknown

<220>
<223>  Rosa26 targeting vector forward primer 3

<400>  17
ggaattcaat aaaatatctt tattttcatt acatc                                35

<210>  18
<211>  59
<212>  DNA
<213>  Unknown

<220>
<223>  Rosa26 targeting vector reverse primer 3

<400>  18
ccttaattaa ggatccacgc gtgtttaaac accggtttta cgagggtagg aagtggtac        59


<210>  19
<211>  37
<212>  DNA
<213>  Unknown

<220>
<223>  AAVS1 targeting vector constructed with AAVS1 hPGK-PuroR-pA donor
       forward primer 1

<400>  19
cccaagcttc tcgagttggg gttgcgcctt ttccaag                                37


<210>  20
<211>  31
<212>  DNA
<213>  Unknown

<220>
<223>  AAVS1 targeting vector constructed with AAVS1 hPGK-PuroR-pA donor
       reverse primer 1

<400>  20
cccaagcttc catagagccc accgcatccc c                                      31


<210>  21
<211>  44
<212>  DNA
<213>  Unknown

<220>
<223>  AAVS1 targeting vector constructed with AAVS1 hPGK-PuroR-pA donor
       forward primer 2

<400>  21
cagggtctag acgccggatc cggtaccctg tgccttctag ttgc                        44


<210>  22
<211>  37
<212>  DNA
<213>  Unknown

<220>
<223>  AAVS1 targeting vector constructed with AAVS1 hPGK-PuroR-pA donor
       reverse primer 2

<400>  22
ggatccggcg tctagaccct ggggagagag gtcggtg                                37

<210>  23
<211>  37
<212>  DNA
<213>  Unknown

<220>
<223>  AAVS1 targeting vector constructed with AAVS1 hPGK-PuroR-pA donor
       forward primer 3

<400>  23
ccgctcgaga ataaaatatc tttattttca ttacatc                                37


<210>  24
<211>  30
<212>  DNA
<213>  Unknown

<220>
<223>  AAVS1 targeting vector constructed with AAVS1 hPGK-PuroR-pA donor
       reverse primer 3

<400>  24
gctctagacc aagtgacgat cacagcgatc                                        30


<210>  25
<211>  22
<212>  DNA
<213>  Unknown

<220>
<223>  Genotyping primer for CRISPR mediated knockin #1

<400>  25
gagctgggac caccttatat tc                                                22


<210>  26
<211>  17
<212>  DNA
<213>  Unknown

<220>
<223>  Genotyping primer for CRISPR mediated knockin #2

<400>  26
ggtgcatgac ccgcaag                                                      17


<210>  27
<211>  21
<212>  DNA
<213>  Unknown

<220>
<223>  Genotyping primer for CRISPR mediated knockin #3

<400>  27
gagagatggc tccaggaaat g                                                 21

<210>  28
<211>  2391
<212>  DNA
<213>  Unknown

<220>
<223>  CFP/YFP FRET sensor with A213R/L238S double mutant of GGBP

<400>  28
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgacctg gggcgtgcag tgcttcagcc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacat cagccacaac gtctatatca ccgccgacaa gcagaagaac     480

ggcatcaagg ccaacttcaa gatccgccac aacatcgagg acggcagcgt gcagctcgcc     540

gaccactacc agcagaacac ccccatcggc gacggccccg tgctgctgcc cgacaaccac     600

tacctgagca cccagtccgc cctgagcaaa gaccccaacg agaagcgcga tcacatggtc     660

ctgctggagt tcgtgaccgc cgccgggatc actctcggca tggacgagct gtacaagggt     720

ggtaccggag gcgccgctga tactcgcatt ggtgtaacaa tctataagta cgacgataac     780

tttatgtctg tagtgcgcaa ggctattgag caagatgcga aagccgcgcc agatgttcag     840

ctgctgatga atgattctca gaatgaccag tccaagcaga acgatcagat cgacgtattg     900

ctggcgaaag gggtgaaggc actggcaatc aacctggttg acccggcagc tgcgggtacg     960

gtgattgaga aagcgcgtgg gcaaaacgtg ccggtggttt tcttcaacaa agaaccgtct    1020

cgtaaggcgc tggatagcta cgacaaagcc tactacgttg gcactgactc caaagagtcc    1080

ggcattattc aaggcgattt gattgctaaa cactgggcgg cgaatcaggg ttgggatctg    1140

aacaaagacg gtcagattca gttcgtactg ctgaaaggtg aaccgggcca tccggatgca    1200

gaagcacgta ccacttacgt gattaaagaa ttgaacgata aaggcatcaa aactgaacag    1260

ttacagttag ataccgcaat gtgggacacc gctcaggcga aagataagat ggacgcctgg    1320

ctgtctggcc cgaacgccaa caaaatcgaa gtggttatcg ccaacaacga tcggatggca    1380

atgggcgcgg ttgaagcgct gaaagcacac aacaagtcca gcattccggt gtttggcgtc    1440

gatgcgtcgc cagaagcgct ggcgctggtg aaatccggtg cactggcggg caccgtactg    1500

aacgatgcta acaaccaggc gaaagcgacc tttgatctgg cgaaaaacct ggccgatggt    1560

aaaggtgcgg ctgatggcac caactggaaa atcgacaaca aagtggtccg cgtaccttat    1620

gttggcgtag ataaagacaa cctggctgaa ttcagcaaga aaggcgccgg taccggtgga 1680

atggtgagca agggcgagga gctgttcacc ggggtggtgc ccatcctggt cgagctggac 1740

ggcgacgtaa acggccacaa gttcagcgtg tccggcgagg gcgagggcga tgccacctac 1800

ggcaagctga ccctgaagtt catctgcacc accggcaagc tgcccgtgcc ctggcccacc 1860

ctcgtgacca ccttcggcta cggcctgcag tgcttcgccc gctaccccga ccacatgaag 1920

cagcacgact tcttcaagtc cgccatgccc gaaggctacg tccaggagcg caccatcttc 1980

ttcaaggacg acggcaacta caagacccgc gccgaggtga agttcgaggg cgacaccctg 2040

gtgaaccgca tcgagctgaa gggcatcgac ttcaaggagg acggcaacat cctggggcac 2100

aagctggagt acaactacaa cagccacaac gtctatatca tggccgacaa gcagaagaac 2160

ggcatcaagg tgaacttcaa gatccgccac aacatcgagg acggcagcgt gcagctcgcc 2220

gaccactacc agcagaacac ccccatcggc gacggccccg tgctgctgcc cgacaaccac 2280

tacctgagct accagtccgc cctgagcaaa gaccccaacg agaagcgcga tcacatggtc 2340

ctgctggagt tcgtgaccgc cgccgggatc actctcggca tggacgagct g 2391

<210> 29
<211> 4146
<212> DNA
<213> Unknown

<220>
<223> Cas9 D10A

<400> 29
gccaccatgg acaagaagta ctccattggg ctcgctatcg gcacaaacag cgtcggctgg 60

gccgtcatta cggacgagta caaggtgccg agcaaaaaat tcaaagttct gggcaatacc 120

gatcgccaca gcataaagaa gaacctcatt ggcgccctcc tgttcgactc cggggagacg 180

gccgaagcca cgcggctcaa aagaacagca cggcgcagat atacccgcag aaagaatcgg 240

atctgctacc tgcaggagat ctttagtaat gagatggcta aggtggatga ctctttcttc 300

cataggctgg aggagtcctt tttggtggag gaggataaaa agcacgagcg ccacccaatc 360

tttggcaata tcgtggacga ggtggcgtac catgaaaagt acccaaccat atatcatctg 420

aggaagaagc ttgtagacag tactgataag gctgacttgc ggttgatcta tctcgcgctg 480

gcgcatatga tcaaatttcg gggacacttc ctcatcgagg gggacctgaa cccagacaac 540

agcgatgtcg acaaactctt tatccaactg gttcagactt acaatcagct tttcgaagag 600

aacccgatca acgcatccgg agttgacgcc aaagcaatcc tgagcgctag gctgtccaaa 660

tcccggcggc tcgaaaacct catcgcacag ctccctgggg agaagaagaa cggcctgttt 720

ggtaatctta tcgccctgtc actcgggctg acccccaact ttaaatctaa cttcgacctg 780

gccgaagatg ccaagcttca actgagcaaa gacacctacg atgatgatct cgacaatctg 840

ctggcccaga tcggcgacca gtacgcagac ctttttttgg cggcaaagaa cctgtcagac 900

gccattctgc tgagtgatat tctgcgagtg aacacggaga tcaccaaagc tccgctgagc 960

gctagtatga tcaagcgcta tgatgagcac caccaagact tgactttgct gaaggccctt 1020

gtcagacagc aactgcctga gaagtacaag gaaattttct tcgatcagtc taaaaatggc 1080

tacgccggat acattgacgg cggagcaagc caggaggaat tttacaaatt tattaagccc 1140

atcttggaaa aaatggacgg caccgaggag ctgctggtaa agcttaacag agaagatctg 1200

ttgcgcaaac agcgcacttt cgacaatgga agcatccccc accagattca cctgggcgaa 1260

ctgcacgcta tcctcaggcg gcaagaggat ttctacccct ttttgaaaga taacagggaa 1320

aagattgaga aaatcctcac atttcggata ccctactatg taggccccct cgcccgggga 1380

aattccagat tcgcgtggat gactcgcaaa tcagaagaga ccatcactcc ctggaacttc 1440

gaggaagtcg tggataaggg ggcctctgcc cagtccttca tcgaaaggat gactaacttt 1500

gataaaaatc tgcctaacga aaaggtgctt cctaaacact ctctgctgta cgagtacttc 1560

acagtttata acgagctcac caaggtcaaa tacgtcacag aagggatgag aaagccagca 1620

ttcctgtctg gagagcagaa gaaagctatc gtggacctcc tcttcaagac gaaccggaaa 1680

gttaccgtga aacagctcaa agaagactat ttcaaaaaga ttgaatgttt cgactctgtt 1740

gaaatcagcg gagtggagga tcgcttcaac gcatccctgg gaacgtatca cgatctcctg 1800

aaaatcatta aagacaagga cttcctggac aatgaggaga acgaggacat tcttgaggac 1860

attgtcctca cccttacgtt gtttgaagat agggagatga ttgaagaacg cttgaaaact 1920

tacgctcatc tcttcgacga caaagtcatg aaacagctca agaggcgccg atatacagga 1980

tgggggcggc tgtcaagaaa actgatcaat gggatccgag acaagcagag tggaaagaca 2040

atcctggatt ttcttaagtc cgatggattt gccaaccgga acttcatgca gttgatccat 2100

gatgactctc tcacctttaa ggaggacatc cagaaagcac aagtttctgg ccagggggac 2160

agtcttcacg agcacatcgc taatcttgca ggtagcccag ctatcaaaaa gggaatactg 2220

cagaccgtta aggtcgtgga tgaactcgtc aaagtaatgg gaaggcataa gcccgagaat 2280

atcgttatcg agatggcccg agagaaccaa actacccaga agggacagaa gaacagtagg 2340

gaaaggatga agaggattga agagggtata aaagaactgg ggtcccaaat ccttaaggaa 2400

cacccagttg aaaacaccca gcttcagaat gagaagctct acctgtacta cctgcagaac 2460

ggcagggaca tgtacgtgga tcaggaactg gacatcaatc ggctctccga ctacgacgtg 2520

gatcatatcg tgccccagtc ttttctcaaa gatgattcta ttgataataa agtgttgaca 2580

agatccgata aaaatagagg gaagagtgat aacgtcccct cagaagaagt tgtcaagaaa 2640

atgaaaaatt attggcggca gctgctgaac gccaaactga tcacacaacg gaagttcgat 2700

aatctgacta aggctgaacg aggtggcctg tctgagttgg ataaagccgg cttcatcaaa 2760

aggcagcttg ttgagacacg ccagatcacc aagcacgtgg cccaaattct cgattcacgc 2820

atgaacacca agtacgatga aaatgacaaa ctgattcgag aggtgaaagt tattactctg 2880

aagtctaagc tggtctcaga tttcagaaag gactttcagt tttataaggt gagagagatc 2940

aacaattacc accatgcgca tgatgcctac ctgaatgcag tggtaggcac tgcacttatc 3000

aaaaaatatc ccaagcttga atctgaattt gtttacggag actataaagt gtacgatgtt 3060

aggaaaatga tcgcaaagtc tgagcaggaa ataggcaagg ccaccgctaa gtacttcttt 3120

tacagcaata ttatgaattt tttcaagacc gagattacac tggccaatgg agagattcgg 3180

aagcgaccac ttatcgaaac aaacggagaa acaggagaaa tcgtgtggga caagggtagg 3240

gatttcgcga cagtccggaa ggtcctgtcc atgccgcagg tgaacatcgt taaaaagacc 3300

gaagtacaga ccggaggctt ctccaaggaa agtatcctcc cgaaaaggaa cagcgacaag 3360

ctgatcgcac gcaaaaaaga ttgggacccc aagaaatacg gcggattcga ttctcctaca 3420

gtcgcttaca gtgtactggt tgtggccaaa gtggagaaag ggaagtctaa aaaactcaaa 3480

agcgtcaagg aactgctggg catcacaatc atggagcgat caagcttcga aaaaaacccc 3540

atcgactttc tcgaggcgaa aggatataaa gaggtcaaaa aagacctcat cattaagctt 3600

cccaagtact ctctctttga gcttgaaaac ggccggaaac gaatgctcgc tagtgcgggc 3660

gagctgcaga aaggtaacga gctggcactg ccctctaaat acgttaattt cttgtatctg 3720

gccagccact atgaaaagct caaagggtct cccgaagata atgagcagaa gcagctgttc 3780

gtggaacaac acaaacacta ccttgatgag atcatcgagc aaataagcga attctccaaa 3840

agagtgatcc tcgccgacgc taacctcgat aaggtgcttt ctgcttacaa taagcacagg 3900

gataagccca tcaggagcaa ggcagaaaac attatccact tgtttactct gaccaacttg 3960

ggcgcgcctg cagccttcaa gtacttcgac accaccatag acagaaagcg gtacacctct 4020

acaaaggagg tcctggacgc cacactgatt catcagtcaa ttacggggct ctatgaaaca 4080

agaatcgacc tctctcagct cggtggagac agcagggctg accccaagaa gaagaggaag 4140

gtgtga 4146

<210> 30
<211> 23
<212> DNA
<213> Unknown

<220>
<223> Rosa26 gRNA1

<400> 30
ggcaggctta aaggctaacc tgg 23

<210>  31
<211>  23
<212>  DNA
<213>  Unknown

<220>
<223>  Rosa26 gRNA2

<400>  31
gactggagtt gcagatcacg agg                                                23


<210>  32
<211>  7695
<212>  DNA
<213>  Unknown

<220>
<223>  Rosa26 targeting vector



<220>
<221>  misc_feature
<222>  (195)..(195)
<223>  n is a, c, g, or t

<220>
<221>  misc_feature
<222>  (230)..(230)
<223>  n is a, c, g, or t

<220>
<221>  misc_feature
<222>  (380)..(380)
<223>  n is a, c, g, or t

<220>
<221>  misc_feature
<222>  (3724)..(3724)
<223>  n is a, c, g, or t

<220>
<221>  misc_feature
<222>  (4213)..(4213)
<223>  n is a, c, g, or t

<220>
<221>  misc_feature
<222>  (4297)..(4297)
<223>  n is a, c, g, or t

<220>
<221>  misc_feature
<222>  (4874)..(4874)
<223>  n is a, c, g, or t

<220>
<221>  misc_feature
<222>  (4931)..(4931)
<223>  n is a, c, g, or t

<220>

<221>  misc_feature
<222>  (4953)..(4953)
<223>  n is a, c, g, or t

<220>
<221>  misc_feature
<222>  (4993)..(4993)
<223>  n is a, c, g, or t

<220>
<221>  misc_feature
<222>  (5644)..(5644)
<223>  n is a, c, g, or t

<220>
<221>  misc_feature
<222>  (6667)..(6667)
<223>  n is a, c, g, or t

<220>
<221>  misc_feature
<222>  (6769)..(6769)
<223>  n is a, c, g, or t

<220>
<221>  misc_feature
<222>  (7147)..(7147)
<223>  n is a, c, g, or t

<220>
<221>  misc_feature
<222>  (7562)..(7562)
<223>  n is a, c, g, or t

<400>  32
ccgcggcagg ccctccgagc gtggtggagc cgttctgtga gacagccggg tacgagtcgt      60

gacgctggaa ggggcaagcg ggtggtgggc aggaatgcgg tccgccctgc agcaaccgga     120

gggggaggga gaagggagcg gaaaagtctc caccggacgc ggccatggct cggggggggg     180

ggggcagcgg aggancgctt ccggccgacg tctcgtcgct gattggcttn ttttcctccc     240

gccgtgtgtg aaaacacaaa tggcgtgttt tggttggcgt aaggcgcctg tcagttaacg     300

gcagccggag tgcgcagccg ccggcagcct cgctctgccc actgggtggg gcgggaggta     360

ggtggggtga ggcgagctgn acgtgcgggc gcggtcggcc tctggcgggg cgggggaggg     420

gagggagggt cagcgaaagt agctcgcgcg cgagcggccg cccaccctcc ccttcctctg     480

ggggagtcgt tttacccgcc gccggccggg cctcgtcgtc tgattggctc tcggggccca     540

gaaaactggc ccttgccatt ggctcgtgtt cgtgcaagtt gagtccatcc gccggccagc     600

gggggcggcg aggaggcgct cccaggttcc ggccctcccc tcggccccgc gccgcagagt     660

ctggccgcgc gcccctgcgc aacgtggcag gaagcgcgcg ctgggggcgg ggacgggcag     720

tagggctgag cggctgcggg gcgggtgcaa gcacgtttcc gacttgagtt gcctcaagag     780

gggcgtgctg agccagacct ccatcgcgca ctccggggag tggagggaag gagcgagggc     840

tcagttgggc tgttttggag gcaggaagca cttgctctcc caaagtcgct ctgagttgtt 900

atcagtaagg gagctgcagt ggagtaggcg gggagaaggc cgcacccttc tccggagggg 960

ggaggggagt gttgcaatac ctttctggga gttctctgct gcctcctggc ttctgaggac 1020

cgccctgggc ctgggagaat cccttgcccc ctcttcccct cgtgatctgc aactccagtc 1080

tttctagtga attcggatcc ttaattaagg cctccgcgcc gggttttggc gcctcccgcg 1140

ggcgcccccc tcctcacggc gagcgctgcc acgtcagacg aagggcgcag cgagcgtcct 1200

gatccttccg cccggacgct caggacagcg gcccgctgct cataagactc ggccttagaa 1260

ccccagtatc agcagaagga cattttagga cgggacttgg gtgactctag ggcactggtt 1320

ttctttccag agagcggaac aggcgaggaa aagtagtccc ttctcggcga ttctgcggag 1380

ggatctccgt ggggcggtga acgccgatga ttatataagg acgcgccggg tgtggcacag 1440

ctagttccgt cgcagccggg atttgggtcg cggttcttgt ttgtggatcg ctgtgatcgt 1500

cacttggtct agacgccacc atggtgtcca agggcgagga ggtgatcaag gagttcatgc 1560

gcttcaaggt gcgcatggag ggctccatga acggccacga gttcgagatc gagggcgagg 1620

gcgagggccg cccctacgag ggcacccaga ccgccaagct gaaggtgacc aagggcggcc 1680

ccctgccctt cgcctgggac atcctgtccc cccagttcat gtacggctcc aaggcctacg 1740

tgaagcaccc cgccgacatc cccgactaca agaagctgtc cttccccgag ggcttcaagt 1800

gggagcgcgt gatgaacttc gaggacggcg gcctggtgac cgtgacccag gactcctccc 1860

tgcaggacgg caccctgatc tacaaggtga agatgcgcgg caccaacttc ccccccgacg 1920

gccccgtgat gcagaagaag accatgggct gggaggcctc caccgagcgc ctgtaccccc 1980

gcgacggcgt gctgaagggc gagatccacc aggccctgaa gctgaaggac ggcggccact 2040

acctggtgga gttcaagacc atctacatgg ccaagaagcc cgtgcagctg cccggctact 2100

actacgtgga caccaagctg gacatcacct cccacaacga ggactacacc atcgtggagc 2160

agtacgagcg ctccgagggc cgccaccacc tgttcctgac cggtgagggc agaggaagcc 2220

ttctaacatg cggtgacgtg gaggagaatc ccggcccttc cgggatgacc gagtacaagc 2280

ccacggtgcg cctcgccacc cgcgacgacg tccccagggc cgtacgcacc ctcgccgccg 2340

cgttcgccga ctaccccgcc acgcgccaca ccgtcgatcc ggaccgccac atcgagcggg 2400

tcaccgagct gcaagaactc ttcctcacgc gcgtcgggct cgacatcggc aaggtgtggg 2460

tcgcggacga cggcgccgcg gtggcggtct ggaccacgcc ggagagcgtc gaagcggggg 2520

cggtgttcgc cgagatcggc ccgcgcatgg ccgagttgag cggttcccgg ctggccgcgc 2580

agcaacagat ggaaggcctc ctggcgccgc accggcccaa ggagcccgcg tggttcctgg 2640

ccaccgtcgg cgtctcgccc gaccaccagg gcaagggtct gggcagcgcc gtcgtgctcc 2700

ccggagtgga ggcggccgag cgcgccgggg tgcccgcctt cctggagacc tccgcgcccc 2760

gcaacctccc cttctacgag cggctcggct tcaccgtcac cgccgacgtc gaggtgcccg 2820

aaggaccgcg cacctggtgc atgacccgca agcccggtgc ctgaatctag gtcgacctgc 2880

agaagcttgc ctcgagcagc gctgctcgag agatctacgg gtggcatccc tgtgacccct 2940

ccccagtgcc tctcctggcc ctggaagttg ccactccagt gcccaccagc cttgtcctaa 3000

taaaattaag ttgcatcatt ttgtctgact aggtgtcctt ctataatatt atggggtgga 3060

ggggggtggt atggagcaag gggcaagttg ggaagacaac ctgtagggcc tgcggggtct 3120

attgggaacc aagctggagt gcagtggcac aatcttggct cactgcaatc tccgcctcct 3180

gggttcaagc gattctcctg cctcagcctc ccgagttgtt gggattccag gcatgcatga 3240

ccaggctcag ctaatttttg tttttttggt agagacgggg tttcaccata ttggccaggc 3300

tggtctccaa ctcctaatct caggtgatct acccaccttg gcctcccaaa ttgctgggat 3360

tacaggcgtg aaccactgct cccttccctg tccttctgat tttgtacccg ggactagaag 3420

atgggcggga gtcttctggg caggcttaaa ggctaacctg gtgtgtgggc gttgtcctgc 3480

aggggaattg aacaggtgta aaattggagg gacaagactt cccacagatt ttcggttttg 3540

tcgggaagtt ttttaatagg ggcaaatagg aaaatggagg ataggagtca tctggggttt 3600

atgcagcaaa actacaggta tattgcttgt atccgcctcg gagatttcca tgaggagata 3660

aagacatgtc acccgagttt atactctcct gcttagatcc tactacagta tgaaatacag 3720

tgtngcgagg tagactatgt aagcagattt aatcatttta aagagcccag tacttcatat 3780

ccatttctcc cgctccttct gcagccttat caaaaggtat ttagaacact cattttagcc 3840

ccattttcat ttattatact ggcttatcca acccctagac agagcattgg cattttccct 3900

ttcctgatct tagaagtctg atgactcatg aaaccagaca gattagttac atacaccaca 3960

aatcgaggct gtagctgggg cctcaacact gcagttcttt tataactcct tagtacactt 4020

tttgttgatc ctttgccttg atccttaatt ttcagtgtct atcacctctc ccgtcaggtg 4080

gtgttccaca tttgggccta ttctcagtcc agggagtttt acaacaatag atgtattgag 4140

aatccaacct aaagcttaac tttccactcc catgaatgcc tctctccttt ttctccatta 4200

taactgagct atnaccatta atggtttcag gtggatgtct cctcccccaa tatacctgat 4260

gtatctacat attgccaggc tgatatttta agacatnaaa ggtatatttc attattgagc 4320

cacatggtat tgattactgc tactaaaatt ttgtcattgt acacatctgt aaaaggtggt 4380

tccttttgga atgcaaagtt caggtgtttg ttgtctttcc tgacctaagg tcttgtgagc 4440

ttgtattttt tctatttaag cagtgctttc tcttggactg gcttgactca tggcattcta 4500

cacgttattg ctggtctaaa tgtgattttg ccaagcttct tcaggaccta taattttgct 4560

tgacttgtag ccaaacacaa gtaaaatgat taagcaacaa atgtatttgt gaagcttggt 4620

ttttaggttg ttgtgttgtg tgtgcttgtg ctctataata atactatcca ggggctggag 4680
aggtggctcg gagttcaaga gcacagactg ctcttccaga agtcctgagt tcaattccca 4740
gcaaccacat ggtggctcac aaccatctgt aatgggatct gatgccctct tctggtgtgt 4800
ctgaagacca caagtgtatt cacattaaat aaataatcct ccttcttctt cttttttttt 4860
ttttaaagag aatnctgtct ccagtagaat tactgaagta atgaaatact ttgtgtttgt 4920
tccaatatgg nagccaataa tcaaatactc ttnagcactg gaaatgtacc aaggaactat 4980
tttatttaag tgnactgtgg acagaggagc cataactgca gacttgtggg atacagaaga 5040
ccaatgcaga cttaatgtct tttctcttac actaagcaat aaagaaataa aaattgaact 5100
tctagtatcc tatttgttaa actgctagct ttactaactt ttgtgcttca tctatacaaa 5160
gctgaaagct aagtctgcag ccattactaa acatgaaagc aagtaatgat aattttggat 5220
ttcaaaaatg tagggccaga gtttagccag ccagtggtgg tgcttgcctt tatgccttaa 5280
tcccagcact ctggaggcag agacaggcag atctctgagt ttgagcccag cctggtctac 5340
acatcaagtt ctatctagga tagccaggaa tacacacaga aaccctgttg gggagggggg 5400
ctctgagatt tcataaaatt ataattgaag cattccctaa tgagccacta tggatgtggc 5460
taaatccgtc tacctttctg atgagatttg ggtattattt tttctgtctc tgctgttggt 5520
tgggtctttt gacactgtgg gctttcttaa agcctccttc cctgccatgt ggtctcttgt 5580
ttgctactaa cttcccatgg cttaaatggc atggcttttt gccttctaag ggcagctgct 5640
gagntttgca gcctgatttc cagggtgggg ttgggaaatc tttcaaacac taaaattgtc 5700
ctttaatttt tttttaaaaa atgggttata taataaacct cataaaatag ttatgaggag 5760
tgaggtggac taatattaat gagtccctcc cctataaaag agctattaag gctttttgtc 5820
ttatactaac ttttttttta aatgtggtat ctttagaacc aagggtctta gagttttagt 5880
atacagaaac tgttgcatcg cttaatcaga ttttctagtt tcaaatccag agaatccaaa 5940
ttcttcacag ccaaagtcaa attaagaatt tctgacttta atgttatttg ctactgtgaa 6000
tataaaatga tagcttttcc tgaggcaggg tctcactatg tatctctgcc tgatctgcaa 6060
caagatatgt agactaaagt tctgcctgct tttgtctcct gaatactaag gttaaaatgt 6120
agtaatactt ttggaacttg caggtcagat tcttttatag gggacacact aaggagcttt 6180
gggtgatagt tggtaaatgt gtttaagtga tgaaaacttg aattattatc accgcaacct 6240
actttttaaa aaaaaaagcc aggcctgtta gagcatgcta agggatccct aggacttgct 6300
gagcacacaa gagtagtact tggcaggctc ctggtgagag catatttcaa aaaacaaggc 6360
agacaaccaa gaaactacag taaggttacc tgtctttaac catctgcata tacacaggga 6420
tattaaaata ttccaaataa tatttcattc aagttttccc ccatcaaatt gggacatgga 6480
tttctccggt gaataggcag agttggaaac taaacaaatg ttggttttgt gatttgtgaa 6540

attgttttca agtgatagtt aaagcccatg agatacagaa caaagctgct atttcgaggt 6600

ctcttggtta tactcagaag cacttctttg ggtttccctg cactatcctg atcatgtgct 6660

aggcctncct taggctgatt gttgttcaaa taacttaagt ttcctgtcag gtgatgtcat 6720

atgatttcat atatcaaggc aaaacatgtt atatatgtta aacatttgna cttaatgtga 6780

aagttaggtc tttgtgggtt ttgattttaa tttcaaaacc tgagctaaat aagtcatttt 6840

acatgtctta catttggtga attgtatatt gtggtttgca ggcaagactc tctgacctag 6900

taaccctcct atagagcact ttgctgggtc acaagtctag gagtcaagca tttcaccttg 6960

aagttgagac gttttgttag tgtatactag ttatatgttg gaggacatgt ttatccagaa 7020

gatattcagg actatttttg actgggctaa ggaattgatt ctgattagca ctgttagtga 7080

gcattgagtg gcctttaggc ttgaattgga gtcacttgta tatctcaaat aatgctggcc 7140

ttttttnaaa agcccttgtt ctttatcacc ctgttttcta cataattttt gttcaaagaa 7200

atacttgttt ggatctcctt ttgacaacaa tagcatgttt tcaagccata ttttttttcc 7260

tttttttttt tttttttggt ttttcgagac agggtttctc tgtatagccc tggctgtcct 7320

ggaactcact ttgtagacca ggctggcctc gaactcagaa atccgcctgc ctctgcctcc 7380

tgagtgccgg gattaaaggc gtgcaccacc acgcctggct aagttggata ttttgtatat 7440

aactataacc aatactaact ccactgggtg gatttttaat tcagtcagta gtcttaagtg 7500

gtctttattg gcccttatta aaatctactg ttcactctaa cagaggctgt tggactagtg 7560

gnactaagca acttcctacg gatatactag cagataaggg tcagggatag aaactagtct 7620

agcgttttgt atacctacca gcttatacta ccttgttctg atagaaatat ttaggacatc 7680

tagcttatcg atccg 7695

<210> 33
<211> 9435
<212> DNA
<213> Unknown

<220>
<223> Rosa26 targeting vector with GGBP

<220>
<221> misc_feature
<222> (195)..(195)
<223> n is a, c, g, or t

<220>
<221> misc_feature
<222> (230)..(230)
<223> n is a, c, g, or t

<220>
<221> misc_feature

<222>  (380)..(380)
<223>  n is a, c, g, or t

<220>
<221>  misc_feature
<222>  (5464)..(5464)
<223>  n is a, c, g, or t

<220>
<221>  misc_feature
<222>  (5953)..(5953)
<223>  n is a, c, g, or t

<220>
<221>  misc_feature
<222>  (6037)..(6037)
<223>  n is a, c, g, or t

<220>
<221>  misc_feature
<222>  (6614)..(6614)
<223>  n is a, c, g, or t

<220>
<221>  misc_feature
<222>  (6671)..(6671)
<223>  n is a, c, g, or t

<220>
<221>  misc_feature
<222>  (6693)..(6693)
<223>  n is a, c, g, or t

<220>
<221>  misc_feature
<222>  (6733)..(6733)
<223>  n is a, c, g, or t

<220>
<221>  misc_feature
<222>  (7384)..(7384)
<223>  n is a, c, g, or t

<220>
<221>  misc_feature
<222>  (8407)..(8407)
<223>  n is a, c, g, or t

<220>
<221>  misc_feature
<222>  (8509)..(8509)
<223>  n is a, c, g, or t

<220>
<221>  misc_feature
<222>  (8887)..(8887)
<223>  n is a, c, g, or t

<220>
<221>  misc_feature
<222>  (9302)..(9302)
<223>  n is a, c, g, or t

<400>  33
ccgcggcagg ccctccgagc gtggtggagc cgttctgtga gacagccggg tacgagtcgt      60

gacgctggaa ggggcaagcg ggtggtgggc aggaatgcgg tccgccctgc agcaaccgga     120

gggggaggga gaagggagcg gaaaagtctc caccggacgc ggccatggct cggggggggg     180

ggggcagcgg aggancgctt ccggccgacg tctcgtcgct gattggcttn ttttcctccc     240

gccgtgtgtg aaaacacaaa tggcgtgttt tggttggcgt aaggcgcctg tcagttaacg     300

gcagccggag tgcgcagccg ccggcagcct cgctctgccc actgggtggg gcgggaggta     360

ggtggggtga ggcgagctgn acgtgcgggc gcggtcggcc tctggcgggg cgggggaggg     420

gagggagggt cagcgaaagt agctcgcgcg cgagcggccg cccaccctcc ccttcctctg     480

ggggagtcgt tttacccgcc gccggccggg cctcgtcgtc tgattggctc tcggggccca     540

gaaaactggc ccttgccatt ggctcgtgtt cgtgcaagtt gagtccatcc gccggccagc     600

gggggcggcg aggaggcgct cccaggttcc ggccctcccc tcggccccgc gccgcagagt     660

ctggccgcgc gcccctgcgc aacgtggcag gaagcgcgcg ctgggggcgg ggacgggcag     720

tagggctgag cggctgcggg gcgggtgcaa gcacgtttcc gacttgagtt gcctcaagag     780

gggcgtgctg agccagacct ccatcgcgca ctccggggag tggagggaag gagcgagggc     840

tcagttgggc tgttttggag gcaggaagca cttgctctcc caaagtcgct ctgagttgtt     900

atcagtaagg gagctgcagt ggagtaggcg gggagaaggc cgcacccttc tccggagggg     960

ggaggggagt gttgcaatac ctttctggga gttctctgct gcctcctggc ttctgaggac    1020

cgccctgggc ctgggagaat cccttgcccc ctcttcccct cgtgatctgc aactccagtc    1080

tttctagtga attcggatcc ttaattaagg cctccgcgcc gggttttggc gcctcccgcg    1140

ggcgcccccc tcctcacggc gagcgctgcc acgtcagacg aagggcgcag cgagcgtcct    1200

gatccttccg cccggacgct caggacagcg gcccgctgct cataagactc ggccttagaa    1260

ccccagtatc agcagaagga cattttagga cgggacttgg gtgactctag ggcactggtt    1320

ttctttccag agagcggaac aggcgaggaa aagtagtccc ttctcggcga ttctgcggag    1380

ggatctccgt ggggcggtga acgccgatga ttatataagg acgcgccggg tgtggcacag    1440

ctagttccgt cgcagccggg atttgggtcg cggttcttgt ttgtggatcg ctgtgatcgt    1500

cacttggtct agaggatccg ggccgcatgg tgagcaaggg cgaggagctg ttcaccgggg    1560

tggtgcccat cctggtcgag ctggacggcg acgtaaacgg ccacaagttc agcgtgtccg    1620

gcgagggcga gggcgatgcc acctacggca agctgaccct gaagttcatc tgcaccaccg    1680

gcaagctgcc cgtgccctgg cccaccctcg tgaccaccct gacctggggc gtgcagtgct    1740

tcagccgcta ccccgaccac atgaagcagc acgacttctt caagtccgcc atgcccgaag    1800

gctacgtcca ggagcgcacc atcttcttca aggacgacgg caactacaag acccgcgccg    1860

aggtgaagtt cgagggcgac accctggtga accgcatcga gctgaagggc atcgacttca 1920

aggaggacgg caacatcctg gggcacaagc tggagtacaa ctacatcagc cacaacgtct 1980

atatcaccgc cgacaagcag aagaacggca tcaaggccaa cttcaagatc cgccacaaca 2040

tcgaggacgg cagcgtgcag ctcgccgacc actaccagca gaacaccccc atcggcgacg 2100

gccccgtgct gctgcccgac aaccactacc tgagcaccca gtccgccctg agcaaagacc 2160

ccaacgagaa gcgcgatcac atggtcctgc tggagttcgt gaccgccgcc gggatcactc 2220

tcggcatgga cgagctgtac aagggtggta ccggaggcgc cgctgatact cgcattggtg 2280

taacaatcta taagtacgac gataacttta tgtctgtagt gcgcaaggct attgagcaag 2340

atgcgaaagc cgcgccagat gttcagctgc tgatgaatga ttctcagaat gaccagtcca 2400

agcagaacga tcagatcgac gtattgctgg cgaaaggggt gaaggcactg gcaatcaacc 2460

tggttgaccc ggcagctgcg ggtacggtga ttgagaaagc gcgtgggcaa aacgtgccgg 2520

tggttttctt caacaaagaa ccgtctcgta aggcgctgga tagctacgac aaagcctact 2580

acgttggcac tgactccaaa gagtccggca ttattcaagg cgatttgatt gctaaacact 2640

gggcggcgaa tcagggttgg gatctgaaca aagacggtca gattcagttc gtactgctga 2700

aaggtgaacc gggccatccg gatgcagaag cacgtaccac ttacgtgatt aaagaattga 2760

acgataaagg catcaaaact gaacagttac agttagatac cgcaatgtgg gacaccgctc 2820

aggcgaaaga taagatggac gcctggctgt ctggcccgaa cgccaacaaa atcgaagtgg 2880

ttatcgccaa caacgatcgg atggcaatgg gcgcggttga agcgctgaaa gcacacaaca 2940

agtccagcat tccggtgttt ggcgtcgatg cgtcgccaga agcgctggcg ctggtgaaat 3000

ccggtgcact ggcgggcacc gtactgaacg atgctaacaa ccaggcgaaa gcgacctttg 3060

atctggcgaa aaacctggcc gatggtaaag gtgcggctga tggcaccaac tggaaaatcg 3120

acaacaaagt ggtccgcgta ccttatgttg gcgtagataa agacaacctg gctgaattca 3180

gcaagaaagg cgccggtacc ggtggaatgg tgagcaaggg cgaggagctg ttcaccgggg 3240

tggtgcccat cctggtcgag ctggacggcg acgtaaacgg ccacaagttc agcgtgtccg 3300

gcgagggcga gggcgatgcc acctacggca agctgaccct gaagttcatc tgcaccaccg 3360

gcaagctgcc cgtgccctgg cccaccctcg tgaccacctt cggctacggc ctgcagtgct 3420

tcgcccgcta ccccgaccac atgaagcagc acgacttctt caagtccgcc atgcccgaag 3480

gctacgtcca ggagcgcacc atcttcttca aggacgacgg caactacaag acccgcgccg 3540

aggtgaagtt cgagggcgac accctggtga accgcatcga gctgaagggc atcgacttca 3600

aggaggacgg caacatcctg gggcacaagc tggagtacaa ctacaacagc cacaacgtct 3660

atatcatggc cgacaagcag aagaacggca tcaaggtgaa cttcaagatc cgccacaaca 3720

tcgaggacgg cagcgtgcag ctcgccgacc actaccagca gaacaccccc atcggcgacg 3780

gccccgtgct gctgcccgac aaccactacc tgagctacca gtccgccctg agcaaagacc 3840

ccaacgagaa gcgcgatcac atggtcctgc tggagttcgt gaccgccgcc gggatcactc 3900

tcggcatgga cgagctgtac aaggaggtgg gcggaagctc cggtgagggc agaggaagcc 3960

ttctaacatg cggtgacgtg gaggagaatc ccggcccttc cgggatgacc gagtacaagc 4020

ccacggtgcg cctcgccacc cgcgacgacg tccccagggc cgtacgcacc ctcgccgccg 4080

cgttcgccga ctaccccgcc acgcgccaca ccgtcgatcc ggaccgccac atcgagcggg 4140

tcaccgagct gcaagaactc ttcctcacgc gcgtcgggct cgacatcggc aaggtgtggg 4200

tcgcggacga cggcgccgcg gtggcggtct ggaccacgcc ggagagcgtc gaagcggggg 4260

cggtgttcgc cgagatcggc ccgcgcatgg ccgagttgag cggttcccgg ctggccgcgc 4320

agcaacagat ggaaggcctc ctggcgccgc accggcccaa ggagcccgcg tggttcctgg 4380

ccaccgtcgg cgtctcgccc gaccaccagg gcaagggtct gggcagcgcc gtcgtgctcc 4440

ccggagtgga ggcggccgag cgcgccgggg tgcccgcctt cctggagacc tccgcgcccc 4500

gcaacctccc cttctacgag cggctcggct tcaccgtcac cgccgacgtc gaggtgcccg 4560

aaggaccgcg cacctggtgc atgacccgca agcccggtgc ctgaatctag gtcgacctgc 4620

agaagcttgc ctcgagcagc gctgctcgag agatctacgg gtggcatccc tgtgacccct 4680

ccccagtgcc tctcctggcc ctggaagttg ccactccagt gcccaccagc cttgtcctaa 4740

taaaattaag ttgcatcatt ttgtctgact aggtgtcctt ctataatatt atggggtgga 4800

ggggggtggt atggagcaag gggcaagttg ggaagacaac ctgtagggcc tgcggggtct 4860

attgggaacc aagctggagt gcagtggcac aatcttggct cactgcaatc tccgcctcct 4920

gggttcaagc gattctcctg cctcagcctc ccgagttgtt gggattccag gcatgcatga 4980

ccaggctcag ctaatttttg tttttttggt agagacgggg tttcaccata ttggccaggc 5040

tggtctccaa ctcctaatct caggtgatct acccaccttg gcctcccaaa ttgctgggat 5100

tacaggcgtg aaccactgct cccttccctg tccttctgat tttgtacccg ggactagaag 5160

atgggcggga gtcttctggg caggcttaaa ggctaacctg gtgtgtgggc gttgtcctgc 5220

aggggaattg aacaggtgta aaattggagg gacaagactt cccacagatt ttcggttttg 5280

tcgggaagtt ttttaatagg ggcaaatagg aaaatggagg ataggagtca tctggggttt 5340

atgcagcaaa actacaggta tattgcttgt atccgcctcg gagatttcca tgaggagata 5400

aagacatgtc acccgagttt atactctcct gcttagatcc tactacagta tgaaatacag 5460

tgtngcgagg tagactatgt aagcagattt aatcatttta aagagcccag tacttcatat 5520

ccatttctcc cgctccttct gcagccttat caaaaggtat ttagaacact cattttagcc 5580

ccattttcat ttattatact ggcttatcca acccctagac agagcattgg cattttccct 5640

ttcctgatct tagaagtctg atgactcatg aaaccagaca gattagttac atacaccaca 5700

aatcgaggct gtagctgggg cctcaacact gcagttcttt tataactcct tagtacactt 5760

tttgttgatc ctttgccttg atccttaatt ttcagtgtct atcacctctc ccgtcaggtg 5820

gtgttccaca tttgggccta ttctcagtcc agggagtttt acaacaatag atgtattgag 5880

aatccaacct aaagcttaac tttccactcc catgaatgcc tctctccttt ttctccatta 5940

taactgagct atnaccatta atggtttcag gtggatgtct cctcccccaa tatacctgat 6000

gtatctacat attgccaggc tgatatttta agacatnaaa ggtatatttc attattgagc 6060

cacatggtat tgattactgc tactaaaatt ttgtcattgt acacatctgt aaaaggtggt 6120

tccttttgga atgcaaagtt caggtgtttg ttgtctttcc tgacctaagg tcttgtgagc 6180

ttgtattttt tctatttaag cagtgctttc tcttggactg gcttgactca tggcattcta 6240

cacgttattg ctggtctaaa tgtgattttg ccaagcttct tcaggaccta taattttgct 6300

tgacttgtag ccaaacacaa gtaaaatgat taagcaacaa atgtatttgt gaagcttggt 6360

ttttaggttg ttgtgttgtg tgtgcttgtg ctctataata atactatcca ggggctggag 6420

aggtggctcg gagttcaaga gcacagactg ctcttccaga agtcctgagt tcaattccca 6480

gcaaccacat ggtggctcac aaccatctgt aatgggatct gatgccctct tctggtgtgt 6540

ctgaagacca caagtgtatt cacattaaat aaataatcct ccttcttctt cttttttttt 6600

ttttaaagag aatnctgtct ccagtagaat tactgaagta atgaaatact ttgtgtttgt 6660

tccaatatgg nagccaataa tcaaatactc ttnagcactg gaaatgtacc aaggaactat 6720

tttatttaag tgnactgtgg acagaggagc cataactgca gacttgtggg atacagaaga 6780

ccaatgcaga cttaatgtct tttctcttac actaagcaat aaagaaataa aaattgaact 6840

tctagtatcc tatttgttaa actgctagct ttactaactt ttgtgcttca tctatacaaa 6900

gctgaaagct aagtctgcag ccattactaa acatgaaagc aagtaatgat aattttggat 6960

ttcaaaaatg tagggccaga gtttagccag ccagtggtgg tgcttgcctt tatgccttaa 7020

tcccagcact ctggaggcag agacaggcag atctctgagt ttgagcccag cctggtctac 7080

acatcaagtt ctatctagga tagccaggaa tacacacaga aaccctgttg gggagggggg 7140

ctctgagatt tcataaaatt ataattgaag cattccctaa tgagccacta tggatgtggc 7200

taaatccgtc tacctttctg atgagatttg ggtattattt tttctgtctc tgctgttggt 7260

tgggtctttt gacactgtgg gctttcttaa agcctccttc cctgccatgt ggtctcttgt 7320

ttgctactaa cttcccatgg cttaaatggc atggcttttt gccttctaag ggcagctgct 7380

gagntttgca gcctgatttc cagggtgggg ttgggaaatc tttcaaacac taaaattgtc 7440

ctttaatttt tttttaaaaa atgggttata taataaacct cataaaatag ttatgaggag 7500

tgaggtggac taatattaat gagtccctcc cctataaaag agctattaag gctttttgtc 7560

ttatactaac ttttttttta aatgtggtat ctttagaacc aagggtctta gagttttagt 7620

atacagaaac tgttgcatcg cttaatcaga ttttctagtt tcaaatccag agaatccaaa 7680

ttcttcacag ccaaagtcaa attaagaatt tctgacttta atgttatttg ctactgtgaa 7740

tataaaatga tagcttttcc tgaggcaggg tctcactatg tatctctgcc tgatctgcaa 7800

caagatatgt agactaaagt tctgcctgct tttgtctcct gaatactaag gttaaaatgt 7860

agtaatactt ttggaacttg caggtcagat tcttttatag gggacacact aagggagctt 7920

gggtgatagt tggtaaatgt gtttaagtga tgaaaacttg aattattatc accgcaacct 7980

actttttaaa aaaaaaagcc aggcctgtta gagcatgcta agggatccct aggacttgct 8040

gagcacacaa gagtagtact tggcaggctc ctggtgagag catatttcaa aaaacaaggc 8100

agacaaccaa gaaactacag taaggttacc tgtctttaac catctgcata tacacaggga 8160

tattaaaata ttccaaataa tatttcattc aagttttccc ccatcaaatt gggacatgga 8220

tttctccggt gaataggcag agttggaaac taaacaaatg ttggttttgt gatttgtgaa 8280

attgttttca agtgatagtt aaagcccatg agatacagaa caaagctgct atttcgaggt 8340

ctcttggtta tactcagaag cacttctttg ggtttccctg cactatcctg atcatgtgct 8400

aggcctncct taggctgatt gttgttcaaa taacttaagt ttcctgtcag gtgatgtcat 8460

atgatttcat atatcaaggc aaaacatgtt atatatgtta aacatttgna cttaatgtga 8520

aagttaggtc tttgtgggtt ttgattttaa tttcaaaacc tgagctaaat aagtcatttt 8580

acatgtctta catttggtga attgtatatt gtggtttgca ggcaagactc tctgacctag 8640

taaccctcct atagagcact ttgctgggtc acaagtctag gagtcaagca tttcaccttg 8700

aagttgagac gttttgttag tgtatactag ttatatgttg gaggacatgt ttatccagaa 8760

gatattcagg actatttttg actgggctaa ggaattgatt ctgattagca ctgttagtga 8820

gcattgagtg gcctttaggc ttgaattgga gtcacttgta tatctcaaat aatgctggcc 8880

ttttttnaaa agcccttgtt ctttatcacc ctgttttcta cataattttt gttcaaagaa 8940

atacttgttt ggatctcctt ttgacaacaa tagcatgttt tcaagccata ttttttttcc 9000

tttttttttt tttttttggt ttttcgagac agggtttctc tgtatagccc tggctgtcct 9060

ggaactcact ttgtagacca ggctggcctc gaactcagaa atccgcctgc ctctgcctcc 9120

tgagtgccgg gattaaaggc gtgcaccacc acgcctggct aagttggata ttttgtatat 9180

aactataacc aatactaact ccactgggtg gatttttaat tcagtcagta gtcttaagtg 9240

gtctttattg gcccttatta aaatctactg ttcactctaa cagaggctgt tggactagtg 9300

gnactaagca acttcctacg gatatactag cagataaggg tcaggatagg aaactagtct 9360

agcgttttgt atacctacca gcttatacta ccttgttctg atagaaatat ttaggacatc 9420

tagcttatcg atccg                                                    9435

<210>  34
<211>  747
<212>  DNA
<213>  Unknown

<220>
<223>  IgGFc (mouse)

<400>  34
atgtacagga tgcaactcct gtcttgcatt gcactaagtc ttgcacttgt cacgaattcg      60

atatcggcca tggttagatc tggttgtaag ccttgcatat gtacagtccc agaagtatca     120

tctgtcttca tcttcccccc aaagcccaag gatgtgctca ccattactct gactcctaag     180

gtcacgtgtg ttgtggtaga catcagcaag gatgatcccg aggtccagtt cagctggttt     240

gtagatgatg tggaggtgca cacagctcag acgcaacccc gggaggagca gttcaacagc     300

actttccgct cagtcagtga acttcccatc atgcaccagg actggctcaa tggcaaggag     360

ttcaaatgca gggtcaacag tgcagctttc cctgccccca tcgagaaaac catctccaaa     420

accaaaggca gaccgaaggc tccacaggtg tacaccattc cacctcccaa ggagcagatg     480

gccaaggata aagtcagtct gacctgcatg ataacagact tcttccctga agacattact     540

gtggagtggc agtggaatgg gcagccagcg gagaactaca agaacactca gcccatcatg     600

gacacagatg gctcttactt cgtctacagc aagctcaatg tgcagaagag caactgggag     660

gcaggaaata ctttcacctg ctctgtgtta catgagggcc tgcacaacca ccatactgag     720

aagagcctct cccactctcc tggtaaa                                         747

<210>  35
<211>  195
<212>  DNA
<213>  Unknown

<220>
<223>  S2-VaTx3 with signal

<400>  35
atgcgccgcg gccgcctgct ggagatcgcc ctgggcttca ccgtgctgct ggcctcctac      60

acctcccacg gcgccgacgc cgctgaattc gagtgccgct ggtacctggg cggctgcaag     120

gaggactccg agtgctgcga gcacctgcag tgccactcct actgggagtg gtgcctgtgg     180

gacggctcct tctaa                                                      195

<210>  36
<211>  324
<212>  DNA
<213>  Unknown

<220>

<223>  S2-DkTx with signal

<400>  36
atgcgccgcg gccgcctgct ggagatcgcc ctgggcttca ccgtgctgct ggcctcctac      60

acctcccacg gcgccgacgc cgctgaattc ggatctgact gcgccaagga gggcgaggtg     120

tgctcctggg gcaagaagtg ctgcgacctg gacaacttct actgccccat ggagttcatc     180

ccccactgca agaagtacaa gccctacgtg cccgtgacca ccaactgcgc caaggagggc     240

gaggtgtgcg gctggggctc caagtgctgc cacggcctgg actgccccct ggccttcatc     300

ccctactgcg agaagtaccg ctaa                                            324


<210>  37
<211>  873
<212>  DNA
<213>  Unknown

<220>
<223>  VatX3 target vector cassette: signal->VaTx3->Furin->IgGFc

<400>  37
atgtacagga tgcaactcct gtcttgcatt gcactaagtc ttgcacttgt cacgaattcg      60

ggatctgagt gccgctggta cctgggcggc tgcaaggagg actccgagtg ctgcgagcac     120

ctgcagtgcc actcctactg ggagtggtgc ctgtgggacg gctccttccg acggaagcga     180

gggatatcgg ccatggttag atctggttgt aagccttgca tatgtacagt cccagaagta     240

tcatctgtct tcatcttccc cccaaagccc aaggatgtgc tcaccattac tctgactcct     300

aaggtcacgt gtgttgtggt agacatcagc aaggatgatc ccgaggtcca gttcagctgg     360

tttgtagatg atgtggaggt gcacacagct cagacgcaac cccgggagga gcagttcaac     420

agcactttcc gctcagtcag tgaacttccc atcatgcacc aggactggct caatggcaag     480

gagttcaaat gcagggtcaa cagtgcagct ttccctgccc ccatcgagaa aaccatctcc     540

aaaaccaaag gcagaccgaa ggctccacag gtgtacacca ttccacctcc caaggagcag     600

atggccaagg ataaagtcag tctgacctgc atgataacag acttcttccc tgaagacatt     660

actgtggagt ggcagtggaa tgggcagcca gcggagaact acaagaacac tcagcccatc     720

atggacacag atggctctta cttcgtctac agcaagctca atgtgcagaa gagcaactgg     780

gaggcaggaa atactttcac ctgctctgtg ttacatgagg gcctgcacaa ccaccatact     840

gagaagagcc tctcccactc tcctggtaaa taa                                  873


<210>  38
<211>  993
<212>  DNA
<213>  Unknown

<220>
<223>  DkTx target vector cassette: signal->DkTx->Furin->IgGFc

<400>  38
atgtacagga tgcaactcct gtcttgcatt gcactaagtc ttgcacttgt cacgaattcg      60

ggatctgact gcgccaagga gggcgaggtg tgctcctggg gcaagaagtg ctgcgacctg     120

gacaacttct actgccccat ggagttcatc ccccactgca agaagtacaa gccctacgtg     180

cccgtgacca ccaactgcgc caaggagggc gaggtgtgcg gctggggctc caagtgctgc     240

cacggcctgg actgccccct ggccttcatc ccctactgcg agaagtaccg ccggaagcga     300

gggatatcgg ccatggttag atctggttgt aagccttgca tatgtacagt cccagaagta     360

tcatctgtct tcatcttccc cccaaagccc aaggatgtgc tcaccattac tctgactcct     420

aaggtcacgt gtgttgtggt agacatcagc aaggatgatc ccgaggtcca gttcagctgg     480

tttgtagatg atgtggaggt gcacacagct cagacgcaac cccgggagga gcagttcaac      540

agcactttcc gctcagtcag tgaacttccc atcatgcacc aggactggct caatggcaag     600

gagttcaaat gcagggtcaa cagtgcagct ttccctgccc ccatcgagaa aaccatctcc     660

aaaaccaaag gcagaccgaa ggctccacag gtgtacacca ttccacctcc caaggagcag     720

atggccaagg ataaagtcag tctgacctgc atgataacag acttcttccc tgaagacatt     780

actgtggagt ggcagtggaa tgggcagcca gcggagaact acaagaacac tcagcccatc     840

atggacacag atggctctta cttcgtctac agcaagctca atgtgcagaa gagcaactgg     900

gaggcaggaa atactttcac ctgctctgtg ttacatgagg gcctgcacaa ccaccatact     960

gagaagagcc tctcccactc tcctggtaaa taa                                  993


<210>  39
<211>  681
<212>  DNA
<213>  Unknown

<220>
<223>  IgGFc (human)

<400>  39
gacaaaactc acacatgccc accgtgccca gcacctgaac tcctgggggg accgtcagtc      60

ttcctcttcc ccccaaaacc caaggacacc ctcatgatct cccggacccc tgaggtcaca     120

tgcgtggtgg tggacgtgag ccacgaagac cctgaggtca agttcaactg gtacgtggac     180

ggcgtggagg tgcataatgc caagacaaag ccgcgggagg agcagtacaa cagcacgtac     240

cgtgtggtca gcgtcctcac cgtcctgcac caggactggc tgaatggcaa ggagtacaag     300

tgcaaggtct ccaacaaagc cctcccagcc cccatcgaga aaaccatctc caaagccaaa     360

gggcagcccc gagaaccaca ggtgtacacc ctgcccccat cccgggagga gatgaccaag     420

aaccaggtca gcctgacctg cctggtcaaa ggcttctatc ccagcgacat cgccgtggag     480

tgggagagca atgggcagcc ggagaacaac tacaagacca cgcctcccgt gctggactcc     540

gacggctcct tcttcctcta cagcaagctc accgtggaca agagcaggtg gcagcagggg 600

aacgtcttct catgctccgt gatgcacgag gctctgcaca accactacac gcagaagagc 660

ctctccctgt ctccgggtaa a 681

<210> 40
<211> 3778
<212> DNA
<213> Unknown

<220>
<223> AAVSI targeting vector

<400> 40
tgctttctct gaccagcatt ctctcccctg ggcctgtgcc gctttctgtc tgcagcttgt 60

ggcctgggtc acctctacgg ctggcccaga tccttccctg ccgcctcctt caggttccgt 120

cttcctccac tccctcttcc ccttgctctc tgctgtgttg ctgcccaagg atgctctttc 180

cggagcactt ccttctcggc gctgcaccac gtgatgtcct ctgagcggat cctccccgtg 240

tctgggtcct ctccgggcat ctctcctccc tcacccaacc ccatgccgtc ttcactcgct 300

gggttccctt ttccttctcc ttctggggcc tgtgccatct ctcgtttctt aggatggcct 360

tctccgacgg atgtctccct tgcgtcccgc ctccccttct tgtaggcctg catcatcacc 420

gtttttctgg acaaccccaa agtaccccgt ctccctggct ttagccacct ctccatcctc 480

ttgctttctt tgcctggaca ccccgttctc ctgtggattc gggtcacctc tcactccttt 540

catttgggca gctcccctac cccccttacc tctctagtct gtgctagctc ttccagcccc 600

ctgtcatggc atcttccagg ggtccgagag ctcagctagt cttcttcctc caacccgggc 660

ccctatgtcc acttcaggac agcatgtttg ctgcctccag ggatcctgtg tccccgagct 720

gggaccacct tatattccca gggccggtta atgtggctct ggttctgggt acttttatct 780

gtcccctcca ccccacagtg gggcaagctt ctcgagttgg ggttgcgcct tttccaaggc 840

agccctgggt ttgcgcaggg acgcggctgc tctgggcgtg gttccgggaa acgcagcggc 900

gccgaccctg ggtctcgcac attcttcacg tccgttcgca gcgtcacccg gatcttcgcc 960

gctacccttg tgggcccccc ggcgacgctt cctgctccgc ccctaagtcg ggaaggttcc 1020

ttgcggttcg cggcgtgccg gacgtgacaa acggaagccg cacgtctcac tagtaccctc 1080

gcagacggac agcgccaggg agcaatggca gcgcgccgac cgcgatgggc tgtggccaat 1140

agcggctgct cagcagggcg cgccgagagc agcggccggg aaggggcggt gcgggaggcg 1200

gggtgtgggg cggtagtgtg ggccctgttc ctgcccgcgc ggtgttccgc attctgcaag 1260

cctccggagc gcacgtcggc agtcggctcc ctcgttgacc gaatcaccga cctctctccc 1320

cagggtctag acgccaccat ggtgtccaag ggcgaggagg tgatcaagga gttcatgcgc 1380

ttcaaggtgc gcatggaggg ctccatgaac ggccacgagt tcgagatcga gggcgagggc 1440

gagggccgcc cctacgaggg cacccagacc gccaagctga aggtgaccaa gggcggcccc 1500

ctgcccttcg cctgggacat cctgtccccc cagttcatgt acggctccaa ggcctacgtg 1560

aagcaccccg ccgacatccc cgactacaag aagctgtcct tccccgaggg cttcaagtgg 1620

gagcgcgtga tgaacttcga ggacggcggc ctggtgaccg tgacccagga ctcctccctg 1680

caggacggca ccctgatcta caaggtgaag atgcgcggca ccaacttccc ccccgacggc 1740

cccgtgatgc agaagaagac catgggctgg gaggcctcca ccgagcgcct gtacccccgc 1800

gacggcgtgc tgaagggcga gatccaccag gccctgaagc tgaaggacgg cggccactac 1860

ctggtggagt tcaagaccat ctacatggcc aagaagcccg tgcagctgcc cggctactac 1920

tacgtggaca ccaagctgga catcacctcc cacaacgagg actacaccat cgtggagcag 1980

tacgagcgct ccgagggccg ccaccacctg ttcctgggat ccgagggcag aggaagcctt 2040

ctaacatgcg gtgacgtgga ggagaatccc ggcccttccg ggatgaccga gtacaagccc 2100

acggtgcgcc tcgccacccg cgacgacgtc cccagggccg tacgcaccct cgccgccgcg 2160

ttcgccgact accccgccac gcgccacacc gtcgatccgg accgccacat cgagcgggtc 2220

accgagctgc aagaactctt cctcacgcgc gtcgggctcg acatcggcaa ggtgtgggtc 2280

gcggacgacg gcgccgcggt ggcggtctgg accacgccgg agagcgtcga agcgggggcg 2340

gtgttcgccg agatcggccc gcgcatggcc gagttgagcg gttcccggct ggccgcgcag 2400

caacagatgg aaggcctcct ggcgccgcac cggcccaagg agcccgcgtg gttcctggcc 2460

accgtcggcg tctcgcccga ccaccagggc aagggtctgg gcagcgccgt cgtgctcccc 2520

ggagtggagg cggccgagcg cgccggggtg cccgccttcc tggagacctc cgcgccccgc 2580

aacctcccct tctacgagcg gctcggcttc accgtcaccg ccgacgtcga ggtgcccgaa 2640

ggaccgcgca cctggtgcat gacccgcaag cccggtgcct gaatctaggt cgacattcta 2700

cttggtaccc tgtgccttct agttgccagc catctgttgt ttgcccctcc cccgtgcctt 2760

ccttgaccct ggaaggtgcc actcccactg tcctttccta ataaaatgag gaaattgcat 2820

cgcattgtct gagtaggtgt cattctattc tggggggtgg ggtggggcag gacagcaagg 2880

gggaggattg ggaagacaat agcaggcatg ctggggatgc ggtgggctct atggaagctt 2940

tactagggac aggattggtg acagaaaagc cccatcctta ggcctcctcc ttcctagtct 3000

cctgatattg ggtctaaccc ccacctcctg ttaggcagat tccttatctg gtgacacacc 3060

cccatttcct ggagccatct ctctccttgc cagaacctct aaggtttgct tacgatggag 3120

ccagagagga tcctgggagg gagagcttgg cagggggtgg gagggaaggg ggggatgcgt 3180

gacctgcccg gttctcagtg gccaccctgc gctaccctct cccagaacct gagctgctct 3240

gacgcggctg tctggtgcgt ttcactgatc ctggtgctgc agcttcctta cacttcccaa 3300

gaggagaagc agtttggaaa aacaaaatca gaataagttg gtcctgagtt ctaactttgg 3360

ctcttcacct ttctagtccc caatttatat tgttcctccg tgcgtcagtt ttacctgtga 3420

gataaggcca gtagccagcc ccgtcctggc agggctgtgg tgaggagggg ggtgtccgtg 3480

tggaaaactc cctttgtgag aatggtgcgt cctaggtgtt caccaggtcg tggccgcctc 3540

tactcccttt ctctttctcc atccttcttt ccttaaagag tccccagtgc tatctgggac 3600

atattcctcc gcccagagca gggtcccgct tccctaaggc cctgctctgg gcttctgggt 3660

ttgagtcctt ggcaagccca ggagaggcgc tcaggcttcc ctgtccccct tcctcgtcca 3720

ccatctcatg cccctggctc tcctgcccct tccctacagg ggttcctggc tctgctct 3778

<210> 41
<211> 602
<212> PRT
<213> Unknown

<220>
<223> Human mutant BChE

<400> 41

Met His Ser Lys Val Thr Ile Ile Cys Ile Arg Phe Leu Phe Trp Phe
1               5                   10                  15

Leu Leu Leu Cys Met Leu Ile Gly Lys Ser His Thr Glu Asp Asp Ile
            20                  25                  30

Ile Ile Ala Thr Lys Asn Gly Lys Val Arg Gly Met Asn Leu Thr Val
        35                  40                  45

Phe Gly Gly Thr Val Thr Ala Phe Leu Gly Ile Pro Tyr Ala Gln Pro
    50                  55                  60

Pro Leu Gly Arg Leu Arg Phe Lys Lys Pro Gln Ser Leu Thr Lys Trp
65                  70                  75                  80

Ser Asp Ile Trp Asn Ala Thr Lys Tyr Ala Asn Ser Cys Cys Gln Asn
                85                  90                  95

Ile Asp Gln Ser Phe Pro Gly Phe His Gly Ser Glu Met Trp Asn Pro
            100                 105                 110

Asn Thr Asp Leu Ser Glu Asp Cys Leu Tyr Leu Asn Val Trp Ile Pro
        115                 120                 125

Ala Pro Lys Pro Lys Asn Ala Thr Val Leu Ile Trp Ile Tyr Gly Gly
    130                 135                 140

Gly Phe Gln Thr Gly Thr Ser Ser Leu His Val Tyr Asp Gly Lys Phe
145                 150                 155                 160

Leu Ala Arg Val Glu Arg Val Ile Val Val Ser Met Asn Tyr Arg Val
                165                 170                 175

Gly Ala Leu Gly Phe Leu Ala Leu Pro Gly Asn Pro Glu Ala Pro Gly
            180                 185                 190

Asn Met Gly Leu Phe Asp Gln Gln Leu Ala Leu Gln Trp Val Gln Lys
        195                 200                 205

Asn Ile Ala Ala Phe Gly Gly Asn Pro Lys Ser Val Thr Leu Phe Gly
    210                 215                 220

Glu Ser Ser Gly Ala Ala Ser Val Ser Leu His Leu Leu Ser Pro Gly
225                 230                 235                 240

Ser His Ser Leu Phe Thr Arg Ala Ile Leu Gln Ser Gly Ser Ala Asn
                245                 250                 255

Ala Pro Trp Ala Val Thr Ser Leu Tyr Glu Ala Arg Asn Arg Thr Leu
            260                 265                 270

Asn Leu Ala Lys Leu Thr Gly Cys Ser Arg Glu Asn Glu Thr Glu Ile
        275                 280                 285

Ile Lys Cys Leu Arg Asn Lys Asp Pro Gln Glu Ile Leu Leu Asn Glu
    290                 295                 300

Ala Phe Val Val Pro Tyr Gly Thr Ala Leu Gly Val Asn Phe Gly Pro
305                 310                 315                 320

Thr Val Asp Gly Asp Phe Leu Thr Asp Met Pro Asp Ile Leu Leu Glu
                325                 330                 335

Leu Gly Gln Phe Lys Lys Thr Gln Ile Leu Val Gly Val Asn Lys Asp
            340                 345                 350

Glu Gly Thr Trp Phe Leu Val Gly Gly Ala Pro Gly Phe Ser Lys Asp
        355                 360                 365

Asn Asn Ser Ile Ile Thr Arg Lys Glu Phe Gln Glu Gly Leu Lys Ile
    370                 375                 380

Phe Phe Pro Gly Val Ser Glu Phe Gly Lys Glu Ser Ile Leu Phe His
385                 390                 395                 400

Tyr Thr Asp Trp Val Asp Asp Gln Arg Pro Glu Asn Tyr Arg Glu Ala
                405                 410                 415

Leu Gly Asp Val Val Gly Asp Tyr Asn Phe Ile Cys Pro Ala Leu Glu
            420                 425                 430

Phe Thr Lys Lys Phe Ser Glu Trp Gly Asn Asn Ala Phe Phe Tyr Tyr
        435                 440                 445

Phe Glu His Arg Ser Ser Lys Leu Pro Trp Pro Glu Trp Met Gly Val
    450                 455                 460

Met His Gly Tyr Glu Ile Glu Phe Val Phe Gly Leu Pro Leu Glu Arg
465                 470                 475                 480

Arg Asp Asn Tyr Thr Lys Ala Glu Glu Ile Leu Ser Arg Ser Ile Val
                485                 490                 495

Lys Arg Trp Ala Asn Phe Ala Lys Tyr Gly Asn Pro Asn Glu Thr Gln
            500                 505                 510

Asn Asn Ser Thr Ser Trp Pro Val Phe Lys Ser Thr Glu Gln Lys Tyr
        515                 520                 525

Leu Thr Leu Asn Thr Glu Ser Thr Arg Ile Met Thr Lys Leu Arg Ala
    530                 535                 540

Gln Gln Cys Arg Phe Trp Thr Ser Phe Phe Pro Lys Val Leu Glu Met
545                 550                 555                 560

Thr Gly Asn Ile Asp Glu Ala Glu Trp Glu Trp Lys Ala Gly Phe His
                565                 570                 575

Arg Trp Asn Asn Tyr Met Met Asp Trp Lys Asn Gln Phe Asn Asp Tyr
            580                 585                 590

Thr Ser Lys Lys Glu Ser Cys Val Gly Leu
        595                 600

<210>  42
<211>  1806
<212>  DNA
<213>  Unknown

<220>
<223>  Nucleic acid sequence encoding the amino acid sequence of SEQ ID
       NO: 41

<400>  42
atgcacagca aggtgaccat catctgcatc aggttcctgt tctggttcct gctgctgtgc      60

atgctgatcg gcaagagcca caccgaggac gacatcatca tcgccaccaa gaacggcaag     120

gtgaggggca tgaacctgac cgtgttcggc ggcaccgtga ccgccttcct gggcatcccc     180

tacgcccagc cccccctggg caggctgagg ttcaagaagc cccagagcct gaccaagtgg     240

agcgacatct ggaacgccac caagtacgcc aacagctgct gccagaacat cgaccagagc     300

ttccccggct tccacggcag cgagatgtgg aaccccaaca ccgacctgag cgaggactgc     360

ctgtacctga acgtgtggat ccccgccccc aagcccaaga acgccaccgt gctgatctgg     420

atctacggcg gcggcttcca gaccggcacc agcagcctgc acgtgtacga cggcaagttc     480

ctggccaggg tggagagggt gatcgtggtg agcatgaact acagggtggg cgccctgggc     540

ttcctggccc tgcccggcaa ccccgaggcc cccggcaaca tgggcctgtt cgaccagcag     600

ctggccctgc agtgggtgca gaagaacatc gccgccttcg gcggcaaccc caagagcgtg     660

accctgttcg gcgagagcag cggcgccgcc agcgtgagcc tgcacctgct gagccccggc     720

agccacagcc tgttcaccag ggccatcctg cagagcggca gcgccaacgc cccctgggcc     780

gtgaccagcc tgtacgaggc caggaacagg accctgaacc tggccaagct gaccggctgc     840

agcagggaga acgagaccga gatcatcaag tgcctgagga acaaggaccc ccaggagatc     900

ctgctgaacg aggccttcgt ggtgccctac ggcaccgccc tgggcgtgaa cttcggcccc     960

accgtggacg gcgacttcct gaccgacatg cccgacatcc tgctggagct gggccagttc    1020

aagaagaccc agatcctggt gggcgtgaac aaggacgagg gcacctggtt cctggtgggc    1080

ggcgcccccg gcttcagcaa ggacaacaac agcatcatca ccaggaagga gttccaggag    1140

ggcctgaaga tcttcttccc cggcgtgagc gagttcggca aggagagcat cctgttccac    1200

tacaccgact gggtggacga ccagaggccc gagaactaca gggaggccct gggcgacgtg    1260

gtgggcgact acaacttcat ctgccccgcc ctggagttca ccaagaagtt cagcgagtgg    1320

ggcaacaacg ccttcttcta ctacttcgag cacaggagca gcaagctgcc ctggcccgag    1380

tggatgggcg tgatgcacgg ctacgagatc gagttcgtgt tcggcctgcc cctggagagg    1440

agggacaact acaccaaggc cgaggagatc ctgagcagga gcatcgtgaa gaggtgggcc    1500

aacttcgcca agtacggcaa ccccaacgag acccagaaca acagcaccag ctggcccgtg    1560

ttcaagagca ccgagcagaa gtacctgacc ctgaacaccg agagcaccag gatcatgacc    1620

aagctgaggg cccagcagtg caggttctgg accagcttct tccccaaggt gctggagatg    1680

accggcaaca tcgacgaggc cgagtgggag tggaaggccg gcttccacag gtggaacaac    1740

tacatgatgg actggaagaa ccagttcaac gactacacca gcaagaagga gagctgcgtg    1800

ggcctg                                                              1806

<210>  43
<211>  603
<212>  PRT
<213>  Unknown

<220>
<223>  Mouse mutant BChE

<400>  43

Met Gln Thr Gln His Thr Lys Val Thr Gln Thr His Phe Leu Leu Trp
1               5                   10                  15

Ile Leu Leu Leu Cys Met Pro Phe Gly Lys Ser His Thr Glu Glu Asp
            20                  25                  30

Phe Ile Ile Thr Thr Lys Thr Gly Arg Val Arg Gly Leu Ser Met Pro
        35                  40                  45

Val Leu Gly Gly Thr Val Thr Ala Phe Leu Gly Ile Pro Tyr Ala Gln
    50                  55                  60

Pro Pro Leu Gly Ser Leu Arg Phe Lys Lys Pro Gln Pro Leu Asn Lys
65                  70                  75                  80

Trp Pro Asp Ile His Asn Ala Thr Gln Tyr Ala Asn Ser Cys Tyr Gln
                85                  90                  95

Asn Ile Asp Gln Ala Phe Pro Gly Phe Gln Gly Ser Glu Met Trp Asn
            100                 105                 110

Pro Asn Thr Asn Leu Ser Glu Asp Cys Leu Tyr Leu Asn Val Trp Ile
        115                 120                 125

Pro Val Pro Lys Pro Lys Asn Ala Thr Val Met Val Trp Ile Tyr Gly
    130                 135                 140

Gly Gly Phe Gln Thr Gly Thr Ser Ser Leu Pro Val Tyr Asp Gly Lys
145                 150                 155                 160

Phe Leu Ala Arg Val Glu Arg Val Ile Val Val Ser Met Asn Tyr Arg
                165                 170                 175

Val Gly Ala Leu Gly Phe Leu Ala Phe Pro Gly Asn Pro Asp Ala Pro
            180                 185                 190

Gly Asn Met Gly Leu Phe Asp Gln Gln Leu Ala Leu Gln Trp Val Gln
        195                 200                 205

Arg Asn Ile Ala Ala Phe Gly Gly Asn Pro Lys Ser Ile Thr Ile Phe
    210                 215                 220

Gly Glu Ser Ser Gly Ala Ala Ser Val Ser Leu His Leu Leu Cys Pro
225                 230                 235                 240

Gln Ser Tyr Pro Leu Phe Thr Arg Ala Ile Leu Glu Ser Gly Ser Ala
                245                 250                 255

Asn Ala Pro Trp Ala Val Lys His Pro Glu Glu Ala Arg Asn Arg Thr
            260                 265                 270

Leu Thr Leu Ala Lys Phe Thr Gly Cys Ser Lys Glu Asn Glu Met Glu
        275                 280                 285

Met Ile Lys Cys Leu Arg Ser Lys Asp Pro Gln Glu Ile Leu Arg Asn
    290                 295                 300

Glu Arg Phe Val Leu Pro Ser Asp Ser Ala Leu Gly Ile Asn Phe Gly
305                 310                 315                 320

Pro Thr Val Asp Gly Asp Phe Leu Thr Asp Met Pro His Thr Leu Leu
                325                 330                 335

Gln Leu Gly Lys Val Lys Lys Ala Gln Ile Leu Val Gly Val Asn Lys
            340                 345                 350

Asp Glu Gly Thr Trp Phe Leu Val Gly Gly Ala Pro Gly Phe Ser Lys
        355                 360                 365

Asp Asn Asp Ser Leu Ile Thr Arg Lys Glu Phe Gln Glu Gly Leu Asn
    370                 375                 380

Met Tyr Phe Pro Gly Val Ser Arg Leu Gly Lys Glu Ala Val Leu Phe
385                 390                 395                 400

Tyr Tyr Val Asp Trp Leu Gly Glu Gln Ser Pro Glu Val Tyr Arg Asp
                405                 410                 415

Ala Leu Asp Asp Val Ile Gly Asp Tyr Asn Ile Ile Cys Pro Ala Leu
            420                 425                 430

Glu Phe Thr Lys Lys Phe Ala Glu Leu Glu Asn Asn Ala Phe Phe Tyr
        435                 440                 445

Phe Phe Glu His Arg Ser Ser Lys Leu Pro Trp Pro Glu Trp Met Gly
    450                 455                 460

Val Met His Gly Tyr Glu Ile Glu Phe Val Phe Gly Leu Pro Leu Gly
465                 470                 475                 480

Arg Arg Val Asn Tyr Thr Arg Ala Glu Glu Ile Phe Ser Arg Ser Ile
                485                 490                 495

Met Lys Thr Trp Ala Asn Phe Ala Lys Tyr Gly His Pro Asn Gly Thr
            500                 505                 510

Gln Gly Asn Ser Thr Met Trp Pro Val Phe Thr Ser Thr Glu Gln Lys
        515                 520                 525

Tyr Leu Thr Leu Asn Thr Glu Lys Ser Lys Ile Tyr Ser Lys Leu Arg
    530                 535                 540

Ala Pro Gln Cys Gln Phe Trp Arg Leu Phe Phe Pro Lys Val Leu Glu
545                 550                 555                 560

Met Thr Gly Asp Ile Asp Glu Thr Glu Gln Glu Trp Lys Ala Gly Phe
                565                 570                 575

His Arg Trp Ser Asn Tyr Met Met Asp Trp Gln Asn Gln Phe Asn Asp
            580                 585                 590

Tyr Thr Ser Lys Lys Glu Ser Cys Thr Ala Leu
        595                 600

<210>  44
<211>  1809
<212>  DNA
<213>  Unknown

<220>
<223>  Nucleic acid sequence encoding the amino acid sequence of SEQ ID
       NO: 43

<400>  44
atgcagaccc agcacaccaa ggtgacccag acccacttcc tgctgtggat cctgctgctg      60

tgcatgccct tcggcaagag ccacaccgag gaggacttca tcatcaccac caagaccggc     120

agggtgaggg gcctgagcat gcccgtgctg ggcggcaccg tgaccgcctt cctgggcatc     180

ccctacgccc agccccccct gggcagcctg aggttcaaga agccccagcc cctgaacaag     240

tggcccgaca tccacaacgc cacccagtac gccaacagct gctaccagaa catcgaccag     300

gccttccccg gcttccaggg cagcgagatg tggaacccca acaccaacct gagcgaggac     360

tgcctgtacc tgaacgtgtg gatccccgtg cccaagccca agaacgccac cgtgatggtg     420

tggatctacg gcggcggctt ccagaccggc accagcagcc tgcccgtgta cgacggcaag     480

ttcctggcca gggtggagag ggtgatcgtg gtgagcatga actacagggt gggcgccctg 540

ggcttcctgg ccttccccgg caaccccgac gcccccggca acatgggcct gttcgaccag 600

cagctggccc tgcagtgggt gcagaggaac atcgccgcct tcggcggcaa ccccaagagc 660

atcaccatct tcggcgagag cagcggcgcc gccagcgtga gcctgcacct gctgtgcccc 720

cagagctacc ccctgttcac cagggccatc ctggagagcg gcagcgccaa cgccccctgg 780

gccgtgaagc accccgagga ggccaggaac aggaccctga ccctggccaa gttcaccggc 840

tgcagcaagg agaacgagat ggagatgatc aagtgcctga ggagcaagga cccccaggag 900

atcctgagga acgagaggtt cgtgctgccc agcgacagcg ccctgggcat caacttcggc 960

cccaccgtgg acggcgactt cctgaccgac atgccccaca ccctgctgca gctgggcaag 1020

gtgaagaagg cccagatcct ggtgggcgtg aacaaggacg agggcacctg gttcctggtg 1080

ggcggcgccc ccggcttcag caaggacaac gacagcctga tcaccaggaa ggagttccag 1140

gagggcctga acatgtactt ccccggcgtg agcaggctgg gcaaggaggc cgtgctgttc 1200

tactacgtgg actggctggg cgagcagagc cccgaggtgt acagggacgc cctggacgac 1260

gtgatcggcg actacaacat catctgcccc gccctggagt tcaccaagaa gttcgccgag 1320

ctggagaaca acgccttctt ctacttcttc gagcacagga gcagcaagct gccctggccc 1380

gagtggatgg gcgtgatgca cggctacgag atcgagttcg tgttcggcct gcccctgggc 1440

aggagggtga actacaccag ggccgaggag atcttcagca ggagcatcat gaagacctgg 1500

gccaacttcg ccaagtacgg ccaccccaac ggcacccagg gcaacagcac catgtggccc 1560

gtgttcacca gcaccgagca gaagtacctg accctgaaca ccgagaagag caagatctac 1620

agcaagctga gggcccccca gtgccagttc tggaggctgt tcttccccaa ggtgctggag 1680

atgaccggcg acatcgacga gaccgagcag gagtggaagg ccggcttcca caggtggagc 1740

aactacatga tggactggca gaaccagttc aacgactaca ccagcaagaa ggagagctgc 1800

accgccctg 1809

<210> 45
<211> 275
<212> PRT
<213> Unknown

<220>
<223> mGLP-1 (GLY8 mutant with IgG-Fc fusion)

<400> 45

Met Tyr Arg Met Gln Leu Leu Ser Cys Ile Ala Leu Ser Leu Ala Leu
1               5                   10                  15

Val Thr Asn Ser His Gly Glu Gly Thr Phe Thr Ser Asp Val Ser Ser
            20                  25                  30

Tyr Leu Glu Gly Gln Ala Ala Lys Glu Phe Ile Ala Trp Leu Val Lys
        35                  40                  45

Gly Arg Gly Arg Ser Gly Cys Lys Pro Cys Ile Cys Thr Val Pro Glu
    50                  55                  60

Val Ser Ser Val Phe Ile Phe Pro Pro Lys Pro Lys Asp Val Leu Thr
65                  70                  75                  80

Ile Thr Leu Thr Pro Lys Val Thr Cys Val Val Val Asp Ile Ser Lys
                85                  90                  95

Asp Asp Pro Glu Val Gln Phe Ser Trp Phe Val Asp Asp Val Glu Val
            100                 105                 110

His Thr Ala Gln Thr Gln Pro Arg Glu Glu Gln Phe Asn Ser Thr Phe
        115                 120                 125

Arg Ser Val Ser Glu Leu Pro Ile Met His Gln Asp Trp Leu Asn Gly
    130                 135                 140

Lys Glu Phe Lys Cys Arg Val Asn Ser Ala Ala Phe Pro Ala Pro Ile
145                 150                 155                 160

Glu Lys Thr Ile Ser Lys Thr Lys Gly Arg Pro Lys Ala Pro Gln Val
                165                 170                 175

Tyr Thr Ile Pro Pro Pro Lys Glu Gln Met Ala Lys Asp Lys Val Ser
            180                 185                 190

Leu Thr Cys Met Ile Thr Asp Phe Phe Pro Glu Asp Ile Thr Val Glu
        195                 200                 205

Trp Gln Trp Asn Gly Gln Pro Ala Glu Asn Tyr Lys Asn Thr Gln Pro
    210                 215                 220

Ile Met Asp Thr Asp Gly Ser Tyr Phe Val Tyr Ser Lys Leu Asn Val
225                 230                 235                 240

Gln Lys Ser Asn Trp Glu Ala Gly Asn Thr Phe Thr Cys Ser Val Leu
                245                 250                 255

His Glu Gly Leu His Asn His His Thr Glu Lys Ser Leu Ser His Ser
            260                 265                 270

Pro Gly Lys

        275

<210>  46
<211>  825
<212>  DNA
<213>  Unknown

<220>
<223>  Nucleic acid sequence encoding the amino acid sequence of SEQ ID
       NO: 45

<400>  46
atgtacagga tgcagctgct gagctgcatc gccctgagcc tggccctggt gaccaacagc      60

cacggcgagg gcaccttcac cagcgacgtg agcagctacc tggagggcca ggccgccaag     120

gagttcatcg cctggctggt gaagggcagg ggcaggagcg gctgcaagcc ctgcatctgc     180

accgtgcccg aggtgagcag cgtgttcatc ttccccccca agcccaagga cgtgctgacc     240

atcaccctga cccccaaggt gacctgcgtg gtggtggaca tcagcaagga cgaccccgag     300

gtgcagttca gctggttcgt ggacgacgtg gaggtgcaca ccgcccagac ccagcccagg     360

gaggagcagt tcaacagcac cttcaggagc gtgagcgagc tgcccatcat gcaccaggac     420

tggctgaacg gcaaggagtt caagtgcagg gtgaacagcg ccgccttccc cgcccccatc     480

gagaagacca tcagcaagac caagggcagg cccaaggccc cccaggtgta caccatcccc     540

ccccccaagg agcagatggc caaggacaag gtgagcctga cctgcatgat caccgacttc     600

ttccccgagg acatcaccgt ggagtggcag tggaacggcc agcccgccga gaactacaag     660

aacacccagc ccatcatgga caccgacggc agctacttcg tgtacagcaa gctgaacgtg     720

cagaagagca actgggaggc cggcaacacc ttcacctgca gcgtgctgca cgagggcctg     780

cacaaccacc acaccgagaa gagcctgagc cacagccccg gcaag                     825


<210>  47
<211>  36
<212>  DNA
<213>  Unknown

<220>
<223>  Primer A

<400>  47
gctctagagc caccatgcag actcagcata ccaagg                                36


<210>  48
<211>  42
<212>  DNA
<213>  Unknown

<220>
<223>  Primer B

<400>  48

cgggatccac cggtttagag agctgtacaa gattctttct tg                42

<210>  49
<211>  36
<212>  DNA
<213>  Unknown

<220>
<223>  Primer C

<400>  49
cccaagcttg ccaccatgca tagcaaagtc acaatc                      36

<210>  50
<211>  38
<212>  DNA
<213>  Unknown

<220>
<223>  Primer D

<400>  50
acgcgtcgac ttagagaccc acacaacttt ctttcttg                    38

<210>  51
<211>  1701
<212>  DNA
<213>  Unknown

<220>
<223>  PAL

<400>  51
atgaagacgc tgtcacaggc ccagtccaag acctcctccc agcagttctc cttcaccggc      60

aactcctccg ccaacgtgat catcggcaac cagaagctga ccatcaacga cgtggcccgc     120

gtggcccgca acggcaccct ggtgtccctg accaacaaca ccgacatcct gcagggcatc     180

caggcctcct gcgactacat caacaacgcc gtggagtccg gcgagcccat ctacggcgtg     240

acctccggct tcggcggcat ggccaacgtg gccatctccc gcgagcaggc ctccgagctg     300

cagaccaacc tggtgtggtt cctgaagacc ggcgccggca acaagctgcc cctggccgac     360

gtgcgcgccg ccatgctgct gcgcgccaac tcccacatgc gcggcgcctc cggcatccgc     420

ctcgagctga tcaagcgcat ggagatcttc ctgaacgccg gcgtgacccc ctacgtgtac     480

gagttcggct ccatcggcgc ctccggcgac ctggtgcccc tgtcctacat caccggctcc     540

ctgatcggcc tggacccctc cttcaaggtg gacttcaacg gcaaggagat ggacgccccc     600

accgccctgc gccagctgaa cctgtccccc ctgaccctgc tgcccaagga gggcctggcc     660

atgatgaacg gcacctccgt gatgaccggc atcgccgcca actgcgtgta cgacacccag     720

atcctgaccg ccatcgccat gggtgtacac gctctggaca tccaggccct gaacggcacc     780

aaccagtcct tccacccctt catccacaac tccaagcccc accccggcca gctgtgggcc     840

gccgaccaga tgatctccct cctcgccaac tcccagctgg tgcgcgacga gctggacggc 900

aagcacgact accgcgacca cgagctgatc caggaccgct actccctgcg ctgcctgccc 960

cagtacctgg gccccatcgt ggacggcatc tcccagatcg ccaagcagat cgagatcgag 1020

atcaactccg tgaccgacaa ccccctgatc gacgtggaca accaggcctc ctaccacggc 1080

ggcaacttcc tgggccagta cgtgggcatg ggcatggacc acctgcgcta ctacatcggc 1140

ctgctggcca agcacctgga cgtgcagatc gccctgctgg cctcccccga gttctccaac 1200

ggcctgcccc cctccctgct gggcaaccgc gagcgcaagg tgaacatggg cctgaagggc 1260

ctgcagatct gcggtaactc gataatgccc ctgctgacct tctacggcaa ctccatcgcc 1320

gaccgcttcc ccacccacgc cgagcagttc aaccagaaca tcaactccca gggctacacc 1380

tccgccaccc tggcccgccg ctccgtggac atcttccaga actacgtggc catcgccctg 1440

atgttcggcg ttcaagctgt agacctgcgc acctacaaga agaccggcca ctacgacgcc 1500

cgcgcctccc tgtcccccgc caccgagcgc ctgtactccg ccgtgcgcca cgtggtgggc 1560

cagaagccca cctccgaccg cccctacatc tggaacgaca acgagcaggg cctggacgag 1620

cacatcgccc gcatctccgc cgacatcgca gcaggtggcg tgatcgtgca ggccgtgcag 1680

gacatcctgc cctccctgca c 1701

<210> 52
<211> 1128
<212> DNA
<213> Unknown

<220>
<223> HSV-TK

<400> 52
atggcttcgt accccggcca tcagcacgcg tctgcgttcg accaggctgc gcgttctcgc 60

ggccatagca accgacgtac ggcgttgcgc cctcgccggc agcaagaagc cacggaagtc 120

cgcccggagc agaaaatgcc cacgctactg cgggtttata tagacggtcc ccacgggatg 180

gggaaaacca ccaccacgca actgctggtg gccctgggtt cgcgcgacga tatcgtctac 240

gtacccgagc cgatgactta ctggcgggtg ctgggggctt ccgagacaat cgcgaacatc 300

tacaccacac aacaccgcct tgaccagggt gagatatcgg ccggggacgc ggcggtggta 360

atgacaagcg cccagataac aatgggcatg ccttatgccg tgaccgacgc cgttctggct 420

cctcatatcg ggggggaggc tgggagctca catgccccgc ccccggccct caccctcatc 480

ttcgaccgcc atcccatcgc cgccctcctg tgctacccgg ccgcgcgata ccttatgggc 540

agcatgaccc cccaggccgt gctggcgttc gtggccctca tcccgccgac cttgcccggc 600

acaaacatcg tgttgggggc ccttccggag gacagacaca tcgaccgcct ggccaaacgc 660

cagcgccccg gcgagcggct tgacctggct atgctggccg cgattcgccg cgtttacggg 720

ctgcttgcca atacggtgcg gtatctgcag ggcggcgggt cgtggcggga ggattgggga 780

cagctttcgg ggacggccgt gccgccccag ggtgccgagc cccagagcaa cgcgggccca 840

cgaccccata tcggggacac gttatttacc ctgtttcggg cccccgagtt gctggccccc 900

aacggcgacc tgtacaacgt gtttgcctgg gccttggacg tcttggccaa acgcctccgt 960

cccatgcacg tctttatcct ggattacgac caatcgcccg ccggctgccg ggacgccctg 1020

ctgcaactta cctccgggat gatccagacc cacgtcacca ccccaggctc cataccgacg 1080

atctgcgacc tggcgcgcac gtttgcccgg gagatggggg aggctaac 1128

<210> 53
<211> 474
<212> DNA
<213> Unknown

<220>
<223> yCD

<400> 53
atggtgaccg gcggcatggc ctccaagtgg gaccagaagg gcatggacat cgcctacgag 60

gaggccgccc tgggctacaa ggagggcggc gtgcccatcg gcggctgcct gatcaacaac 120

aaggacggct ccgtgctggg ccgcggccac aacatgcgct tccagaaggg ctccgccacc 180

ctgcacggcg agatctccac cctggagaac tgcggccgcc tggagggcaa ggtgtacaag 240

gacaccaccc tgtacaccac cctgtccccc tgcgacatgt gcaccggcgc catcatcatg 300

tacggcatcc cccgctgcgt ggtgggcgag aacgtgaact tcaagtccaa gggcgagaag 360

tacctgcaga cccgcggcca cgaggtggtg gtggtggacg acgagcgctg caagaagatc 420

atgaagcagt tcatcgacga gcgcccccag gactggttcg aggacatcgg cgag 474

<210> 54
<211> 1356
<212> DNA
<213> Unknown

<220>
<223> PAH

<400> 54
atgtccactg cggtcctgga aaacccaggc ttgggcagga aactctctga ctttggacag 60

gaaacaagct atattgaaga caactgcaat caaaatggtg ccatatcact gatcttctca 120

ctcaaagaag aagttggtgc attggccaaa gtattgcgct tatttgagga gaatgatgta 180

aacctgaccc acattgaatc tagaccttct cgtttaaaga aagatgagta tgaatttttc 240

acccatttgg ataaacgtag cctgcctgct ctgacaaaca tcatcaagat cttgaggcat 300

gacattggtg ccactgtcca tgagctttca cgagataaga agaaagacac agtgccctgg 360

ttcccaagaa ccattcaaga gctggacaga tttgccaatc agattctcag ctatggagcg 420

gaactggatg ctgaccaccc tggttttaaa gatcctgtgt accgtgcaag acggaagcag 480

tttgctgaca ttgcctacaa ctaccgccat gggcagccca tccctcgagt ggaatacatg 540

gaggaagaaa agaaaacatg gggcacagtg ttcaagactc tgaagtcctt gtataaaacc 600

catgcttgct atgagtacaa tcacattttt ccacttcttg aaaagtactg tggcttccat 660

gaagataaca ttccccagct ggaagacgtt tctcaattcc tgcagacttg cactggtttc 720

cgcctccgac ctgtggctgg cctgctttcc tctcgggatt tcttgggtgg cctggccttc 780

cgagtcttcc actgcacaca gtacatcaga catggatcca agcccatgta tacccccgaa 840

cctgacatct gccatgagct gttgggacat gtgcccttgt tttcagatcg cagctttgcc 900

cagttttccc aggaaattgg ccttgcctct ctgggtgcac ctgatgaata cattgaaaag 960

ctcgccacaa tttactggtt tactgtggag tttgggctct gcaaacaagg agactccata 1020

aaggcatatg gtgctgggct cctgtcatcc tttggtgaat tacagtactg cttatcagag 1080

aagccaaagc ttctccccct ggagctggag aagacagcca tccaaaatta cactgtcacg 1140

gagttccagc ccctgtatta cgtggcagag agttttaatg atgccaagga gaaagtaagg 1200

aactttgctg ccacaatacc tcggcccttc tcagttcgct acgacccata cacccaaagg 1260

attgaggtct tggacaatac ccagcagctt aagattttgg ctgattccat taacagtgaa 1320

attggaatcc tttgcagtgc cctccagaaa ataaag 1356

<210> 55
<211> 723
<212> DNA
<213> Unknown

<220>
<223> GTPCH

<400> 55
atggagaagc cgcggggagt caggtgcacc aatgggttct ccgagcgtga actacctcgt 60

cctggagcaa gcccacctgc agagaagtcc cgacctcctg aagcaaaggg cgcacagccg 120

gccgacgcct ggaaggcagg gcggcaccgc agcgaggagg aaaaccaggt gaacctcccc 180

aaactggcgg ccgcttactc gtccattctg ctctcgctgg gcgaggaccc ccagcggcag 240

gggctgctca agacgccctg gagggcggcc accgccatgc agtacttcac caagggatac 300

caggagacca tctcagatgt cctgaatgat gctatatttg atgaagatca tgacgagatg 360

gtgattgtga aggacataga tatgttctcc atgtgtgagc atcaccttgt tccatttgta 420

ggaagggtcc atattggcta tcttcctaac aagcaagtcc ttggtctcag taaacttgcc 480

aggattgtag aaatctacag tagacgacta caagttcaag agcgcctcac caaacagatt 540

gcggtggcca tcacagaagc cttgcagcct gctggcgttg gagtagtgat tgaagcgaca 600

cacatgtgca tggtaatgcg aggcgtgcag aaaatgaaca gcaagactgt cactagcacc 660

atgctgggcg tgttccggga agaccccaag actcgggagg agttcctcac actaatcagg 720

agc 723

<210> 56
<211> 432
<212> DNA
<213> Unknown

<220>
<223> PTPS

<400> 56
atgagcgctg ctggtgatct tcgtcgtcgt gcgcgactgt cgcgcctcgt gtccttcagc 60

gcgagccacc ggctgcacag cccatctctg agcgatgaag agaacttaag agtgtttggg 120

aaatgcaaca atccgaatgg ccacgggcac aactataaag ttgtggtgac agtccatgga 180

gagattgatc ctgttacagg aatggttatg aatttgaccg acctcaaaga atacatggag 240

gaggccatca tgaagcctct tgatcacaag aacctggacc tggatgtgcc gtactttgcg 300

gatgctgtga gcacgacaga aaatgtagct gtctacatct gggaaagcct ccagaaactt 360

cttccagtgg gagctcttta taaagtaaaa gtgtttgaaa ccgacaacaa catcgtagtc 420

tataaaggag aa 432

<210> 57
<211> 783
<212> DNA
<213> Unknown

<220>
<223> SR (sepiapterin reductase)

<400> 57
atggagggcg ggctggggcg tgctgtgtgc ttgctgaccg gggcctcccg cggcttcggc 60

cggacgctgg ccccgctcct ggcctcgctg ctgtcgcccg gctccgtgct tgtccttagc 120

gcccgcaacg acgaggcact gcgccagctg gaggccgagc tgggcgccga gcggtctggc 180

ctgcgcgtgg tgcgggtgcc cgccgacctg ggcgccgagg ccggcttgca gcagctgctc 240

ggcgccctgc gcgagctccc ccggcccaag gggctgcagc gactgctgct tatcaacaac 300

gcgggctctc ttggggatgt gtccaaaggc ttcgtggacc tgagtgactc cactcaagtg 360

aacaactact gggcactgaa cttgacctcc atgctctgcc tgacttccag cgtcctgaag 420

gccttcccgg acagtcctgg cctcaacaga accgtggtta acatctcgtc cctctgtgcc 480

ctgcaacctt tcaaaggctg ggcgctgtac tgtgcaggaa aggctgctcg tgatatgctg 540

ttccaggtcc tggcgctgga ggaacctaat gtgagggtgc tgaactatgc cccaggtcct 600

ctggacacag acatgcagca gttggcccgg gagacctccg tggacccaga catgcgaaaa 660

gggctgcagg agctgaaggc aaaggggaag ctggtggatt gcaaggtgtc agcccagaaa 720

ctgctgagct tactggaaaa ggacgagttc aagtctggag cccacgtgga cttctatgac 780

aaa 783

<210> 58
<211> 4413
<212> DNA
<213> Unknown

<220>
<223> Factor VIII minus B

<400> 58
atgcaaatag agctctccac ctgcttcttt ctgtgccttt tgcgattctg ctttagtgcc 60

accagaagat actacctggg tgcagtggaa ctgtcatggg actatatgca aagtgatctc 120

ggtgagctgc ctgtggacgc aagatttcct cctagagtgc caaaatcttt tccattcaac 180

acctcagtcg tgtacaaaaa gactctgttt gtagaattca cggatcacct tttcaacatc 240

gctaagccaa ggccaccctg gatgggtctg ctaggtccta ccatccaggc tgaggtttat 300

gatacagtgg tcattacact taagaacatg gcttcccatc ctgtcagtct tcatgctgtt 360

ggtgtatcct actggaaagc ttctgaggga gctgaatatg atgatcagac cagtcaaagg 420

gagaaagaag atgataaagt cttccctggt ggaagccata catatgtctg gcaggtcctg 480

aaagagaatg gtccaatggc ctctgaccca ctgtgcctta cctactcata tctttctcat 540

gtggacctgg taaaagactt gaattcaggc ctcattggag ccctactagt atgtagagaa 600

gggagtctgg ccaaggaaaa gacacagacc ttgcacaaat ttatactact ttttgctgta 660

tttgatgaag ggaaaagttg gcactcagaa acaaagaact ccttgatgca ggatagggat 720

gctgcatctg ctcgggcctg gcctaaaatg cacacagtca atggttatgt aaacaggtct 780

ctgccaggtc tgattggatg ccacaggaaa tcagtctatt ggcatgtgat tggaatgggc 840

accactcctg aagtgcactc aatattcctc gaaggtcaca catttcttgt gaggaaccat 900

cgccaggcgt ccttggaaat ctcgccaata actttcctta ctgctcaaac actcttgatg 960

gaccttggac agtttctact gttttgtcat atctcttccc accaacatga tggcatggaa 1020

gcttatgtca aagtagacag ctgtccagag gaaccccaac tacgaatgaa aaataatgaa 1080

gaagcggaag actatgatga tgatcttact gattctgaaa tggatgtggt caggtttgat 1140

gatgacaact ctccttcctt tatccaaatt cgctcagttg ccaagaagca tcctaaaact 1200

tgggtacatt acattgctgc tgaagaggag gactgggact atgctccctt agtcctcgcc 1260

cccgatgaca gaagttataa aagtcaatat ttgaacaatg gccctcagcg gattggtagg 1320

aagtacaaaa aagtccgatt tatggcatac acagatgaaa cctttaagac tcgtgaagct 1380
attcagcatg aatcaggaat cttgggacct ttactttatg gggaagttgg agacacactg 1440
ttgattatat ttaagaatca agcaagcaga ccatataaca tctaccctca cggaatcact 1500
gatgtccgtc ctttgtattc aaggagatta ccaaaaggtg taaaacattt gaaggatttt 1560
ccaattctgc caggagaaat attcaaatat aaatggacag tgactgtaga agatgggcca 1620
actaaatcag atcctcggtg cctgacccgc tattactcta gtttcgttaa tatggagaga 1680
gatctagctt caggactcat tggccctctc ctcatctgct acaaagaatc tgtagatcaa 1740
agaggaaacc agataatgtc agacaagagg aatgtcatcc tgttttctgt atttgatgag 1800
aaccgaagct ggtacctcac agagaatata caacgctttc tccccaatcc agctggagtg 1860
cagcttgagg atccagagtt ccaagcctcc aacatcatgc acagcatcaa tggctatgtt 1920
tttgatagtt tgcagttgtc agtttgtttg catgaggtgg catactggta cattctaagc 1980
attggagcac agactgactt cctttctgtc ttcttctctg gatatacctt caaacacaaa 2040
atggtctatg aagacacact caccctattc ccattctcag gagaaactgt cttcatgtcg 2100
atggaaaacc caggtctatg gattctgggg tgccacaact cagactttcg gaacagaggc 2160
atgaccgcct tactgaaggt ttctagttgt gacaagaaca ctggtgatta ttacgaggac 2220
agttatgaag atatttcagc atacttgctg agtaaaaaca atgccattga accaagaagc 2280
ttctcccaga attcaagaca ccctagcact aggcaaaagc aatttaatgc caccccacca 2340
gtcttgaaac gccatcaacg ggaaataact cgtactactc ttcagtcaga tcaagaggaa 2400
attgactatg atgataccat atcagttgaa atgaagaagg aagattttga catttatgat 2460
gaggatgaaa atcagagccc ccgcagcttt caaaagaaaa cacgacacta ttttattgct 2520
gcagtggaga ggctctggga ttatgggatg agtagctccc cacatgttct aagaaacagg 2580
gctcagagtg gcagtgtccc tcagttcaag aaagttgttt tccaggaatt tactgatggc 2640
tcctttactc agcccttata ccgtggagaa ctaaatgaac atttgggact cctggggcca 2700
tatataagag cagaagttga agataatatc atggtaactt tcagaaatca ggcctctcgt 2760
ccctattcct tctattctag ccttatttct tatgaggaag atcagaggca aggagcagaa 2820
cctagaaaaa actttgtcaa gcctaatgaa accaaaactt acttttggaa agtgcaacat 2880
catatggcac ccactaaaga tgagtttgac tgcaaagcct gggcttattt ctctgatgtt 2940
gacctggaaa aagatgtgca ctcaggcctg attggacccc ttctggtctg ccacactaac 3000
acactgaacc ctgctcatgg gagacaagtg acagtacagg aatttgctct gtttttcacc 3060
atctttgatg agaccaaaag ctggtacttc actgaaaata tggaaagaaa ctgcagggct 3120
ccctgcaata tccagatgga agatcccact tttaaagaga attatcgctt ccatgcaatc 3180
aatggctaca taatggatac actacctggc ttagtaatgg ctcaggatca aaggattcga 3240

tggtatctgc tcagcatggg cagcaatgaa aacatccatt ctattcattt cagtggacat 3300

gtgttcactg tacgaaaaaa agaggagtat aaaatggcac tgtacaatct ctatccaggt 3360

gtttttgaga cagtggaaat gttaccatcc aaagctggaa tttggcgggt ggaatgcctt 3420

attggcgagc atctacatgc tgggatgagc acactttttc tggtgtacag caataagtgt 3480

cagactcccc tgggaatggc ttctggacac attagagatt ttcagattac agcttcagga 3540

caatatggac agtgggcccc aaagctggcc agacttcatt attccggatc aatcaatgcc 3600

tggagcacca aggagccctt ttcttggatc aaggtggatc tgttggcacc aatgattatt 3660

cacggcatca agacccaggg tgcccgtcag aagttctcca gcctctacat ctctcagttt 3720

atcatcatgt atagtcttga tgggaagaag tggcagactt atcgaggaaa ttccactgga 3780

accttaatgg tcttctttgg caatgtggat tcatctggga taaaacacaa tatttttaac 3840

cctccaatta ttgctcgata catccgtttg cacccaactc attatagcat tcgcagcact 3900

cttcgcatgg agttgatggg ctgtgattta aatagttgca gcatgccatt gggaatggag 3960

agtaaagcaa tatcagatgc acagattact gcttcatcct actttaccaa tatgtttgcc 4020

acctggtctc cttcaaaagc tcgacttcac ctccaaggga ggagtaatgc ctggagacct 4080

caggtgaata atccaaaaga gtggctgcaa gtggacttcc agaagacaat gaaagtcaca 4140

ggagtaacta ctcagggagt aaaatctctg cttaccagca tgtatgtgaa ggagttcctc 4200

atctccagca gtcaagatgg ccatcagtgg actctctttt ttcagaatgg caaagtaaag 4260

gtttttcagg gaaatcaaga ctccttcaca cctgtggtga actctctaga cccaccgtta 4320

ctgactcgct accttcgaat tcacccccag agttgggtgc accagattgc cctgaggatg 4380

gaggttctgg gctgcgaggc acaggacctc tac 4413

<210> 59
<211> 7053
<212> DNA
<213> Unknown

<220>
<223> Factor VIII

<400> 59
atgcaaatag agctctccac ctgcttcttt ctgtgccttt tgcgattctg ctttagtgcc 60

accagaagat actacctggg tgcagtggaa ctgtcatggg actatatgca aagtgatctc 120

ggtgagctgc ctgtggacgc aagatttcct cctagagtgc caaaatcttt tccattcaac 180

acctcagtcg tgtacaaaaa gactctgttt gtagaattca cggatcacct tttcaacatc 240

gctaagccaa ggccaccctg gatgggtctg ctaggtccta ccatccaggc tgaggtttat 300

gatacagtgg tcattacact taagaacatg gcttcccatc ctgtcagtct tcatgctgtt 360

ggtgtatcct actggaaagc ttctgaggga gctgaatatg atgatcagac cagtcaaagg 420

gagaaagaag atgataaagt cttccctggt ggaagccata catatgtctg gcaggtcctg 480

aaagagaatg gtccaatggc ctctgaccca ctgtgcctta cctactcata tctttctcat 540

gtggacctgg taaaagactt gaattcaggc ctcattggag ccctactagt atgtagagaa 600

gggagtctgg ccaaggaaaa gacacagacc ttgcacaaat ttatactact ttttgctgta 660

tttgatgaag ggaaaagttg gcactcagaa acaaagaact ccttgatgca ggatagggat 720

gctgcatctg ctcgggcctg gcctaaaatg cacacagtca atggttatgt aaacaggtct 780

ctgccaggtc tgattggatg ccacaggaaa tcagtctatt ggcatgtgat tggaatgggc 840

accactcctg aagtgcactc aatattcctc gaaggtcaca catttcttgt gaggaaccat 900

cgccaggcgt ccttggaaat ctcgccaata actttcctta ctgctcaaac actcttgatg 960

gaccttggac agtttctact gttttgtcat atctcttccc accaacatga tggcatggaa 1020

gcttatgtca aagtagacag ctgtccagag gaaccccaac tacgaatgaa aaataatgaa 1080

gaagcggaag actatgatga tgatcttact gattctgaaa tggatgtggt caggtttgat 1140

gatgacaact ctccttcctt tatccaaatt cgctcagttg ccaagaagca tcctaaaact 1200

tgggtacatt acattgctgc tgaagaggag gactgggact atgctccctt agtcctcgcc 1260

cccgatgaca gaagttataa aagtcaatat ttgaacaatg gccctcagcg gattggtagg 1320

aagtacaaaa aagtccgatt tatggcatac acagatgaaa cctttaagac tcgtgaagct 1380

attcagcatg aatcaggaat cttgggacct ttactttatg gggaagttgg agacacactg 1440

ttgattatat ttaagaatca agcaagcaga ccatataaca tctaccctca cggaatcact 1500

gatgtccgtc ctttgtattc aaggagatta ccaaaaggtg taaaacattt gaaggatttt 1560

ccaattctgc caggagaaat attcaaatat aaatggacag tgactgtaga agatgggcca 1620

actaaatcag atcctcggtg cctgacccgc tattactcta gtttcgttaa tatggagaga 1680

gatctagctt caggactcat tggccctctc ctcatctgct acaaagaatc tgtagatcaa 1740

agaggaaacc agataatgtc agacaagagg aatgtcatcc tgttttctgt atttgatgag 1800

aaccgaagct ggtacctcac agagaatata caacgctttc tccccaatcc agctggagtg 1860

cagcttgagg atccagagtt ccaagcctcc aacatcatgc acagcatcaa tggctatgtt 1920

tttgatagtt tgcagttgtc agtttgtttg catgaggtgg catactggta cattctaagc 1980

attggagcac agactgactt cctttctgtc ttcttctctg gatatacctt caaacacaaa 2040

atggtctatg aagacacact caccctattc ccattctcag gagaaactgt cttcatgtcg 2100

atggaaaacc caggtctatg gattctgggg tgccacaact cagactttcg gaacagaggc 2160

atgaccgcct tactgaaggt ttctagttgt gacaagaaca ctggtgatta ttacgaggac 2220

agttatgaag atatttcagc atacttgctg agtaaaaaca atgccattga accaagaagc 2280

ttctcccaga attcaagaca ccctagcact aggcaaaagc aatttaatgc caccacaatt 2340

ccagaaaatg acatagagaa gactgaccct tggtttgcac acagaacacc tatgcctaaa 2400

atacaaaatg tctcctctag tgatttgttg atgctcttgc gacagagtcc tactccacat 2460

gggctatcct tatctgatct ccaagaagcc aaatatgaga ctttttctga tgatccatca 2520

cctggagcaa tagacagtaa taacagcctg tctgaaatga cacacttcag gccacagctc 2580

catcacagtg gggacatggt atttacccct gagtcaggcc tccaattaag attaaatgag 2640

aaactgggga caactgcagc aacagagttg aagaaacttg atttcaaagt ttctagtaca 2700

tcaaataatc tgatttcaac aattccatca gacaatttgg cagcaggtac tgataataca 2760

agttccttag gacccccaag tatgccagtt cattatgata gtcaattaga taccactcta 2820

tttggcaaaa agtcatctcc ccttactgag tctggtggac ctctgagctt gagtgaagaa 2880

aataatgatt caaagttgtt agaatcaggt ttaatgaata gccaagaaag ttcatgggga 2940

aaaaatgtat cgtcaacaga gagtggtagg ttatttaaag ggaaaagagc tcatggacct 3000

gctttgttga ctaaagataa tgccttattc aaagttagca tctctttgtt aaagacaaac 3060

aaaacttcca ataattcagc aactaataga aagactcaca ttgatggccc atcattatta 3120

attgagaata gtccatcagt ctggcaaaat atattagaaa gtgacactga gtttaaaaaa 3180

gtgacacctt tgattcatga cagaatgctt atggacaaaa atgctacagc tttgaggcta 3240

aatcatatgt caaataaaac tacttcatca aaaaacatgg aaatggtcca acagaaaaaa 3300

gagggcccca ttccaccaga tgcacaaaat ccagatatgt cgttctttaa gatgctattc 3360

ttgccagaat cagcaaggtg gatacaaagg actcatggaa agaactctct gaactctggg 3420

caaggcccca gtccaaagca attagtatcc ttaggaccag aaaaatctgt ggaaggtcag 3480

aatttcttgt ctgagaaaaa caaagtggta gtaggaaagg gtgaatttac aaaggacgta 3540

ggactcaaag agatggtttt tccaagcagc agaaacctat ttcttactaa cttggataat 3600

ttacatgaaa ataatacaca caatcaagaa aaaaaaattc aggaagaaat agaaaagaag 3660

gaaacattaa tccaagagaa tgtagttttg cctcagatac atacagtgac tggcactaag 3720

aatttcatga agaacctttt cttactgagc actaggcaaa atgtagaagg ttcatatgac 3780

ggggcatatg ctccagtact tcaagatttt aggtcattaa atgattcaac aaatagaaca 3840

aagaaacaca cagctcattt ctcaaaaaaa ggggaggaag aaaacttgga aggcttggga 3900

aatcaaacca agcaaattgt agagaaatat gcatgcacca caaggatatc tcctaataca 3960

agccagcaga attttgtcac gcaacgtagt aagagagctt tgaaacaatt cagactccca 4020

ctagaagaaa cagaacttga aaaaaggata attgtggatg acacctcaac ccagtggtcc 4080

aaaaacatga aacatttgac cccgagcacc ctcacacaga tagactacaa tgagaaggag 4140

aaaggggcca ttactcagtc tcccttatca gattgcctta cgaggagtca tagcatccct 4200

caagcaaata gatctccatt acccattgca aaggtatcat catttccatc tattagacct 4260

atatatctga ccagggtcct attccaagac aactcttctc atcttccagc agcatcttat 4320

agaaagaaag attctggggt ccaagaaagc agtcatttct tacaaggagc caaaaaaaat 4380

aacctttctt tagccattct aaccttggag atgactggtg atcaaagaga ggttggctcc 4440

ctggggacaa gtgccacaaa ttcagtcaca tacaagaaag ttgagaacac tgttctcccg 4500

aaaccagact tgcccaaaac atctggcaaa gttgaattgc ttccaaaagt tcacatttat 4560

cagaaggacc tattccctac ggaaactagc aatgggtctc ctggccatct ggatctcgtg 4620

gaagggagcc ttcttcaggg aacagaggga gcgattaagt ggaatgaagc aaacagacct 4680

ggaaaagttc cctttctgag agtagcaaca gaaagctctg caaagactcc ctccaagcta 4740

ttggatcctc ttgcttggga taaccactat ggtactcaga taccaaaaga agagtggaaa 4800

tcccaagaga agtcaccaga aaaaacagct tttaagaaaa aggataccat tttgtccctg 4860

aacgcttgtg aaagcaatca tgcaatagca gcaataaatg agggacaaaa taagcccgaa 4920

atagaagtca cctgggcaaa gcaaggtagg actgaaaggc tgtgctctca aaacccacca 4980

gtcttgaaac gccatcaacg ggaaataact cgtactactc ttcagtcaga tcaagaggaa 5040

attgactatg atgataccat atcagttgaa atgaagaagg aagattttga catttatgat 5100

gaggatgaaa atcagagccc ccgcagcttt caaaagaaaa cacgacacta ttttattgct 5160

gcagtggaga ggctctggga ttatgggatg agtagctccc cacatgttct aagaaacagg 5220

gctcagagtg gcagtgtccc tcagttcaag aaagttgttt tccaggaatt tactgatggc 5280

tcctttactc agcccttata ccgtggagaa ctaaatgaac atttgggact cctggggcca 5340

tatataagag cagaagttga agataatatc atggtaactt tcagaaatca ggcctctcgt 5400

ccctattcct tctattctag ccttatttct tatgaggaag atcagaggca aggagcagaa 5460

cctagaaaaa actttgtcaa gcctaatgaa accaaaactt acttttggaa agtgcaacat 5520

catatggcac ccactaaaga tgagtttgac tgcaaagcct gggcttattt ctctgatgtt 5580

gacctggaaa aagatgtgca ctcaggcctg attggacccc ttctggtctg ccacactaac 5640

acactgaacc ctgctcatgg gagacaagtg acagtacagg aatttgctct gtttttcacc 5700

atctttgatg agaccaaaag ctggtacttc actgaaaata tggaaagaaa ctgcagggct 5760

ccctgcaata tccagatgga agatcccact tttaaagaga attatcgctt ccatgcaatc 5820

aatggctaca taatggatac actacctggc ttagtaatgg ctcaggatca aaggattcga 5880

tggtatctgc tcagcatggg cagcaatgaa aacatccatt ctattcattt cagtggacat 5940

gtgttcactg tacgaaaaaa agaggagtat aaaatggcac tgtacaatct ctatccaggt 6000

gtttttgaga cagtggaaat gttaccatcc aaagctggaa tttggcgggt ggaatgcctt 6060

attggcgagc atctacatgc tgggatgagc acactttttc tggtgtacag caataagtgt 6120

cagactcccc tgggaatggc ttctggacac attagagatt ttcagattac agcttcagga 6180

caatatggac agtgggcccc aaagctggcc agacttcatt attccggatc aatcaatgcc 6240

tggagcacca aggagccctt ttcttggatc aaggtggatc tgttggcacc aatgattatt 6300

cacggcatca agacccaggg tgcccgtcag aagttctcca gcctctacat ctctcagttt 6360

atcatcatgt atagtcttga tgggaagaag tggcagactt atcgaggaaa ttccactgga 6420

accttaatgg tcttctttgg caatgtggat tcatctggga taaaacacaa tatttttaac 6480

cctccaatta ttgctcgata catccgtttg cacccaactc attatagcat tcgcagcact 6540

cttcgcatgg agttgatggg ctgtgattta aatagttgca gcatgccatt gggaatggag 6600

agtaaagcaa tatcagatgc acagattact gcttcatcct actttaccaa tatgtttgcc 6660

acctggtctc cttcaaaagc tcgacttcac ctccaaggga ggagtaatgc ctggagacct 6720

caggtgaata atccaaaaga gtggctgcaa gtggacttcc agaagacaat gaaagtcaca 6780

ggagtaacta ctcagggagt aaaatctctg cttaccagca tgtatgtgaa ggagttcctc 6840

atctccagca gtcaagatgg ccatcagtgg actctctttt ttcagaatgg caaagtaaag 6900

gtttttcagg gaaatcaaga ctccttcaca cctgtggtga actctctaga cccaccgtta 6960

ctgactcgct accttcgaat tcacccccag agttgggtgc accagattgc cctgaggatg 7020

gaggttctgg gctgcgaggc acaggacctc tac 7053

<210> 60
<211> 1827
<212> DNA
<213> Unknown

<220>
<223> Albumin

<400> 60
atgaagtggg taacctttat ttcccttctt tttctcttta gctcggctta ttccaggggt 60

gtgtttcgtc gagatgcaca caagagtgag gttgctcatc ggtttaaaga tttgggagaa 120

gaaaatttca aagccttggt gttgattgcc tttgctcagt atcttcagca gtgtccattt 180

gaagatcatg taaaattagt gaatgaagta actgaatttg caaaaacatg tgttgctgat 240

gagtcagctg aaaattgtga caaatcactt catacccttt ttggagacaa attatgcaca 300

gttgcaactc ttcgtgaaac ctatggtgaa atggctgact gctgtgcaaa acaagaacct 360

gagagaaatg aatgcttctt gcaacacaaa gatgacaacc caaacctccc ccgattggtg 420

agaccagagg ttgatgtgat gtgcactgct tttcatgaca atgaagagac atttttgaaa 480

aaatacttat atgaaattgc cagaagacat ccttactttt atgccccgga actccttttc 540

tttgctaaaa ggtataaagc tgcttttaca gaatgttgcc aagctgctga taaagctgcc 600

tgcctgttgc caaagctcga tgaacttcgg gatgaaggga aggcttcgtc tgccaaacag 660

agactcaagt gtgccagtct ccaaaaattt ggagaaagag ctttcaaagc atgggcagta 720

gctcgcctga gccagagatt tcccaaagct gagtttgcag aagtttccaa gttagtgaca 780

gatcttacca aagtccacac ggaatgctgc catggagatc tgcttgaatg tgctgatgac 840

agggcggacc ttgccaagta tatctgtgaa aatcaagatt cgatctccag taaactgaag 900

gaatgctgtg aaaaacctct gttggaaaaa tcccactgca ttgccgaagt ggaaaatgat 960

gagatgcctg ctgacttgcc ttcattagct gctgattttg ttgaaagtaa ggatgtttgc 1020

aaaaactatg ctgaggcaaa ggatgtcttc ctgggcatgt ttttgtatga atatgcaaga 1080

aggcatcctg attactctgt cgtgctgctg ctgagacttg ccaagacata tgaaaccact 1140

ctagagaagt gctgtgccgc tgcagatcct catgaatgct atgccaaagt gttcgatgaa 1200

tttaaacctc ttgtggaaga gcctcagaat ttaatcaaac aaaattgtga gctttttgag 1260

cagcttggag agtacaaatt ccagaatgcg ctattagttc gttacaccaa gaaagtaccc 1320

caagtgtcaa ctccaactct tgtagaggtc tcaagaaacc taggaaaagt gggcagcaaa 1380

tgttgtaaac atcctgaagc aaaaagaatg ccctgtgcag aagactatct atccgtggtc 1440

ctgaaccagt tatgtgtgtt gcatgagaaa acgccagtaa gtgacagagt caccaaatgc 1500

tgcacagaat ccttggtgaa caggcgacca tgcttttcag ctctggaagt cgatgaaaca 1560

tacgttccca aagagtttaa tgctgaaaca ttcaccttcc atgcagatat atgcacactt 1620

tctgagaagg agagacaaat caagaaacaa actgcacttg ttgagctcgt gaaacacaag 1680

cccaaggcaa caaaagagca actgaaagct gttatggatg atttcgcagc ttttgtagag 1740

aagtgctgca aggctgacga taaggagacc tgctttgccg aggagggtaa aaaacttgtt 1800

gctgcaagtc aagctgcctt aggctta 1827

<210> 61
<211> 1389
<212> DNA
<213> Unknown

<220>
<223> Factor IX

<400> 61
gccaccatgc agcgcgtgaa catgatcatg gcagaatcac caggcctcat caccatctgc 60

cttttaggat atctactcag tgctgaatgt acagtttttc ttgatcatga aaacgccaac 120

aaaattctga atcggccaaa gaggtataat tcaggtaaat tggaagagtt tgttcaaggg 180

aaccttgaga gagaatgtat ggaagaaaag tgtagttttg aagaagcacg agaagttttt 240

gaaaacactg aaagaacaac tgaattttgg aagcagtatg ttgatggaga tcagtgtgag 300

tccaatccat gtttaaatgg cggcagttgc aaggatgaca ttaattccta tgaatgttgg 360

tgtccctttg gatttgaagg aaagaactgt gaattagatg taacatgtaa cattaagaat 420

ggcagatgcg agcagttttg taaaaatagt gctgataaca aggtggtttg ctcctgtact 480

gagggatatc gacttgcaga aaaccagaag tcctgtgaac cagcagtgcc atttccatgt 540

ggaagagttt ctgtttcaca aacttctaag ctcacccgtg ctgagactgt ttttcctgat 600

gtggactatg taaattctac tgaagctgaa accattttgg ataacatcac tcaaagcacc 660

caatcattta atgacttcac tcgggttgtt ggtggagaag atgccaaacc aggtcaattc 720

ccttggcagg ttgttttgaa tggtaaagtt gatgcattct gtggaggctc tatcgttaat 780

gaaaaatgga ttgtaactgc tgcccactgt gttgaaactg gtgttaaaat tacagttgtc 840

gcaggtgaac ataatattga ggagacagaa catacagagc aaaagcgaaa tgtgattcga 900

attattcctc accacaacta caatgcagct attaataagt acaaccatga cattgccctt 960

ctggaactgg acgaaccctt agtgctaaac agctacgtta cacctatttg cattgctgac 1020

aaggaataca cgaacatctt cctcaaattt ggatctggct atgtaagtgg ctggggaaga 1080

gtcttccaca aagggagatc agctttagtt cttcagtacc ttagagttcc acttgttgac 1140

cgagccacat gtcttcgatc tacaaagttc accatctata acaacatgtt ctgtgctggc 1200

ttccatgaag gaggtagaga ttcatgtcaa ggagatagtg gggaccccca tgttactgaa 1260

gtggaaggga ccagtttctt aactggaatt attagctggg gtgaagagtg tgcaatgaaa 1320

ggcaaatatg gaatatatac caaggtatcc cggtatgtca actggattaa ggaaaaaaca 1380

aagctcact 1389

<210> 62
<211> 432
<212> DNA
<213> Unknown

<220>
<223> H-FABP

<400> 62
atggtggacg ctttcctggg cacctggaag ctagtggaca gcaagaattt cgatgactac 60

atgaagtcac tcgctcatat actcataacc ttccccctac cctcaggtgt gggttttgct 120

accaggcagg tggccagcat gaccaagcct accacaatca tcgaaaagaa tggggacatt 180

ctcaccctaa aaacacacag caccttcaag aacacagaga tcagctttaa gttgggggtg 240

gagttcgatg agacaacagc agatgacagg aaggtcaagt ccattgtgac actggatgga 300

gggaaacttg ttcacctgca gaaatgggac gggcaagaga ccacacttgt gcgggagcta 360

attgatggaa aactcatcct gacactcacc cacggcactg cagtttgcac tcgcacttat 420

gagaaagagg ca                                                          432

<210>  63
<211>  462
<212>  DNA
<213>  Unknown

<220>
<223>  Myocardial myoglobin

<400>  63
atggggctca gcgacgggga atggcagttg gtgctgaacg tctgggggaa ggtggaggct      60

gacatcccag gccatgggca ggaagtcctc atcaggctct ttaagggtca cccagagact     120

ctggagaagt ttgacaagtt caagcacctg aagtcagagg acgagatgaa ggcgtctgag     180

gacttaaaga agcatggtgc caccgtgctc accgccctgg gtggcatcct taagaagaag     240

gggcatcatg aggcagagat taagcccctg gcacagtcgc atgccaccaa gcacaagatc     300

cccgtgaagt acctggagtt catctcggaa tgcatcatcc aggttctgca gagcaagcat     360

cccggggact ttggtgctga tgcccagggg gccatgaaca aggccctgga gctgttccgg     420

aaggacatgg cctccaacta caaggagctg ggcttccagg gc                        462

<210>  64
<211>  1293
<212>  DNA
<213>  Unknown

<220>
<223>  GFAP

<400>  64
atggagagga gacgcatcac ctccgctgct cgccgctcct acgtctcctc aggggagatg      60

atggtggggg gcctggctcc tggccgccgt ctgggtcctg gcacccgcct ctccctggct     120

cgaatgcccc ctccactccc gacccgggtg gatttctccc tggctggggc actcaatgct     180

ggcttcaagg agacccgggc cagtgagcgg gcagagatga tggagctcaa tgaccgcttt     240

gccagctaca tcgagaaggt tcgcttcctg gaacagcaaa acaaggcgct ggctgctgag     300

ctgaaccagc tgcgggccaa ggagcccacc aagctggcag acgtctacca ggctgagctg     360

cgagagctgc ggctgcggct cgatcaactc accgccaaca gcgcccggct ggaggttgag     420

agggacaatc tggcacagga cctggccact gtgaggcaga agctccagga tgaaaccaac     480

ctgaggctgg aagccgagaa caacctggct gcctatagac aggaagcaga tgaagccacc     540

ctggcccgtc tggatctgga gaggaagatt gagtcgctgg aggaggagat ccggttcttg     600

aggaagatcc acgaggagga ggttcgggaa ctccaggagc agctggcccg acagcaggtc     660

catgtggagc ttgacgtggc caagccagac ctcaccgcag ccctgaaaga gatccgcacg     720

cagtatgagg caatggcgtc cagcaacatg catgaagccg aagagtggta ccgctccaag     780

tttgcagacc tgacagacgc tgctgcccgc aacgcggagc tgctccgcca ggccaagcac 840

gaagccaacg actaccggcg ccagttgcag tccttgacct gcgacctgga gtctctgcgc 900

ggcacgaacg agtccctgga gaggcagatg cgcgagcagg aggagcggca cgtgcgggag 960

gcggccagtt atcaggaggc gctggcgcgg ctggaggaag aggggcagag cctcaaggac 1020

gagatggccc gccacttgca ggagtaccag gacctgctca atgtcaagct ggccctggac 1080

atcgagatcg ccacctacag gaagctgcta gagggcgagg agaaccggat caccattccc 1140

gtgcagacct tctccaacct gcagattcga gggggcaaaa gcaccaaaga cggggaaaat 1200

cacaaggtca caagatatct caaaagcctc acaatacgag ttataccaat acaggctcac 1260

cagattgtaa atggaacgcc gccggctcgc ggt 1293

<210> 65
<211> 276
<212> DNA
<213> Unknown

<220>
<223> S100B

<400> 65
atgtctgagc tggagaaggc catggtggcc ctcatcgacg ttttccacca atattctgga 60

agggagggag acaagcacaa gctgaagaaa tccgaactga aggagctcat caacaatgag 120

ctttcccatt tcttagagga aatcaaagag caggaggttg tggacaaagt catggaaaca 180

ctggacaatg atggagacgg cgaatgtgac ttccaggaat tcatggcctt tgttgccatg 240

gttactactg cctgccacga gttctttgaa catgag 276

<210> 66
<211> 1069
<212> DNA
<213> Unknown

<220>
<223> PYY (Homo sapiens)

<400> 66
gcccctggag gaactgaacc cactatcggt catggggccg agactaaatg tggcgggttg 60

tctttaatct gctgccaaga ggaaactcat tcaggcaagt tcagcccttt atgaggaatt 120

cccctgtggt cacattccaa ttcctggacc tgctgccacc ctcagaactg catgctcctt 180

cttcagactt tctaagaatg actcaggtca ttggtggagt gaagtcaaga tttccaactc 240

agtcacctga agagatggag ataccattca tggagctgga ggtccctgga gatttgggaa 300

ttcagataac aagctaagat aaggagtttg cctacctctg tcctagagcg aagcctgagc 360

cttgggcgcg cagcacacca caagtatctg ttactgtgtt ttgcagaagc ttcaggcggg 420

gatataagcc ccacaaggaa agcgctgagc agaggaggcc tcagcttgac ctgcggcagt 480

gcagcccttg ggacttccct cgccttccac ctcctgctcg tctgcttcac aagctatcgc 540

tatggtgttc gtgcgcaggc cgtggcccgc cttgaccaca gtgcttctgg ccctgctcgt 600

ctgcctaggg gcgctggtcg acgcctaccc catcaaaccc gaggctcccg gcgaagacgc 660

ctcgccggag gagctgaacc gctactacgc ctccctgcgc cactacctca acctggtcac 720

ccggcagcgg tatgggaaaa gagacggccc ggacacgctt ctttccaaaa cgttcttccc 780

cgacggcgag gaccgccccg tcaggtcgcg gtcggagggc ccagacctgt ggtgaggacc 840

cctgaggcct cctgggagat ctgccaacca cgcccacgtc atttgcatac gcactcccga 900

ccccagaaac ccggattctg cctcccgacg gcggcgtctg ggcagggttc gggtgcggcc 960

ctccgcccgc gtctcggtgc cccgcccccc tgggctggag ggctgtgtgt ggtccttccc 1020

tggtcccaaa ataaagagca aattccacag aaacggaaaa aaaaaaaaa 1069

<210> 67
<211> 3670
<212> DNA
<213> Unknown

<220>
<223> TIMP2 (Homo sapiens)

<400> 67
cgcagcaaac acatccgtag aaggcagcgc ggccgccgag aaccgcagcg ccgctcgccc 60

gccgcccccc accccgccgc cccgcccggc gaattgcgcc ccgcgcccct cccctcgcgc 120

ccccgagaca aagaggagag aaagtttgcg cggccgagcg gggcaggtga ggagggtgag 180

ccgcgcggga ggggcccgcc tcggccccgg ctcagccccc gcccgcgccc ccagcccgcc 240

gccgcgagca gcgcccggac cccccagcgg cggcccccgc ccgcccagcc ccccggcccg 300

ccatgggcgc cgcggcccgc accctgcggc tggcgctcgg cctcctgctg ctggcgacgc 360

tgcttcgccc ggccgacgcc tgcagctgct ccccggtgca cccgcaacag gcgttttgca 420

atgcagatgt agtgatcagg gccaaagcgg tcagtgagaa ggaagtggac tctggaaacg 480

acatttatgg caaccctatc aagaggatcc agtatgagat caagcagata aagatgttca 540

aagggcctga gaaggatata gagtttatct acacggcccc ctcctcggca gtgtgtgggg 600

tctcgctgga cgttggagga aagaaggaat atctcattgc aggaaaggcc gagggggacg 660

gcaagatgca catcaccctc tgtgacttca tcgtgccctg ggacaccctg agcaccaccc 720

agaagaagag cctgaaccac aggtaccaga tgggctgcga gtgcaagatc acgcgctgcc 780

ccatgatccc gtgctacatc tcctccccgg acgagtgcct ctggatggac tgggtcacag 840

agaagaacat caacgggcac caggccaagt tcttcgcctg catcaagaga agtgacggct 900

cctgtgcgtg gtaccgcggc gcggcgcccc ccaagcagga gtttctcgac atcgaggacc 960

cataagcagg cctccaacgc ccctgtggcc aactgcaaaa aaagcctcca agggtttcga 1020

ctggtccagc tctgacatcc cttcctggaa acagcatgaa taaaacactc atcccatggg 1080

tccaaattaa tatgattctg ctcccccctt ctccttttag acatggttgt gggtctggag 1140

ggagacgtgg gtccaaggtc ctcatcccat cctccctctg ccaggcacta tgtgtctggg 1200

gcttcgatcc ttgggtgcag gcagggctgg gacacgcggc ttccctccca gtccctgcct 1260

tggcaccgtc acagatgcca agcaggcagc acttagggat ctcccagctg ggttagggca 1320

gggcctggaa atgtgcattt tgcagaaact tttgagggtc gttgcaagac tgtgtagcag 1380

gcctaccagg tccctttcat cttgagaggg acatggccct tgttttctgc agcttccacg 1440

cctctgcact ccctgcccct ggcaagtgct cccatcgccc cggtgcccac catgagctcc 1500

cagcacctga ctccccccac atccaagggc agcctggaac cagtggctag ttcttgaagg 1560

agccccatca atcctattaa tcctcagaat tccagtggga gcctccctct gagccttgta 1620

gaaatgggag cgagaaaccc cagctgagct gcgttccagc ctcagctgag tctttttggt 1680

ctgcacccac ccccccaccc cccccccccc gcccacatgc tccccagctt gcaggaggaa 1740

tcggtgaggt cctgtcctga ggctgctgtc cggggccggt ggctgccctc aaggtccctt 1800

ccctagctgc tgcggttgcc attgcttctt gcctgttctg gcatcaggca cctggattga 1860

gttgcacagc tttgctttat ccgggcttgt gtgcagggcc cggctgggct ccccatctgc 1920

acatcctgag gacagaaaaa gctgggtctt gctgtgccct cccaggctta gtgttccctc 1980

cctcaaagac tgacagccat cgttctgcac ggggctttct gcatgtgacg ccagctaagc 2040

atagtaagaa gtccagccta ggaagggaag gattttggag gtaggtggct ttggtgacac 2100

actcacttct ttctcagcct ccaggacact atggcctgtt ttaagagaca tcttattttt 2160

ctaaaggtga attctcagat gataggtgaa cctgagttgc agatatacca acttctgctt 2220

gtatttctta aatgacaaag attacctagc taagaaactt cctagggaac tagggaacct 2280

atgtgttccc tcagtgtggt ttcctgaagc cagtgatatg ggggttagga taggaagaac 2340

tttctcggta atgataagga gaatctcttg tttcctccca cctgtgttgt aaagataaac 2400

tgacgatata caggcacatt atgtaaacat acacacgcaa tgaaaccgaa gcttggcggc 2460

ctgggcgtgg tcttgcaaaa tgcttccaaa gccaccttag cctgttctat tcagcggcaa 2520

ccccaaagca cctgttaaga ctcctgaccc ccaagtggca tgcagccccc atgcccaccg 2580

ggacctggtc agcacagatc ttgatgactt ccctttctag ggcagactgg gagggtatcc 2640

aggaatcggc ccctgcccca cgggcgtttt catgctgtac agtgacctaa agttggtaag 2700

atgtcataat ggaccagtcc atgtgatttc agtatataca actccaccag acccctccaa 2760

cccatataac accccacccc tgttcgcttc ctgtatggtg atatcatatg taacatttac 2820

tcctgtttct gctgattgtt tttttaatgt tttggtttgt ttttgacatc agctgtaatc 2880

attcctgtgc tgtgtttttt attacccttg gtaggtatta gacttgcact tttttaaaaa 2940

aaggtttctg catcgtggaa gcatttgacc cagagtggaa cgcgtggcct atgcaggtgg 3000

attccttcag gtctttcctt tggttctttg agcatctttg ctttcattcg tctcccgtct 3060

ttggttctcc agttcaaatt attgcaaagt aaaggatctt tgagtaggtt cggtctgaaa 3120

ggtgtggcct ttatatttga tccacacacg ttggtctttt aaccgtgctg agcagaaaac 3180

aaaacaggtt aagaagagcc gggtggcagc tgacagagga agccgctcaa ataccttcac 3240

aataaatagt ggcaatatat atatagttta agaaggctct ccatttggca tcgtttaatt 3300

tatatgttat gttctaagca cagctctctt ctcctatttt catcctgcaa gcaactcaaa 3360

atatttaaaa taaagtttac attgtagtta ttttcaaatc tttgcttgat aagtattaag 3420

aaatattgga cttgctgccg taatttaaag ctctgttgat tttgtttccg tttggatttt 3480

tgggggaggg gagcactgtg tttatgctgg aatatgaagt ctgagacctt ccggtgctgg 3540

gaacacacaa gagttgttga aagttgacaa gcagactgcg catgtctctg atgctttgta 3600

tcattcttga gcaatcgctc ggtccgtgga caataaacag tattatcaaa gagaaaaaaa 3660

aaaaaaaaaa 3670

## Description

### STATEMENT REGARDING FEDERALLY SPONSORED RESEARCH

This invention was made with government support under grant number R01AR063630 awarded by the National Institutes of Health. The government has certain rights in the invention.

### BACKGROUND OF THE INVENTION

### Field of the Invention

This disclosure relates to genetically engineered tissue organoids capable of noninvasively reporting sensed biochemical signals in an individual and/or providing a therapeutic agent to the individual to monitor and/or treat a disease or improve an individual's health.

### Description of Related Art

Obesity and diabetes are examples of acute and growing global health concerns that can require careful monitoring and therapeutic intervention (see Ahima, R. S. Digging deeper into obesity. *J Clin Invest* **121**, 2076-2079, 2011). Diabetes, in particular, often requires daily monitoring of blood glucose levels. However, such constant blood monitoring can be inconvenient, time consuming, and painful.

In addition, there are numerous other diseases such as phenylketonuria, substance addiction, and heart disease, to name a few, that exhibit elevated blood concentrations of specific disease-associated indicators or blood factors (e.g., amino acids, illicit chemicals, proteins, lipids, enzymes, metabolites, etc.). However, while certain diseases like diabetes can require constant indicator monitoring (*i.e*., blood glucose levels), others may only require periodic monitoring such as once a week or once a month or may even only require sporadic, on-demand monitoring.

Recent advances in blood factor monitoring have provided improved options for individuals, such as diabetics needing to monitor blood glucose levels. For example, implantable glucose monitors enable diabetics to continuously monitor blood glucose levels remotely using compatible smart devices. Indeed, biointegrated sensors can address various monitoring challenges in medicine by transmitting a wide variety of biological signals, including electrophysiological, physiological, and biochemical information continuously (see Kim et al. Flexible and stretchable electronics for biointegrated devices. Annual review of biomedical engineering 14, 113-128, 2012).

Concomitant with the requirements of blood monitoring for some individuals is the need to receive therapeutic agents to counter the underlying disease causing the elevated concentrations of disease-associated indicators. For example, diabetic individuals often require insulin supplementation. Insulin supplementation is typically self-administered by injection, which similar to blood monitoring, can be inconvenient, difficult to remember, time consuming, and painful. Fortunately, as with advances in blood monitoring technology, new biointegrated devices are becoming available that provide greater convenience to individuals requiring periodic, self-administered monitoring and administration of therapeutic agents. For example, devices combining glucose monitors and insulin pumps are available that constantly monitor glucose levels and periodically deliver rapid- or short-acting insulin through a catheter, as required. Further, "smart insulin patches" are being developed that integrate thin polymeric platforms covered with needles with live beta cells.

There are still other diseases where proteins or metabolites are either non-functional or expressed at insufficient levels but do not increase concentrations of a particular, telltale, disease-associated indicator. For example, hemophilia A and hemophilia B are genetic diseases where individuals express insufficient amounts of clotting factors VIII and IX, respectively. Yet, hemophiliacs can be treated by replacing these missing clotting factors which can be harvested from human blood or made recombinantly.

While advancements in biointegrated device design and fabrication using novel nano-materials has greatly accelerated development for applications *in vivo,* including brain, heart, and skin (see Kim *et al.* and Choi et al. Recent Advances in Flexible and Stretchable Bio-Electronic Devices Integrated with Nanomaterials. Advanced materials 28, 4203-4218, 2016), the requisite biomechanical interfaces for these devices still remain problematic. For example, stability, biocompatibility, and potential infection from the implanted devices remain technically challenging to the field. Moreover, current biointegrated devices either require replacement of batteries and/or therapeutic agent reservoirs and/or must be replaced themselves once their useful lifespan has been reached.

In light of the foregoing, there is a need for improved, stable, and permanent means for constantly monitoring a disease state and/or administering therapeutic agents and that can be physiologically tailored for a specific individual.

### SUMMARY OF THE INVENTION

Provided herein are physiologically-tailored tissue organoids and methods of their use for monitoring and treating diseases.

In a first aspect, the invention provides a physiologically-tailored tissue organoid that includes a plurality of genetically engineered cells including at least one recombinant gene encoding a reporter molecule. The reporter molecule produces a detectable signal when associated with and/or contacting a predetermined blood factor. In one embodiment, tissue organoid includes a stratified skin graft grown from cells taken from an individual. In another embodiment, the tissue organoid includes a cultured skin graft grown from embryonic stem cells, human induced pluripotent stem cells, epidermal stem cells, or keratinocytes. In one embodiment, when the tissue organoid is biontegrated into an individual, the tissue organoid expresses the reporter molecule in proportion to a concentration of a blood factor in the individual's blood. In a further embodiment, the blood factor includes a cell, an enzyme, a protein, a polypeptide, an amino acid, a polynucleotide, a nucleic acid, a sugar, a lipid, a metabolite, a synthetic chemical compound, a naturally occurring chemical compound, a mineral, a metal, a bacterium, a virus, a prion, a disease indicator, or combinations thereof. In one embodiment, the physiologically-tailored tissue organoid is biointegrated into an individual. The individual can be an animal.

In a second aspect, the invention provides a physiologically-tailored tissue organoid includes a plurality of genetically engineered cells comprising at least one recombinant gene encoding a therapeutic agent. When the therapeutic agent is administered to an individual in need thereof, the therapeutic agent improves the individual's health. In one embodiment, the therapeutic agent comprises an enzyme, a protein, a clotting factor, a vitamin, a peptide, a lipid, a toxin, or a combination thereof. In another embodiment, expression of the therapeutic agent is inducible by an inducer. The inducer can be one or more of heat, cold, light, a protein, a hormone, a lipid, a chemical, a metabolic change, an electric potential or field, and combinations thereof. In one embodiment, when expression of the therapeutic agent is induced, the therapeutic agent is released into the individual's circulation. In another embodiment, the physiologically-tailored tissue organoid can biointegrated into an individual. The individual can be an animal. The predetermined blood factor can be glucose.

In a third aspect, the invention provides a physiologically-tailored tissue organoid including a plurality of genetically engineered cells comprising at least one recombinant gene encoding a reporter molecule and at least one recombinant gene encoding a therapeutic agent. The therapeutic agent is expressed as a function of expression of the reporter molecule.

In a fourth aspect, the invention provides a method of monitoring a blood factor including biointegrating a tissue organoid according to any of the preceding aspects or embodiments into an individual and detecting a detectable signal produced by a reporter molecule expressed by the tissue organoid in response to a blood factor. The detectable signal is proportional to a concentration of the blood factor in the individual. In one embodiment of the method, the tissue organoids are biointegrated into an individual by grafting or surgical implantation. In another embodiment of the method, the detectable signal is detected by one or more of a fluorometer, a colorimeter, a bioluminescence monitoring system, and an electrical system with proper electrodes.

In a fifth aspect, the invention provides a method of treating a disease in an individual in need thereof. The method includes biointegrating a tissue organoid according to any of the preceding aspects or embodiments into an individual and administering a therapeutic agent to the individual. In one embodiment, the individual is a human.

In a sixth aspect, the invention provides a physiologically-tailored tissue organoid includes a population of genetically engineered cells comprising at least one recombinant gene encoding a therapeutic agent. The therapeutic agent comprises at least one of mGLP1 and hBChE, and when the therapeutic agent is administered to an individual in need thereof, the therapeutic agent improves the individual's health. In one embodiment, the therapeutic agent improves the individual's health by reducing the individual's seeking and/or consumption of at least one of nicotine, alcohol, cocaine, and amphetamine.

In a seventh aspect, the invention provides a method of treating cocaine addiction in an individual in need thereof, the method including contacting a tissue organoid to the individual, the tissue organoid comprising a population of genetically engineered cells comprising at least one recombinant gene encoding a therapeutic agent, wherein the therapeutic agent comprises hBChE. In one embodiment, the tissue organoid is transplanted into the individual, In one embodiment, hBChE is expressed constitutively or by induction with an inducer. In one embodiment, the expression of hBChE hydrolyzes cocaine in the blood of the individual. In one embodiment, the expression of hBChE causes decreased cocaine seeking and/or consumption by the individual.

In an eighth aspect, the invention provides a method of treating AUD in an individual in need thereof, the method including contacting a tissue organoid to the individual, the tissue organoid comprising a population of genetically engineered cells comprising at least one recombinant gene encoding a therapeutic agent, wherein the therapeutic agent comprises mGLP1 or an analog thereof. In one embodiment, the tissue organoid is transplanted into the individual. In one embodiment, the mGLP1 or the analog thereof is expressed constitutively or by induction with an inducer. In one embodiment, the expression of mGLP1 or the analog thereof causes decreased alcohol seeking and/or consumption by the individual.

In a ninth aspect, the invention provides a method of treating nicotine addiction in an individual in need thereof, the method including contacting a tissue organoid to the individual, the tissue organoid comprising a population of genetically engineered cells comprising at least one recombinant gene encoding a therapeutic agent, wherein the therapeutic agent comprises mGLP1 or an analog thereof. In one embodiment, the tissue organoid is transplanted into the individual. In one embodiment, mGLP1 or the analog thereof is expressed constitutively or by induction with an inducer. In one embodiment, the expression of mGLP1 or the analog thereof causes decreased nicotine seeking and consumption by the individual.

In a tenth aspect, the invention provides a method of treating amphetamine addiction in an individual in need thereof, the method including contacting a tissue organoid to the individual, the tissue organoid comprising a population of genetically engineered cells comprising at least one recombinant gene encoding a therapeutic agent, wherein the therapeutic agent comprises mGLP1 or an analog thereof. In one embodiment, the tissue organoid is transplanted into the individual. In one embodiment, mGLP1 or the analog thereof is expressed constitutively or by induction with an inducer. In one embodiment, the expression of mGLP1 causes decreased amphetamine seeking and consumption by the individual.

In an eleventh aspect, the invention provides a method of treating obesity in an individual in need thereof, the method including contacting a tissue organoid to the individual, the tissue organoid comprising a population of genetically engineered cells comprising at least one recombinant gene encoding a therapeutic agent, wherein the therapeutic agent comprises peptide tyrosine tyrosine (PYY) or an analog thereof. In one embodiment, the tissue organoid is transplanted into the individual. In one embodiment, PYY or the analog thereof is expressed constitutively or by induction with an inducer. In one embodiment, the expression of PYY causes decreased food seeking and consumption by the individual.

In a twelfth aspect, the invention provides a method of treating the effects of aging in an individual in need thereof, the method including contacting a tissue organoid to the individual, the tissue organoid comprising a population of genetically engineered cells comprising at least one recombinant gene encoding a therapeutic agent, wherein the therapeutic agent comprises tissue inhibitor of metalloproteinases 2 (TIMP2) or an analog thereof. In one embodiment, the tissue organoid is transplanted into the individual. In one embodiment, TIMP2 or the analog thereof is expressed constitutively or by induction with an inducer. In one embodiment, the expression of TIMP2 causes a decrease in the negative effects of aging in an individual. In one embodiment, the negative effects of aging include memory loss, reduced vascular response, and/or reduced immune response.

These and other features and advantages of the present invention will be more fully understood from the following detailed description taken together with the accompanying claims. It is noted that the scope of the claims is defined by the recitations therein and not by the specific discussion of features and advantages set forth in the present description.

### DESCRIPTION OF DRAWINGS

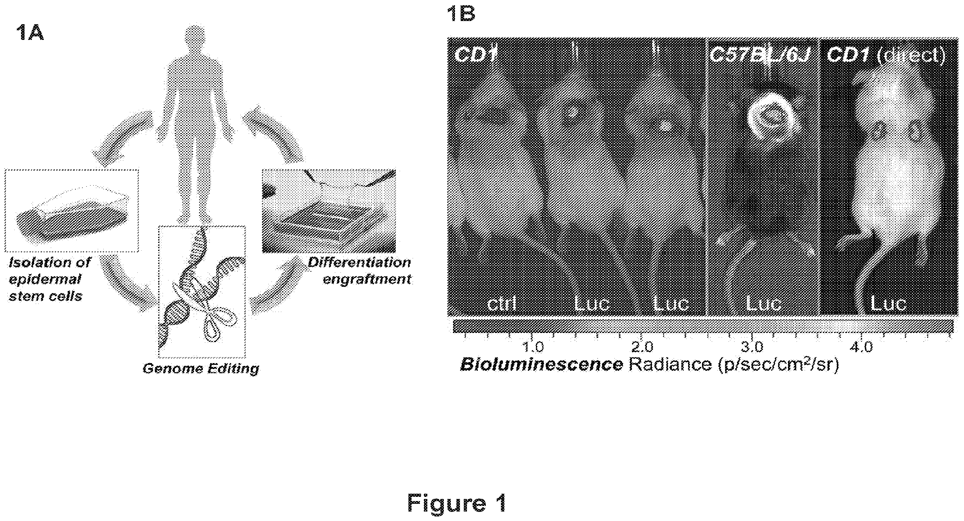

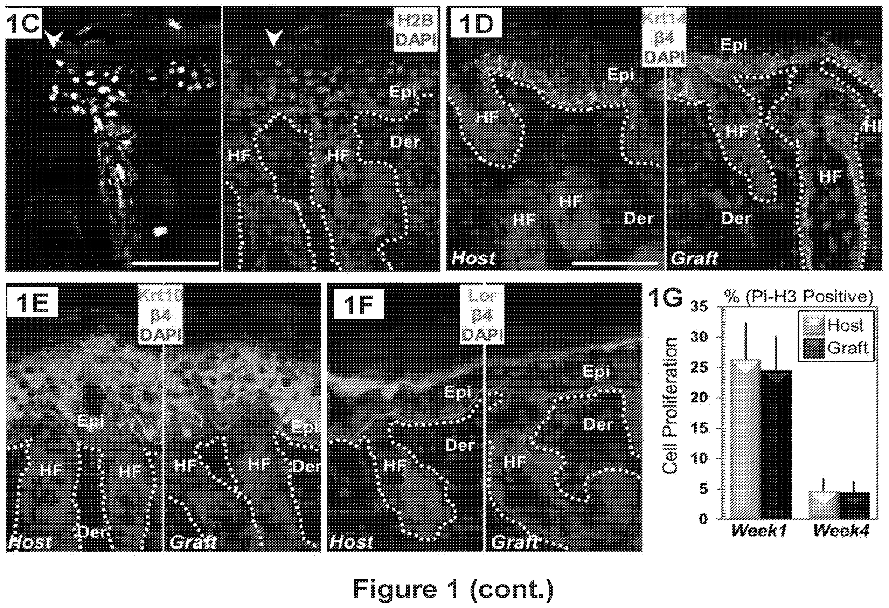

**FIG. 1****. Development of a mouse-to-mouse cutaneous gene transfer model with immunocompetent hosts.** **FIG. 1A****:** Diagram of the cutaneous gene transfer strategy. Primary epidermal stem cells are isolated and cultured from patients' skin biopsy. The cells are genetically modified with genome editing technology, and the resultant cells are used to generate skin organoids and transplant to the same patient for clinical applications. **FIG. 1B****:** Images of immunocompetent mice (CD1 strain or C57BL/6J strain) grafted with isogenic skin organoids with (left two panels) or without (right panel, direct grafting) the assistance of skin dome chamber for transplantation. Intravital imaging shows efficient incorporation of grafted cells expressing *luciferase* (Luc) upon engraftment. Ctrl: control. **FIG. 1C**: Sections of CD1 skin with grafted *H2B-RFP*-expressing keratinocytes were stained with DAPI. Dotted lines denote dermal-epidermal boundaries. Arrow heads denote the boundary between grafted skin and host skin. Scale bar=50 µm. Epi: epidermis, Der: dermis, HF: hair follicle. FIGS. D-F: Sections of grafted skin and adjacent host skin were immunostained with different antibodies as indicated (Krt14: keratin 14, Krt10: keratin 10, Lor: Loricrin, β4: β4-integrin, CD104). Scale bar=50 µm. FIG. 1G: Proliferation of epidermal cells in host or grafted skin one or four weeks post skin transplantation was determined and quantified by immunohistological staining with antibody against phosphor-histone H3. A significant decrease in cell proliferation was observed over time after skin grafting, most likely due to skin wound healing. There is no significant change in cell proliferation between the grafted skin and adjacent host skin (P > 0.05). Error bar represents s.d. (standard deviation) unless otherwise indicated. The sample size (*n*) = 7 (7 representative sections obtained from 3 different animals for each group).

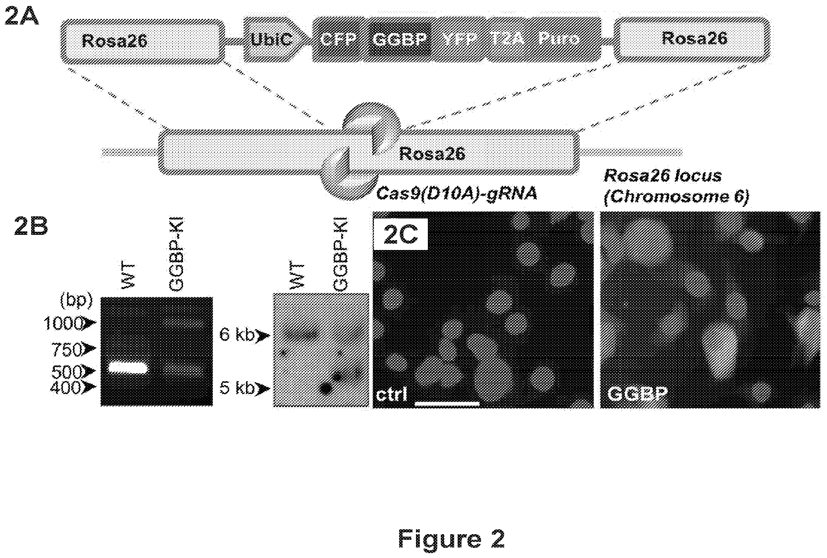

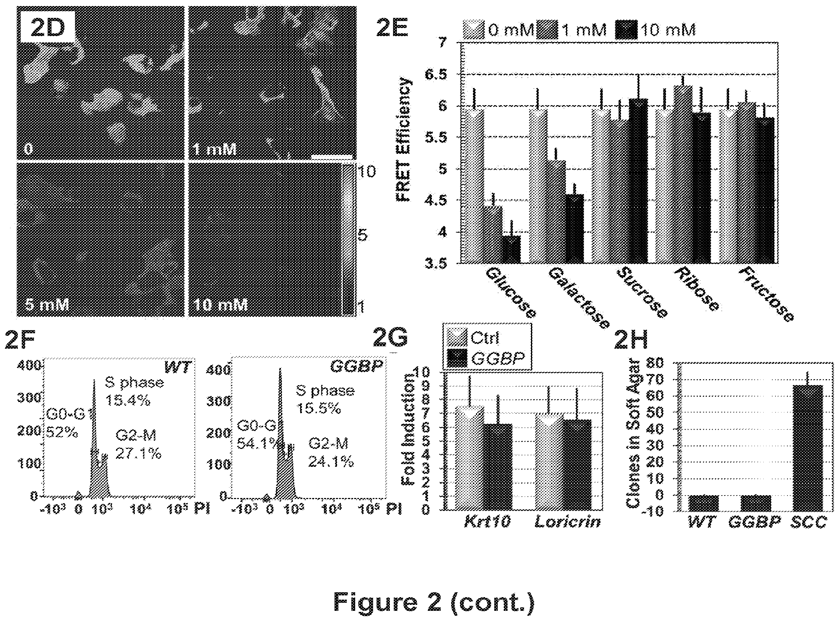

**FIG. 2****. Engineering skin epidermal stem cells with CRISPR.** **FIG. 2A****:** Diagram showing the Rosa26 targeting strategy for expression of glucose sensor *GGBP*, The targeting vector contains two *Rosa26* homology arms, flanking the expression cassette for GGBP and a selection marker (puromycin resistant gene, Puro) by a constitutive promoter UbiC (Ubiquitin C promoter). GGBP and Puro are separated by a self-cleavable peptide T2A. **FIG. 2B****:** Integration of the targeting vector into *Rosa26* locus was verified by PCR (left panel) and southern blotting (right panel). Positive clones displayed an additional band of the expected size. FIG. 2C: Expression of GGBP sensor was confirmed in targeted cells by fluorescence imaging. Scale bar = 20 µm. FIG. 2D: FRET ratio images were pseudocolored to demonstrate glucose-dependent ratio changes in engineered cells. Red indicates high (H) FRET efficiency, and blue represents low (L) efficiency. M: medium FRET efficiency. Integration of the ratio over the entire cells was used to quantify the FRET ratio changes. FIG. 2E: The FRET ratio change of GGBP reporter was determined in the presence of various monosaccharides or oligosaccharides at different concentration. Note: only glucose and galactose led to significant FRET ratio changes (P < 0.01). *n* > 6 (individual cells). **FIG. 2F****:** FACS (fluorescence activated cell sorting) demonstrated similar cell cycle profiles for WT (wild type) and GGBP-expressing epidermal stem cells. PI: propidium iodine. FIG. 2G: Western blotting analysis of early (Krt10) and late (loricrin) differentiation marker expression in WT and GGBP-expressing cells upon calcium shift. Band intensity was determined by densitometry and fold of induction was quantified, *n* = 4 (4 independent tests). *P* > 0.05. FIG. 2H: WT cells or *GGBP* cells were tested for anchorage independent growth in soft agar. Note: no growth for WT or GGBP cells, but tumor initiating cells isolated from skin SCC (squamous cell carcinoma) can readily produce colonies in soft agar plate. *n* = 3. P < 0.01 (between WT and SCC or GGBP and SCC).

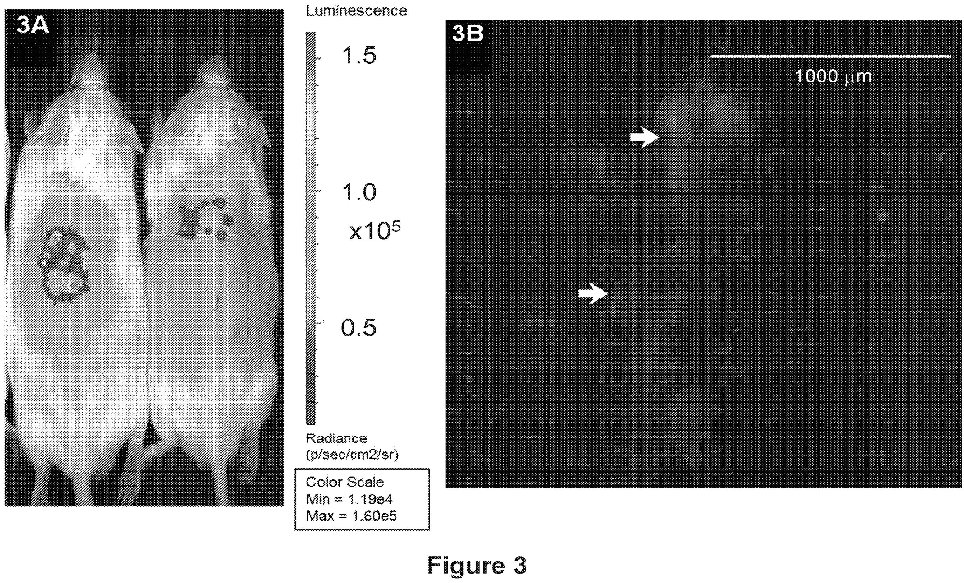

FIG. 3. Transfecting skin cells *in vivo* with electroporation. FIG. 3A: CD1 mice were electroporated intradermally with plasmid DNA encoding luciferase. Expression of *luciferase* was determined by bioluminescence imaging two days after treatment. FIG. 3B: CD1 mice were electroporated intradermally with plasmid DNA encoding *tdTomato.* Expression of red fluorescence protein was determined by intravital imaging with two-photon microscope. Arrows denote *tdTomato-*expressing cells in skin.

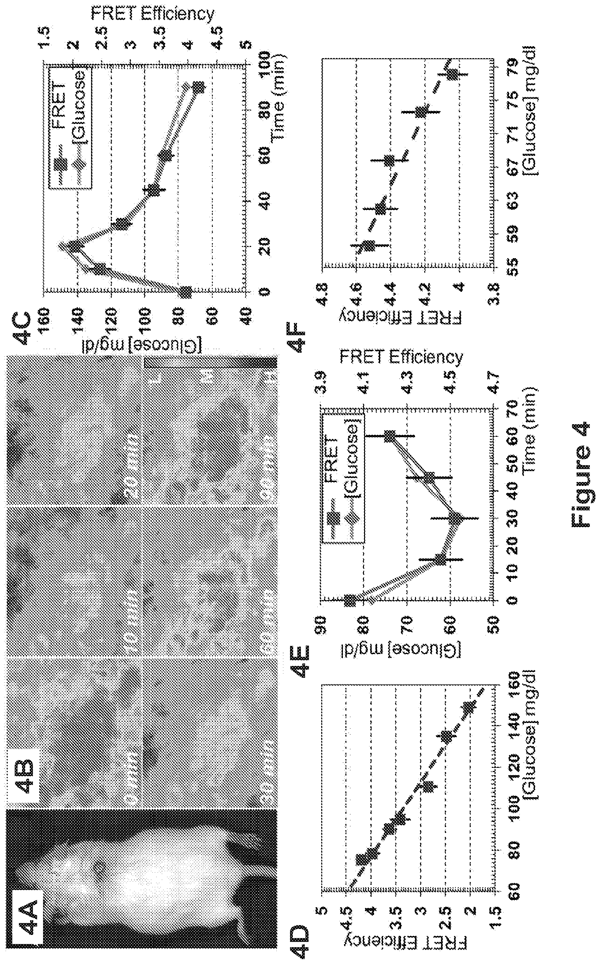

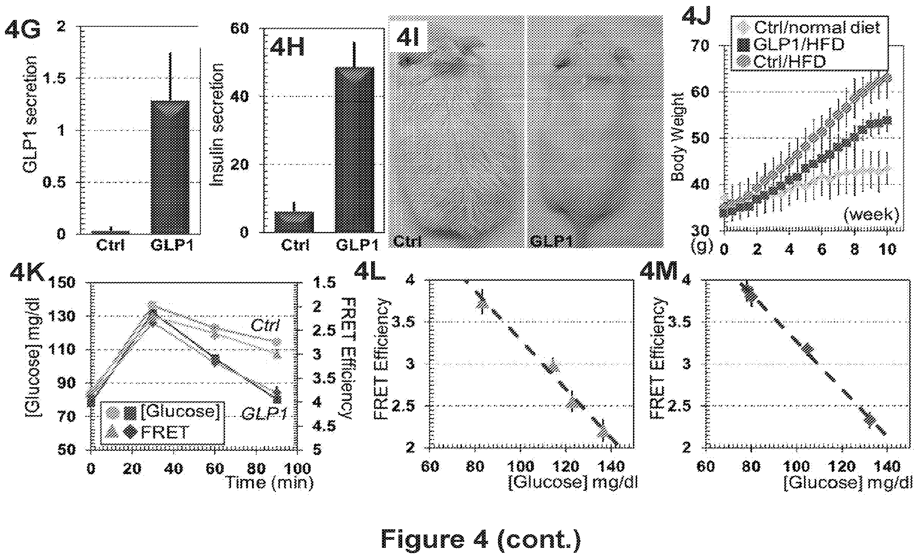

**FIG. 4****. Monitoring changes of blood glucose level with GGBP reporter *in vivo.*** **FIG. 4A**: Skin organoids were developed from control or GGBP-producing cells, and transplanted to CD1 mice. **FIG. 4B****:** Glucose fluctuation was induced in grafted animal with IPGTT (intraperitoneal glucose tolerance test). FRET ratio images were pseudocolored to demonstrate glucose-dependent ratio changes in grafted skin. Red indicates high FRET efficiency, and blue represents low efficiency. **FIG. 4C and 4D****:** Correlation of FRET ratio with blood glucose concentration upon IPGTT (intraperitoneal glucose tolerance test). *n* = 9 (integrated FRET value from 9 different fields at each time point). FIG. 4E and 4F: Insulin injection was used to induce hypoglycemia in the grafted animals. Intravital imaging with the grafted skin demonstrated the correlation between FRET ratios of GGBP reporter with blood glucose concentration. *n* = 9 (integrated FRET value from 9 different fields at each time point).
FIG. 4G: Secretion of *GLP1* in cell culture medium was determined by ELISA (enzyme-linked immunosorbent assay), *n* = 3 (3 independent tests). *P* < 0.01. FIG. 4H: Conditioned medium was collected from different cell cultures and used to treat starved insulinoma cells. Secretion of insulin *in vitro* was determined by ELISA. *n* = 4 (4 independent tests). P < 0.01. **FIG. 4I**: Images of control and GLP1 animals fed with HFD (high fat diet). FIG. 4J: Body weight change of different cohorts of mice measured from ~10 weeks of age. Note that the HFD induced significant obesity in control mice (P < 0.01, between control mice with normal diet or HFD for week 8-10) but that expression of *GLP1* inhibited weight gain (P < 0.05, between GLP1/HFD and control/HFD groups for week 8-10). *n* = 5 (animals). FIG. 4K: Correlation of FRET ratio with blood glucose concentration over time upon IPGTT in control and *GLP1* mice, *n* = 9 (integrated FRET value from 9 different fields at each time point). FIG. 4L and 4M: Correlation of blood glucose concentration with GGBP FRET changes in control (L) and GLP1 (M) mice. *n* = 9 (integrated FRET value from 9 different fields at each time point).

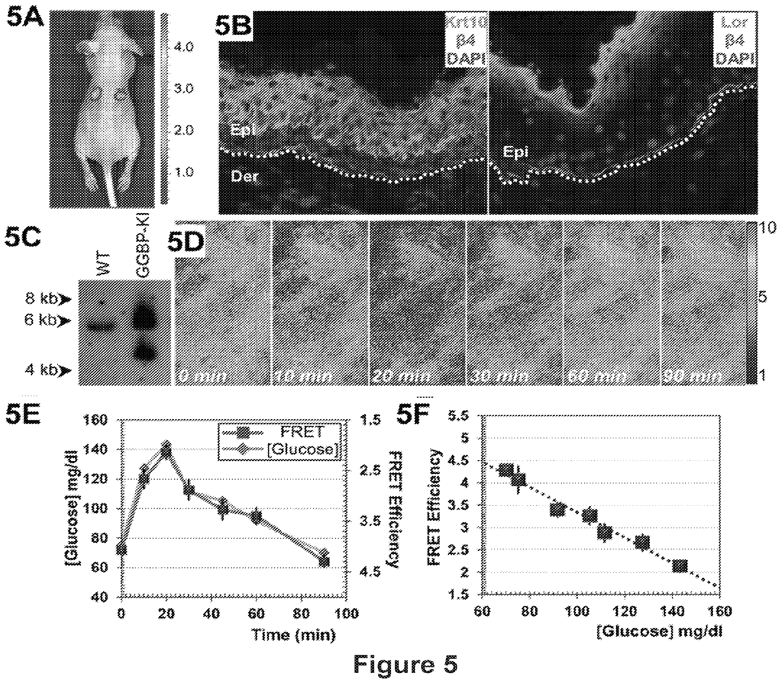

**FIG. 5****. Expression of GGBP *reporter* in human epidermal stem cells with** CRISPR. FIG. 5A: Image of nude mouse grafted with organotypic human skin culture. Intravital imaging shows efficient incorporation of grafted cells expressing *luciferase.* FIG. 5B: Sections of grafted skin and adjacent host skin were immunostained with different antibodies as indicated. Scale bar=50 µm. FIG. 5C: Integration of the targeting vector into *AAVS1* locus was verified by southern blotting. Positive clones display an additional band of the expected size. FIG. 5D: Glucose fluctuation was induced in grafted animal with an IPGTT (intraperitoneal glucose tolerance test). FRET ratio images were pseudocolored to demonstrate glucose-dependent ratio changes in grafted skin. Red indicates high FRET efficiency, and blue represents low efficiency. **FIG. 5E and 5F****:** Correlation of FRET ratio with blood glucose concentration upon IPGTT. *n* = 9 (integrated FRET value from 9 different fields at each time point).

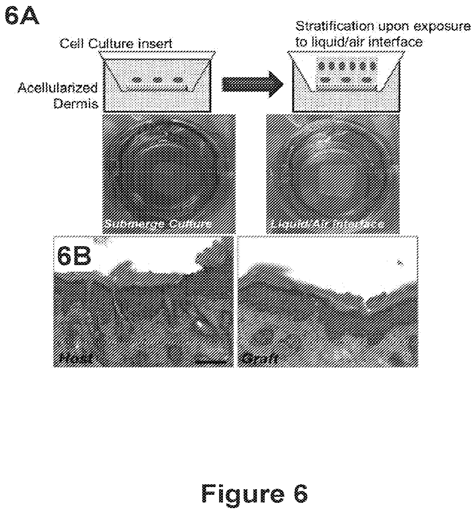

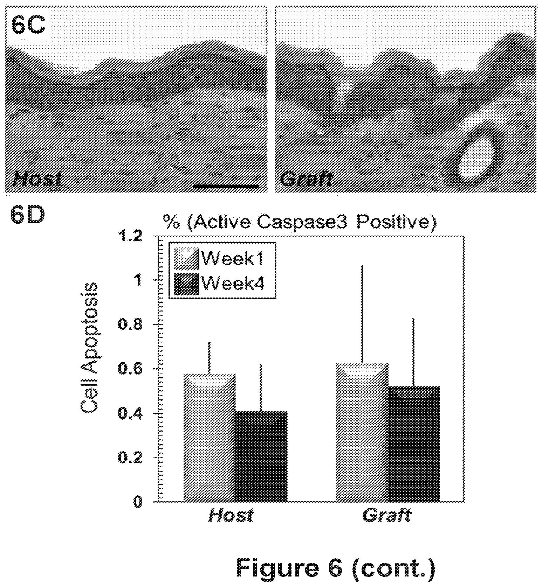

FIG. 6. Development of a mouse-to-mouse cutaneous gene transfer model with immunocompetent hosts. FIG. 6A: Diagram demonstrating the procedure for skin organotypic culture *in vitro.* Epidermal progenitor cells were plated on top of acellularized dermis, and then exposed to air/liquid interphase to induce differentiation and stratification as skin epidermis *in vivo.* FIGS. 6B and 6C: H/E (haematoxylin and eosin) staining of grafted skin and adjacent host skin one (B) or four (C) weeks after skin transplantation. Scale bar = 50 µm. **FIG. 6D****:** Apoptosis of epidermal cells in host or grafted skin one or four weeks post skin transplantation was determined and quantified by immunohistological staining with antibody against active caspase 3. Error bar represents s.d. (standard deviation) unless otherwise indicated. *n* = 4 (4 independent tests). *P* > 0.05.

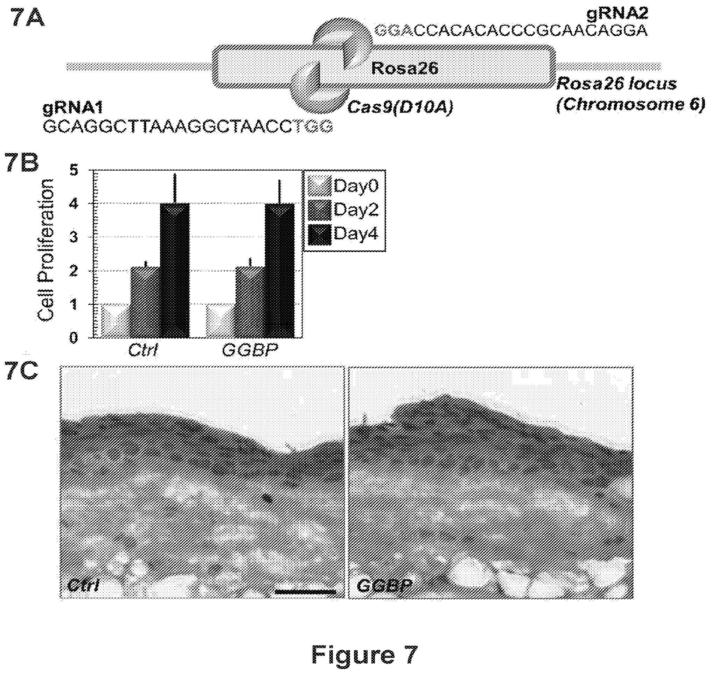

FIG. 7. Engineering GGBP-producing skin epidermal progenitor cells with **CRISPR**. **FIG. 7A**: Diagram showing Rosa26 targeting strategy for expression of *GGBP.* Expression vector encoding D10A mutant of cas9 and two gRNAs targeting Rosa26 locus is used to create the cleavage in the chromosomal DNA and enhance integration of *GGBP* targeting vector. **FIG. 7B****:** Cell proliferation of control (Ctrl) and GGBP-expressing cells. Fold increase of cell numbers is quantified for all cell types. *n* = 3 (4 independent tests). *P* > 0.05 for each time point between ctrl and GGBP groups. FIG. 7C: Control or GGBP targeted epidermal progenitor cells can produce similar skin organoids *in vitro.* Scale bar = 50 µm.

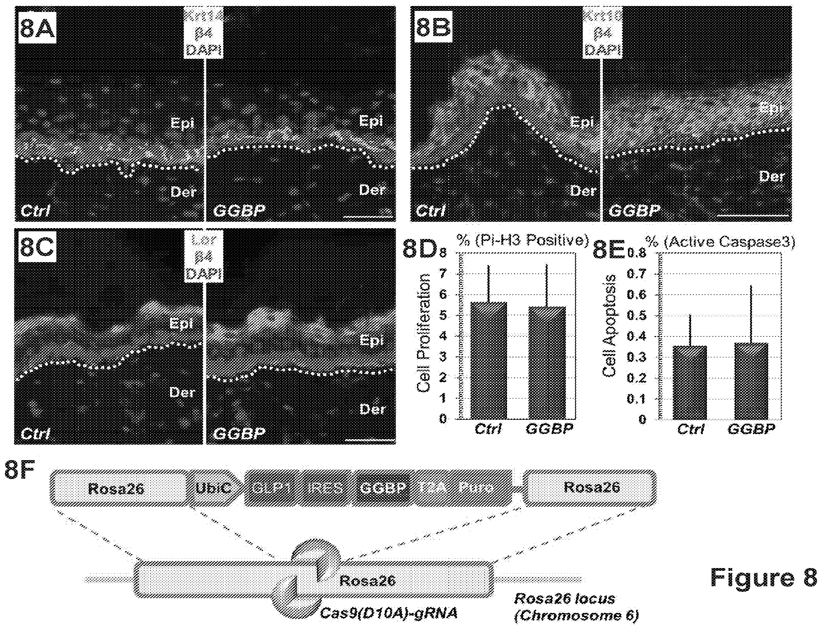

**FIG. 8****. Engraftment of *GGBP*-expressing cells *in vivo.*** **FIGS. 8A-8C****:** Sections of grafted skin were immunostained with different antibodies as indicated (Krt14: keratin 14, Krt10: keratin 10, Lor: Loricrin, β4: β4-integrin, CD104). Dotted lines denote dermal-epidermal boundaries. Epi: epidermis; Der: dermis. Scale bar = 50 µm. **FIG. 8D**: Proliferation of epidermal cells in control or GGBP skin grafts was determined and quantified by immunohistological staining with antibody against phosphor-histone H3. *n* = 7 (7 sections obtained from 2 animals for each group). P > 0.05. FIG. 8E: Apoptosis of epidermal cells in host or grafted skin one or four weeks post skin transplantation was determined and quantified by immunohistological staining with antibody against active caspase 3. *n* = 6 (6 sections obtained from 2 animals for each group). P > 0.05. FIG. 8F: Diagram showing Rosa26 targeting strategy for expression of *GGBP* and GLP1 simultaneously. Coding sequences of GGBP and *GLP1* is separated by IRES (internal ribosome entry site).

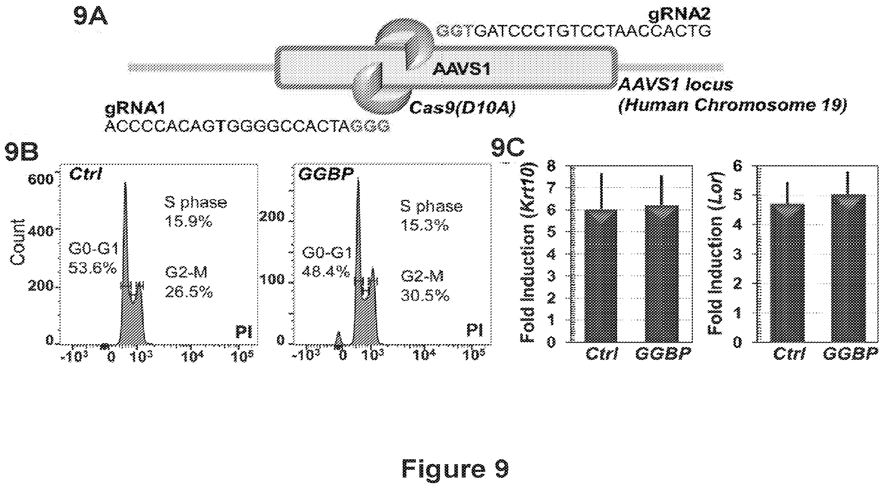

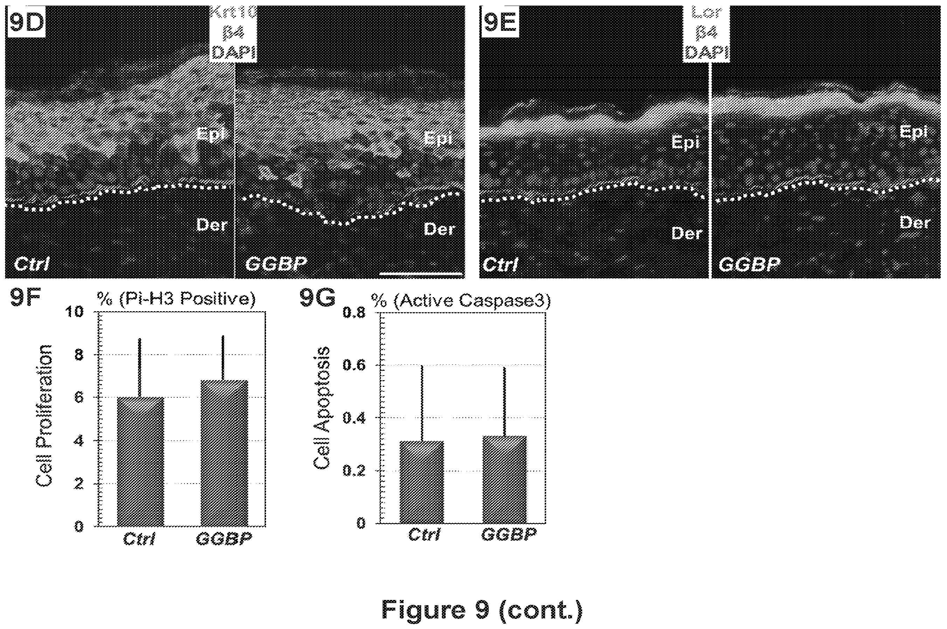

**FIG. 9****. Expression of *GGBP* in human epidermal progenitor cells with CRISPR**. **FIG. 9A****:** Diagram showing *AAVS1* targeting strategy for expression of GGBP. Expression vector encoding D10A mutant of *cas9* and two gRNAs targeting *AAVS1* locus is used to create the cleavage in the chromosomal DNA and enhance integration of GGBP targeting vector. **FIG.** 9B: FACS (fluorescence activated cell sorting) demonstrates similar cell cycle profiles of WT (wild type) and GGBP-expressing epidermal progenitor cells before and after doxycycline treatment. PI: propidium iodine. FIG. 9C: Western blotting analysis of early (C) and late (D) differentiation marker expression in WT and GGBP-expressing cells upon calcium shift. Band intensity was determined by densitometry and fold of induction is quantified. Krt10: keratin 10; Lor: loricrin. *n* = 4 (4 independent tests). *P* > 0.05. FIGS. **9D and** 9E: Sections of grafted skin were immunostained with different antibodies as indicated. Scale bar=50 µm. FIGS. **9F and 9G**: Proliferation and apoptosis of epidermal cells in control or GGBP skin grafts was determined and quantified by immunohistological staining with antibody against phosphor-histone H3 and active caspase 3 respectively. For proliferation, *n* = 7 (7 sections obtained from 2 animals for each group). P > 0.05. For apoptosis, *n* = 6 (6 sections obtained from 2 animals for each group). *P* > 0.05.

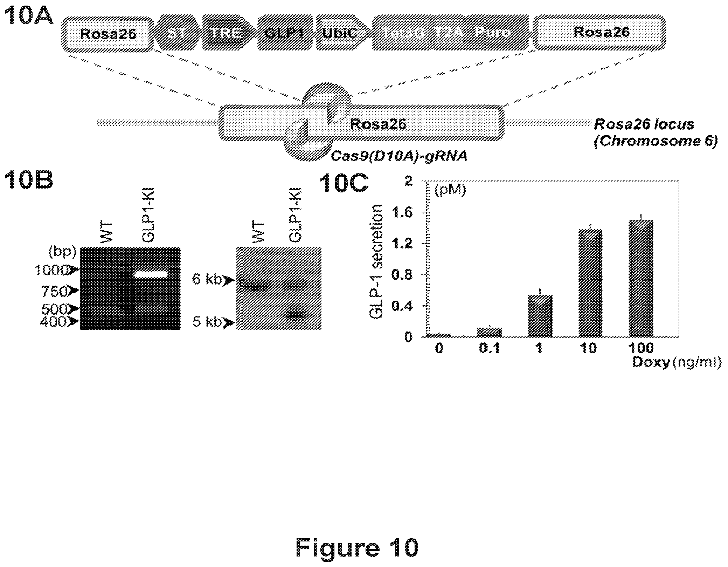

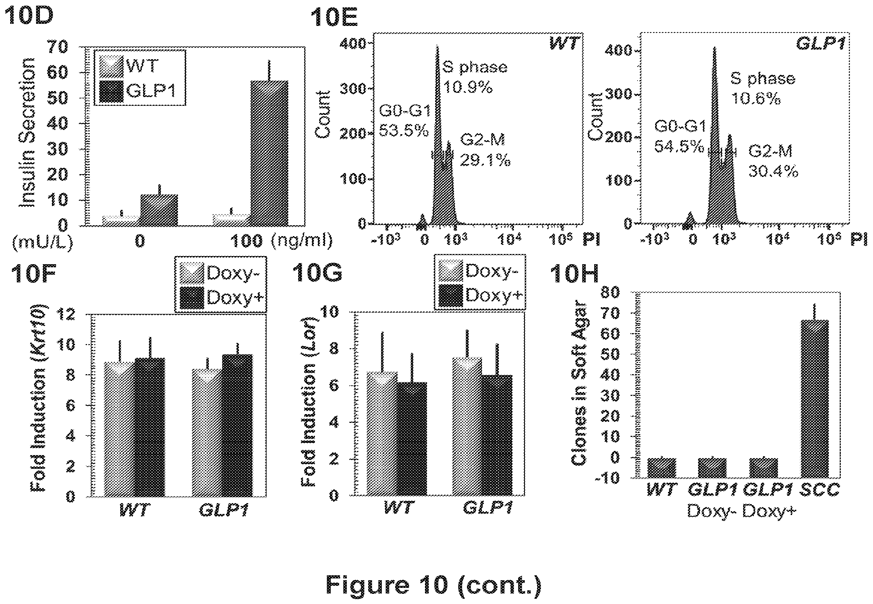

FIG. 10. Engineering ***GLP1*-producing** skin epidermal stem cells with **CRISPR**. FIG. **10A**: Diagram showing the Rosa26 targeting strategy for expression of *GLP1.* The targeting vector contains two Rosa26 homology arms, flanking the expression cassette for *GLP1*. The tetracycline-inducible expression cassette drives expression of Tet3G (tetracycline transactivator) protein and a selection marker (puromycin resistant gene, Puro) by a constitutive promoter UbiC (Ubiquitin C promoter). Tet3G and Puro are separated by a self-cleavable peptide T2A. Expression of the *GLP1* fusion protein is controlled by TRE (tet-on) promoter A transcriptional stop signal (ST) is included in front of the TRE promoter to eliminate the leakage expression of *GLP1.* FIG. **10B**: Integration of the targeting vector into *Rosa26* locus is verified by PCR (left panel) and southern blotting (right panel). Positive clones display an additional band of the expected size. FIG. **10C**: Secretion of *GLP1* in cell culture medium is determined by ELISA (enzyme-linked immunosorbent assay) upon stimulation with different amount of doxycycline (Doxy). FIG. 10D: Conditioned medium is collected from different cell cultures and used to treat starved insulinoma cells. Secretion of insulin *in vitro* is determined by ELISA. FIG. **10E**: FACS (fluorescence activated cell sorting) demonstrates similar cell cycle profiles for WT (wild type) and *GLP1*-expressing epidermal stem cells after doxycycline treatment. PI: propidium iodine. FIGS. 10F and **10G**: Western blotting analysis of early (F) and late (G) differentiation marker expression in WT and *GLP1*-expressing cells upon calcium shift. Band intensity was determined by densitometry and fold of induction is quantified. Krt10: keratin 10; Lor: loricrin. FIG. 10H: WT cells or *GLP1* cells with or without doxycycline treatment are tested for anchorage independent growth in soft agar. Note no growth for WT or GLP1 cells, but tumor initiating cells isolated from skin SCC (squamous cell carcinoma) can readily produce colonies in soft agar plate.

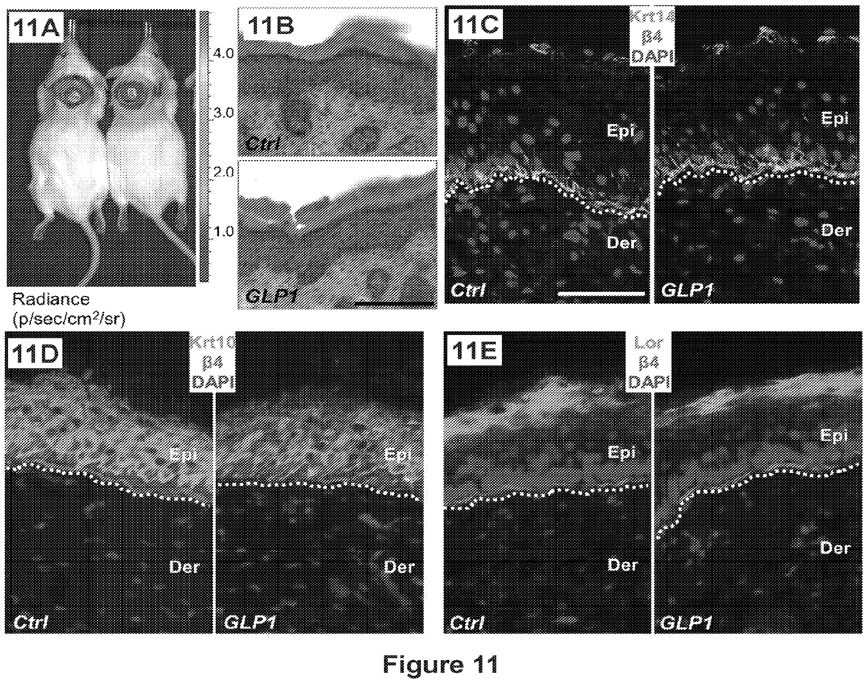

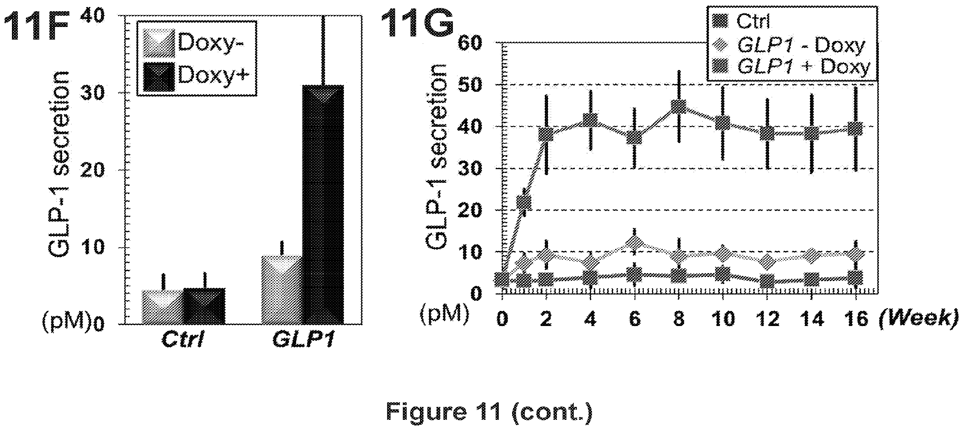

FIG. 11. Stable delivery of *GLP1 in **vivo*** through mouse-to-mouse skin transplantation. **FIG.** 11A: Images of immunocompetent mice (CD1 strain) grafted with isogenic skin organoids generated from *GLP1*-expressing cells. Cells are infected with lentivirus encoding *Luciferase* before grafting, and intravital imaging shows efficient incorporation of grafted cells. FIG. 11B: Histological examination of grafted GLP1 skin and adjacent host skin as control (Ctrl). Scale bar=50 µm. Epi: epidermis, Der: dermis, HF: hair follicle. **FIGS. 11C-11E****:** Sections of grafted skin and adjacent host skin (ctrl) were immunostained with different antibodies as indicated (Krt14: keratin 14, Krt10: keratin 10, Lor: Loricrin, β4: β4-integrin, CD104). Scale bar=50 µm. **FIG. 11F**: Skin organoids are developed from control or GLP1-producing cells, and transplanted to CD1 mice. The level of GLP1 in blood is determined by ELISA. Doxycycline-containing food can activate *GLP1* secretion *in vivo.* FIG. 11G: CD1 mice are grafted with control or *GLP1* skin organoids, and treated with or without doxycycline. Presence of GLP1 in blood is determined by ELISA for 16 weeks after engraftment.

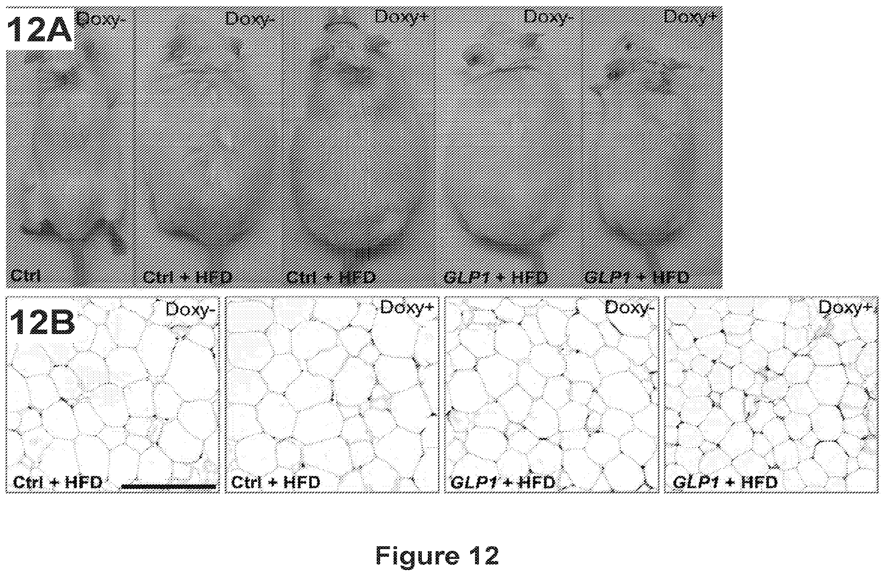

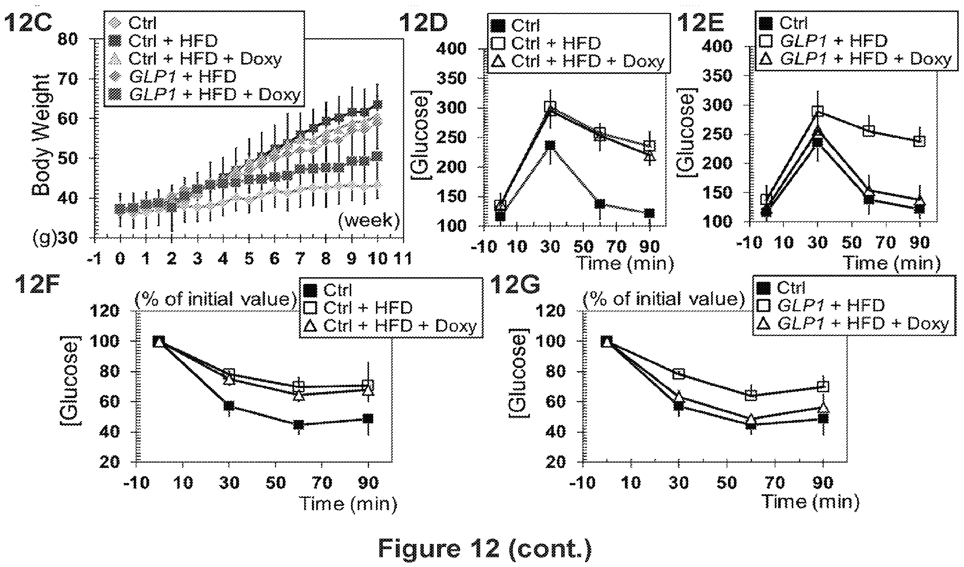

**FIG. 12****. Expression of *GLP1* in epidermal stem cells improves body weight and glucose homeostasis *in vivo.*** **FIG. 12A****:** Images of control and grafted animals fed a regular diet or a HFD (high fat diet). FIG. 12B: Representative images of white fat tissue histological examinations. Scale bar= 100 µm. **FIG. 12C****:** Body weight change of different cohorts of mice measured from -10 weeks of age. Note that the HFD induced significant obesity in control mice but that expression of *GLP1* by doxycycline stimulation inhibited weight gain. FIGS. 12D-12E: IPGTT (intraperitoneal glucose tolerance test) for control (D) and *GLP1* grafted (E) animals. Blood glucose concentration as a function of time following intraperitoneal injection of glucose showed improved glucose tolerance in *GLP1*-expressing mice fed a HFD. FIGS. 12F and 12G: ITT (insulin tolerance test). Profile of glucose concentration (percentage of initial value) as a function of time following intraperitoneal injection of insulin shows reduced insulin resistance in *GLP1*-expressing mice.

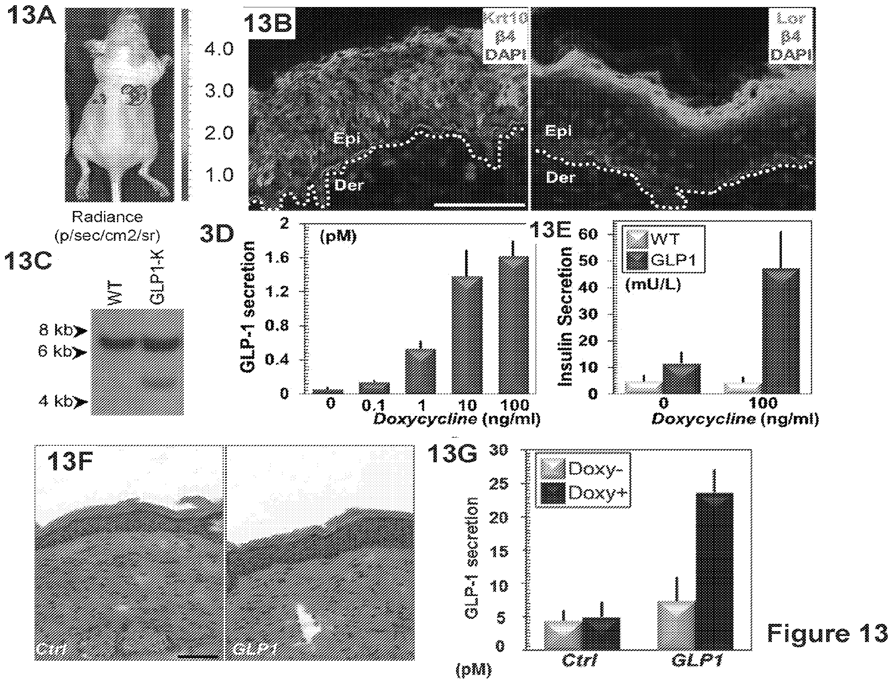

FIG. 13. Expression of *GLP1* in human epidermal stem cells with CRISPR. FIG. 13A: Image of nude mouse grafted with organotypic human skin culture. Intravital imaging shows efficient incorporation of grafted cells expressing *luciferase* upon engraftment. FIG. 13B: Sections of grafted skin and adjacent host skin were immunostained with different antibodies as indicated. Scale bar=50 µm. FIG. 13C: Integration of the targeting vector into *AAVS1* locus is verified by southern blotting. Positive clones display an additional band of the expected size. FIG. 13D: Secretion of *GLP1* into the culture medium was determined by the ELISA upon stimulation with different concentrations of doxycycline (Doxy). **FIG.** 13E: Conditioned medium was collected from control and GLP1-expressing cells, cultured in the presence and absence of Doxy, and used to treat starved insulinoma cells. Secretion of insulin *in vitro* was determined by ELISA. **FIG. 13F****:** H&E staining of skin organoids developed from control or GLP1-producing human cells, and transplanted to *nude* mice. FIG. 13G: Level of *GLP1* was determine by ELISA in blood from control and grafted *nude* mice fed either a standard or Doxycycline-containing food to activate GLP-1 secretion in vivo.

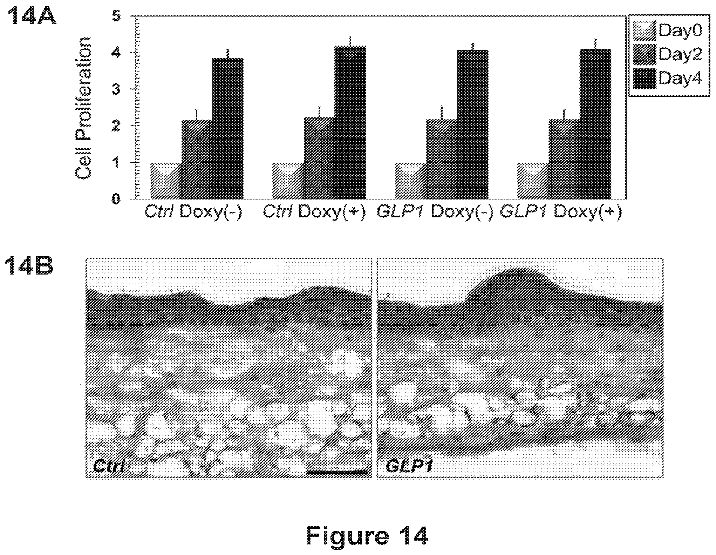

**FIG. 14****.** Engineering ***GLP1*-producing** skin epidermal progenitor cells with **CRISPR**. **FIG. 14A****:** Cell proliferation of control (Ctrl) and GLP1-expressing cells, Fold increase of cell numbers is quantified for all cell types. FIG. 14B: Control or *GLP1* targeted epidermal progenitor cells can produce similar skin organoids *in vitro.* Scale bar=50 µm.

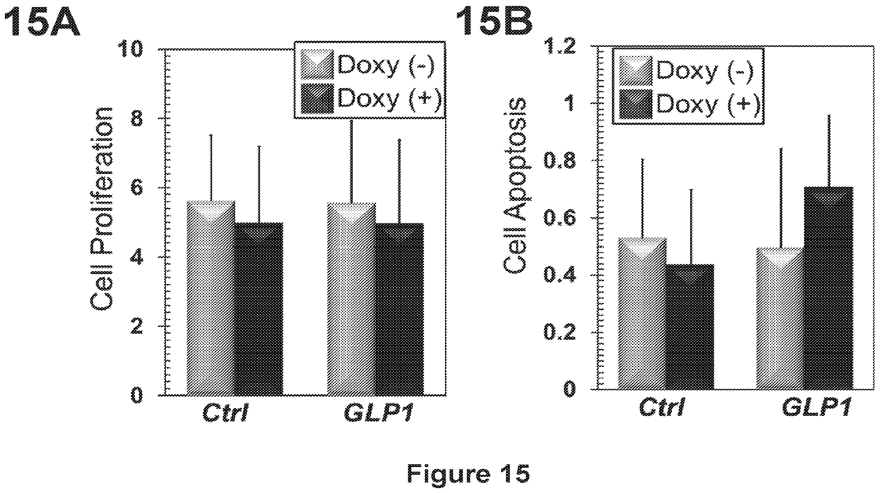

FIG. 15. Stable delivery of GLP1 *in vivo* through mouse-to-mouse skin transplantation. FIG. 15A: Proliferation of epidermal cells in control or *GLP1* skin grafts was determined and quantified by immunohistological staining with antibody against phosphor-histone H3. FIG. **15B****:** Apoptosis of epidermal cells in host or grafted skin one or four weeks post skin transplantation was determined and quantified by immunohistological staining with antibody against active caspase 3.

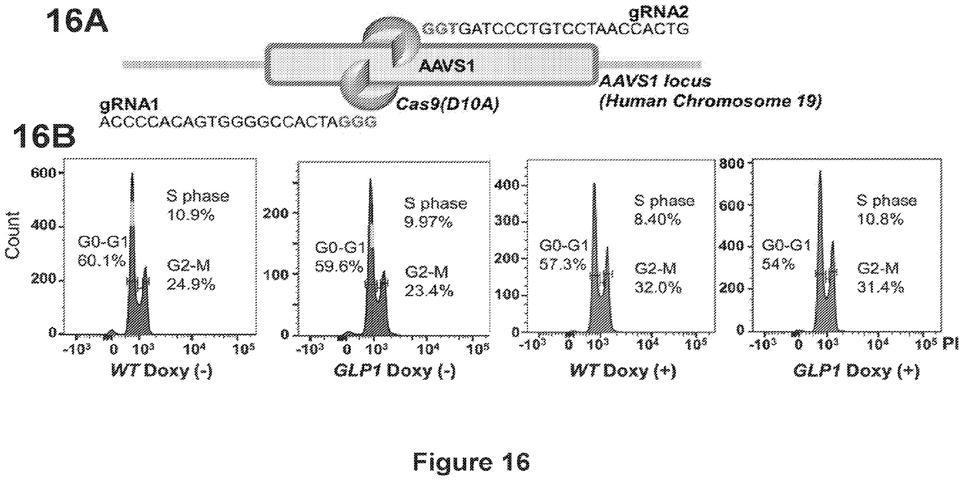

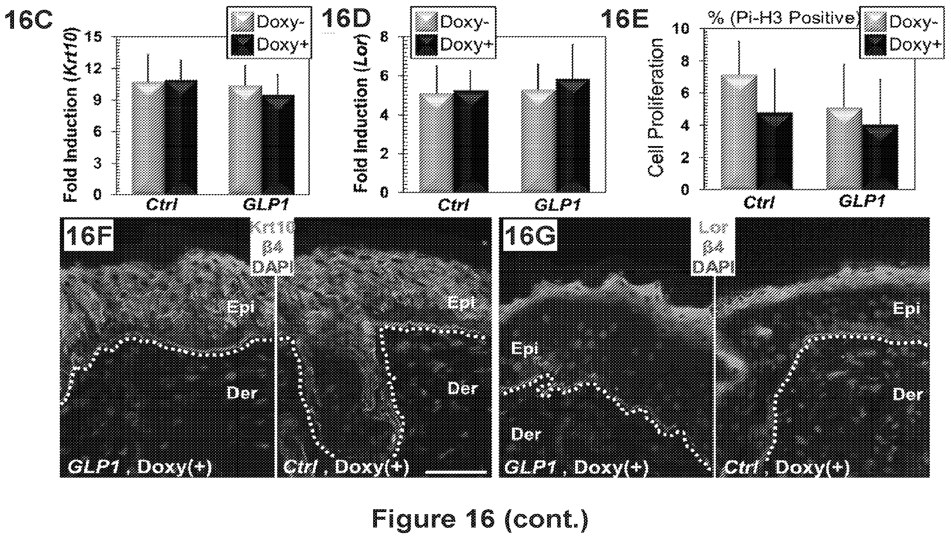

**FIG. 16****. Expression of *GLP1* in human epidermal progenitor cells with CRISPR**. **FIG. 16A****:** Diagram showing *AAVS1* targeting strategy for expression of *GLP1.* Expression vector encoding D10A mutant of cas9 and two gRNAs targeting *AAVS1* locus is used to create the cleavage in the chromosomal DNA and enhance integration of *GLP1* targeting vector. **FIG. 16B****:** FACS (fluorescence activated cell sorting) demonstrates similar cell cycle profiles of WT (wild type) and *GLP1-*expressing epidermal progenitor cells before and after doxycycline treatment. PI: propidium iodine. **FIGS. 16C and 16D****:** Western blotting analysis of early (C) and late (D) differentiation marker expression in WT and *GLP1*-expressing cells upon calcium shift. Band intensity was determined by densitometry and fold of induction is quantified. Krt10: keratin 10; Lor: loricrin. **FIG. 16E****:** Proliferation of epidermal cells in control or *GLP1* skin grafts was determined and quantified by immunohistological staining with antibody against phosphor-histone H3. **FIGS. 16F and 16G****:** Sections of grafted skin were immunostained with different antibodies as indicated. Scale bar = 50 µm.

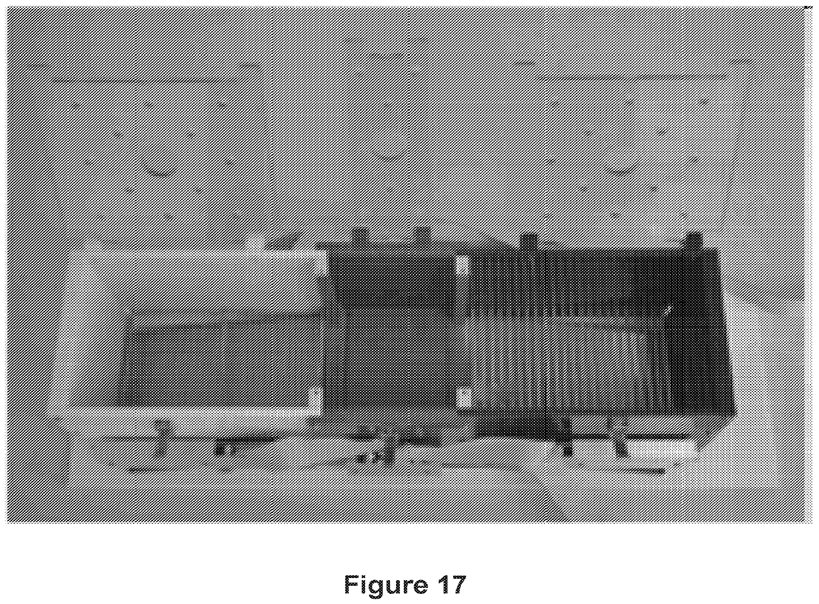

**FIG. 17****. CPP apparatus.** FIG. 17 shows a three compartment CPP apparatus that includes two large conditioning compartments with different colors and floor textures. The CPP apparatus (Med Associates, E. Fairfield, VT, USA) consisted of two larger chambers (16.8×12.7×12.7 cm), which were separated by a smaller chamber (7.2×12.7×12.7 cm) as previously described (Yan et al, 2013). Each chamber had a unique combination of visual and tactile properties (one large chamber had black walls and a rod floor, the other larger chamber had white walls with a mesh floor, whereas the middle chamber had gray walls and a solid gray floor). Each compartment had a light embedded in a clear, Plexiglas hinged lid. Time spent in each chamber was measure via photobeam breaks and recorded. CPP was determined on testing days via time spent in the drug-paired side minus time spent in the saline-paired side.

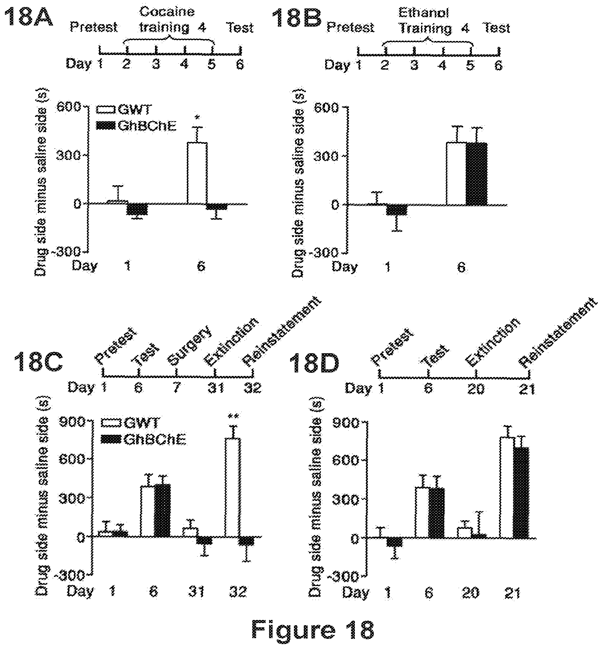

**FIG. 18****. Engraftment of *hBChE*-expressing cells can attenuate CPP acquisition and reinstatement induced by cocaine. 18A:** After engraftment, GhBChE (grafted hBChE expressing cells) and GWT (grafted wild type cells) mice underwent pretest (Day 1), cocaine conditioning (Day 2 to Day 5) and CPP expression test (Day 6). Data represent mean±SEM (n=9 in each group, treatment×days interaction: *F*_{1,16}=4.94, P < 0.05). **18B**: After engraftment, GhBChE and GWT mice underwent pretest (Day 1), ethanol conditioning (Day 2 to Day 5) and CPP testing (day 6). Data represent mean±SEM (n=8 in each group, treatment×days interaction: *F* _{1,17}=0.07, not significant). ** P<0.01 compared to pretest (Fisher's *t*-test). **18C**: Mice acquired similar levels of cocaine CPP after pretest, cocaine conditioning and test and underwent engrafting surgery on Day 7. Following 10 days of recovery, GhBChE and GWT mice underwent extinction till Day 31. During reinstatement on Day 32, GhBChE and GWT mice were given a cocaine injection and CPP was measured again. Data show mean±SEM (n=8 in each group, treatment×days interaction: *F*_{3, 42}=12.34, *P* < 0.001). **18D:** Mice acquired similar levels of ethanol CPP after pretest, ethanol conditioning and test from Day 1 to Day 6, and underwent extinction till Day 20. During reinstatement on Day 21, were given an ethanol injection and CPP was recorded (mean±SEM) (n=8 in each group, treatment×days interaction: *F*_{3, 42}=0.05, not significant). * P<0.05 compared to last extinction (Fisher's *t*-test).

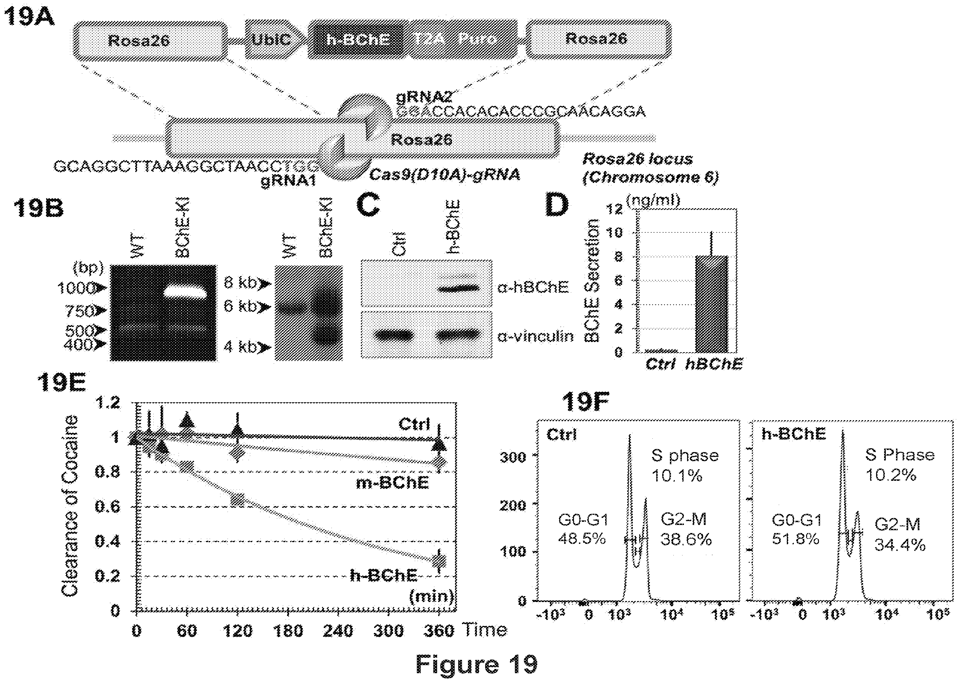

FIG. 19. Expression of engineered hBChE via genome editing in skin epidermal stem cells. FIG. 19A: Targeting strategy for the expression of engineered *hBChE.* The targeting vector contains two Rosa26 homology arms, flanking the expression cassette for *hBChE* and a selection marker (puromycin resistant gene, Puro) by a constitutive promoter UbiC (Ubiquitin C promoter). hBChE and Puro were separated by a self-cleavable peptide T2A. gRNA: guide RNA. FIG. 19B: Integration of the targeting vector into Rosa26 locus was verified by PCR (left panel) and southern blotting (right panel). Positive clones displayed an additional band of the expected size. **FIG. 19C****:** Confirmation of *hBChE* expression in targeted cells by immunoblots with different antibodies. **FIG. 19D****:** Confirmation of secretion of engineered hBChE in the culture media by ELISA. **FIG. 19E****:** Cocaine hydrolysis activity *in vitro.* Cell cultured supernatants were collected from cells targeted by *hBChE* or *mBChE.* Cocaine hydrolysis activity was examined by a clearance assay *in vitro.* **FIG. 19F****;** Cell cycle profiles. FACS (fluorescence activated cell sorting) of control (Ctrl) and *hBChE*-expressing epidermal stem cells. PI: propidium iodine.

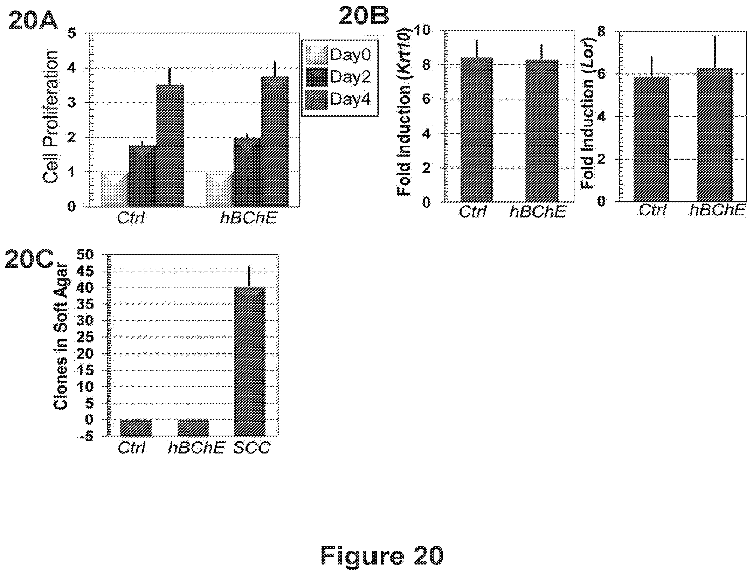

**Figure 20****. Engineering hBChE-producing skin epidermal progenitor cells with CRISPR. 20A:** Cell proliferation of control (Ctrl) and *hBChE*-expressing cells. Fold increase of cell numbers is quantified for all cell types. **20B:** Western blotting analysis of early (Krt10: keratin 10) and late (Lor: loricrin) differentiation marker expression in WT and *hBChE-*expressing cells upon calcium shift. Band intensity was determined by densitometry and fold of induction is quantified. **20C:** WT cells or *hBChE* cells were tested for anchorage independent growth in soft agar. Note no growth for WT or *hBChE* cells, but tumor initiating cells isolated from skin SCC (squamous cell carcinoma) can readily produce colonies in soft agar plate.

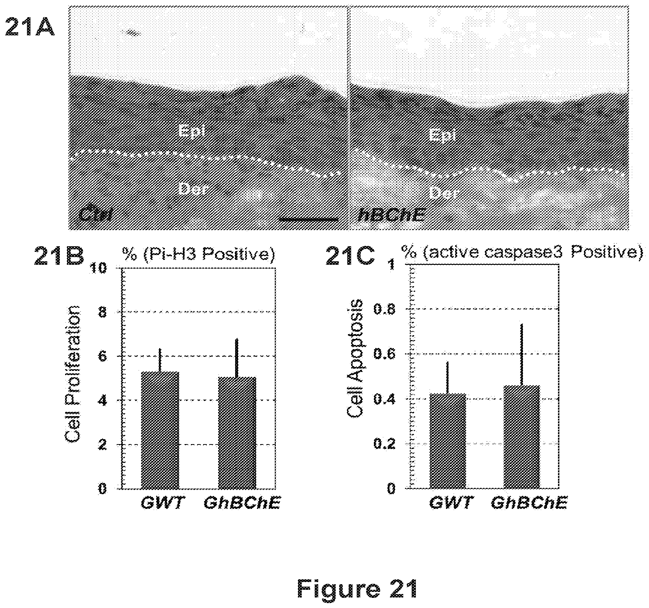

**Figure 21****. Stable delivery of engineered hBChE *in vivo* through mouse-to-mouse skin transplantation. 21A:** Control or *hBChE* targeted epidermal progenitor cells can produce similar skin organoids *in vitro.* Scale bar=50 µm. **21B**: Grafted skins were collected from mice grafted with control (GWT) or *hBChE* skin organoids (GhBChE). Cell proliferation was determined and quantified by immunohistological staining with antibody against phospho-histone 3. **21C**: Apoptosis of epidermal cells in control or hBChE skin grafts was determined and quantified by immunohistological staining with antibody against active caspase 3.

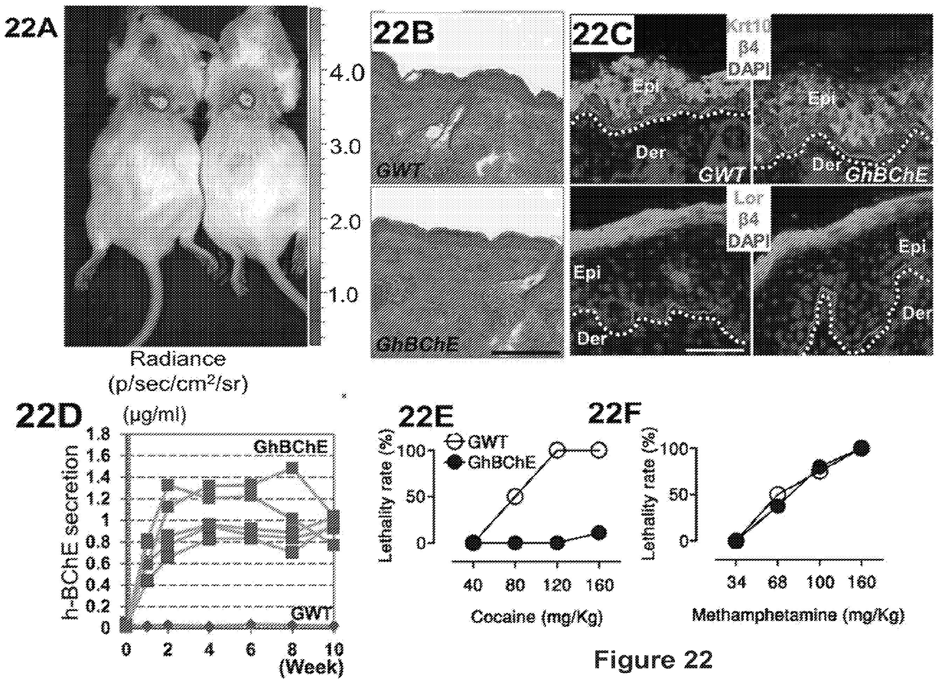

**Figure 22****. Engraftment of *hBChE*-expressing cells can protect against cocaine overdose. 22A:** Skin organoids are developed from control or *hBChE*-producing cells, and transplanted to the host mice. Cells were infected with lentivirus encoding *firefly luciferase* before engraftment to allow intravital imaging of the skin grafts. **22B:** Histological examination of grafted skin collected from mice grafted with control (GWT) or *hBChE* skin organoids (GhBChE). Scale bar=50 µm). **22C:** Sections of grafted skin were immunostained with different antibodies as indicated (Krt10: keratin 10, a marker for early epidermal differentiation, Lor: Loricrin, a marker for early epidermal differentiation, β4: β4-integrin, CD104, a marker for skin basement membrane). Dashed lines denote the basement of skin. Epi: epidermis, Der: dermis. Scale bar=50 µm. **22D**: Mice are grafted with control or *hBChE* skin organoids. Presence of hBChE in blood was determined by ELISA for 10 weeks after engraftment (n=5 mice in each group). 22E: Lethality rates after injection of 40, 80, 120, 160 mg/kg cocaine in GhBChE and GWT mice (n=8 in each group). 22F: Lethality rate after injections of 34, 68, 100, 160 mg/kg METH (methamphetamine) in GhBChE and GWT mice (n=8 in each group).

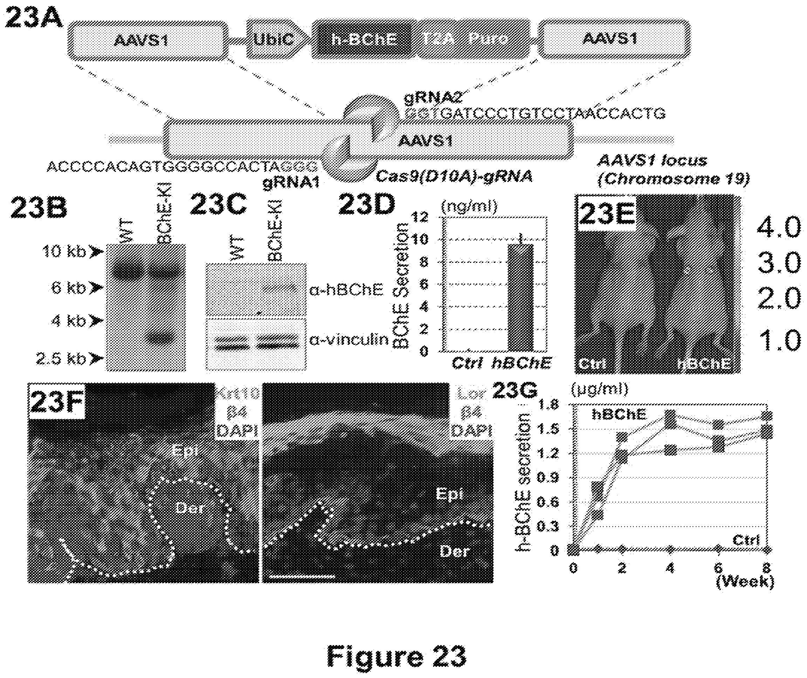

**FIG. 23****. Expression of *hBChE* in human epidermal stem cells with CRISPR**. **23A:** The *AAVS1* targeting strategy for expression of engineered *hBChE.* The targeting vector contains two *AAVS1* homology arms, flanking the expression cassette for *hBChE* and a selection marker (puromycin resistant gene, Puro) by a constitutive promoter UbiC (Ubiquitin C promoter). hBChE and Puro are separated by a self-cleavable peptide T2A. **23B** Integration of the targeting vector into *AAVS1* locus is verified by southern blotting. Positive clones display an additional band of the expected size. **23C:** Expression of *hBChE* is confirmed in targeted cells by immunoblots with different antibodies as indicated. **23D:** Secretion of engineered hBChE in the culture media is confirmed by ELISA. **23E:** Image of nude mouse grafted with organotypic human skin culture, Intravital imaging shows efficient incorporation of grafted cells expressing *luciferase* (right side) or control cells (left side) upon engraftment. **23F:** Sections of grafted skin were immunostained with different antibodies as indicated. Dashed lines denote the basement of skin. Scale bar=50 µm. **23G:** Mice are grafted with control or *hBChE* skin organoids. Presence of hBChE in blood was determined by ELISA for 8 weeks after engraftment (n=3 mice in each group).

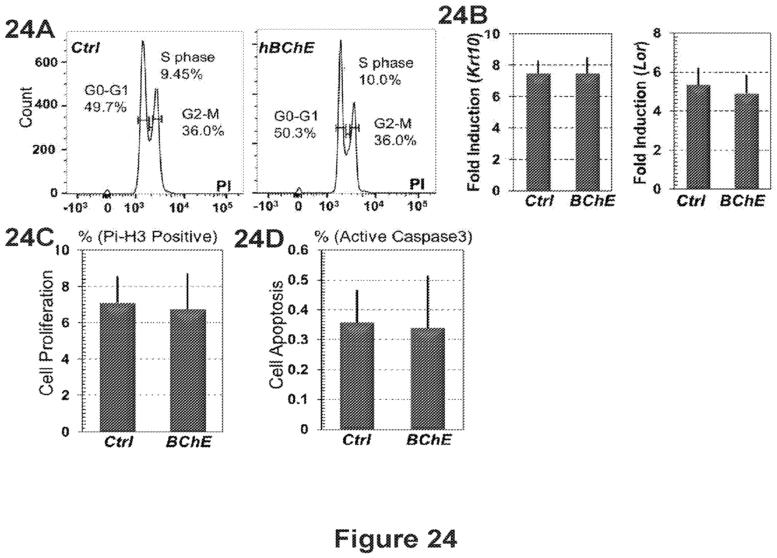

**FIG. 24****. Expression of *hBChE* in human epidermal progenitor cells with CRISPR**. **24A:** FACS (fluorescence activated cell sorting) demonstrates similar cell cycle profiles for control (Ctrl) and *hBChE*-expressing epidermal stem cells. PI: propidium iodine. **24B:** Western blotting analysis of early (Krt10: keratin10) and late (Lor: loricrin) differentiation marker expression in WT and *hBChE*-expressing human epidermal stem cells upon calcium shift. Band intensity was determined by densitometry and fold of induction is quantified. **24C:** Grafted skins were collected from mice grafted with control or *hBChE* skin organoids. Cell proliferation was determined and quantified by immunohistological staining with antibody against phospho-histone 3. **24D:** Apoptosis of epidermal cells in control or hBChE skin grafts was determined and quantified by immunohistological staining with antibody against active caspase 3.

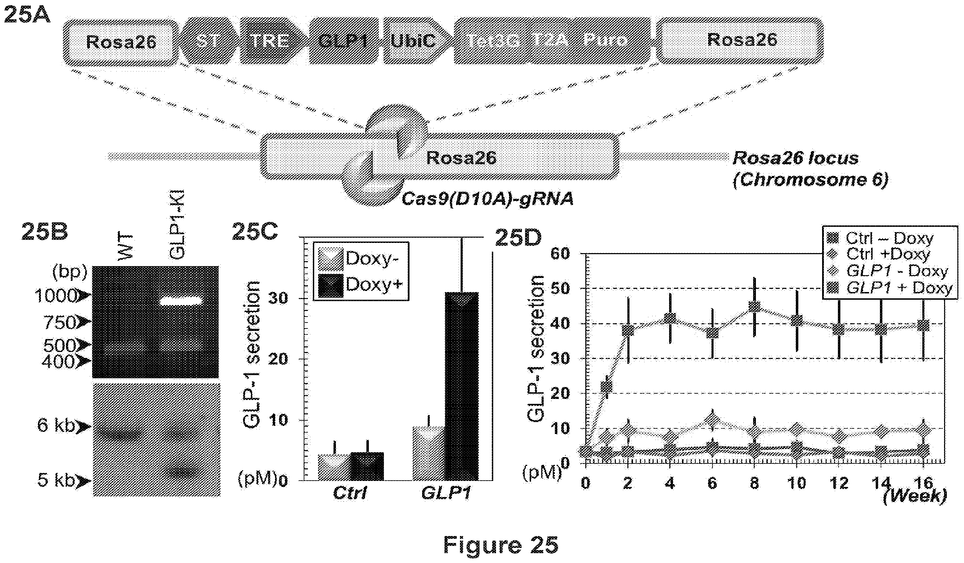

**FIG. 25****. Engineering *mGLP1*-producing skin epidermal stem cells with CRISPR.** CRISPR-mediated knockin of *DImGLP1* in mouse epidermal progenitor cells and dox-regulated mGLP1 expression. **FIG. 25A****:** Targeting vector contains two Rosa26 homology arms flanking a dox-responsive expression cassette encoding mGLP1. Expression of Tet3G (a transactivator) and Puromycin resistance (Puro) connected by a T2A peptide is controlled by an Ubiquitin C (Ubi) promoter. ST is a signal to increase TRE promoter specificity. FIG. **25B****:** PCR and southern verification of knockin of *DlmGLP1.* **FIG. 25C****:** Dox-induced mGLP1 expression in plasma of GLP1 mice. **FIG. 25D****:** Long-term mGLP1 expression in GLP1 mice.

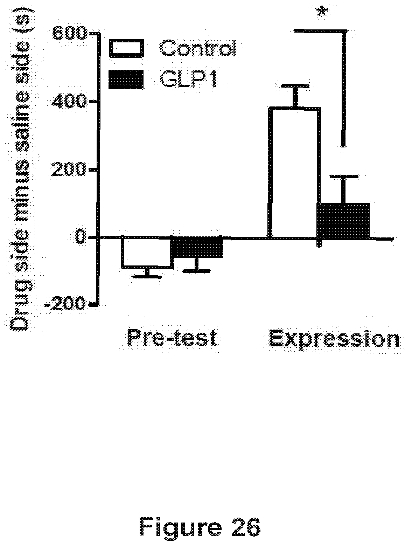

**FIG. 26****. mGLP1 expression on ethanol-induced CPP.** GLP1 mice did not exhibit significant ethanol-induced CPP. Following 2 free explorations (Pre-test) on day 1, separate groups of GLP1 and GWT mice (n = 9 each) received alternative ethanol (2 g/kg) and saline i.p. injections twice daily for the next 4 days, as previously described (Chen et al., Dopamine D1 and D3 receptors are differentially involved in cue-elicited cocaine seeking. J. Neurochem. 114, 530-541 (2010); Kong et al., Activation of dopamine D3 receptors inhibits reward-related learning induced by cocaine. Neurosci. 176, 152-161 (2011)). CPP expression was tested on day 6. Results represent mean ± SEM time spent on the drug-paired side minus the saline-paired side. Repeated-measures ANOVA with test days as the within group factor and status of grafting as the between-subject factor were used (Chen *et al.,* 2010: Kong *et a*/*.,* 2011). F value was calculated and Newman-Keuls post-hoc test was performed (Chen et *al.,* 2010; Kong *et al.,* 2011). GLP1 and WT mice were on dox food for the entire duration.

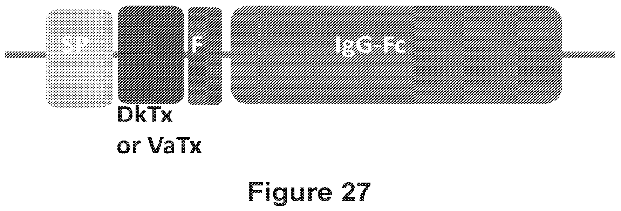

**FIG. 27****. Expression of Spider-derived pain peptides in human epidermal progenitor cells with CRISPR.** Diagram showing a contemplated targeting vector for treatment of alcoholism by cutaneous expression of spider derived pain peptides (toxins), DkTx (SEQ ID NO: 36) or VaTx (SEQ ID NO: 35). SP: signal peptide; F: furin cleavage site; IgG-Fc: mouse (SEQ ID NO: 34) or human IgG-Fc (SEQ ID NO: 39) fragment. Contemplated VatX3 and DkTx target vector cassettes are represented by SEQ ID NOS: 37 and 38, respectively.

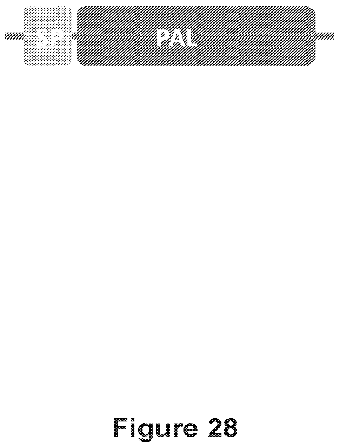

**FIG. 28****. Expression of *PAL* in human epidermal progenitor cells with CRISPR**. Diagram showing a contemplated targeting vector for treatment of PKU by cutaneous expression of *PAL.* SP: signal peptide; PAL: coding sequence for PAL.

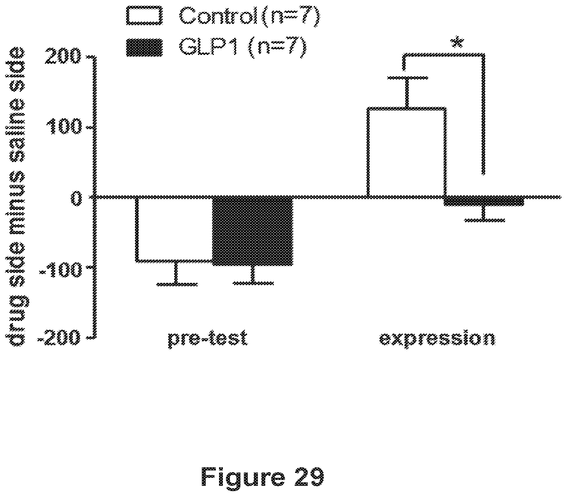

**FIG. 29** **mGLP1 expression on nicotine-induced CPP**. Figure legend: GLP1 mice did not exhibit significant nicotine-induced CPP. Following 2 free explorations (Pre-test) on day 1, separate groups of GLP1 and GWT mice (n = 7 each) received alternative nicotine (0.4 mg/kg) and saline i.p. injections twice daily for the next 4 days, as previously described (Chen *et al.,* 2010; Kong et *al.,* 2011). CPP expression was tested on day 6. Results represent mean ± SEM time spent on the drug-paired side minus the saline-paired side. Repeated-measures ANOVA with test days as the within group factor and status of grafting as the between-subject factor were used (Chen *et al.,* 2010; Kong *et al.,* 2011). F value was calculated and Newman-Keuls post-hoc test was performed (Chen *et al.,* 2010; Kong *et al.,* 2011). GLP1 and WT mice were on dox food for the entire duration.

### DETAILED DESCRIPTION

All publications, patents, and patent applications cited herein are hereby expressly incorporated by reference in their entirety for all purposes.

Before describing the present invention in detail, a number of terms will be defined. As used herein, the singular forms "a," "an," and "the" include plural referents unless the context clearly dictates otherwise. For example, reference to "a metabolite" means one or more metabolites.

It is noted that terms like "preferably," "commonly," and "typically" are not utilized herein to limit the scope of the claimed invention or to imply that certain features are critical, essential, or even important to the structure or function of the claimed invention. Rather, these terms are merely intended to highlight alternative or additional features that can or cannot be utilized in a particular embodiment of the present invention,

For the purposes of describing and defining the present invention it is noted that the term "substantially" as used herein represents the inherent degree of uncertainty that can be attributed to any quantitative comparison, value, measurement, or other representation. The term "substantially" is also used herein to represent the degree by which a quantitative representation can vary from a stated reference without resulting in a change in the basic function of the subject matter at issue.

Methods well known to those skilled in the art can be used to construct genetic expression constructs, targeting vectors, and genetically engineered cells according to this invention. These methods include *in vitro* recombinant DNA techniques, synthetic techniques, *in vivo* recombination techniques, polymerase chain reaction (PCR) techniques, and others. See, for example, techniques as described in Green & Sambrook, 2012, MOLECULAR CLONING: A LABORATORY MANUAL, Fourth Edition, Cold Spring Harbor Laboratory, New York; Ausubel et al., 1989, CURRENT PROTOCOLS IN MOLECULAR BIOLOGY, Greene Publishing Associates and Wiley Interscience, New York, and PCR Protocols: A Guide to Methods and Applications (Innis et al., 1990, Academic Press, San Diego, CA).

As used herein, the terms "polynucleotide," "nucleotide," "oligonucleotide," and "nucleic acid" can be used interchangeably to refer to nucleic acid comprising DNA, RNA, derivatives thereof, or combinations thereof.

As used herein, the term "genetically engineered" refers to the genetic manipulation of one or more cells, whereby the genome of the one or more cells has been augmented by at least one DNA sequence. Candidate DNA sequences include but are not limited to genes that are not naturally present, DNA sequences that are not normally transcribed into RNA or translated into a protein ("expressed"), and other genes or DNA sequences which one desires to introduce into the one or more cells. It will be appreciated that typically the genome of genetically engineered cells described herein is augmented through stable introduction of one or more recombinant genes. Generally, introduced DNA is not originally resident in the genetically engineered cell that is the recipient of the DNA, but it is within the scope of this disclosure to isolate a DNA segment from a given genetically engineered cell, and to subsequently introduce one or more additional copies of that DNA into the same genetically engineered cell, e.g., to enhance production of the product of a gene or alter the expression pattern of a gene. In some instances, the introduced DNA will modify or even replace an endogenous gene or DNA sequence by, e.g., homologous recombination, site-directed mutagenesis, and/or genome editing technology, including CRISPR (clustered regularly-interspaced short palindromic repeats), and/or mammalian transposon technology, such as by using the *piggyBac^{™}* transposon. In some instances, the introduced DNA is introduced into the recipient via viral vectors, including vectors derived from retrovirus, lentivirus, and adeno-associated virus. In some instances, the introduced DNA is introduced into the recipient skin directly with electroporation without skin stem cell isolation, culture, CRISPR editing, or grafting.

As used herein, the term "recombinant gene" refers to a gene or DNA sequence that is introduced into a genetically engineered cell, regardless of whether the same or a similar gene or DNA sequence may already be present in such a host. "Introduced," or "augmented" in this context, is known in the art to mean introduced or augmented by the hand of man. Thus, a recombinant gene can be a DNA sequence from another species, or can be a DNA sequence that originated from or is present in the same species, but has been incorporated into a cell by methods to form a genetically engineered cell. It will be appreciated that a recombinant gene that is introduced into a cell can be identical to a DNA sequence that is normally present in the cell being transformed, and is introduced to provide one or more additional copies of the DNA to thereby permit overexpression or modified expression of the gene product of that DNA. Recombinant genes can also be introduced with different driving promoters or associated sequences that can alter the gene's expression level or pattern. Said recombinant genes are particularly encoded by cDNA. Non-coding sequences, such as short hairpin RNAs, microRNAs, or long non-coding RNAs, may also be included.

It is further contemplated that recombinant genes can be codon optimized to maximize protein expression in genetically engineered cells by increasing the translation efficiency of a particular gene. Codon optimization can be achieved, for example, by transforming nucleotide sequences of one species into the genetic sequence of a different species. Optimal codons help to achieve faster translation rates and high accuracy. As a result of these factors, translational selection is expected to be stronger in highly expressed genes. However, while optimal codon usage is contemplated herein for expression of disclosed proteins, all possible codons are contemplated for use herein for nucleic acids encoding any disclosed protein.

As used herein, the term "about" refers to ±10% of any particular value.

As used herein, the terms "or" and "and/or" are utilized to describe multiple components in combination or exclusive of one another. For example, "x, y, and/or z" can refer to "x" alone, "y" alone, "z" alone, "x, y, and z," "(x and y) or z," "x or (y and z)," or "x or y or z."

As used herein, the term "blood factor" refers to any specific disease-associated indicator or factor that can circulate in the body, such as in the blood and/or lymphatic system. For example, a blood factor can be, without limitation, a cell, an enzyme, a protein, a polypeptide, an amino acid, a polynucleotide, a nucleic acid, a sugar, a lipid, a metabolite, a synthetic chemical compound, a naturally occurring chemical compound, a mineral, a metal, a bacterium, a virus, a prion, a disease indicator, and combinations or variations thereof.

As used herein, the term "reporter molecule" refers to any compound that can be produced biosynthetically by a genetically engineered host cell. For example, a reporter molecule can be an enzyme, a protein, a polypeptide, an amino acid, a polynucleotide, a nucleic acid, a sugar, a lipid, a metabolite, a synthetic chemical compound, a naturally occurring chemical compound, and combinations thereof. In one particular example, a reporter molecule can be a fluorescent protein, a fret-based biosensor, and/or a bioluminescent protein.

In one embodiment, the reporter molecule can be inducibly expressed, such as when a blood factor is perceived by the tissue organoid. Induction of expression can also be caused by administration of an inducer, as described herein elsewhere. When expressed by induction, the increased concentration of the reporter molecule functions to "report" the presence of the blood factor in question by producing a detectable signal. "Reporting" can be by any detectable means, such as, for example, fluorescing, producing a FRET signal, producing an electrical signal, and/or undergoing a conformational change.

Alternatively, a reporter molecule can be constitutively expressed but only "reports" when a signal produced by the reporter molecule is changed as a function of the perception of a blood factor by the reporter molecule. In this context, a change (increase or decrease) in signal can be proportionally associated with an increase in concentration of the blood factor.

In one embodiment, an external measurement device targeted at a tissue organoid expressing a reporter molecule can be used to noninvasively measure the relative amount of a blood factor in the patient. In one embodiment, it is contemplated that the reporter molecule can directly or indirectly associate with or contact a blood factor to produce a detectable signal that is proportional to the concentration of the blood factor in individual.

As used herein, the term "therapeutic agent" refers to a substance that when administered to an individual in need thereof, improves the individual's health. For example, a therapeutic agent can be, without limitation, an enzyme, a protein, a clotting factor, a vitamin, a peptide, a lipid, a toxin, a hormone, a polysaccharide, and combinations thereof. In one particular example, a therapeutic agent can be insulin or an analogue thereof that when administered to an individual in need thereof improves the individual's health by regulating the individual's blood sugar levels. In another particular example, a therapeutic agent can be a hormone, such as GLP1, that when administered to an individual in need thereof improves an individual's health by inducing the individual's satiety response, for example, to help regulate food intake. In a further particular example, a therapeutic agent can be an enzyme, such as *phenylalanine hydroxylase* (*PAH*), that when administered to an individual in need thereof improves an individual's health by reducing the concentration of phenylalanine in the patient's blood. Moreover, a therapeutic agent can be any compound that can be produced biosynthetically by a genetically engineered host cell, such as an epidermal cell. Therapeutic agents can include proteins and other substances derived from one species and administered to another species in native and/or modified forms.

As used herein, the term "individual" refers to any animal. Examples of individuals include humans, domesticated animals, household pets, and other animals without limitation. Further examples of individuals include animals having a disease.

As used herein, the term "physiologically tailored" refers to a state in which a tissue organoid has been created to be physiologically and/or immunologically compatible with an individual. For example, a physiologically tailored tissue organoid can be a tissue organoid grown from an individual's own cells or from cells that are physiologically compatible and that do not trigger an immune response from the individual when the individual's immune system is exposed to the tissue organoid, such as when the tissue organoid is surgically grafted into or onto the individual or otherwise biointegrated.

As used herein, the term "tissue organoid" refers to a collection of cells forming a tissue that has been genetically modified and that can be biointegrated *in vivo* via surgical transplantation or grafting (for example, on an individual's skin). For example, a tissue organoid can be a cultured, stratified skin graft grown from genetically engineered stem cells or keratinocytes taken from an individual. In addition to *in vitro* construction, it is envisioned that tissue organoids could be constructed *in situ* on an individual.

The cultured skin graft can be engineered to express one or more proteins or other molecules of interest under predetermined conditions, such as in response to the presence, absence, or change in levels of one or more blood factors. The protein or other molecule of interest can be a reporter molecule, a therapeutic agent, an inducer, and/or any other molecule or compound that can be produced biosynthetically by a genetically engineered host cell.

As used herein, the term "inducer" refers to a physical stimulus and/or chemical stimulant that induces expression of one or more genes within a tissue organoid and/or activation and/or release of a reporter molecule or therapeutic agent from a tissue organoid. Non-exciusive examples of inducers can include heat, cold, light, a protein, a peptide, a hormone, a lipid, a chemical, a metabolic change, a metabolite, an electric potential or field, and combinations thereof. Specific examples of inducers include doxycycline, a reporter molecule, and ethanol. It is further contemplated that inducers can induce expression and/or release of a reporter molecule or therapeutic agent in a dose-dependent manner.

In one embodiment, the present disclosure is directed to a physiologically tailored, biointegratable tissue organoid that can monitor levels of one or more blood factors in an individual. The tissue organoid can be transplanted or grafted onto the individual to provide a permanent or temporary (e.g., for one, two, three, six, twelve, or sixty months or longer) continuous monitor of one or more blood factors and/or source of therapeutic agent(s). For example, the tissue organoid can be a fully stratified skin graft cultured from the individual's own epidermal stem cells that is surgically grafted onto the individual's skin. Once biointegrated into the patient's skin, the tissue organoid forms a part of the patient's skin thereby preventing potential infections by eliminating the need for piercing the skin to monitor blood factor concentration. Further, because the tissue organoid is derived from the patient's own cells, the risk of host rejection of the tissue organoid is reduced.

Tissue organoids when biointegrated are nourished like any other tissue of the individual and concomitantly are exposed to circulating blood factors. In this context, tissue organoids can be genetically engineered to carry one or more stable genetic modifications (e.g., genome-integrated modifications) such as one or more genes encoding a reporter molecule that are expressed when a blood factor is perceived by or comes in contact with the tissue organoid.

In another embodiment, a reporter molecule can be constitutively expressed within the tissue organoid but only "reports" by producing a detectable signal (e.g., fluoresces, produces a FRET signal, produces an electrical signal, bioluminesces, produces a colorimetric change, and/or undergoes a conformational change) when associated with and/or contacting a predetermined blood factor. It is contemplated that the reporter molecule may directly or indirectly associate with or contact a blood factor to produce a detectable signal. External measurement devices targeted at the tissue organoid can be used to noninvasively measure the relative amount of perceived blood factor in the patient. In another embodiment, it is contemplated that reporter molecules produced by the tissue organoid are released systemically and can therefore be detected in another part of the body.

In one specific embodiment, a contemplated tissue organoid is a blood glucose monitor that expresses a reporter molecule in proportion to the relative blood glucose concentration of an individual. For example, the tissue organoid can express a fluorescent or bioluminescent reporter protein in relative proportion to glucose blood concentrations. The relative amount of the reporter protein can externally be measured, such as by a fluorometer or colorimeter and the concentration of the blood factor can therefore be determined. It is further contemplated that an inverse relationship between a reporter molecule and a particular blood factor is possible such that the relative amount of reporter molecule decreases in response to an increase in the blood factor concentration.

It is further contemplated that a tissue organoid could produce a reporter molecule that can be detected by measuring the reporter molecule in sweat, tears, mucus, plasma, urine, feces, or combinations thereof.

In another embodiment, the present disclosure is directed to a physiologically tailored, biointegratable tissue organoid that can express a therapeutic agent that passes the epidermal/dermal barrier to reach the circulation and have a therapeutic effect.

In a specific embodiment, the tissue organoid can be induced to express a therapeutic agent constitutively or by administration of an inducer to the individual. For example, a tissue organoid can express *GLP1* upon stimulation with an inducer, such as doxycycline in a dose-dependent manner. In this way, the amount of therapeutic agent expressed by a tissue organoid can be tailored to an individual's specific need at a particular time.

In one embodiment, a tissue organoid expresses both a reporter molecule and a therapeutic agent.

### Genetic constructs

Tissue organoids can include genetically engineered cells capable of expressing reporter molecules and/or therapeutic agents. Genes encoding reporter molecules and/or therapeutic agents can be stably introduced into the genomes of cells using any technology that permits genome editing, such as CRISPR**.** However, other approaches are contemplated herein. When using CRISPR for genetically engineering cells, any integration locus suitable for genome editing can be used. Examples of integration loci include *AAVSI* (adeno-associated virus integration site 1), *HPRT1* (hypoxanthine phosphoribosyltransferase-1), and/or human *Rosa26* locus. Use of the *HPRT1* locus offers the advantage that correctly integrated cells can be selected based on their resistance to 6-TG (2-amino-6-captopurine). Further, CRISPR targeting vectors can incorporate a drug resistance gene (puromycin; "puro") for cell selection, which may elicit an immune reaction *in vivo.* If this occurs, the targeting vector could be modified so that a puro coding sequence would be flanked with two LoxP sites. The puro sequence can be removed *in vitro* by transient expression of Cre recombinase after selection of targeted clones.

### Cell Selection and Tissue Growth

Suitable cells that can be used for tissue organoid construction include epidermal stem cells, such as those isolated from human skin. Other sources for epidermal stem cells include induced human pluripotent stem cells. Further examples of suitable cells include embryonic stem cells and human induced pluripotent stem cells.

Once isolated, the stem cells can be transfected with a targeting vector carrying one or more reporter molecule coding genes and/or one or more therapeutic agent coding genes and selected for correct integration of the targeting vector by Southern blot and other available methods. Correctly integrated genetically engineered epidermal stem cells can then be induced to differentiate to form stratified skin tissue when seeded on decellularized dermis and exposed to an air/liquid interface within a cell culture insert. Once grafts are ready, they can be transplanted to donor patients with well-established protocols.

### Transplantation

Tissue organoids can be implanted into skin of individuals via known surgical procedures, such as skin grafting. Other suitable procedures include direct application of engineered skin stem cells to patient skin. Once implanted, the tissue organoids can be allowed to heal and fully biointegrate into the individual's skin.

### Reporter Molecule Detection

Detection of reporter molecules of biointegrated tissue organoids can be performed by any means known in the art suitable for detecting the reporter molecule to be measured. For example, fluorometers and/or colorimeters can be used to measure changes in fluorescence, color, or luminescence associated with reporter molecule expression. Further, intravital bioluminescence imaging can be performed using a bioluminescence monitoring system, such as Xenogen. Additional examples of measurement devices that can be used to measure reporter molecules include electrical systems with proper electrodes.

### Tissue Organoid Standardization

Once a tissue organoid is biointegrated into an individual, for example, a blood glucose monitoring tissue organoid, the response of the organoid to blood glucose concentrations can be standardized such that a given reporter response is indicative of a specific blood glucose concentration. This can be accomplished by measuring different blood glucose concentrations using a standard blood glucose meter and associating the specific concentrations measured with the relative reporter molecule signals at each concentration. One example of such as standardization test is the intraperitoneal glucose tolerance test. Similar clinical standardization techniques can be performed for tissue organoids that are engineered to detect other specific blood factors. It is envisioned that once the tissue organoid "readout" is correlated to blood factor concentration, no further direct blood testing will be necessary.

### Therapeutic Platform Development

The present disclosure is also directed to tissue organoids for treating multiple medical issues simultaneously or as needed. For example, a physiologically tailored, biointegratable tissue organoid is envisioned that can express one or more therapeutic agents designed to address an individual's specific medical needs and thereby form a biointegratable therapeutic platform. For example, therapeutic platforms can be designed to express multiple therapeutic agents such as 2, 3, 4, 5, 6, 7, 8, 9, 10, or more therapeutic agents at one time or at different times depending upon an individual's needs. For example, each therapeutic agent to be expressed by the therapeutic platform can be individually and separately induced by an inducer such that for each therapeutic agent to be expressed, a separate inducer must be introduced to cause expression of the therapeutic agent. Alternatively, therapeutic agents that could advantageously be expressed at the same time, such as 2 or more therapeutic agents, can each be inducible by the same inducer.

Further, while some embodiments described herein are directed to expression of therapeutic agents for treatment of an existing illness, disease, and/or deficiency or absence of a required physiological substance (e.g., an enzyme, a protein, a clotting factor, a vitamin, a peptide, a lipid, etc.), it is further envisioned that a therapeutic agent can be expressed in anticipation of a physiological insult or stress. For example, tissue organoids made to express a therapeutic agent to counter the effects of a harmful chemical or substance could be induced to express the therapeutic agent in anticipation of exposure to the harmful chemical. In this way, the therapeutic agent has been expressed in the individual before the harmful chemical or substance is encountered by the individual. In another example, tissue organoids can be made to express a therapeutic agent in anticipation of blood loss, a low oxygen environment, and/or other physiological insult.

In another embodiment, the present disclosure is directed to physiologically tailored, biointegratable tissue organoids that can express therapeutic agents with multiple therapeutic effects. For example, a tissue organoid can be designed to express a single therapeutic agent, such as GLP1 that can be used to combat both alcohol abuse and nicotine abuse.

In one particular embodiment, it is envisioned that a tissue organoid designed to express GLP-1 (an anti-alcohol and anti-nicotine therapeutic agent) and BChE (an anti-cocaine therapeutic agent) can be biointegrated into an individual suffering from substance abuse or that is at risk for a relapse to eliminate or minimize the addictive effects of alcohol, nicotine, and cocaine at the same time.

In a further embodiment, GLP-1 analogs are contemplated for use herein. For example, contemplated analogs for use herein include Exendin-4 and Exendin-based therapies (*e,g*., Exenatide and Exenatide LAR), DPP-IV-resistant GLP-1 analogs (e.g., albiglutide), semaglutide (NN9535), liraglutide, taspoglutide, dulaglutide (GLP-1Fc, Trulicity^{®}) (LY2189265), and derivatives thereof (see Gupta, V. Glucagon-like peptide-1 analogues: An overview, Indian J Endocrinol Metab. 17(3): 413-421 (2013)).

Relatedly, it is envisioned that pharmaceutical compositions including one or more inducers can be administered to an individual to cause expression of one or more therapeutic agents that are inducible by the administered inducers. For example, a formulation in a pharmaceutically-acceptable form, such as an oral, parenteral, inhalable, and/or topical medication, can contain 2 or more inducers each specific for a separate therapeutic agent to be expressed by a tissue organoid. In this way, tissue organoids can be designed to express multiple therapeutic agents and tailored therapeutic agent expression can be obtained.

In one particular example, a tissue organoid can be biointegrated into an individual where the tissue organoid is designed to express 5 different therapeutic agents, TA1, TA2, TA3, TA4, and TA5, each upon induction by a separate inducer, I1, I2, I3, I4, and I5, respectively. When needed, the individual can be administered a pharmaceutical composition including inducers I2 I3 and I5, for example, which causes expression of therapeutic agents TA2, TA3, and TA5. Alternatively, the individual can be administered a pharmaceutical composition including inducers I1, I2 and I4, for example, which causes expression of therapeutic agents TA1, TA2, and TA4. Multiple variations of therapeutic agent induction are envisioned without limitation. Moreover, multiple variations of pharmaceutical dosage forms are contemplated such as immediate release, delayed release, and/or extended release forms. In this way, a particular pharmaceutical composition can include, for example, inducers I1, I2 I3, 14, and 15, where inducers I1 and I5 are formulated for immediate release, inducers I2 and I3 are formulated for delayed release, and inducer II4 is formulated for extended release. Additional dosage forms such as implantable depots are contemplated. Combinations of any inducers in any dosage form or formulation are contemplated herein without limitation,

It is further envisioned that a tissue organoid can be designed to express multiple therapeutic agents where expression of one or more of the therapeutic agents is inducible and expression of one or more of the therapeutic agents is constitutive.

It is further envisioned that a tissue organoid can be cultured that include multiple populations of transformed cells where each population is designed to express a different therapeutic agent than the other populations within the tissue organoid. Any number of separate populations of cells is envisioned.

The invention will be further described in the following examples, which do not limit the scope of the invention described in the claims.

### EXAMPLES

The Examples that follow are illustrative of specific embodiments of the invention, and various uses thereof. They are set forth for explanatory purposes only and are not taken as limiting the invention.

### Example No. 1: Development of an Intrinsic Skin Sensor for Blood Glucose Level with CRISPR-mediated Genome Editing in Epidermal Stem Cells

### INTRODUCTION

Current integrated biosensors exhibit limitations in stability, biocompatibility, and increased risk of infection. One possibility to overcome these limitations is to transform a small portion of endogenous tissue into a biointegrated, long-lasting sensor for physiological and biochemical signals via genome editing technology (see Hsu et al. Development and applications of CRISPR-Cas9 for genome engineering. Cell 157, 1262-1278 (2014); Maeder et al. Genome-editing Technologies for Gene and Cell Therapy. Mol Ther 24, 430-446 (2016); and Wright et al. Biology and Applications of CRISPR Systems: Harnessing Nature's Toolbox for Genome Engineering. Cell 164, 29-44 (2016)). In this regard, the human skin and skin epidermal stem cells (see Blanpain et al. Epidermal stem cells of the skin. Annu Rev Cell Dev Biol 22, 339-373 (2006) and Watt, F. M. Mammalian skin cell biology: at the interface between laboratory and clinic. Science 346, 937-940 (2014)) have several unique advantages, making them particularly suited for genetic engineering and applications *in vivo* (FIG. 1A).

The procedure to isolate and culture primary epidermal stem cells is well established. Cultured epidermal stem cells can be induced to stratify and differentiate *in vitro,* and transplantation of epidermal autografts is minimally invasive, safe, and stable *in vivo.* Autologous skin grafts have been clinically used for treatment of burn wounds for decades (*see* Carsin, H. et al. Cultured epithelial autografts in extensive burn coverage of severely traumatized patients: a five year single-center experience with 30 patients. Burns : journal of the international Society for Burn Injuries 26, 379-387 (2000) and Coleman et al. Cultured epidermal autografts: a life-saving and skin-saving technique in children. Journal of pediatric surgery 27, 1029-1032 (1992)). In this example, tissue organoids derived from genome-edited epidermal stem cells are shown to be useful for continuous monitoring of blood glucose level *in vivo.*

### EXPERIMENTAL PROCEDURES

### Reagents and Plasmid DNA Constructions

Guinea pig anti K5, rabbit anti K14, rabbit anti K10 and Loricrin antibodies were generous gifts from Dr. Elaine Fuchs at the Rockefeller University. Rat monoclonal p4-integrin (CD104, BD 553745) was obtained from BD Pharmingen^{®} (Franklin lakes, NJ). Ser10 phohistone antibody was obtained from EMD Miilipore^{®} (06-570, Billerica, MA). Cleaved caspase-3 antibody was obtained from Cell Signaling Technology^{®} (#9661, Danvers, MA). Insulin ELISA kit was obtained from EMD Millipore^{®} Corp. (EZRMI-13K, Billerica, MA). GLP-1 ELISA kit was obtained from Sigma^{®} (RAB0201-1kt, St. Louis, MO). Other chemicals or reagents were obtained from Sigma^{®}, unless otherwise indicated.

Lentiviral vector encoding *Luciferase* (SEQ ID NO: 1) and *H2B-RFP* (SEQ ID NO: 2) has been described before (Liu *et al*., 2015; Yue, 2016). Plasmid encoding *hCas9*-D10A mutant was a gift from George Church, obtained from Addgene (plasmid #41816). Plasmid encoding gRNA expression cassette was constructed with primers: AAG GAA AAA AGC GGC CGC TGT ACA AAA AAG CAG G (SEQ ID NO: 3); and gGA ATT CTA ATG CCA ACT TTG TAC (SEQ ID NO: 4), using gBlock as a template. *Rosa26*-targeting gRNA is constructed with primers: ACA CCG GCA GGC TTA AAG GCT AAC CG (SEQ ID NO: 5), AAA ACG GTT AGC CTT TAA GCC TGC CG (SEQ ID NO: 6), ACA CCG AGG ACA ACG CCC ACA CAC Cg (SEQ ID NO: 7), AAA ACG GTG TGT GGG CGT TGT CCT CG (SEQ ID NO: 8). *AAVS1*-targeting gRNA is constructed with primers: ACA CCG TCA CCA ATC CTG TCC CTA GG (SEQ ID NO: 9), AAA ACC TAG GGA CAG GAT TGG TGA CG (SEQ ID NO: 10), ACA CCG CCC CAC AGT GGG GCC ACT AG (SEQ ID NO: 11), AAA ACT AGT GGC CCC ACT GTG GGG CG (SEQ ID NO: 12). *Rosa26* targeting vector is constructed with pRosa26-GT as template (a gift from Liqun Luo, addgene plasmid 40025) using primers: GAC TAG TGA ATT CGG ATC CTT AAT TAA GGC CTC CGC GCC GGG TTT TGG CG (SEQ ID NO: 13), GAC TAG TCC CGG GGG ATC CAC CGG TCA GGA ACA GGT GGT GGC GGC CC (SEQ ID NO: 14), CGG GAT CCA CCG GTG AGG GCA GAG GAA GCC TTC TAA C (SEQ ID NO: 15), TCC CCC GGG TAC AAA ATC AGA AGG ACA GGG AAG (SEQ ID NO: 16), GGA ATT CAA TAA AAT ATC TTT ATT TTC ATT ACA TC (SEQ ID NO: 17), CCT TAA TTA AGG ATC CAC GCG TGT TTA AAC ACC GGT TTT ACG AGG GTA GGA AGT GGT AC (SEQ ID NO: 18). AAVS1 targeting vector (SEQ ID NO: 40) was constructed with AAVS1 hPGK-PuroR-pA donor (a gift from Rudolf Jaenisch, addgene plasmid 22072) as template using primers: CCC AAG CTT CTC GAG TTG GGG TTG CGC CTT TTC CAA G (SEQ ID NO: 19), CCC AAG CTT CCA TAG AGC CCA CCG CAT CCC C (SEQ ID NO: 20), CAG GGT CTA GAC GCC GGA TCC GGT ACC CTG TGC CTT CTA GTT GC (SEQ ID NO: 21), GGA TCC GGC GTC TAG ACC CTG GGG AGA GAG GTC GGT G (SEQ ID NO: 22), CCG CTC GAG AAT AAA ATA TCT TTA TTT TCA TTA CAT C (SEQ ID NO: 23), GCT CTA GAC CAA GTG ACG ATC ACA GCG ATC (SEQ ID NO: 24). Genotyping primers for CRISPR mediated knockin: GAG CTG GGA CCA CCT TAT ATT C (SEQ ID NO: 25), GGT GCA TGA CCC GCA AG (SEQ ID NO: 26), GAG AGA TGG CTC CAG GAA ATG (SEQ ID NO: 27).

### Culture of mouse and human primary keratinocytes

Primary mouse keratinocytes were isolated from the epidermis of newborn mice using trypsin, after prior separation of the epidermis from the dermis by an overnight dispase treatment. Keratinocytes were plated on mitomycin C-treated 3T3 fibroblast feeder cells until passage 3. Cells were cultured in E-media supplemented with 15% serum and a final concentration of 0.05 mM Ca²⁺.

Primary human neonatal epidermal keratinocytes were obtained from Thermo Fischer^{®} (C0015C), and cultured with Epilife^{®} medium (Thermo Fischer, M-EPICF-500) with manufacturer's recommended procedures. Calcium shift was performed to induce differentiation of primary keratinocytes by increasing the calcium concentration in culture media to 1.5 mM.

Cells are routinely screened for the presence of mycoplasma using the ATCC Universal Mycoplasma Detection Kit (Catalogue # 30-1012K). Cells are screened every 6 months and any mycoplasma contamination will result in the cells being discarded and replaced with previous, mycoplasma-free passages.

### Cell Cycle Analysis:

Propidium iodide (PI) staining followed by flow cytometry were used to determine the effect of cell cycle profiles. Mouse and human epidermal cells were cultured in two 6 cm cell culture dish for 24 hours, respectively. Cells were trypsinized, and 1×10⁵ cells from each dish were collected, followed by one PBS wash, Fixation of cells was carried out using 70% (v/v) ice cold ethanol for 1 hour. Then, the fixed cells were centrifuged at 500 g at 4°C for 10 minutes, followed by PBS wash for two times. The cells were then treated with 75 µg RNAse A in 100 µL PBS and incubated at 37°C for 1 hour. After incubation, the cells were collected by centrifuging at 500 g at 4°C for 10 minutes, followed by another PBS wash. The cell pellet was re-suspended in 200 µL PBS, in addition of PI solution at a final concentration of 25 ng/µL. After staining, the cells were analyzed immediately using flow cytometer BD FACSCanto^{™} II (BD Biosciences, San Jose, CA) with an excitation wavelength at 488 nm and emission at 585 nm. DNA content and histograms of cell cycle distribution were analyzed using FlowJo^{™} software, version 10 (FLOWJO LLC, OR).

### Protein biochemical analysis

Western blotting was performed as described previously (Blanpain *et a*/*.).* Briefly, equal amounts of the cell lysates were separated on a SDS-polyacrylamide gel electrophoresis (PAGE) and electroblotted onto a nitrocellulose membrane. The immunoblot was incubated with Odyssey^{®} blocking buffer (Li-Cor) at room temperature for 1 h, followed by an overnight incubation with primary antibody. Blots were washed three times with Tween 20/Tris-buffered saline (TBST) and incubated with a 1:10000 dilution of secondary antibody for 1 h at room temperature. Blots were washed three times with TBST again. Visualization and quantification was carried out with the LI-COR Odyssey^{®} scanner and software (LI-COR Biosciences).

### Skin organoid culture and transplantation

Decellularized dermis (circular shape with 1cm diameter) was prepared by EDTA treatment of newborn mouse skin (Maeder *et al*.). An aliquot containing 1.5 × 10⁶ cultured keratinocytes was seeded onto the dermis in cell culture insert. After overnight attachment, the skin culture was exposed to air/liquid interface.

For grafting with skin organoids, CD1 (isogenic mouse keratinocyte transplantation) males or Nude (human keratinocyte transplantation) females with the ages of 6-8 weeks were anesthetized. A silicone chamber bottom with the interior diameter of 0.8 cm and the exterior diameter of 1.5 cm was implanted on its shaved dorsal mid-line skin, which was used to hold the skin graft. A chamber cap was installed to seal the chamber right after a piece of graft was implanted. About one week later, the chamber cap was removed to expose the graft to air. A single dose of 0.2 mg α-CD4 (GK1.5) and 0.2 mg α-CD8 (2.43.1) antibodies was administered intraperitoneally for skin grafting.

### Obesity induced by high fat diet and glucose tolerance test

Male CD-1 mice with skin transplants were housed (5 per cage, ~8 weeks old) in a central-controlled animal facility for air, humidity and temperature. These mice were fed either a regular chow or an HFD (60% kcal from fats, 20% from carbohydrates, and 20% from proteins) purchased from Bio-Serv (Frenchtown, NJ). Body weight and food intake were measure biweekly.

For glucose tolerance testing, an intraperitoneal glucose tolerance test (IPGTT) was performed on mice fed an HFD for 10 weeks. Mice were fasted for 6 h before the test. Animals were injected (1 g/kg glucose/body weight, i.p.) with glucose dissolved in saline, and blood glucose was measured at 0, 10, 20, 30, 60 and 90 minutes using glucose test strips and glucose meters.

To induce hypoglycemia, CD1 mice with skin grafts were fasted for 4 h and injected (2 U/kg, i.p.) with insulin purchased from Sigma (St. Louis, MO). Blood glucose levels were determined thereafter at 0, 15, 30, 45, and 60 minutes.

### Intravital imaging of mice

Optical imaging was performed in the integrated small animal imaging research resource (iSAIRR) at the University of Chicago. Bioluminescence images were acquired on an IVIS Spectrum (Caliper Life Sciences^{®}, Alameda, CA) after animal was injected with luciferin (100 mg/kg). Acquisition and image analysis were performed with Living Image 4.3.1 software.

Wound healing in grafted skin were imaged by multiphoton microscope in the light microscopy center at the University of Chicago. Images were analyzed with Image J software.

### Histology and Immunofluorescence

Skin or wound samples were embedded in OCT, frozen, sectioned, and fixed in 4% formaldehyde. For paraffin sections, samples were incubated in 4% formaldehyde at 4°C overnight, dehydrated with a series of increasing concentrations of ethanol and xylene, and then embedded in paraffin. Paraffin sections were rehydrated in decreasing concentrations of ethanol and subjected to antigen unmasking in 10 mM Citrate, pH 6.0. Sections were subjected to hematoxylin and eosin staining or immunofluorescence staining, as described in Wright *et al.* Antibodies were diluted according to manufacturer's instruction, unless otherwise indicated.

### Statistical analysis

Statistical analysis was performed using Excel or OriginLab software. Box plots are used to describe the entire population without assumptions on the statistical distribution. A student t test was used to assess the statistical significance (P value) of differences between two experimental conditions (2 tailed distribution unless specified). All experiments were repeated at least three times, unless otherwise specified. For all figures, statistical tests are justified and meet the assumption of the tests. The variance between different test groups that are being statistically compared is similar.

For all the experiments, the sample size was chosen based upon our preliminary test and previous research. There is no sample exclusion for all the *in vitro* analysis. For *in vivo* experiments, animals that died before the end of the experiment were excluded. The exclusion criteria is pre-established. No randomization or blinding was used in this study.

### RESULTS AND DISCUSSION

Despite the potential clinical applications, research in skin epidermal stem cells has been greatly hampered by the lack of an appropriate model. Although it has been shown that mouse skin or human skin can be transplanted onto immunodeficient mice, the lack of an intact immune system in this model forecloses prediction of potential outcomes of this procedure *in vivo.* Immune clearance of engineered cells has been a major complication for somatic gene therapy (*see* Collins, M. & Thrasher, A. Gene therapy: progress and predictions. Proceedings. Biological sciences / The Royal Society 282 (2015)). Additionally, it remains technically challenging to perform skin organoid culture with mouse epidermal stem cells and generate mouse skin substitute for transplantation. To resolve these issues, a new organotypic culture model with mouse epidermal stem cells *in vitro* was developed by culturing the cells on top of acellularized mouse dermis (FIG. 6A) (*see* Liu, H. et a/. Regulation of Focal Adhesion Dynamics and Cell Motility by the EB2 and Hax1 Protein Complex. J Biol Chem 290, 30771-30782 (2015) and Yue, J. et al. In vivo epidermal migration requires focal adhesion targeting of ACF7. Nat Commun 7, 11692 (2016).

### Tissue Organoids

Exposure to the air/liquid interface can induce stratification of cultured epidermal cells to generate a skin-like organoid *in vitro.* Transplantation of such cultured skin organoids to *nude* hosts leads to efficient skin engraftments (see Liu, H *et al.* 2015 and Yue, J *et al.* 2016). Using a modified surgical procedure and skin graft maintenance protocol, engrafting the isogenic mouse skin substitute onto an immunocompetent host (CD1 and C57BL/6J strains) with or without the silicone dome chamber for skin transplantation (FIG. 1B and FIGS. 6B and 6C) was achieved.

Grafted cells in immunocompetent hosts readily expressed exogenous genes, such as *Luciferase* and *Histone H2B-RFP* (FIG. 1B-C), which were transduced to the cells with lentivirus. The grafted tissue exhibited normal skin stratification (FIG. 1D-F) when stained for basal epidermal stem cells (Keratin 14) or early (Keratin 10) and later (Loricrin) skin differentiation markers. In addition, skin grafts displayed similar cell proliferation and cell death when compared with adjacent host skin (FIG. 1G and FIG. 6D). Importantly, the tissue organoid with expression of exogenous gene, such as *Luciferase* and *H2B-RFP,* was stable *in vivo* for more than 5 months in immunocompetent hosts, as determined by intravital bioluminescence imaging and tissue histology. Together, these results demonstrate that tissue organoids are a new model for epidermal stem cell engineering and transplantation, and strongly suggest that genetically modified skin epidermal cells are not immunogenic and well tolerated *in vivo*,

### Engineered Blood Glucose Monitors

A biointegrated sensor for noninvasive monitoring of blood glucose level *in vivo* could remove the need for diabetic patients to draw blood multiple times a day. Additionally, continuous monitoring of glucose allows patients to better maintain blood glucose levels by altering insulin dosage or diet according to the prevailing glucose values. Currently, most continuous glucose monitoring sensors are enzyme electrodes or microdialysis probes implanted under skin. These sensors usually require oxygen for activity, and are insufficiently stable *in vivo* and poorly accurate under low glucose condition. Presence of interfering electroactive substances in tissues can also cause impaired responses and signal drift *in vivo,* which necessitate frequent calibrations of current sensors. A fluorescence-based glucose sensor in skin would likely be more stable, have improved sensitivity, and resolve the issue of electrochemical interference from the tissue (see Pickup et al. Fluorescence-based glucose sensors. Biosensors & bioelectronics 20, 2555-2565 (2005)).

To engineer epidermal stem cells for glucose sensing, intracellular expression of a sugar binding protein, glucose/galactose-binding protein (GGBP) (Jeffery, C. J. Engineering periplasmic ligand binding proteins as glucose nanosensors. Nano reviews 2, 2011) was examined. GGBP transports glucose within the periplasm of *E. coli*, and binding with glucose can lead to a large conformational change in the protein (Jeffery, 2011). This property of GGBP has been exploited to develop protein sensors for glucose based on FRET (fluorescence resonance energy transfer) or bioluminescence imaging (see Fehr et al. In vivo imaging of the dynamics of glucose uptake in the cytosol of COS-7 cells by fluorescent nanosensors. J Biol Chem 278, 19127-19133, (2003); Saxl et al. A fluorescence lifetime-based fibre-optic glucose sensor using glucose/galactose-binding protein. The Analyst 136, 968-972 (2011): Teasley Hamorsky et al. A bioluminescent molecular switch for glucose. Angewandte Chemie 47, 3718-3721 (2008); Tian et al. Structure-based design of robust glucose biosensors using a Thermotoga maritima periplasmic glucose-binding protein. Protein science: a publication of the Protein Society 16, 2240-225 (2007) and Veetil et al. A glucose sensor protein for continuous glucose monitoring. Biosensors & bioelectronics 26, 1650-1655 (2010). WT (wild type) GGBP has very high glucose binding affinity (*K_{d}* = 0.2 µm). To generate a probe corresponding to physiologically relevant range of glucose, a CFP/YFP FRET sensor with A213R/L238S double mutant of GGBP (SEQ ID NO: 28) (*K_{d}* = 10 mM) (Amiss, T. J., Sherman, D. B., Nycz, C. M., Andaluz, S. A. & Pitner, J. B. Engineering and rapid selection of a low-affinity glucose/galactose-binding protein for a glucose biosensor. Protein science : a publication of the Protein Society 16, 2350-2359) was engineered via CRISPR-mediated genome editing in mouse epidermal stem cells. DNA vectors encoding the D10A mutant of Cas9 (CRISPR associated protein 9) (Ran, F. A. et al. Double nicking by RNA-guided CRISPR Cas9 for enhanced genome editing specificity. Cell 154, 1380-1389 (2013) (SEQ ID NO: 29), two gRNAs (guide RNA) (SEQ ID NO: 30), (SEQ ID NO: 31) targeting the mouse *Rosa26* locus, and a *Rosa26*-targeting vector (SEQ ID NO: 32) were developed. The targeting vector contained two homology arms for the *Rosa26* locus, flanking an expression cassette that encoded a *GGBP* fusion protein (FIG. 2A and FIG.7A) (SEQ ID NO: 33).

Primary epidermal keratinocytes were isolated from CD1 newborn mice, and electroporated with the *Rosa26* targeting vector together with plasmids encoding Cas9 and *Rosa26*-specific gRNAs. Clones were isolated upon selection, and the correct integration to the *Rosa26* locus was confirmed by both PCR screening and southern blotting analysis (FIG. 2B). Engineered epidermal cells exhibited robust expression of GGBP fusion proteins in the cytosol (FIG. 2C).

To test whether intradermal electroporation would also be an effective means of introducing a reporter, CD1 mice were electroporated intradermally with plasmid DNA encoding luciferase and tdTomato and expression was assayed by bioluminescence imaging (FIG. 3A) and intravital imaging with a two-photon microscope (FIG. 3B), respectively. Electroporation resulted in expression of both luciferase and tdTomato.

To test glucose sensing *in vitro,* the cells were exposed to medium containing increasing amounts of glucose. Quantification of FRET ratio by microscopic imaging showed an excellent correlation of FRET ratio with extracellular glucose concentration, ranging from 0-10 mM (FIG. 2D). Further analysis revealed that the GGBP reporter responded to extracellular glucose or galactose, but not to other sugars, such as sucrose, fructose, or ribose (FIG. 2E). The intracellular GGBP probe responded to the change of glucose concentration rapidly. The FRET ratio changed within 30 seconds after replacement of the medium, and remained stable in the same medium. Together, these results indicate that epidermal stem cells expressing a GGBP reporter can faithfully sense the extracellular glucose concentration.

Expression of the GGBP fusion protein in epidermal cells did not significantly change cell proliferation (FIG. 2F and FIG. 7B) or differentiation (FIG. 2G) *in vitro.* To confirm that modified epidermal cells are not tumorigenic, anchorage-independent growth of cells was assayed and confirmed that epidermal stem cells with GGBP targeting could not grow in suspension (FIG. 2H). As a positive control, cancer initiating cells isolated from mouse SCC (squamous cell carcinoma) exhibited robust colony formation in soft agar medium (FIG. 2H). Expression of GGBP reporter did not affect the ability of epidermal stem cells to stratify. When subjected to skin organoid culture, the targeted cells readily produced stratified epithelial tissue (FIG. 7C).

To investigate the potential applicability *in vivo* of the engineered blood glucose monitor, a skin organoid culture was prepared with engineered epidermal stem cells, and the organoid was grafted onto CD1 host animals (FIG. 4A). No significant rejection of the skin grafts was observed after transplantation. The grafted organoids exhibited normal epidermal stratification, proliferation and cell death (FIGS. 8A-8E). To test the glucose sensing capability *in vivo,* an intraperitoneal glucose tolerance test (IPGTT) was performed in grafted animals. Fasted animals received a bolus of glucose intraperitoneally. Fluorescence (FRET) change in the grafted skin was monitored by intravital imaging (FIG. 4B), and blood glucose level was measured by a commercial glucose monitoring system (Bayer Contour) with blood samples taken from the snipped tail. FIGS. 4C and 4D show the correlation between the measured glucose concentration and the FRET ratio changes over time. The FRET ratio exhibited a nearly linear correlation with the glucose concentration *in vivo* (FIG. 4D, R²=0.977). In contrast, traditional glucose sensors cannot accurately measure low glucose level *in vivo.*

To test the skin sensor for lower glucose concentration, we induced hypoglycemia by insulin administration to fasted animals. Intravital imaging of the grafted skin again showed excellent correlation of the FRET ratio changes with glucose level (FIG. 4E and 4F). Together, these results illustrate that engineered skin organoid with GGBP reporter can accurately sense the blood glucose level *in vivo.*

### Engineered Tissue Organoids

If an engineered blood glucose monitor could be engineered to produce or regulate insulin levels in response to blood glucose levels, it could approximate an "artificial endocrine pancreas" that would automatically maintain glucose level in patients. Thus, introduction of an expression cassette into epidermal stem cells that encodes both a GGBP reporter and a therapeutic protein could achieve continuous glucose monitoring and diabetes treatment with a single skin transplantation. GLP1 (glucagon-like peptide 1) is released from the gut upon food intake and acts both as a satiety signal to reduce food consumption and as an incretin hormone to stimulate insulin release and inhibit glucagon secretion. Indeed, GLP1 receptor agonists have previously been used to treat type 2 diabetes. Therefore, a new *Rosa26* targeting vector containing an expression cassette that encodes a *GLP1* and mouse *IgG-Fc* fragment (for enhanced stability and secretion of GLP1) fusion protein together with the GGBP reporter was developed (FIG. 8F).

Engineered epidermal cells exhibited robust GLP1 production and secretion (FIG. 4G). The secreted GLP1 fusion protein was functional as the conditioned medium significantly induced secretion of insulin when added to cultured insulinoma cells (FIG. 4H).

To examine the potential applicability in diet-induced obesity and diabetes, *GLP1*/*GGBP*-expressing cells and control cells (GGBP alone) were transplanted into two cohorts of CD1 adult male mice. The mice were fed a high fat diet (HFD) to induce obesity in grafted animals. Compared with animals on a regular chow diet, the HFD greatly accelerated body weight gain in mice grafted with control cells. By contrast, *GLP1* expression led to significant inhibition in body weight increase (FIG. 41; quantified in FIG. 4J).

IPGTT was performed to examine glucose homeostasis. Expression of *GLP1* significantly reduced glycemic excursion *in vivo* as determined by both direct measurement of blood glucose or intravital imaging of the GGBP reporter (FIGS. 4K-4M). Noninvasive monitoring with the GGBP reporter exhibited excellent correlation with conventional glucose measurement in both diabetic animals and GLP1-treated animals (FIGS. 4L and 4M).

### Human Tissue Organoids

To test the feasibility of glucose sensing with human epidermal stem cells, human skin organoids were cultured from primary epidermal keratinocytes isolated from human newborn foreskin. The human epidermal keratinocytes readily produce organoids *in vitro,* which can be transplanted to *nude* mice. When infected with lentivirus, the grafted human cells exhibited robust expression of the exogenous *Luciferase* gene (FIG. 5A). The grafted tissue shows normal skin stratification when stained for early or late epidermal differentiation markers (FIG. 5B).

For CRISPR-mediated genome editing, vectors encoding two gRNAs targeting the human *AAVS1* (adeno-associated virus integration site 1) locus and an *AAVS1*-targeting vector (FIG. 9A) that encodes the GGBP reporter protein were developed. Human epidermal keratinocytes were electroporated with the targeting vector together with plasmids encoding *Cas9* and the gRNAs. Clones were isolated and correct integration was confirmed by southern blotting analysis (FIG. 5C).

Expression of the GGBP fusion protein did not significantly change cell proliferation (FIG. 9B) or differentiation (FIG. 9C) *in vitro.* The engineered cells stratified and formed skin organoids *in vitro,* which were successfully transplanted onto *nude* hosts. Grafted tissue organoids exhibited normal epidermal stratification and proliferation *in vivo* (FIGS. 9D-9G). IPGTT and intravital imaging of the GGBP reporter were performed and a similar correlation of FRET ratio changes in the grafted skin with blood glucose level was observed (FIG. 5D - 5F). These data indicate that these human tissue organoids could be used for monitoring of blood glucose levels in humans.

In this study, technical hurdles were overcome to establish a unique mouse-to-mouse skin transplantation model with immunocompetent hosts. The results provide key evidence supporting the feasibility of cutaneous monitoring of blood glucose level *in vivo.* The same platform can be also exploited for development of other biosensors and ex *vivo* cutaneous gene therapy for stable delivery of therapeutic proteins *in vivo,* such as GLP1, providing a promising treatment for many otherwise terminal or severely disabling diseases (see Christensen, R. et al. Skin genetically engineered as a bioreactor or a 'metabolic sink'. Cells, tissues, organs 172, 96-104, (2002) and Del Rio, M. et al. Current approaches and perspectives in human keratinocyte-based gene therapies. Gene therapy 11 Suppl 1, S57-63, (2004)).

### Example No. 2: Treatment of Diabetes and Obesity with CRISPR-mediated

### Genome Editing in Epidermal Stem Cells

### INTRODUCTION

In this report, by combining CRISPR-mediated genome editing with epidermal stem cell platform, a skin graft with controllable release of GLP1 (glucagon-like peptide-1) was developed and demonstrated therapeutic effect *in vivo* by reducing glycemic excursions in diet-induced obese and diabetic mice. GLP1 is a major physiological incretin that controls homeostasis of blood glucose by stimulation of glucose-dependent insulin secretion, inhibition of glucagon secretion, delay of gastric emptying, and protection of islet beta-cell mass (see Ross *et al.* 2010, Sandoval *et al.* 2015). However, native GLP1 must be delivered through a parenteral route to achieve its effect as it has an extremely short circulating half-life. Thus, somatic gene transfer may provide a more effective way for long term and stable delivery of GLP1 *in vivo* in order to treat diabetes (Prud'homme *et al.* 2007; Rowzee *et al.* 2011).

The recent development of genome editing technology, including CRISPR (clustered regularly-interspaced short palindromic repeats) system, has made it possible to perform precise genetic engineering, providing an ideal tool for somatic gene therapy (Cox et *al.*, 2015; Hotta *et al.* 2015; Wright *et al.* 2016). However, clinical application of CRISPR technology has been challenging due to inadequate efficacy *in vivo* using conventional delivery approach. Thus, the development of an ex *vivo* platform that can combine both precise genome editing *in vitro* with effective application of engineered cells *in vivo* will provide significant benefits for the treatment of many human diseases.

Skin epidermal stem cells (Blanpain and Fuchs, 2006; Watt, 2014) have several unique advantages, making them particularly suited for somatic gene therapy ex *vivo: **i)*** Human skin is the largest and most accessible organ in the body, offering availability for collection of epidermal stem cells with well-established procedures (Rasmussen *et al*., 2013; Rheinwald and Green, 1975, 1977). Moreover, it is easy to monitor the skin for potential off-target effects of gene targeting and, if necessary, to remove it in case of an adverse consequence. ***ii)*** Cultured epidermal stem cells can be readily induced to differentiate and the resultant stratified skin tissue can be transplanted to donor patients with well-established protocols (Blanpain and Fuchs, 2006; Watt, 2014). Comparing with other somatic gene therapy approach, autologous skin grafts are relatively inexpensive, and the procedure is minimally invasive, safe, and has been clinically used for treating burn wounds for decades (Carsin *et a*/*.* 2000: Coleman and Siwy, 1992). ***iii)*** Somatic gene therapy with epidermal stem cells is tissue specific. Anatomically, skin epidermis is not directly vascularized but receives nutrients from blood vessels located in the underlying dermal tissue. The physical separation by the basement membrane precludes potential dissemination of genetically modified cells *in vivo,* making it extremely tissue specific and safe for the cutaneous gene therapy. ***iv)*** Epidermal stem cells can withstand long-term culture *in vitro* without losing stemness Rheinwald and Green, 1975), making it possible to perform precise genome editing with non-viral approaches. Potential genotoxicity, particularly from viral vectors, has been a significant hurdle for somatic gene therapy (Kotterman *et a*/*.* 2015; Kustikova *et al.* 2010). ***v)*** Epidermal stem cells have low immunogenicity. Gene therapy-derived products can be recognized as foreign antigens by the host immune system, which may mount an immune response leading to clearance of genetically modified cells. However, skin autograft or allograft developed from cultured epidermal stem cells can achieve long term and stable transplantation in human patients without eliciting significant immune reaction (Centanni *et al*., 2011; Zaulyanov and Kirsner, 2007). ***vi)*** It has been well documented that proteins secreted by skin epidermal cells, such as ApoE (apolipoprotein E) and large blood clotting proteins Factor VIII and Factor IX, can cross the epidermal/dermal barrier and reach circulation to achieve therapeutic effect in a systematic manner (Christensen *et al*., 2002; Del Rio *et a*/*.,* 2004; Fakharzadeh *et al*., 2000; Fenjves *et al*., 1989; Gerrard *et al*., 1993; Morgan *et al*., 1987). Thus, the potential applicability of skin stem cell therapy is broad, and beyond the skin diseases.

This example demonstrates that genome-edited epidermal stem cells can be exploited for robust, controllable delivery of GLP1 *in vivo* and effective treatment of diabetes and obesity in a clinically-relevant setting.

### EXPERIMENTAL PROCEDURES

### Reagents and Plasmid DNA Constructions

Guinea pig anti K5, rabbit anti K14, rabbit anti K10 and Loricrin antibodies were generous gifts from Dr. Elaine Fuchs at the Rockefeller University. Rat monoclonal p4-integrin (CD104, BD 553745) was obtained from BD Pharmingen (Franklin lakes, NJ). Ser10 pho-histone antibody was obtained from EMD Millipore (Billerica, MA). Cleaved caspase-3 antibody was obtained from Cell Signaling Technology (Danvers, MA). Insulin ELISA kit was obtained from EMD Millipore Corp (Billerica, MA). GLP-1 ELISA kit was obtained from Sigma (St. Louis, MO). Other chemicals or reagents were obtained from Sigma, unless otherwise indicated.

Lentiviral vector encoding *Luciferase* (SEQ ID NO: 1) and *H2B-RFP* (SEQ ID NO: 2) has been described before (Liu *et al*., 2015; Yue, 2016). Plasmid encoding *hCas9*-D10A mutant was a gift from George Church, obtained from Addgene (plasmid #41816). Plasmid encoding gRNA expression cassette was constructed with primers: AAG GAA AAA AGC GGC CGC TGT ACA AAA AAG CAG G (SEQ ID NO: 3); and gGA ATT CTA ATG CCA ACT TTG TAC (SEQ ID NO: 4), using gBlock as a template. *Rosa26*-targeting gRNA is constructed with primers: ACA CCG GCA GGC TTA AAG GCT AAC CG (SEQ ID NO: 5), AAA ACG GTT AGC CTT TAA GCC TGC CG (SEQ ID NO: 6), ACA CCG AGG ACA ACG CCC ACA CAC Cg (SEQ ID NO: 7), AAA ACG GTG TGT GGG CGT TGT CCT CG (SEQ ID NO: 8). *AAVS1*-targeting gRNA is constructed with primers: ACA CCG TCA CCA ATC CTG TCC CTA GG (SEQ ID NO: 9), AAA ACC TAG GGA CAG GAT TGG TGA CG (SEQ ID NO: 10), ACA CCG CCC CAC AGT GGG GCC ACT AG (SEQ ID NO:11), AAA ACT AGT GGC CCC ACT GTG GGG CG (SEQ ID NO: 12). Rosa26 targeting vector is constructed with pRosa26-GT as template (a gift from Liqun Luo, addgene plasmid 40025) using primers: GAC TAG TGA ATT CGG ATC CTT AAT TAA GGC CTC CGC GCC GGG TTT TGG CG (SEQ ID NO: 13), GAC TAG TCC CGG GGG ATC CAC CGG TCA GGA ACA GGT GGT GGC GGC CC (SEQ ID NO: 14), CGG GAT CCA CCG GTG AGG GCA GAG GAA GCC TTC TAA C (SEQ ID NO: 15), TCC CCC GGG TAC AAA ATC AGA AGG ACA GGG AAG (SEQ ID NO: 16), GGA ATT CAA TAA AAT ATC TTT ATT TTC ATT ACA TC (SEQ ID NO: 17), CCT TAA TTA AGG ATC CAC GCG TGT TTA AAC ACC GGT TTT ACG AGG GTA GGA AGT GGT AC (SEQ ID NO: 18). AAVS1 targeting vector (SEQ ID NO: 40) was constructed with AAVS1 hPGK-PuroR-pA donor (a gift from Rudolf Jaenisch, addgene plasmid 22072) as template using primers: CCC AAG CTT CTC GAG TTG GGG TTG CGC CTT TTC CM G (SEQ ID NO: 19), CCC AAG CTT CCA TAG AGC CCA CCG CAT CCC C (SEQ ID NO: 20), CAG GGT CTA GAC GCC GGA TCC GGT ACC CTG TGC CTT CTA GTT GC (SEQ ID NO: 21), GGA TCC GGC GTC TAG ACC CTG GGG AGA GAG GTC GGT G (SEQ ID NO: 22), CCG CTC GAG AAT AAA ATA TCT TTA TTT TCA TTA CAT C (SEQ ID NO: 23), GCT CTA GAC CAA GTG ACG ATC ACA GCG ATC (SEQ ID NO: 24). Genotyping primers for CRISPR mediated knockin: GAG CTG GGA CCA CCT TAT ATT C (SEQ ID NO: 25), GGT GCA TGA CCC GCA AG (SEQ ID NO: 26), GAG AGA TGG CTC CAG GAA ATG (SEQ ID NO: 27).

### Skin organoid culture and transplantation

Decellularized dermis (circular shape with 1 cm diameter) was prepared by EDTA treatment of newborn mouse skin (Prunieras *et al*., 1983). An aliquot of 1.5 × 10⁶ cultured keratinocytes was seeded onto the dermis in a cell culture insert. After overnight attachment, the skin culture was exposed to air/liquid interface.

For grafting with skin organoids, CD1 males with the ages of 6-8 weeks were anesthetized. A silicone chamber bottom with the interior diameter of 0.8 cm and exterior diameter of 1.5 cm was implanted on its shaved dorsal mid-line skin, which was used to hold the skin graft. A chamber cap was installed to seal the chamber immediately after a piece of graft was implanted. About one week later, the chamber cap was removed to expose the graft to air. A single dose of 0.2 mg α-CD4 (GK1.5) and 0.2 mg α-CD8 (2.43.1) antibodies was administered intraperitoneally for skin grafting.

### Histology and Immunofluorescence

Skin or wound samples were embedded in OCT, frozen, sectioned, and fixed in 4% formaldehyde. For paraffin sections, samples were incubated in 4% formaldehyde at 4°C overnight, dehydrated with a series of increasing concentrations of ethanol and xylene, and then embedded in paraffin. Paraffin sections were rehydrated in decreasing concentrations of ethanol and subjected to antigen unmasking in 10 mM Citrate, pH 6.0. Sections were subjected to hematoxylin and eosin staining or immunofluorescence staining. Antibodies were diluted according to manufacturer instructions, unless otherwise indicated.

### Cell Cycle Analysis:

Propidium iodide (PI) staining followed by flow cytometry were used to determine the effect of cell cycle profiles. Mouse and human epidermal cells were cultured in two 6 cm cell culture dish for 24 hours, respectively. Cells were trypsinized, and 1×10⁵ cells from each dish were collected, followed by one PBS wash. Fixation of cells was carried out using 70% (v/v) ice cold ethanol for 1 hour. Then, fixed cells were centrifuged at 500 g at 4°C for 10 minutes, followed by 2x PBS wash. Cells were then treated with 75 µg RNAse A in 100 µl PBS and incubated at 37°C for 1 hour. After incubation, the cells were collected by centrifuging at 500 g at 4°C for 10 minutes, followed by another PBS wash. The cell pellet was resuspended in 200 µl PBS with PI solution at a final concentration of 25 ng/µl. After staining, the cells were analyzed immediately using flow cytometer BD FACSCanto^{™} II (BD Biosciences, San Jose, CA) with an excitation wavelength at 488 nm and emission at 585 nm. DNA content and histograms of cell cycle distribution were analyzed using FlowJo^{™} software, version 10 (FLOWJO LLC, OR).

### Protein biochemical analysis

Western blotting was performed to assess protein biochemistry. Briefly, equal amounts of cell lysates were separated on an SDS-polyacrylamide gel electrophoresis (PAGE) and electroblotted onto a NC membrane. The immunoblot was incubated with Odyssey blocking buffer (Li-Cor) at room temperature for 1 h, followed by an overnight incubation with primary antibody. Blots were washed three times with Tween 20/Tris-buffered saline (TBST) and incubated with a 1:10000 dilution of secondary antibody for 1 h at room temperature. Blots were washed three times with TBST again. Visualization and quantification were carried out with the LI-COR Odyssey scanner and software (LI-COR Biosciences).

### Obesity induced by high fat diet and glucose and insulin tolerance tests

Male CD-1 mice were housed (5 per cage, ~8 weeks old) in a central-controlled animal facility for air, humidity, and temperature. These mice were fed either a regular chow or an HFD (60% kcal from fats, 20% from carbohydrates, and 20% from proteins) purchased from Bio-Serv (Frenchtown, NJ). Body weight and food intake were measure biweekly.

For glucose and insulin tolerance tests, an intraperitoneal glucose tolerance test (IPGTT) was performed on mice fed an HFD for 10 weeks. Mice were fasted for 6 h before the test. Animals were injected (1 g/kg glucose/body weight, i.p.) with glucose dissolved in saline, and blood glucose was measured at 0, 30, 60 and 90 min using glucose test strips and glucose meters. An intraperitoneal insulin tolerance test was carried out 1 week after IPGTT. Mice were fasted for 4 h and injected (2 U/kg, i.p.) with insulin purchased from Sigma (St. Louis, MO). Blood glucose levels were determined thereafter at 0, 30, 60 and 90 min.

### Statistical analysis

Statistical analysis was performed using Excel or OriginLab software. Box plots were used to describe the entire population without assumptions on the statistical distribution. A student t test was used to assess the statistical significance (P value) of differences between two experimental conditions.

### RESULTS

### Ectopic expression of GLP1 in epidermal stem cells via CRISPR-mediated genome editing

By genetic engineering of skin epidermal stem cells, skin can potentially be transformed into an *in vivo* reactor that produces GLP1 in a controllable manner (FIG. 1A). Although skin stem cells are very susceptible for manipulation with viral vectors, viral infection could lead to genotoxicity and may raise significant safety concern for the potential gene therapy (Kotterman *et al*., 2015; Kustikova *et al*., 2010). The CRISPR system presents a novel approach to carry out site-specific modification of target genomes non-virally (Cox *et a*/*.,* 2015; Hotta and Yamanaka, 2015; Wright *et a*/*.,* 2016).

To test CRISPR-mediated genome editing in mouse epidermal stem cells, DNA vectors encoding the D10A mutant of Cas9 (CRISPR associated protein 9) (Ran *et al*., 2013), two gRNAs (guide RNA) targeting the mouse *Rosa26* locus, and a *Rosa26*-targeting vector were developed. The targeting vector contains two homology arms for the *Rosa26* locus, flanking an expression cassette that encodes a *GLP-1* and mouse *IgG-Fc* fragment fusion protein (FIG. 10A and FIG. 2A). Fusion with IgG-Fc enhances the stability and secretion of GLP-1 when ectopically expressed in epidermal cells (Kumar *et al.* 2007). To control the level of GLP-1 release, we further modified the targeting vector so the expression of GLP-1 fusion protein is driven by a tetracycline-dependent promoter (FIG. 10A).

Primary epidermal keratinocytes were isolated from CD1 newborn mice, and electroporated with the *Rosa26* targeting vector together with plasmids encoding Cas9 and *Rosa26*-specific gRNAs. Clones were isolated upon selection, and the correct integration to the *Rosa26* locus was confirmed by both PCR screening and southern blotting analysis (FIG. 10B). Engineered epidermal cells exhibited robust GLP1 production upon stimulation with doxycycline in a dose-dependent manner (FIG. 10C). The secreted GLP1 fusion protein was functional as the conditioned medium can significantly induce secretion of insulin when added to insulinoma cells cultured *in vitro* (FIG. 10D). Expression of *GLP1* fusion protein in epidermal cells did not significantly change cell proliferation (FIG. 10E and FIG. 14A) or differentiation (FIG. 10F and 10G) *in vitro.*

To confirm that modified epidermal cells were not tumorigenic, the potential anchorage-independent growth of cells was examined, and the results indicated that epidermal stem cells with *GLP1* targeting cannot grow in suspension with or without doxycycline stimulation (FIG. 10H). As a positive control, cancer initiating cells (Schober and Fuchs, 2011) isolated from mouse SCC (squamous cell carcinoma) exhibited robust colony formation in soft agar medium (FIG. 10H). Expression of *GLP1* did not affect the ability of epidermal stem cells to stratify. When subjected to skin organoid culture, the targeted cells readily produced stratified epithelial tissue (FIG. 14B).

### Stable delivery of GLP1 in vivo through skin transplantation

To investigate the potential therapeutic effect of GLP1 *in vivo,* skin organoid cultures with epidermal keratinocytes targeted with a *GLP1*-expression vector or a control vector, and transplant the organoids to CD1 host animals (FIGS. 11A and 11B) were prepared. No significant rejection of the skin grafts has been observed after transplantation, suggesting that the targeted epidermal stem cells were well tolerated immunologically *in vivo.* Grafted skin exhibited normal epidermal stratification, proliferation and cell death regardless of doxycycline treatment (FIGS. 11C-11E, and FIGS. 15A and 15B). When fed with food containing doxycycline, the mice that were grafted with *GLP1*-expressing cells displayed significantly enhanced level of GLP1 in the blood (FIG. 11F). Expression of *GLP1* in grafted animals was stable for more than 3 months (FIG. 11G). Consistent with previous observations (Christensen *et al*., 2002; Del Rio *et a*/., 2004; Fakharzadeh *et a*/., 2000: Fenjves *et a*/., 1989; Gerrard *et a*/., 1993; Morgan *et a*/*.,* 1987; Sebastiano *et al*., 2014), these results confirm that a skin-derived therapeutic protein can cross the basement membrane barrier and achieve a systematic effect *in vivo.*

### Cutaneous gene transfer with GLP1 can achieve therapeutic effects in vivo

To examine the potential effect in diet-induced obesity and diabetes, *GLP1-*expressing cells and control cells were grafted onto two cohorts of CD1 adult mice, and a high fat diet (HFD) was used to induce obesity in the grafted animals. Doxycycline was applied to half of the animals to induce expression of *GLP1.* To minimize gender difference, only male animals were used. Compared with animals on regular chow diet, HFD greatly accelerated body weight gain in mice grafted with control cells or *GLP1* cells without doxycycline treatment. Induction of *GLP1* expression with doxycycline led to a significant decrease in body weight in mice grafted with *GLP1* cells but not control cells (FIG. 12A and quantified in FIG. 1C). Consistently, histological examination of white fat tissue demonstrated that HFD progressively increased the size of adipocytes and induced a significant level of adipocyte hypertrophy at the end of the experiment in the control groups (FIG. 12B). By contrast, induction of *GLP1* expression dramatically suppressed this effect (FIG. 128).

To examine glucose homeostasis, IPGTT (intraperitoneal glucose tolerance test) and ITT (insulin tolerance test) were performed. HFD resulted in decreased glucose tolerance in control mice or *GLP1*-grafted mice without doxycycline treatment FIGS. 12D and 12E). By contrast, expression of *GLP1* significantly reduced glycemic excursion *in vivo* (FIGS. 12D and 12E). Consistent with glucose homeostasis analysis, expression of *GLP1* in grafted skin significantly reduced insulin resistance compared with control mice or GLP1 mice without doxycycline treatment (FIGS. 12F and 12G). Together, these data strongly suggest that cutaneous gene therapy with inducible expression of *GLP1* can be used for the treatment and prevention of diet-induced obesity and pathologies.

### Cutaneous delivery of GLP1 with human epidermal stem cells

To test the feasibility of cutaneous gene therapy with human epidermal stem cells, human skin organoids were cultured from primary epidermal keratinocytes isolated from human newborn foreskin. When infected with lentivirus, the grafted human cells exhibited robust expression of the exogenous *Luciferase* gene (FIG. 13A). The grafted tissue showed normal skin stratification when stained for early or late epidermal differentiation markers (FIG. 13B).

To examine CRISPR-mediated genome editing in human epidermal cells, vectors encoding two gRNAs targeting human *AAVS1* (adeno-associated virus integration site 1) locus, and an *AAVS1*-targeting vector (FIG. 16A) that harbors a tetracycline-inducible expression cassette encoding the *GLP-1* and *IgG-Fc* fragment fusion protein were used. Human epidermal keratinocytes were transfected with the targeting vector together with plasmids encoding Cas9 and the gRNAs. Clones were isolated and correct integration confirmed by southern blotting analysis (FIG. 13C). Like mouse cells, engineered human epidermal cells exhibited strong GLP1 production upon dose-dependent stimulation with doxycycline (FIG. 13D). The GLP1 fusion protein secreted from these cells induced insulin secretion *in vitro* (FIG. 13E), confirming the release of functional GLP1 from these cells.

Expression of the *GLP1* fusion protein in human cells did not significantly change cell proliferation (FIG. 16B) or differentiation (FIGS. 16C and 16D) *in vitro.* The engineered cells stratified and formed skin organoids *in vitro,* which were transplanted to *nude* hosts (FIG. 13F). Grafted skin exhibited normal epidermal stratification and proliferation regardless of doxycycline treatment (FIGS. 16E-16G). When fed with food containing doxycycline, significant secretion of GLP1 was detected in the blood from the *nude* mice that were grafted with *GLP1-*expressing cells (FIG. 13G).

### DISCUSSION

Somatic gene therapy provides a promising therapeutic approach for treatment of a variety of otherwise terminal or severely disabling diseases (Collins and Thrasher, 2015). Skin epidermal stem cells represent an ideal platform for ex *vivo* gene therapy, allowing efficient genetic manipulation with minimal risk of tumorigenesis or other detrimental complications in *vivo* (Christensen *et al*., 2002; Del Rio *et a*/*.,* 2004). In addition to expression of therapeutic agent (*e*.*g*., hormones and/or protein factors), such as *GLP-1,* ectopic expression of metabolic enzymes in skin epidermal cells can also transform engineered tissue organoids into a potential "metabolic sink" for correction of various metabolic disorders (Christensen *et al*., 2002). Thus, the applicability of such a cutaneous gene therapy platform is very broad. Because of the minimally invasive and safe nature of skin transplantation, should an efficient preclinical model for cutaneous gene therapy be established and encouraging results be obtained from animal models, human clinical tests could start relatively easily.

Diabetes is a major health issue worldwide (Ahima, 2011; Ashcroft and Rorsman, 2012). The peptide hormone GLP1 has the essential requisite properties to maintain homeostatic levels of glucose in order to effectively treat diabetes. Compounds that elongate half-life of endogenous GLP1 or synthetic GLP1 receptor agonists have already been clinically used for adjunctive antidiabetic treatments (Ross and Ekoe, 2010; Sandoval and D'Alessio, Physiology of proglucagon peptides: role of glucagon and GLP-1 in health and disease. *Physio. Rev.* **95**, 513-548 (2015)). Somatic gene transfer that can stably deliver GLP1 to the patients has been proposed as a more effective way for diabetes treatment (Prud'homme *et al*., 2007; Rowzee *et a*/*.,* 2011). In this regard, skin constitutes a tempting target organ, providing a long-lasting, safe, and affordable way for GLP1 delivery. Here, controllable release of GLP1 was demonstrated in engineered tissue organoids and proven to be therapeutically effective *in vivo,* thus laying the essential groundwork for developing novel therapeutic approach for combating obesity and diabetes.

Gene therapy-derived products can be recognized as foreign antigens by the host immune system, which may mount an immune response leading to the clearance of genetically modified cells (Collins and Thrasher, 2015). However, the model developed in this study provides a unique approach. Within normal skin epidermis, the Langerhans cells function as the sole cell type that expresses *major histocompatibility complex (MHC)* class II and presents antigen (Haniffa *et al*., 2015). Epidermal keratinocytes only express *MHC* class I molecules on their cell surface, and are considered as "non-professional" antigen presenting cells. Thus, in the engineered tissue organoids described here (generated from epidermal stem cells), without the presence of Langerhans cells or leukocytes as antigen presenting cells, potential antigenicity and immunogenicity are significantly reduced. It has been shown that skin allografts developed from cultured human keratinocytes can been clinically used for the treatment of wounds, without eliciting significant immune reaction (Centanni *et al*., 2011; Zaulyanov and Kirsner, 2007). The present results demonstrate for the first time that grafted skin stem cells expressing therapeutic proteins can be efficiently and stably grafted to host mice with intact immune systems. The present results further demonstrate the potential of cutaneous gene therapy for the treatment of various human diseases in the future.

### Example No. 3: Tissue Organoids for Treating Cocaine Addiction

### INTRODUCTION

Drug addiction is characterized by the development of compulsive drug-seeking and taking and a high likelihood of relapse when an addicted individual is exposed to drugs or drug-associated cues, even long after abstention (Kalivas et al. Drug addiction as a pathology of staged neuroplasticity. Neuropsychopharmacology: official publication of the American College of Neuropsychopharmacology 33: 166-180 (2008); Koob et al. Neurocircuitry of addiction. Neuropsychopharmacology: official publication of the American College of Neuropsychopharmacology 35: 217-238 (2010); O'Brien et al. Conditioning factors in drug abuse: can they explain compulsion? Journal of psychopharmacology 12: 15-22 (1998)). Cocaine is a commonly abused drug that causes significant morbidity and mortality. Although a variety of pharmacological targets and behavioral interventions have been explored, there are currently no FDA-approved medications for treating cocaine use or relapse in users, and there are no effective interventions for the acute emergencies that result from cocaine overdose (Heard et al. Mechanisms of acute cocaine toxicity. The open pharmacology journal 2: 70-78, (2008); Zimmerman, J. L. Cocaine intoxication. Critical care clinics 28: 517-526 (2012)). We recently demonstrated that the epidermal progenitor cells of the skin can be readily genome edited *in vitro* using CRISPR (clustered regularly interspaced short palindromic repeats) and transplanted back into donor mice (see *Liu, H et al.,* 2015; Yue, J *et a*/*.,* 2016; Yue *et al.* Engineered epidermal progenitor cells can correct diet-induced obesity and diabetes. *Cell stem* cell (2017)). This unique ex *vivo* platform can provide a long-lasting, effective, and safe way for somatic gene delivery. Here, we report that this skin stem cell-based platform for long-term delivery of a cocaine hydrolase *in vivo* can efficiently and specifically protect against cocaine-seeking and acute overdose.

BChE (butyrylcholinesterase) is a natural enzyme that is present in hepatocytes and plasma and hydrolyzes its normal substrate acetylcholine. BChE can also hydrolyze cocaine at low catalytic efficiency into benzoic acid and ecgonine methylester, which are low in toxicity and rewarding properties, i.e., they are not addictive substances. Recent advances in protein engineering have greatly enhanced catalytic potency and substrate specificity of BChE for cocaine hydrolysis, and for protecting against cocaine-induced behaviors, including acquisition and reinstatement of IVSA (intravenous self-administration). The modified hBChE (E30-6) has more than 4400 times higher catalytic efficiency (*k_{cat}*/*K_{M}*) than wild-type (WT) hBChE with significantly reduced activity for acetylcholine (see Zheng et al. A highly efficient cocaine-detoxifying enzyme obtained by computational design. Nat Commun. 5: 3457 (2014)). However, purified recombinant hBChE has a short half-life *in vivo* after i.v. injection, making it useful only for acute treatment of cocaine abuse. In recent clinical trials, an hBChE-albumin fusion protein, TV-1380, was ineffective in facilitating cocaine-abstinence in dependent individuals, most likely due to the short half-life of the protein and also the inefficient intramuscular route of injection (Cohen-Barak et al. Safety, pharmacokinetics, and pharmacodynamics of TV-1380, a novel mutated butyrylcholinesterase treatment for cocaine addiction, after single and multiple intramuscular injections in healthy subjects. Journal of clinical pharmacology 55: 573-583 (2015); Gilgun-Sherki et a/. Placebo-controlled evaluation of a bioengineered, cocaine-metabolizing fusion protein, TV-1380 (AlbuBChE), in the treatment of cocaine dependence. Drug and alcohol dependence 166: 13-20 (2016)). Therefore, the ability to stably deliver engineered hBChE *in vivo* to allow continuous activity could lead to cocaine abstinence in dependent individuals and prevent establishment of cocaine dependence in others.

### EXPERIMENTAL PROCEDURES

### Reagents and Plasmid DNA Constructions

Unless indicated to the contrary, reagents and experimental procedures follow those described in Example No. 2 above. Human BChE quantikine ELISA kit was obtained from R&D systems (Minneapolis, MN).

Human (SEQ ID NO: 41) and mouse *BChE* (SEQ ID NO: 43) with point mutations {Zheng, 2014 #794} were codon-optimized and synthesized from IDT (integrated DNA Technology, Coralville, IA), and PCR amplified with primers A-D, respectively: GCT CTA GAG CCA CCA TGC AGA CTC AGC ATA CCA AGG (SEQ ID NO: 47), CGG GAT CCA CCG GTT TAG AGA GCT GTA CAA GAT TCT TTC TTG (SEQ ID NO: 48), CCC AAG CTT GCC ACC ATG CAT AGC AAA GTC ACA ATC (SEQ ID NO: 49), ACG CGT CGA CTT AGA GAC CCA CAC AAC TTT CTT TCT TG (SEQ ID NO: 50).

### Skin organoid culture and transplantation

Skin organoid culture and transplantation was performed following the procedures described in Example No. 2 above.

### Engraftment

Engraftment followed the procedures described in Example No. 2 above unless otherwise indicated below.

### Cocaine-induced behaviors

For all behavioral experiments except where noted, C57BL/6J mice were used. Roughly equal numbers of adult male and female mice were group-housed until surgery. Mice were maintained under controlled temperature and humidity conditions on a 12 h:12 h light:dark cycle (lights on at 7:00). Water and food were available *ad libitum.* Mice weighed around 25-30 g at the beginning of the experiments. All procedures followed National Institutes of Health Guide for the Care and Use of Laboratory Animal and were approved by *the* University of Chicago Institutional Animal Care and Use Committee.

***Drug:*** Cocaine HCl and methamphetamine HCl (Sigma-Aldrich, Saint Louis, MO) were dissolved in sterile saline and delivered intraperitoneally at appropriates doses in a volume of 10 mL/kg. Ethanol (Sigma-Aldrich, Saint Louis, MO, 95%, density=0.816) was prepared in 20% (v/v) diluted in sterile saline and delivered intraperitoneally at appropriate doses. Vehicle (sterile saline) was intraperitoneally administered at 10 mL/kg as a control.

***CPP apparatus:*** The CPP apparatus (FIG. 17; Med Associates, E. Fairfield, VT, USA) consisted of two larger chambers (16.8×12.7×12.7 cm), which were separated by a smaller chamber (7.2×12.7×12.7 cm) as previously described (Yan *et a*/*.,* 2013). Each chamber had a unique combination of visual and tactile properties (one large chamber had black walls and a rod floor, the other larger chamber had white walls with a mesh floor, whereas the middle chamber had gray walls and a solid gray floor). Each compartment had a light embedded in a clear, Plexiglas hinged lid. Time spent in each chamber was measure via photobeam breaks and recorded. CPP was determined on testing days via time spent in the drug-paired side minus time spent in the saline-paired side.

***Acquisition of CPP**:* A biased CPP procedure was used similar to that from a previous study (Yan *et al*., 2013). Acquisition of CPP consisted of three sequential procedures-pretest, conditioning, and test. After 7-12 days of recovery from engrafting surgeries, mice underwent pretest on Day 1, where mice were allowed to freely explore the entire chamber for 20 min once daily. Mice that spent more than 500 s in the grey compartment or more than 800 s in either of the large compartments were excluded from the study. Following the pretest day, mice underwent conditioning and testing on Days 2-5. Starting on Day 2, mice received an i.p. injection of drug (10 mg/kg cocaine in cocaine CPP or 2 g/kg ethanol in ethanol CPP) and were confined to the white chamber for 30 min. At least 5 hours after in the same day, mice received an i.p. injection of saline and were confined to the black compartment for 30 min. On Day 6 (test day) mice were allowed to explore the entire chambers for 20 min and time spent in each area was recorded.

***Extinction and reinstatement of CPP:*** Following CPP acquisition, mice underwent extinction, in which the procedure was identical to that in the test day. In each extinction day, mice were allowed to explore the entire chambers for 20 min and time spent in each area was recorded. Extinction was performed until the CPP decreased to a level that was not different from that of the pretest in consecutive two days. On the following day of the last extinction, mice underwent reinstatement procedures, in which mice that were trained for cocaine CPP received an i.p. injection of 15 mg/kg cocaine, and mice that were trained for ethanol CPP received an i.p. injection of 1 g/kg ethanol. Immediate after injection, mice were allowed to explore the entire chambers for 20 min and time spent in each area was recorded.

***Acute drug overdose test:*** Two weeks after grafting surgery, 4 groups of GhBChE and 4 groups of GWT mice (n=8 each in each group) received i.p. injections of cocaine at 40, 80, 120, and 160 mg/kg. As a control, 4 groups of GhBChE and 4 groups of GWT mice (n=8 each) received i.p. injections of methamphetamine (METH) at 34, 68 (LD50), 100, and 160 mg/kg. Two each of GhBChE and GWT mice with CD1 mice as hosts were also used to videotape acute cocaine (80 mg/kg) induced behaviors. Mice were monitored for 2 h following injection and percent of cocaine- and METH-induced lethality was calculated.

***Specific methods:*** One group of GhBChE and one group of GWT mice (n=9 in each group) were trained for cocaine CPP 7 days after engraftment (FIG. 18A). Mice underwent pretest on Day 1, four days of cocaine conditioning day 2 to Day 5, and CPP test on Day 6. One group of GhBChE and one group of GWT mice (n=8 in each group) were trained for ethanol CPP 7 days after engraftment (FIG. 18B). Mice underwent pretest on Day 1, four ethanol conditioning day 2 to Day 5, and CPP test on Day 6. Two groups of drug-naïve wildtype mice (n=8 in each group) were trained for cocaine CPP from day 1 to Day 6 (FIG. 18C). On the following day, mice underwent engrafting surgery. The behavioral procedure resumed after engraftment surgery from Day 18. Extinction was performed from Day 18 to Day 31. On Day 32, mice underwent reinstatement induced by i.p. injection of cocaine. One group of GhBChE and one group of GWT mice (n=8 in each group) were trained for ethanol CPP from day 1 to Day 6. Extinction was performed from Day 7 to Day 20 (FIG. 18D). On Day 21, mice underwent reinstatement procedure induced by i.p. injection of ethanol.

### Statistical analysis

Statistical analysis was performed using Excel or OriginLab software. Box plots are used to describe the entire population without assumptions on the statistical distribution. A student *t* test was used to assess the statistical significance (P value) of differences between two experimental conditions. For cocaine behavioral analysis, CPP results were analyzed using repeated-measures ANOVA with within factor time (testing days) and between factor treatment (engraftment). Significant effects were further analyzed with Fishers t-tests.

### RESULTS AND DISCUSSION

To carry out CRISPR-mediated genome editing in mouse epidermal progenitor cells, DNA vectors were developed encoding the D10A mutant of Cas9 (CRISPR associated protein 9; Ran *et al*., 2013), two gRNAs (guide RNA) targeting the mouse *Rosa26* locus, and a *Rosa26*-targeting vector. The targeting vector contains two homology arms for the *Rosa26* locus, flanking an expression cassette that encodes the modified *hBChE* gene (FIG. 19A). Primary epidermal basal cells were isolated from newborn mice, and electroporated with the Rosa26 targeting vector together with plasmids encoding Cas9 and *Rosa26*-specific gRNAs. Clones were isolated upon selection and the correct integration to the *Rosa26* locus was confirmed by both PCR screening and southern blotting analysis (FIG. 19B). Engineered epidermal cells exhibited robust expression and secretion of hBChE as shown by immunoblots and ELISA (enzyme-linked immunosorbent assay) (FIGS. 19C and 19D). The secreted hBChE protein was functional as the conditioned medium collected from hBChE-expressing cells but not the control cells significantly induced degradation of cocaine *in vitro* (FIG. 19E). Consistent with previous reports, similar mutations in *mBChE* (mouse *BChE*) led to only residual activity in cocaine hydrolysis (Chen, X. et a/. Kinetic characterization of a cocaine hydrolase engineered from mouse butyrylcholinesterase. The Biochemical journal 466, 243-251 (2015); FIG. 19E). Expression of *hBChE* in epidermal stem cells did not significantly change cell proliferation (FIG. 19F and FIG. 20A) or differentiation (FIG. 20B) *in vitro.* To confirm that modified epidermal cells were not tumorigenic, the potential anchorage-independent growth of cells was examined. The results indicated that epidermal stem cells with *hBChE* targeting cannot grow in suspension. By contrast, cancer initiating cells (Schober, M. et al. Tumor-initiating stem cells of squamous cell carcinomas and their control by TGF-beta and integrin/focal adhesion kinase (FAK) signaling. Proc Natl Acad Sci U S A 108, 10544-10549 (2011)) isolated from mouse SCC (squamous cell carcinoma) exhibited robust colony forming efficiency in soft agar medium (FIG. 20C).

To efficiently transplant mouse epidermal progenitor cells, a new organotypic culture model with mouse epidermal progenitor cells was developed *in vitro* by culturing the cells on top of acellularized mouse dermis. Exposure to the air/liquid interface can induce stratification of cultured cells to generate a skin-like organoid *in vitro.* Expression of *hBChE* did not change the ability of epidermal stem cells to stratify (FIG. 21A). To investigate the potential therapeutic effect of *hBChE* expression *in vivo,* we transplanted the organoids to isogenic host animals (CD1 and C57BL/6) (FIGS. 22A and 22B). No significant rejection of the skin grafts was observed for at least 5 months after transplantation, suggesting that the targeted epidermal stem cells were well tolerated immunologically *in vivo.* Grafted skin exhibited normal epidermal stratification, proliferation, and cell death (FIGS. 21B, 21C, and 22C). The mice that were grafted with *hBChE*-expressing cells displayed significantly elevated levels of human BChE in the blood (FIG. 22D). Expression of *hBChE* in grafted animals was stable for more than 10 weeks (FIG. 22D). Consistent with previous observations, our results confirm that a skin-derived therapeutic protein can cross the basement membrane barrier and enter circulation *in vivo.*

To first determine whether engrafting hBChE-expressing cells protects mice from acute systemic toxicity of cocaine, we delivered different doses of cocaine to grafted mice and calculated the lethality rates of cocaine. Doses of 40, 80, 120, 160 mg/kg of cocaine had nearly 0 lethality in mice grafted with *hBChE*-expressing cells (GhBChE), whereas 80 mg/kg of cocaine induced roughly 50% lethality and 120 and 160 mg/kg cocaine induced 100% lethality in control mice grafted with WT epidermal cells (GWT) (FIG. 22E). Parallel testing was conducted to test the toxicity of a related stimulant METH (methamphetamine) in GhBChE and GWT mice. There was no difference in lethality induced by various doses of METH between GhBChE and GWT animals (FIG. 22F). This finding shows that engraftment of *hBChE-*expressing cells can lead to significant release of functional hBChE *in* vivo and protect mice from the toxicity of an acute cocaine overdose.

Next, protection against development of cocaine-seeking was assessed using the conditioned place preference (CPP) paradigm, which is thought to model reward learning and seeking because experimental animals approach and remain in contact with cues that have been paired with the effects of the reward. *hBChE*-expressing cells were grafted to cocaine-naïve mice with GWT animals as controls. After 4 days of place conditioning, GWT mice spent significantly more time in environments previously associated with cocaine, whereas GhBChE mice showed no such preference (FIG. 18A). As an additional control, ethanol CPP was measured after 4 days of conditioning in GhBChE and GWT mice. In contrast, both GhBChE and GWT mice spent significantly more time in ethanol-paired environments (FIG. 18B). This finding indicates that engraftment of hBChE-expressing cells efficiently and specifically attenuated cocaine-induced rewarding effect.

To determine whether engrafting hBChE-expressing cells affect cocaine-induced reinstatement of drug-seeking, *hBChE*-expressing cells were grafted in mice that previously acquired cocaine CPP. Following 10 days of recovery, we performed extinction training and drug-elicited reinstatement. After a priming dose of cocaine injection, the preference for the previously cocaine-associated environment was restored in the GWT mice but not in the GhBChE mice (FIG.18C). Because hBChE-expression did not prevent CPP induction by ethanol (FIG. 18B), these GhBChE and GWT mice were used to perform extinction training followed by reinstatement. In contrast to those induced by cocaine, ethanol CPP was similarly reinstated in both GhBChE and GWT mice (FIG. 18D). These results suggest that skin-derived hBChE efficiently and specifically disrupts cocaine-elicited reinstatement.

To test the feasibility of cutaneous gene therapy with human epidermal progenitor cells, we cultured human skin organoids from primary epidermal keratinocytes isolated from human newborn foreskin. To perform CRISPR-mediated genome editing in human cells, we developed vectors encoding two gRNAs targeting human *AAVS1* (adeno-associated virus integration site 1) locus, and an *AAVS1*-targeting vector (FIG. 23A) that harbors the expression cassette encoding engineered *hBChE.* Human epidermal keratinocytes were electroporated with the targeting vector together with plasmids encoding Cas9 and the gRNAs. Clones were isolated and correct integration confirmed by southern blotting analysis (FIG. 23B). Like mouse cells, engineered human epidermal cells exhibited strong hBChE production as determined by immunoblots and ELISA (FIGS. 23C and 23D). Expression of the *hBChE* protein in human cells did not significantly change cell proliferation (FIG. 24A) or differentiation (FIG. 24B) *in vitro.* The engineered cells stratified and formed skin organoids *in vitro,* which were transplanted to *nude* host (FIG. 23E). Grafted skin exhibited normal epidermal stratification, proliferation, and apoptosis *in vivo* (FIG. 23F and FIGS. 24C-D). Together, these results indicate that CRISPR editing of human epidermal progenitor cells does not significantly alter cellular dynamics and persistence *in vivo.* ELISA confirmed that the mice with engraftment of *hBChE*-expressing cells had significantly levels of human BChE in the blood, whose expression was stable for more than 8 weeks *in vivo* (FIG. 23G). Our results suggest the potential clinical relevance of cutaneous gene delivery for treatment of cocaine abuse and overdose in the future.

This study demonstrates for the first time that grafted skin stem cells expressing hBChE can be highly drug-specific in addressing several key aspects of cocaine abuse including reducing development of cocaine-seeking, preventing cocaine-induced reinstatement of drug-seeking and protecting against acute cocaine overdose. Because of the high levels of hBChE present, this approach is very efficient with little individual variation. Cutaneous gene delivery with engineered epidermal stem cells may provide therapeutic opportunities for drug abuse or co-abuse beyond cocaine.

For instance, glucagon-like peptide 1 (GLP1) is a major physiological incretin that controls food intake and glucose homeostasis (Sandoval *et a*/*.,* 2015). Several GLP1 receptor agonists have been approved by the FDA to treat type II diabetes. Our recent study indicates that skin-derived expression of *GLP1* can effectively correct diet-induced obesity and diabetes in mice (Yue *et al.,* 2017). Interestingly, GLP1 receptor agonists can also attenuate the reinforcing properties of alcohol and nicotine in rodents (Skibicka, K.P., The central GLP-1: implications for food and drug reward. Front Neurosci. 7, 181 (2013); Egecioglu et al. The glucagon-like peptide 1 analogue Exendin-4 attenuates alcohol mediated behaviors in rodents. Psychoneuroendocrinol. 38, 1259-1270 (2013); Shirazi et a/., Gut peptide GLP-1 and its analogue, Exendin-4, decrease alcohol intake and reward. PLoS One. 8, e61965 (2013); Vallöf et a/. The glucagon-like peptide 1 receptor agonist liraglutide attenuates the reinforcing properties of alcohol in rodents. Addict. Biol. 21, 422-437 (2016)). Thus, future studies will determine whether a constitutive or inducible expression of *GLP1* from skin transplants can reduce alcohol and nicotine use and relapse in patients with alcohol use disorder and nicotine dependence. Additionally, it will be important to investigate whether co-expression of *hBChE* and *GLP1* in skin can be used for treatment of alcohol and cocaine and ethanol and nicotine co-abuse, which occurs with high frequency and significantly increases the risk of drug-related morbidity and mortality.

Epidermal stem cells of the skin provide an ideal platform for ex *vivo* gene therapy, allowing efficient genetic manipulation with minimal risk of tumorigenesis or other detrimental complications *in* vivo. Cultured human epidermal progenitor cells have been used to generate CEA (cultured epidermal autograft), which has been clinically used to treat massive burn wounds for decades. Engineered skin stem cells and CEA have also been used to treat other skin diseases, including vitiligo and skin genetic disorders, such as epidermolysis bullosa. The regenerated skin is stable *in vivo* and can last for long term in the clinical follow-up studies. As such, the cutaneous gene therapy is long-lasting, minimally invasive and safe.

Gene therapy-derived products can be recognized as foreign antigens by the host immune system, which may mount an immune response leading to the neutralization of the therapeutic molecules or the clearance of genetically modified cells (Collins and Thrasher, 2015). Our skin transplantation model built with WT isogenic animals provides a unique approach to examine this process *in* vivo. Skin epidermal keratinocytes have low immunogenicity. Within normal skin epidermis, the Langerhans cells function as the only cell type that expresses *major histocompatibility complex* (*MHC*) class II and presents antigen. Epidermal keratinocytes are considered as "non-professional" antigen presenting cells. Thus, in skin substitute generated from epidermal progenitor cells, without the presence of Langerhans cells or leukocytes as antigen presenting cells, potential antigenicity and immunogenicity are significantly reduced. Consequently, engineered skin grafts are generally well taken and immunologically tolerated in WT isogenic animals. Indeed, skin-derived expression of *hBChE* in host mice with intact immune systems can be stable for more than 10 weeks without significant decrease in the serum level of engineered enzyme, strongly suggesting that the low immunogenicity of skin environment may help to reduce the antigenicity and immune reaction toward *hBChE.* Moreover, the oldest GhBChE mice are 6 months old and healthy with no tissue rejections lending further support for the long-lasting feasibility of cutaneous gene therapy targeting cocaine abuse. Taken together, these results show promise for cutaneous gene therapy as a safe and cost-effective therapeutic option for cocaine abuse in the future.

### Example No. 4: Treatment of Alcohol Abuse

### INTRODUCTION

Alcohol use disorder (AUD) is one of the foremost public health problems. AUD involves problems controlling drinking, continuing to use alcohol even when it causes problems, having to drink more to get the same effect, or having withdrawal symptoms when one decreases or stops drinking (Koob et al. Neurobiology of addiction: a neurocircuitry analysis. Lancet Psychiatry 3, 760-773 (2016); Koob, G.F. Neurocircuitry of alcohol addiction: synthesis from animal models. Handb. Clin. Neurol. 125, 33-54 (2014)). 7.2 percent or 17 million adults in the United States ages 18 and older had an AUD in 2012 (Grant et al. Epidemiology of DSM-5 alcohol use disorder: results from the national epidemiologic survey on alcohol and related conditions III. JAMA Psychiatry 72, 757-766 (2015)). There are three FDA-approved medications and behavioral counseling for stopping or reducing drinking and preventing relapse in humans (Johnson, B.A. Update on neuropharmacological treatments for alcoholism: scientific basis and clinical findings. Biochem. Pharmacol. 75, 34-56 (2008)). However, only 1.3 million receive treatment (NIAAA, 2015). Moreover, not all people respond to these medications and types of treatment, and not all people follow the treatment regimens offered (Cohen et al. Alcohol treatment utilization: findings from the national epidemiologic survey on alcohol and related conditions. Drug Alcohol Depend. 86, 214-221 (2007)). Consequently, additional approaches are needed for combating AUD. Indeed, if a treatment were available that prevented an individual from desiring to consume alcohol, such a treatment could ultimately lead to the individual stopping consumption of alcohol.

Glucagon-like peptide 1 (GLP1) is a gastrointestinal peptide and a major physiological incretin that controls food intake and glucose homeostasis (Sandoval *et al.* 2015). Several GLP1 receptor agonists have been approved by the FDA to treat type II diabetes. Interestingly, GLP1 receptor agonists can also attenuate the reinforcing properties of alcohol in rodents (Skibicka, K.P., The central GLP-1: implications for food and drug reward. Front Neurosci. 7, 181 (2013); Egecioglu et al. The glucagon-like peptide 1 analogue Exendin-4 attenuates alcohol mediated behaviors in rodents. Psychoneuroendocrinol. 38, 1259-1270 (2013); Shirazi et al., Gut peptide GLP-1 and its analogue, Exendin-4, decrease alcohol intake and reward. PLoS One. 8, e61965 (2013); Vallöf et al. The glucagon-like peptide 1 receptor agonist liraglutide attenuates the reinforcing properties of alcohol in rodents. Addict. Biol. 21, 422-437 (2016); Sørensen et al. Effects of the GLP-1 agonist Exendin-4 on intravenous ethanol self-administration in mice. Alcohol Clin. Exp. Res. 40, 2247-2252 (2016); Suchankova et al. The glucagon-like peptide-1 receptor as a potential treatment target in alcohol use disorder: evidence from human genetic association studies and a mouse model of alcohol dependence. Transl. Psychi. 5, e583 (2015)).

While GLP1 may be potentially used in treating AUD (Suchankova *et al.* 2015), the native GLP1 must be delivered through a parenteral route to achieve its effect, and it has an extremely short circulating half-life (Sandoval and D'Alessio, 2015). Therefore, the somatic gene transfer approach used in Example No. 2 was used to determine the efficacy of GLP1 in treating AUD in mice.

### EXPERIMENTAL PROCEDURES

Unless indicated to the contrary, reagents and experimental procedures follow those described in Example No. 2 above.

### Modified GLP1

The GLP1 gene (*mGLP1* or DImGLP1) was modified to produce a novel protein with longer half-life *in vivo* (Kumar et al., Gene therapy of diabetes using a novel GLP-1/lgG1-Fc fusion construct normalizes glucose levels in db/db mice. Gene therapy 14, 162-172, (2007)). To be able to stably deliver *mGLP1 in vivo* and control its expression, we made a Rosa26-targeting vector encoding a Gly8-mutant mGLP1 and mouse IgG-Fc fragment fusion protein (SEQ ID NO: 45) driven by a dox-dependent promoter (FIG. 25A; Yue et al. Treatment of diabetes and obesity with CRISPR-mediated genome editing in epidermal progenitor cells. Cell Stem Cell, In press (2017); Kumar *et al.,* 2007). The glycine mutation renders the peptide resistant to DPP-IV-mediated cleavage (Mentlein et al., Dipeptidyl-peptidase IV hydrolyses gastric inhibitory polypeptide, glucagon-like peptide-1 (7-36) amide, peptide histidine methionine and is responsible for their degradation in human serum. Eur. J. Biochem. 214, 829-835 (1993)).

Fusion with IgG-Fc further enhances mGLP1 stability and circulation half-life when expressed in epidermal cells (Kumar *et al.,* 2007), and it can pass the blood-brain barrier to reach the brain (Pardridge, W.M., CSF, blood-brain barrier, and brain drug delivery. Expert Opin. Drug Deliv. 13, 963-975 (2016)). The Rosa26 allele has been widely used as a safe locus for gene targeting in mice (Soriano, P., Generalized lacZ expression with the ROSA26 Cre reporter strain. Nat. Genetics 21, 70-71, (1999)). To use CRISPR-mediated genome editing, vectors encoding the D10A mutant Cas9 (CRISPR associated protein 9) (Ran *et a*/*.,* 2013) and two gRNA (guide RNA) targeting the mouse Rosa26 allele were developed. The D10A mutation in Cas9 and double nicking system reduce the undesired off-target mutagenesis in the genome to a minimum level (Ran *et al.,* 2013).

### Skin organoid culture and transplantation

The epidermal progenitor cells were isolated from newborn pups of CD1 or C57BL/6J mice. Cells were transfected with the targeting vector and plasmids encoding Cas9 and Rosa26-specific gRNAs. Targeted clones were selected in the medium containing puromycin, and correct incorporation into the Rosa26 locus was confirmed by PCR and southern blots (FIG. 25B). Mouse skin substitute was prepared by seeding the targeted cells to the acellularized newborn dermis and differentiation upon exposure to the air/liquid interphase and the resultant tissues were transplanted to CD1 or C57BL/6J mice (GLP1).

When fed with dox food, GGLP1 mice began to display significantly enhanced levels of mGLP1 in the blood within 3 days (FIG. 25C; Yue *et al.,* 2017). There was a dose-dependent release of mGLP1 in plasma (not shown). Expression of mGLP1 in GLP1 mice was stable for up to 4 months in the presence of dox (FIG. 25D).

### RESULTS AND DISCUSSION

mGLP1 expression attenuated ethanol-induced CPP in GLP1 mice (FIG. 26) compared to mock grafted (GWT).

GLP1 mice did not exhibit significant ethanol-induced CPP. Following 2 free explorations (Pre-test) on day 1, separate groups of GLP1 and GWT mice (n = 9 each) received alternative ethanol (2 g/kg) and saline i.p. injections twice daily for the next 4 days, as previously described (Chen et a/., Dopamine D1 and D3 receptors are differentially involved in cue-elicited cocaine seeking. J. Neurochem. 114, 530-541 (2010); Kong et al., Activation of dopamine D3 receptors inhibits reward-related learning induced by cocaine. Neurosci. 176, 152-161 (2011)). CPP expression was tested on day 6. Results represent mean ± SEM time spent on the drug-paired side minus the saline-paired side. Repeated-measures ANOVA with test days as the within group factor and status of grafting as the between-subject factor were used (Chen *et al.,* 2010; Kong *et al.,* 2011). F value was calculated and Newman-Keuls post-hoc test was performed (Chen *et al.,* 2010; Kong *et al.,* 2011). GLP1 and GWT mice were on dox food for the entire duration.

These results demonstrate that *mGLP1* expression attenuated ethanol-induced CPP. Therefore, tissue organoids expressing mGLP1 have the potential for treating AUD. In addition, Egecioglu *et a*/*.,* also reported that GLP-1 receptor agonist, Exendin-4 (Ex4) attenuates nicotine-induced locomotor stimulation, accumbal dopamine release, and the expression of conditioned place preference in mice (Egecioglu *et al.,* 2013). Therefore, it is believed that tissue organoids expressing mGLP1 can also be used to treat nicotine addiction. It follows that tissue organoids expressing mGLP1 can also be used to treat individuals with AUD who are addicted to nicotine at the same time. Moreover, it is believed that tissue organoids designed to express multiple therapeutic agents, such as mGLP1 and hBChE can be used to reduce incidents of cocaine and ethanol and/or nicotine co-abuse and potentially reduce abuse and co-abuse of other drugs, such as amphetamines (see Skibicka K.P. The central GLP-1: implications for food and drug reward, Front Neurosci. 7:181 (2013)). It is also contemplated herein to that GLP-1 analogs (see above) can be employed in a similar fashion.

### Example No. 5: Tissue Organoids for Treating Alcohol Abuse

Alcoholism is a debilitating disease characterized by dependence on alcohol consumption and is often associated with social and/or health problems. Medications are available to treat alcoholism that discourage alcohol consumption by causing nausea when consuming alcohol, by reducing the pleasure associated with drinking, or by reducing the craving for alcohol. However, as with all medications, treatment compliance is difficult. Treatment compliance is further complicated when the medications are for breaking an addiction and require self-administration. Therefore, automatic administration of a therapeutic agent for combatting alcoholism induced when alcohol is consumed would be desirable.

Therefore, a tissue organoid engineered to express one or more alcohol-inducible genes encoding a spider-derived pain peptides, such as DkTx (S2-DkTx; SEQ ID NO: 36) or VaTx (S2-VaTx3; SEQ ID NO: 35) is prepared as described above (see FIG. 27). In addition, a GLP-1 and/or a GLP-1 analog can be further added to the tissue organoid for simultaneous or on-demand expression along with DkTx or VaTx. It is envisioned that any of these therapeutic agents can be expressed, and/or administered, individually or in any combination.

After engineering an alcohol-inducible therapeutic tissue organoid, the organoid is transplanted into mice before and after training mice in a two-bottle choice paradigm. This paradigm tests how well the system works for protecting mice from acquiring or relapsing into alcohol drinking. In the mouse housing cages, there are two liquid bottles with one containing regular drinking water, and the other a certain percentage of an alcohol solution. The amount of alcohol or water consumption is measured daily. Previous work has established that mice prefer drinking alcohol over water over a period, reflecting the rewarding effects of alcohol.

Therefore, with an alcohol-inducible therapeutic tissue organoid implanted, mice are expected to never develop the preference (acquisition) or relapse into alcohol drinking (relapse).

Similarly, a human patient with a biointegrated tissue organoid, according to this example, experiences 1) pain or discomfort from the expressed toxin and 2) lack of reward from the expressed GLP-1/GLP-1 analog when alcohol metabolites activate the expression of the therapeutic agent (e.g., a spider-derived toxin and GLP-1 or GLP-1 analog). In this way, the therapeutic agents expressed in response to alcohol metabolites work synergistically, and the patient is disinclined to drink.

### Example No. 6: Tissue Organoids for Monitoring Silent Heart Attack

Silent heart attacks have few or no overt "classical" symptoms, such as chest pressure, chest heaviness, arm pain, neck pain, jaw pain, shortness of breath, sweating, extreme fatigue, dizziness, and nausea. However, though nearly half of heart attacks are silent, they are correlated with similar risk of death as "overt" heart attacks, and if successfully diagnosed, they may treated with similar medications and lifestyle changes. Therefore, the ability to monitor factors indicative of silent heart attacks would be beneficial, such as Heart-type Fatty Acid-Binding Protein (H-FABP) and myocardial myoglobin.

A tissue organoid engineered to report occurrence of a silent heart attack is prepared as described above using a targeting vector harboring genes encoding reporter molecules specific for H-FABP (SEQ ID NO: 62) and myocardial myoglobin (SEQ ID NO: 63).

### Example No. 7: Tissue Organoids for Monitoring Stroke

Cerebrovascular accidents or "strokes" are the sudden death of brain cells due to lack of oxygen when the blood flow to the brain is impaired by blockage or blood vessel rupture. Glial fibrillary acidic protein (GFAP) (SEQ ID NO: 64) is a biomarker associated with symptoms of acute stroke. S100B (SEQ ID NO: 65) is a marker associated with astrocyte damage and increased BBB permeability associated with stroke. The ability to monitor such markers of strokes would permit earlier intervention when an individual experiences a stroke.

Therefore, a tissue organoid engineered to report occurrence of a stroke is prepared as described herein using a targeting vector harboring genes encoding reporter molecules specific for GFAP (SEQ ID NO: 64) and S100B (SEQ ID NO: 65).

### Example No. 8: Tissue Organoids for Treating Phenylketonuria (PKU)

### INTRODUCTION

PKU is the most prevalent inherited disease involving the metabolism of amino acids. It results from mutations in the gene encoding a key hepatic enzyme, *phenylalanine hydroxylase (PAH*), which catalyzes the hydroxylation reaction that converts phenylalanine to tyrosine. If untreated, PKU will lead to a dramatic increase of plasma phenylalanine levels, causing profound and irreversible mental disability, epilepsy, and other behavioral problems. The current treatment of PKU is stringent dietary restriction of natural protein intake and supplementation of amino acids other than phenylalanine by a chemically manufactured protein substitute, which can prevent most of the complications of the disease after birth. However, it has been well documented that neuropsychological deficits still exist for patients with dietary restriction, and maintaining the dietary control has been proven difficult, especially in adolescents, young adults, and pregnant women. Somatic gene therapy holds the potential for development of more effective treatment for PKU.

Although PAH is naturally a hepatic enzyme, it has been speculated that circulating phenylalanine could be adequately cleared by ectopic expression of *PAH* in tissues other than liver, such as T cells, muscle cells, and skin epidermal cells. However, a potential issue is that catalytic activity of PAH requires a non-protein cofactor, BH4 (tetrahydrobiopterin), which is synthesized de novo from guanosine triphosphate in hepatocytes by a three-enzyme pathway involving GTP cyclohydrolase I (GTPCH), 6-pyruvoyltetrahydrobiopterin synthase (PTPS), and sepiapterin reductase (SR). Without sufficient supplement of BH4 or reconstitution of the BH4 synthetic pathway, ectopic expression of *PAH* cannot function properly. Transgenic mice that express both *PAH* and *GTPCH*, the rate limiting enzyme for BH4 synthesis, under various skin specific promoters cannot sufficiently rescue the phenylalanine defects *in vivo,* suggesting that alternative approaches must be pursued to convert skin epidermis to an effective "phenylalanine sink."

Enzyme replacement therapy has been proposed for treatment of PKU. Phenylalanine ammonia-lyase (PAL) is an attractive alternative for clearance of phenylalanine. PAL can carry out non-oxidative deamination of L-phenylalanine to form ammonia and trans-cinnamic acid (cinnamate), which can be excreted as hippurate in urine, along with small amounts of cinnamic and benzoic acid. Unlike PAH, PAL is an autocatalytic enzyme that requires no cofactors, making it a simple and effective way to remove plasma phenylalanine.

Preliminary results in mouse epidermal keratinocytes show efficient expression of *PAL* upon infection with lentivirus encoding *PAL* and degradation of phenylalanine in culture media much faster than cells expressing *PAH*. Exogenous expression of *PAL* in epidermal progenitor cells does not significantly alter cell proliferation or differentiation *in vitro,* and the cells readily generate skin organoids when cultured on top of acellularized dermis. Therefore, using CRISPR-mediated genome editing with epidermal progenitor cell technology the possibility for treatment of PKU by cutaneous expression of *PAL* is investigated.

The mouse model of PKU has been developed by ENU (N-ethyl-N-nitrosourea)-mediated mutagenesis in BTBR strain (Jax strain 002232).

With establishment of skin engraftment in *PAH* mutant mice, plasma phenylalanine level are monitored. Three weeks post skin grafting with *PAL* expressing cells or control cells (BTBR epidermal progenitor cells treated with control vectors), blood samples are collected from the tail clipping biweekly for up to 30 weeks. Plasma is separated by centrifugation. Phenylalanine concentration is determined by established fluorometric protocol. Lowering of phenylalanine level restores melanin biosynthesis, and leads to a change of fur color. Potential alteration in fur coat color is recorded weekly for all the grafted animals. It has been demonstrated before that treatment of PKU has gender-dependent response, for both viral vector-mediated therapy and enzyme replacement therapy with modified PAL. Thus, two different cohorts of animals (male and female) are established to test the potential gender-dependent effect of cutaneous gene therapy *in vivo.*

At the endpoint of the experiment (30 weeks), grafted animals are euthanized and tissue samples (grafted skin, host skin, and liver) are collected. Presence of enzyme activity in the tissues is determined by PAH and PAL activity assay.

*In vitro* analysis demonstrated that exogenous expression of *PAL* in mouse epidermal cells can clear ~120 nmol of phenylalanine/hour/10⁶ cells. Thus, two 1 cm² epidermal grafts that contain ~2×10⁷ metabolically active cells is likely to remove 57 µmol of phenylalanine per day, which would be sufficient for a complete reversal of hyperphenylalaninemia (*PAH* mutant mice usually have - 2mM of plasma phenylalanine). A complete phenotypic change from brown hair coat to dark black fur is also expected. For tissue enzymatic analysis, PAL activity detection in grafted skin is expected but not in other tissues. PAH activity is absent in the liver of the mutant mice. Female mice are more resistant to PKU therapy. If this is the case for cutaneous gene therapy, female *PAH* mutant mice are tested to determine whether they require larger skin grafts or more grafts than male mice for correction of hyperphenylalaninemia.

One potential issue is that overexpression of *PAL* can lead to excessive removal of phenylalanine in mice and cause hypophenylalaninemia, which has been reported for human PKU patients receiving enzyme replacement therapy with recombinant *PAL.* If this occurs, a Tet (tetracycline)-inducible system to drive *PAL* expression as described above can be used. Expression level of *PAL* can be controlled by administration of different dose of Doxycycline.

### Approach 1: Express PAL gene in grafted cells

Although exogenous expression of *PAL* does not alter cell proliferation or differentiation program in mouse epidermal keratinocytes, expression of *PAL* could impair human keratinocyte growth or skin regeneration. If this occurs, a Tet-inducible system to drive *PAL* gene expression is used (see FIG. 28).

### Approach 2: Immune Intolerance of PAL

If exogenous expression of *PAL* indeed leads to a severe immune response that cannot be suppressed, expression of *PAH*, the natural phenylalanine processing enzyme, is tested for use in treatment of PKU. Although PAH requires BH4 as cofactor, it has been shown that co-expression of *GTPCH* and *PTPS* together with *PAH* in muscle can result in stable and long-term reduction of plasma phenylalanine in mice model. Therefore, a *Rosa26* targeting vector harboring a triple-cistronic expression cassette encoding *PAH* (SEQ ID NO: 54), *GTPCH* (SEQ ID NO: 55), and *PTPS* (SEQ ID NO: 56) is developed.

Targeted epidermal progenitor cells are examined both *in vitro* and *in vivo* for phenylalanine clearance and potential therapeutic effect for PKU. If co-expression of *PAH, GTPCH,* and *PTPS* is not sufficient, a *Rosa26* targeting vector harboring a quadruple-cistronic expression cassette encoding *PAH, GTPCH, PTPS,* and SR (SEQ ID NO: 57) is developed to reconstitute the entire BH4 de *novo* synthesis pathway in a tissue organoid.

### Example No. 9: Tissue Organoids for Treating Hemophilia

### INTRODUCTION

Hemophilia is an inherited blood clotting disorder caused by a deficiency of clotting *Factor VIII* (type A) or *IX* (type B) in the blood plasma. The unstoppable bleeding itself can be life-threatening, but hemophiliacs usually also suffer from recurrent bleeding into soft tissues, joints and muscles, leading to chronic synovitis, crippling arthropathy and physical disability. Hemophilia is an excellent candidate for cutaneous gene therapy because both Factor VIII and IX are secreted proteins and, therefore, their exogenous expression in epidermal cells could correct the deficiency. In addition, particularly for hemophilia A, the coding sequence of *Factor VIII* is more than 7 kb (kilobase), far beyond the packaging limitation of typical viral vectors used for gene therapy, such as AAV (adeno-associated virus) vectors. Somatic gene therapy in skin provides a more sustained and affordable treatment for hemophilia A than the current standard therapy by intravenous infusions with purified Factor VIII.

Previous work has demonstrated that *Factor VIII* and *IX* can be expressed in human or mouse epidermal keratinocytes, and exogenously expressed clotting factors can pass the epidermal/dermal barrier to reach the circulation. Particularly, when transgenic skin that expresses *Factor VIII* under the involucrin promoter is grafted onto immunocompromised hosts (*Factor VIII* and *Rag1* double knockout), epidermal expression of *Factor VIII* significantly restores the plasma Factor VIII level, strongly supporting the feasibility of treatment of hemophilia A with cutaneous gene therapy approach.

A Factor VIII expression gene with B domain deletion (SEQ ID NO: 58) is envisioned, where the B domain, which is a long internal domain and functionally disposable for blood coagulation is removed. The transfection targeting vector is envisioned to further incorporate a Tet-inducible system to control excessive expression of Factor VIII.

Development of an inhibitory antibody against Factor VIII is the most severe complication of current enzyme replacement therapy. If expression of mouse *Factor VIII* in the mutant mice (*Factor VIII-*deficient) induces antibody production, three approaches are employed:
1) Instead of B domain-deleted *Factor VIII,* a full length *Factor VIII* (SEQ ID NO: 59) for cutaneous gene therapy is used. Although B domain is not essential for blood coagulation, meta-analysis of prospective clinical results suggests that deletion of B domain can shorten the half-life of recombinant Factor VIII, increase bleeding incidence in patients, and significantly increase the risk for development of neutralizing antibody.
2) The Fc domain of immunoglobulin (mouse IgG1 Fc (SEQ ID NO: 34) or human IgG1 Fc (SEQ ID NO: 39)) is conjugated with *Factor VIII.* The Fc domain of IgG can interact with neonatal Fc receptor, which can protect IgG from catabolism and elongate its half-life in circulation. It has been shown that fusion of human *Factor VIII* with IgG1 Fc domain can significantly increase its half-life in patients. In addition, current clinical results suggest that Fc conjugation of Factor VIII will not induce neutralizing antibody production in patients. Consistently, it has been shown that the Fc-Factor VIII conjugate elicits much lower immune reaction compared to non-conjugated Factor VIII in hemophilia A mouse model.
3) *Factor VIII* is coupled with albumin (SEQ ID NO: 60). Albumin is the most abundant plasma protein and a natural carrier for a variety of molecules in circulation. Albumin has very long half-life in blood and low immunogenicity. Hence, conjugation with albumin will likely enhance the effectiveness of cutaneous gene therapy for hemophilia A. It has been demonstrated that albumin conjugation with Factor IX (SEQ ID NO: 61) can significantly protect Factor IX in circulation and reduce its immunogenicity. Although fusion of Factor VIII with albumin has not been successfully pursued before, different strategies of fusion are employed (e.g., conjugation to amino- or carboxyl-terminal of *Factor VIII* and/or using different linker peptide for fusion) and effects *in vitro* and *in vivo* are examined.

For Hemophilia B, similar approaches are taken as those described above using Factor IX.

### Example No. 10: Evaluating Immune Response on Skin Grafting

Development of skin engraftment procedures with immunocompetent mice provides a unique opportunity to evaluate potential immune responses after cutaneous gene therapy. To examine potential inflammatory infiltration *in vivo* upon tissue organoid engraftment, grafted skin tissues are collected at different time points post engraftment. Potential infiltration of immune cells are assessed by immunofluorescence staining with α-CD3, α-CD4, α-CD8 (T cell), α-B220 (B cell), α-CD11c (dendritic cell), α-Mac-1 (macrophage), α-CD335 (NK cells), and α-Gr1 (granulocytes) antibodies. The presence of Langerhans cells in the skin grafts is determined by staining with an α-CD1a antibody. In addition, skin samples are stained for MHC class I and II antigens, including HLA-ABC and HLA-DR, as indicators for potential graft rejection, tissue antigenicity, and epidermal reactivity.

As a secondary approach, immune responses are assessed by flow cytometry (florescence-activated cell sorting), which allows for a more comprehensive and quantitative analysis of immune cell populations. For this purpose, specific panels of antibodies are developed to more closely examine the phenotypes of immune cells that are changing in response to engraftment. These experiments are guided by results from immunofluorescence staining of sections. For example, if differences in the number and/or location of macrophages in stainings are seen, then a 'macrophage panel' is set up that allows determination of whether the recruited macrophages adopt a pro-inflammatory M1 (CD38, CD274, CD319) or an antiinflammatory M2 (CD206, CD163, TFRC) phenotype using surface markers. Similarly, if the staining experiments indicate a T-cell response, then a 'T cell panel' comprised of CD3 (all T cells), CD4 (helper T cell), CD8 (killer T cell), FoxP3/ CD25 (regulatory T cell), and CD44/CD62L (activated T cell) is implemented. Understanding the precise nature of the immune response (*i.e.*, immunogenic vs. tolerogenic) is required to determine whether the host response to engraftment requires modulation.

To determine whether engraftment of modified-epidermis can increase the host white blood cells specific for grafted keratinocytes, *in vitro* cellular proliferation assay are carried out. Peripheral blood mononuclear cells (PBMC) are isolated before and after skin transplantation, and assessed for cellular proliferation assay with either irradiated autologous PBMC or irradiated epidermal progenitor cells with *modified expression.* Phytohemagglutinin is used as a positive control.

To examine NK (natural killer) cells activity toward epidermal progenitor cells, PBMC isolated from grafted animals are used. An *in vitro* cytotoxicity assay is performed to determine the activity of NK cells at different effector: target ratios. A NK-sensitive cell line is used as a positive control.

To examine the immunogenicity of the modified protein, blood samples from grafted animals at different time points (up to 6 months) are collected. Serum antibody levels are determined by ELISA (enzyme-linked immunosorbent assay) analysis. As a positive control, an intravenous injection of the recombinant modified protein is injected into the mice.

### Example No. 11: Removal of skin grafts non-surgically

Biointegrated tissue organoids can be for temporary use and removed surgically. However, skin stem cell technology permits another non-invasive and effective way to achieve clearance of grafted cells.

To test this possibility, a Rosa26 targeting vector encoding phenylalanine ammonia lyase (*PAL*) (SEQ ID NO: 51) together with inducible "suicide" genes *HSV-TK* (Herpes Simplex Virus Thymidine Kinase) (SEQ ID NO: 52) and yCD (yeast cytosine deaminase) (SEQ ID NO: 53) was used for epidermal progenitor cell transfection. Results (data not shown) indicate that both suicide genes were expressed safely in epidermal progenitor cells, and efficiently induced cell death upon treatment with prodrugs (ganciclovir and 5-fluorocytosine, respectively).

A potential issue is that high level of suicide gene expression (*TK* or *yCD*) may lead to bystander effect that causes cell death in the surrounding tissue. To prevent this, a Tet promoter can be used to drive the suicide gene expression resulting in suicide gene expression levels being controlled by administration of doxycycline.

Therefore, tissue organoids are contemplated that encode a reporter molecule and/or a therapeutic agent along with an inducible suicide gene to remove the biointegrated tissue organoid by non-surgical means.

### Example No. 12: Tissue Organoids for treating obesity

Recent reports have shown that nearly 40% of American adults and approximately 20% of children are obese. Peptide YY (PYY) (SEQ ID NO: 66) is associated with hunger and satiation and is released from cells in the ileum and colon in response to feeding. Increased levels of PYY are believed to be associated with satiation. The administration of PYY, in accordance with the present disclosure, may allow those that suffer from obesity to decrease their food intake, thereby making it possible for them to not only lose weight, but maintain weight loss.

Therefore, a tissue organoid engineered to treat and/or prevent obesity is prepared as described herein using a targeting vector encoding PYY (SEQ ID NO: 66). It is anticipated that increased PYY levels will decrease craving in individuals receiving the PYY-expressing tissue organoid. As a result, it is anticipated that the individuals will experience decreased food seeking and consumption thereby treating and/or preventing obesity.

### Example No. 13: Tissue Organoids for Anti-Aging Effects

The global anti-aging market is estimated to be $250 Billion. Effects of aging include, but are not limited to, vascular and heart diseases and decreases in immune response. Tissue inhibitor of metalloproteinases 2 (TIMP2) (SEQ ID NO: 67) encodes a protein that is a natural inhibitor of matrix metalloproteinases (MMP). TIMP2 is found in high concentrations very early in age but declines with age. Memory studies with mice have shown that older mice perform comparably with younger mice following injections with TIMP2 (Castellano et al., Nature, 544, 488-492 (2017)). Controlled administration of TIMP2 may have anti-aging effects such as memory improvement.

Therefore, a tissue organoid engineered to treat and/or prevent the effects of aging is prepared as described herein using a targeting vector encoding TIMP2 (SEQ ID NO: 67). It is anticipated that increased TIMP2 levels will exhibit anti-aging effects in individuals receiving the TIMP2-expressing tissue organoid. As a result, it is anticipated that treated individuals can experience a decrease in the negative effects of aging, which can include memory loss, reduced vascular response, and/or reduced immune response.

### Example No. 14: Tissue Organoids for Nicotine Abuse

Similarly, tobacco use is a major cause of death from cardiovascular disease, pulmonary disease and cancer (Nature, 362, 2295-2303 (2010)). The Center for Disease Control claims that nearly 480,000 people die each year from tobacco-related deaths. It has been long determined that nicotine is responsible for the additive properties of tobacco. Nicotine replacement therapies which include the transdermal nicotine patch and nicotine gum were the first FDA approved treatments for use in smoking cessation. While nicotine replacement is commonly used, other forms of treatment are available which include antibdepressant bupropion (National Institute on Drug Abuse, Tobacco/Nicotine, Research Report Series (2012). Despite the numerous treatments available, nicotine abuse still remains prevalent. Egecioglu *et al.,* reported that GLP-1 receptor agonist, Exendin-4 (Ex4) attenuates nicotine-induced locomotor stimulation, accumbal dopamine release, and the expression of conditioned place preference in mice (Egecioglu *et al.,* 2013). Therefore, it is believed that tissue organoids expressing mGLP1 can also be used to treat nicotine addiction.

### EXPERIMENTAL PROCEDURES

Unless indicated to the contrary, reagents and experimental procedures follow those described in Example No. 2 above.

### Modified GLP1

The GLP1 gene (*mGLP1*) was modified to produce a novel protein with longer half-life *in vivo* (Kumar et al., Gene therapy of diabetes using a novel GLP-1/IgG1-Fc fusion construct normalizes glucose levels in db/db mice. Gene therapy 14, 162-172, (2007)). To be able to stably deliver *mGLP1* in *vivo* and control its expression, we made a Rosa26-targeting vector encoding a Gly8-mutant mGLP1 and mouse IgG-Fc fragment fusion protein (SEQ ID NO: 45) driven by a dox-dependent promoter (FIG. 25A; Yue et al. Treatment of diabetes and obesity with CRISPR-mediated genome editing in epidermal progenitor cells. Cell Stem Cell, In press (2017); Kumar *et a*/*.,* 2007). The glycine mutation renders the peptide resistant to DPP-IV-mediated cleavage (Mentlein et al., Dipeptidyl-peptidase IV hydrolyses gastric inhibitory polypeptide, glucagon-like peptide-1 (7-36) amide, peptide histidine methionine and is responsible for their degradation in human serum. Eur. J. Biochem. 214, 829-835 (1993)).

Fusion with IgG-Fc further enhances mGLP1 stability and circulation half-life when expressed in epidermal cells (Kumar *et al.,* 2007), and it can pass the blood-brain barrier to reach the brain (Pardridge, W.M., CSF, blood-brain barrier, and brain drug delivery. Expert Opin. Drug Deliv. 13, 963-975 (2016)). The Rosa26 allele has been widely used as a safe locus for gene targeting in mice (Soriano, P., Generalized lacZ expression with the ROSA26 Cre reporter strain. Nat. Genetics 21, 70-71, (1999)). To use CRISPR-mediated genome editing, vectors encoding the D10A mutant Cas9 (CRISPR associated protein 9) (Ran *et a*/*.,* 2013) and two gRNA (guide RNA) targeting the mouse Rosa26 allele were developed. The D10A mutation in Cas9 and double nicking system reduce the undesired off-target mutagenesis in the genome to a minimum level (Ran *et al.,* 2013).

### Skin organoid culture and transplantation

The epidermal progenitor cells were isolated from newborn pups of CD1 or C57BL/6J mice. Cells were transfected with the targeting vector and plasmids encoding Cas9 and Rosa26-specific gRNAs. Targeted clones were selected in the medium containing puromycin, and correct incorporation into the Rosa26 locus was confirmed by PCR and southern blots (FIG. 25B). Mouse skin substitute was prepared by seeding the targeted cells to the acellularized newborn dermis and differentiation upon exposure to the air/liquid interphase and the resultant tissues were transplanted to CD1 or C57BL/6J mice (GLP1).

When fed with dox food, mGLP1 mice began to display significantly enhanced levels of mGLP1 in the blood within 3 days (FIG. 25C; Yue *et al.,* 2017). There was a dose-dependent release of GLP1 in plasma (not shown). Expression of mGLP1 in mGLP1 mice was stable for up to 4 months in the presence of dox (FIG. 25D).

### RESULTS AND DISCUSSION

GLP1 expression attenuated nicotine-induced CPP in GLP1 mice (FIG. 29) compared to mock grafted (GWT).

GLP1 mice did not exhibit significant nicotine-induced CPP. Following 2 free explorations (Pre-test) on day 1, separate groups of mGLP1 and GWT (n= 7 each) received alternative nicotine (0.4 mg/kg) and saline i.p. injections twice daily for the next 4 days, as previously described (Chen et al., Dopamine D1 and D3 receptors are differentially involved in cue-elicited cocaine seeking. J. Neurochem. 114, 530-541 (2010); Kong et al., Activation of dopamine D3 receptors inhibits reward-related learning induced by cocaine. Neurosci. 176, 152-161 (2011)). CPP expression was tested on day 6. Results represent mean ± SEM time spent on the drug-paired side minus the saline-paired side. Repeated-measures ANOVA with test days as the within group factor and status of grafting as the between-subject factor were used (Chen *et al.,* 2010; Kong *et al.,* 2011). F value was calculated and Newman-Keuls post-hoc test was performed (Chen *et a*/*.,* 2010; Kong *et a*/*.,* 2011). GLP1 and GWT mice were on dox food for the entire duration.

These results demonstrate that *mGLP1* expression attenuated nicotine-induced CPP. Therefore, tissue organoids expressing mGLP1 have the potential for treating nicotine abuse. Moreover, it is believed that tissue organoids designed to express multiple therapeutic agents, such as mGLP1 and hBChE can be used to reduce incidents of cocaine and ethanol and/or nicotine co-abuse and potentially reduce abuse and co-abuse of other drugs, such as amphetamines (see Skibicka K.P. The central GLP-1: implications for food and drug reward, Front Neurosci. 7:181 (2013)). It is also contemplated herein to that GLP-1 analogs (see above) can be employed in a similar fashion.

**Sequences**

| | |
|---|---|
| SEQ ID NO: 1 | Luciferase |
| SEQ ID NO: 2 | H2B-RFP |
| SEQ ID NO: 3 | gRNA expression cassette forward primer |
| SEQ ID NO: 4 | gRNA expression cassette reverse primer |
| SEQ ID NO: 5 | *Rosa*26-targeting gRNA forward primer 1 |
| SEQ ID NO: 6 | *Rosa*26-targeting gRNA reverse primer 1 |
| SEQ ID NO: 7 | *Rosa*26-targeting gRNA forward primer 2 |
| SEQ ID NO: 8 | *Rosa*26-targeting gRNA reverse primer 2 |
| SEQ ID NO: 9 | *AAVS1*-targeting gRNA forward primer 1 |
| SEQ ID NO: 10 | *AAVS1*-targeting gRNA reverse primer 1 |
| SEQ ID NO: 11 | *AAVS1*-targeting gRNA forward primer 2 |
| SEQ ID NO: 12 | *AAVS1*-targeting gRNA reverse primer 2 |
| SEQ ID NO: 13 | Rosa26 targeting vector forward primer 1 |
| SEQ ID NO: 14 | Rosa26 targeting vector reverse primer 1 |
| SEQ ID NO: 15 | Rosa26 targeting vector forward primer 2 |
| SEQ ID NO: 16 | *Rosa26* targeting vector reverse primer 2 |
| SEQ ID NO: 17 | Rosa26 targeting vector forward primer 3 |
| SEQ ID NO: 18 | Rosa26 targeting vector reverse primer 3 |
| SEQ ID NO: 19 | *AAVS1* targeting vector constructed with AAVS1 hPGK-PuroR-pA donor forward primer 1 |
| SEQ ID NO: 20 | *AAVS1* targeting vector constructed with AAVS1 hPGK-PuroR-pA donor reverse primer 1 |
| SEQ ID NO: 21 | *AAVS1* targeting vector constructed with AAVS1 hPGK-PuroR-pA donor forward primer 2 |
| SEQ ID NO: 22 | *AAVS1* targeting vector constructed with AAVS1 hPGK-PuroR-pA donor reverse primer 2 |
| SEQ ID NO: 23 | *AAVS1* targeting vector constructed with AAVS1 hPGK-PuroR-pA donor forward primer 3 |
| SEQ ID NO: 24 | *AAVS1* targeting vector constructed with AAVS1 hPGK-PuroR-pA donor reverse primer 3 |
| SEQ ID NO: 25 | Genotyping primer for CRISPR mediated knockin #1 |
| SEQ ID NO: 26 | Genotyping primer for CRISPR mediated knockin #2 |
| SEQ ID NO: 27 | Genotyping primer for CRISPR mediated knockin #3 |
| SEQ ID NO: 28 | CFP/YFP FRET sensor with A213R/L238S double mutant of GGBP |
| SEQ ID NO: 29 | Cas9 D10A |
| SEQ ID NO: 30 | Rosa26 qRNA1 |
| SEQ ID NO: 31 | Rosa26 gRNA2 |
| SEQ ID NO: 32 | Rosa26 targeting vector |
| SEQ ID NO: 33 | Rosa26 targeting vector with GGBP |
| SEQ ID NO: 34 | IqG Fc (mouse) |
| SEQ ID NO: 35 | S2-VaTx3 with siqnal |
| SEQ ID NO: 36 | S2-DkTx with siqnal |
| SEQ ID NO: 37 | VatX3 target vector cassette: signal->VaTx3->Furin->IgGFc |
| SEQ ID NO: 38 | DkTx target vector cassette: signal->DkTx->Furin->IqGFc |
| SEQ ID NO: 39 | IgG Fc (human) |
| SEQ ID NO: 40 | AAVSI targeting vector |
| SEQ ID NO: 41 | Human mutant BChE |
| SEQ ID NO: 42 | Nucleic acid sequence encoding the amino acid sequence of SEQ ID NO: 41 |
| SEQ ID NO: 43 | Mouse mutant BChE |
| SEQ ID NO: 44 | Nucleic acid sequence encoding the amino acid sequence of SEQ ID NO: 43 |
| SEQ ID NO: 45 | mGLP-1 (GLY8 mutant with IgG-Fc fusion) |
| SEQ ID NO: 46 | Nucleic acid sequence encoding the amino acid sequence of SEQ ID NO: 45 |
| SEQ ID NO: 47 | Primer A |
| SEQ ID NO: 48 | Primer B |
| SEQ ID NO: 49 | Primer C |
| SEQ ID NO: 50 | Primer D |
| SEQ ID NO: 51 | PAL |
| SEQ ID NO: 52 | HSV-TK |
| SEQ ID NO: 53 | yCD |
| SEQ ID NO: 54 | PAH |
| SEQ ID NO: 55 | GTPCH |
| SEQ ID NO: 56 | PTPS |
| SEQ ID NO: 57 | SR (sepiapterin reductase) |
| SEQ ID NO: 58 | Factor VIII minus B |
| SEQ ID NO: 59 | Factor VIII |
| SEQ ID NO: 60 | Albumin |
| SEQ ID NO: 61 | Factor IX |
| SEQ ID NO: 62 | H-FABP |
| SEQ ID NO: 63 | Myocardial myoglobin |
| SEQ ID NO: 64 | GFAP |
| SEQ ID NO: 65 | S100B |
| SEQ ID NO: 66 | PYY (Homo sapiens) |
| SEQ ID NO: 67 | TIMP2 (Homo sapiens) |

| **Name** | **Seguence** |
|---|---|
| SEQ ID NO: 1 | ATGCCAGAGCCAGCGAAGTCTGCTCCCGCCCCGAAAAAGGGCTCC AAGAAGGCGGTGACTAAGGCGCAGAAGAAAGGCGGCAAGAAGCGC AAGCGCAGCCGCAAGGAGAGCTATTCCATCTATGTGTACAAGGTTC TGAAGCAGGTCCACCCTGACACCGGCATTTCGTCCAAGGCCATGGG CATCATGAACTCGTTTGTGAACGACATTTTCGAGCGCATCGCAGGTG AGGCTTCCCGCCTGGCGCATTACAACAAGCGCTCGACCATCACCTC CAGGGAGATCCAGACGGCCGTGCGCCTGCTGCTGCCTGGGGAGTT GGCCAAGCACGCCGTGTCCGAGGGTACTAAGGCCATCACCAAGTAC ACCAGCGCTAAGGATCCACCGGTCGCCACCATGGCCTCCTCCGAG GACGTCATCAAGGAGTTCATGCGCTTCAAGGTGCGCATGGAGGGCT CCGTGAACGGCCACGAGTTCGAGATCGAGGGCGAGGGCGAGGGC CGCCCCTACGAGGGCACCCAGACCGCCAAGCTGAAGGTGACCAAG GGCGGCCCCCTGCCCTTCGCCTGGGACATCCTGTCCCCTCAGTTCC AGTACGGCTCCAAGGCCTACGTGAAGCACCCCGCCGACATCCCCG ACTACTTGAAGCTGTCCTTCCCCGAGGGCTTCAAGTGGGAGCGCGT GATGAACTTCGAGGACGGCGGCGTGGTGACCGTGACCCAGGACTC CTCCCTGCAGGACGGCGAGTTCATCTACAAGGTGAAGCTGCGCGG CACCAACTTCCCCTCCGACGGCCCCGTAATGCAGAAGAAGACCATG GGCTGGGAGGCCTCCACCGAGCGGATGTACCCCGAGGACGGCGC CCTGAAGGGCGAGATCAAGATGAGGCTGAAGCTGAAGGACGGCGG CCACTACGACGCCGAGGTCAAGACCACCTACATGGCCAAGAAGCCC GTGCAGCTGCCCGGCGCCTACAAGACCGACATCAAGCTGGACATCA CCTCCCACAACGAGGACTACACCATCGTGGAACAGTACGAGCGCGC CGAGGGCCGCCACTCCACCGGCGCC |
| SEQ ID NO: 2 | ATGGAAGACGCCAAAAACATAAAGAAAGGCCCGGCGCCATTCTATC CGCTGGAAGATGGAACCGCTGGAGAGCAACTGCATAAGGCTATGAA GAGATACGCCCTGGTTCCTGGAACAATTGCTTTTACAGATGCACATA TCGAGGTGGACATCACTTACGCTGAGTACTTCGAAATGTCCGTTCG GTTGGCAGAAGCTATGAAACGATATGGGCTGAATACAAATCACAGAA TCGTCGTATGCAGTGAAAACTCTCTTCAATTCTTTATGCCGGTGTTG GGCGCGTTATTTATCGGAGTTGCAGTTGCGCCCGCGAACGACATTT ATAATGAACGTGAATTGCTCAACAGTATGGGCATTTCGCAGCCTACC GTGGTGTTCGTTTCCAAAAAGGGGTTGCAAAAAATTTTGAACGTGCA AAAAAAGCTCCCAATCATCCAAAAAATTATTATCATGGATTCTAAAAC GGATTACCAGGGATTTCAGTCGATGTACACGTTCGTCACATCTCATC TACCTCCCGGTTTTAATGAATACGATTTTGTGCCAGAGTCCTTCGAT AGGGACAAGACAATTGCACTGATCATGAACTCCTCTGGATCTACTGG TCTGCCTAAAGGTGTCGCTCTGCCTCATAGAACTGCCTGCGTGAGA TTCTCGCATGCCAGAGATCCTATTTTTGGCAATCAAATCATTCCGGA TACTGCGATTTTAAGTGTTGTTCCATTCCATCACGGTTTTGGAATGTT TACTACACTCGGATATTTGATATGTGGATTTCGAGTCGTCTTAATGTA TAGATTTGAAGAAGAGCTGTTTCTGAGGAGCCTTCAGGATTACAAGA TTCAAAGTGCGCTGCTGGTGCCAACCCTATTCTCCTTCTTCGCCAAA |
| | AGCACTCTGATTGACAAATACGATTTATCTAATTTACACGAAATTGCT TCTGGTGGCGCTCCCCTCTCTAAGGAAGTCGGGGAAGCGGTTGCCA AGAGGTTCCATCTGCCAGGTATCAGGCAAGGATATGGGCTCACTGA GACTACATCAGCTATTCTGATTACACCCGAGGGGGATGATAAACCG GGCGCGGTCGGTAAAGTTGTTCCATTTTTTGAAGCGAAGGTTGTGG ATCTGGATACCGGGAAAACGCTGGGCGTTAATCAAAGAGGCGAACT GTGTGTGAGAGGTCCTATGATTATGTCCGGTTATGTAAACAATCCGG AAGCGACCAACGCCTTGATTGACAAGGATGGATGGCTACATTCTGG AGACATAGCTTACTGGGACGAAGACGAACACTTCTTCATCGTTGACC GCCTGAAGTCTCTGATTAAGTACAAAGGCTATCAGGTGGCTCCCGC TGAATTGGAATCCATCTTGCTCCAACACCCCAACATCTTCGACGCAG GTGTCGCAGGTCTTCCCGACGATGACGCCGGTGAACTTCCCGCCG CCGTTGTTGTTTTGGAGCACGGAAAGACGATGACGGAAAAAGAGAT CGTGGATTACGTCGCCAGTCAAGTAACAACCGCGAAAAAGTTGCGC GGAGGAGTTGTGTTTGTGGACGAAGTACCGAAAGGTCTTACCGGAA AACTCGACGCAAGAAAAATCAGAGAGATCCTCATAAAGGCCAAGAA GGGCGGAAAGATCGCCG |
| SEQ ID NO: 3 | AAG GAA AAA AGC GGC CGC TGT ACA AAA AAG CAG G |
| SEQ ID NO: 4 | gGA ATT CTA ATG CCA ACT TTG TAC |
| SEQ ID NO: 5 | ACA CCG GCA GGC TTA AAG GCT AAC CG |
| SEQ ID NO: 6 | AAA ACG GTT AGC CTT TAA GCC TGC CG |
| SEQ ID NO: 7 | ACA CCG AGG ACA ACG CCC ACA CAC Cg |
| SEQ ID NO: 8 | AAA ACG GTG TGT GGG CGT TGT CCT CG |
| SEQ ID NO: 9 | ACA CCG TCA CCA ATC CTG TCC CTA GG |
| SEQ ID NO: 10 | AAA ACC TAG GGA CAG GAT TGG TGA CG |
| SEQ ID NO: 11 | ACA CCG CCC CAC AGT GGG GCC ACT AG |
| SEQ ID NO: 12 | AAA ACT AGT GGC CCC ACT GTG GGG CG |
| SEQ ID NO: 13 | GAC TAG TGA ATT CGG ATC CTT AAT TAA GGC CTC CGC GCC GGG TTT TGG CG |
| SEQ ID NO: 14 | GAC TAG TCC CGG GGG ATC CAC CGG TCA GGA ACA GGT GGT GGC GGC CC |
| SEQ ID NO: 15 | CGG GAT CCA CCG GTG AGG GCA GAG GAA GCC TTC TAA C |
| SEQ ID NO: 16 | TCC CCC GGG TAC AAA ATC AGA AGG ACA GGG AAG |
| SEQ ID NO: 17 | GGA ATT CAA TAA AAT ATC TTT ATT TTC ATT ACA TC |
| SEQ ID NO: 18 | CCT TAA TTA AGG ATC CAC GCG TGT TTA AAC ACC GGT TTT ACG AGG GTA GGA AGT GGT AC |
| SEQ ID NO: 19 | CCC AAG CTT CTC GAG TTG GGG TTG CGC CTT TTC CAA G |
| SEQ ID NO: 20 | CCC AAG CTT CCA TAG AGC CCA CCG CAT CCC C |
| SEQ ID NO: 21 | CAG GGT CTA GAC GCC GGA TCC GGT ACC CTG TGC CTT CTA GTT GC |
| SEQ ID NO: 22 | GGA TCC GGC GTC TAG ACC CTG GGG AGA GAG GTC GGT G |
| SEQ ID NO: 23 | CCG CTC GAG AAT AAA ATA TCT TTA TTT TCA TTA CAT C |
| SEQ ID NO: 24 | GCT CTA GAC CAA GTG ACG ATC ACA GCG ATC |
| SEQ ID NO: 25 | GAG CTG GGA CCA CCT TAT ATT C |
| SEQ ID NO: 26 | GGT GCA TGA CCC GCA AG |
| SEQ ID NO: 27 | GAG AGA TGG CTC CAG GAA ATG |
| SEQ ID NO: 28 | ATGGTGAGCAAGGGCGAGGAGCTGTTCACCGGGGTGGTGCCCATC CTGGTCGAGCTGGACGGCGACGTAAACGGCCACAAGTTCAGCGTG TCCGGCGAGGGCGAGGGCGATGCCACCTACGGCAAGCTGACCCTG AAGTTCATCTGCACCACCGGCAAGCTGCCCGTGCCCTGGCCCACCC TCGTGACCACCCTGACCTGGGGCGTGCAGTGCTTCAGCCGCTACCC CGACCACATGAAGCAGCACGACTTCTTCAAGTCCGCCATGCCCGAA |
| | GGCTACGTCCAGGAGCGCACCATCTTCTTCAAGGACGACGGCAACT ACAAGACCCGCGCCGAGGTGAAGTTCGAGGGCGACACCCTGGTGA ACCGCATCGAGCTGAAGGGCATCGACTTCAAGGAGGACGGCAACAT CCTGGGGCACAAGCTGGAGTACAACTACATCAGCCACAACGTCTAT ATCACCGCCGACAAGCAGAAGAACGGCATCAAGGCCAACTTCAAGA TCCGCCACAACATCGAGGACGGCAGCGTGCAGCTCGCCGACCACT ACCAGCAGAACACCCCCATCGGCGACGGCCCCGTGCTGCTGCCCG ACAACCACTACCTGAGCACCCAGTCCGCCCTGAGCAAAGACCCCAA CGAGAAGCGCGATCACATGGTCCTGCTGGAGTTCGTGACCGCCGC CGGGATCACTCTCGGCATGGACGAGCTGTACAAGGGTGGTACCGG AGGCGCCGCTGATACTCGCATTGGTGTAACAATCTATAAGTACGAC GATAACTTTATGTCTGTAGTGCGCAAGGCTATTGAGCAAGATGCGAA AGCCGCGCCAGATGTTCAGCTGCTGATGAATGATTCTCAGAATGAC CAGTCCAAGCAGAACGATCAGATCGACGTATTGCTGGCGAAAGGGG TGAAGGCACTGGCAATCAACCTGGTTGACCCGGCAGCTGCGGGTAC GGTGATTGAGAAAGCGCGTGGGCAAAACGTGCCGGTGGTTTTCTTC AACAAAGAACCGTCTCGTAAGGCGCTGGATAGCTACGACAAAGCCT ACTACGTTGGCACTGACTCCAAAGAGTCCGGCATTATTCAAGGCGAT TTGATTGCTAAACACTGGGCGGCGAATCAGGGTTGGGATCTGAACA AAGACGGTCAGATTCAGTTCGTACTGCTGAAAGGTGAACCGGGCCA TCCGGATGCAGAAGCACGTACCACTTACGTGATTAAAGAATTGAACG ATAAAGGCATCAAAACTGAACAGTTACAGTTAGATACCGCAATGTGG GACACCGCTCAGGCGAAAGATAAGATGGACGCCTGGCTGTCTGGC CCGAACGCCAACAAAATCGAAGTGGTTATCGCCAACAACGATcgGAT GGCAATGGGCGCGGTTGAAGCGCTGAAAGCACACAACAAGTCCAG CATTCCGGTGTTTGGCGTCGATGCGtcGCCAGAAGCGCTGGCGCTG GTGAAATCCGGTGCACTGGCGGGCACCGTACTGAACGATGCTAACA ACCAGGCGAAAGCGACCTTTGATCTGGCGAAAAACCTGGCCGATGG TAAAGGTGCGGCTGATGGCACCAACTGGAAAATCGACAACAAAGTG GTCCGCGTACCTTATGTTGGCGTAGATAAAGACAACCTGGCTGAATT CAGCAAGAAAGGCGCCGGTACCGGTGGAATGGTGAGCAAGGGCGA GGAGCTGTTCACCGGGGTGGTGCCCATCCTGGTCGAGCTGGACGG CGACGTAAACGGCCACAAGTTCAGCGTGTCCGGCGAGGGCGAGGG CGATGCCACCTACGGCAAGCTGACCCTGAAGTTCATCTGCACCACC GGCAAGCTGCCCGTGCCCTGGCCCACCCTCGTGACCACCTTCGGC TACGGCCTGCAGTGCTTCGCCCGCTACCCCGACCACATGAAGCAGC ACGACTTCTTCAAGTCCGCCATGCCCGAAGGCTACGTCCAGGAGCG CACCATCTTCTTCAAGGACGACGGCAACTACAAGACCCGCGCCGAG GTGAAGTTCGAGGGCGACACCCTGGTGAACCGCATCGAGCTGAAG GGCATCGACTTCAAGGAGGACGGCAACATCCTGGGGCACAAGCTG GAGTACAACTACAACAGCCACAACGTCTATATCATGGCCGACAAGCA GAAGAACGGCATCAAGGTGAACTTCAAGATCCGCCACAACATCGAG GACGGCAGCGTGCAGCTCGCCGACCACTACCAGCAGAACACCCCC ATCGGCGACGGCCCCGTGCTGCTGCCCGACAACCACTACCTGAGC TACCAGTCCGCCCTGAGCAAAGACCCCAACGAGAAGCGCGATCACA TGGTCCTGCTGGAGTTCGTGACCGCCGCCGGGATCACTCTCGGCAT GGACGAGCTG |
| SEQ ID NO: 29 | GCCACCATGGACAAGAAGTACTCCATTGGGCTCGCTATCGGCACAA ACAGCGTCGGCTGGGCCGTCATTACGGACGAGTACAAGGTGCCGA GCAAAAAATTCAAAGTTCTGGGCAATACCGATCGCCACAGCATAAAG AAGAACCTCATTGGCGCCCTCCTGTTCGACTCCGGGGAGACGGCC |
| | GAAGCCACGCGGCTCAAAAGAACAGCACGGCGCAGATATACCCGC AGAAAGAATCGGATCTGCTACCTGCAGGAGATCTTTAGTAATGAGAT GGCTAAGGTGGATGACTCTTTCTTCCATAGGCTGGAGGAGTCCTTTT TGGTGGAGGAGGATAAAAAGCACGAGCGCCACCCAATCTTTGGCAA TATCGTGGACGAGGTGGCGTACCATGAAAAGTACCCAACCATATATC ATCTGAGGAAGAAGCTTGTAGACAGTACTGATAAGGCTGACTTGCG GTTGATCTATCTCGCGCTGGCGCATATGATCAAATTTCGGGGACACT TCCTCATCGAGGGGGACCTGAACCCAGACAACAGCGATGTCGACAA ACTCTTTATCCAACTGGTTCAGACTTACAATCAGCTTTTCGAAGAGAA CCCGATCAACGCATCCGGAGTTGACGCCAAAGCAATCCTGAGCGCT AGGCTGTCCAAATCCCGGCGGCTCGAAAACCTCATCGCACAGCTCC CTGGGGAGAAGAAGAACGGCCTGTTTGGTAATCTTATCGCCCTGTC ACTCGGGCTGACCCCCAACTTTAAATCTAACTTCGACCTGGCCGAA GATGCCAAGCTTCAACTGAGCAAAGACACCTACGATGATGATCTCGA CAATCTGCTGGCCCAGATCGGCGACCAGTACGCAGACCTTTTTTTG GCGGCAAAGAACCTGTCAGACGCCATTCTGCTGAGTGATATTCTGC GAGTGAACACGGAGATCACCAAAGCTCCGCTGAGCGCTAGTATGAT CAAGCGCTATGATGAGCACCACCAAGACTTGACTTTGCTGAAGGCC CTTGTCAGACAGCAACTGCCTGAGAAGTACAAGGAAATTTTCTTCGA TCAGTCTAAAAATGGCTACGCCGGATACATTGACGGCGGAGCAAGC CAGGAGGAATTTTACAAATTTATTAAGCCCATCTTGGAAAAAATGGA CGGCACCGAGGAGCTGCTGGTAAAGCTTAACAGAGAAGATCTGTTG CGCAAACAGCGCACTTTCGACAATGGAAGCATCCCCCACCAGATTC ACCTGGGCGAACTGCACGCTATCCTCAGGCGGCAAGAGGATTTCTA CCCCTTTTTGAAAGATAACAGGGAAAAGATTGAGAAAATCCTCACAT TTCGGATACCCTACTATGTAGGCCCCCTCGCCCGGGGAAATTCCAG ATTCGCGTGGATGACTCGCAAATCAGAAGAGACCATCACTCCCTGG AACTTCGAGGAAGTCGTGGATAAGGGGGCCTCTGCCCAGTCCTTCA TCGAAAGGATGACTAACTTTGATAAAAATCTGCCTAACGAAAAGGTG CTTCCTAAACACTCTCTGCTGTACGAGTACTTCACAGTTTATAACGA GCTCACCAAGGTCAAATACGTCACAGAAGGGATGAGAAAGCCAGCA TTCCTGTCTGGAGAGCAGAAGAAAGCTATCGTGGACCTCCTCTTCAA GACGAACCGGAAAGTTACCGTGAAACAGCTCAAAGAAGACTATTTCA AAAAGATTGAATGTTTCGACTCTGTTGAAATCAGCGGAGTGGAGGAT CGCTTCAACGCATCCCTGGGAACGTATCACGATCTCCTGAAAATCAT TAAAGACAAGGACTTCCTGGACAATGAGGAGAACGAGGACATTCTT GAGGACATTGTCCTCACCCTTACGTTGTTTGAAGATAGGGAGATGAT TGAAGAACGCTTGAAAACTTACGCTCATCTCTTCGACGACAAAGTCA TGAAACAGCTCAAGAGGCGCCGATATACAGGATGGGGGCGGCTGT CAAGAAAACTGATCAATGGGATCCGAGACAAGCAGAGTGGAAAGAC AATCCTGGATTTTCTTAAGTCCGATGGATTTGCCAACCGGAACTTCA TGCAGTTGATCCATGATGACTCTCTCACCTTTAAGGAGGACATCCAG AAAGCACAAGTTTCTGGCCAGGGGGACAGTCTTCACGAGCACATCG CTAATCTTGCAGGTAGCCCAGCTATCAAAAAGGGAATACTGCAGACC GTTAAGGTCGTGGATGAACTCGTCAAAGTAATGGGAAGGCATAAGC CCGAGAATATCGTTATCGAGATGGCCCGAGAGAACCAAACTACCCA GAAGGGACAGAAGAACAGTAGGGAAAGGATGAAGAGGATTGAAGA GGGTATAAAAGAACTGGGGTCCCAAATCCTTAAGGAACACCCAGTT GAAAACACCCAGCTTCAGAATGAGAAGCTCTACCTGTACTACCTGCA GAACGGCAGGGACATGTACGTGGATCAGGAACTGGACATCAATCGG CTCTCCGACTACGACGTGGATCATATCGTGCCCCAGTCTTTTCTCAA AGATGATTCTATTGATAATAAAGTGTTGACAAGATCCGATAAAAATAG AGGGAAGAGTGATAACGTCCCCTCAGAAGAAGTTGTCAAGAAAATG |
| | AAAAATTATTGGCGGCAGCTGCTGAACGCCAAACTGATCACACAAC GGAAGTTCGATAATCTGACTAAGGCTGAACGAGGTGGCCTGTCTGA GTTGGATAAAGCCGGCTTCATCAAAAGGCAGCTTGTTGAGACACGC CAGATCACCAAGCACGTGGCCCAAATTCTCGATTCACGCATGAACA CCAAGTACGATGAAAATGACAAACTGATTCGAGAGGTGAAAGTTATT ACTCTGAAGTCTAAGCTGGTCTCAGATTTCAGAAAGGACTTTCAGTT TTATAAGGTGAGAGAGATCAACAATTACCACCATGCGCATGATGCCT ACCTGAATGCAGTGGTAGGCACTGCACTTATCAAAAAATATCCCAAG CTTGAATCTGAATTTGTTTACGGAGACTATAAAGTGTACGATGTTAG GAAAATGATCGCAAAGTCTGAGCAGGAAATAGGCAAGGCCACCGCT AAGTACTTCTTTTACAGCAATATTATGAATTTTTTCAAGACCGAGATT ACACTGGCCAATGGAGAGATTCGGAAGCGACCACTTATCGAAACAA ACGGAGAAACAGGAGAAATCGTGTGGGACAAGGGTAGGGATTTCGC GACAGTCCGGAAGGTCCTGTCCATGCCGCAGGTGAACATCGTTAAA AAGACCGAAGTACAGACCGGAGGCTTCTCCAAGGAAAGTATCCTCC CGAAAAGGAACAGCGACAAGCTGATCGCACGCAAAAAAGATTGGGA CCCCAAGAAATACGGCGGATTCGATTCTCCTACAGTCGCTTACAGTG TACTGGTTGTGGCCAAAGTGGAGAAAGGGAAGTCTAAAAAACTCAAA AGCGTCAAGGAACTGCTGGGCATCACAATCATGGAGCGATCAAGCT TCGAAAAAAACCCCATCGACTTTCTCGAGGCGAAAGGATATAAAGAG GTCAAAAAAGACCTCATCATTAAGCTTCCCAAGTACTCTCTCTTTGAG CTTGAAAACGGCCGGAAACGAATGCTCGCTAGTGCGGGCGAGCTG CAGAAAGGTAACGAGCTGGCACTGCCCTCTAAATACGTTAATTTCTT GTATCTGGCCAGCCACTATGAAAAGCTCAAAGGGTCTCCCGAAGAT AATGAGCAGAAGCAGCTGTTCGTGGAACAACACAAACACTACCTTGA TGAGATCATCGAGCAAATAAGCGAATTCTCCAAAAGAGTGATCCTCG CCGACGCTAACCTCGATAAGGTGCTTTCTGCTTACAATAAGCACAGG GATAAGCCCATCAGGGAGCAGGCAGAAAACATTATCCACTTGTTTAC TCTGACCAACTTGGGCGCGCCTGCAGCCTTCAAGTACTTCGACACC ACCATAGACAGAAAGCGGTACACCTCTACAAAGGAGGTCCTGGACG CCACACTGATTCATCAGTCAATTACGGGGCTCTATGAAACAAGAATC GACCTCTCTCAGCTCGGTGGAGACAGCAGGGCTGACCCCAAGAAG AAGAGGAAGGTGTGA |
| SEQ ID NO: 30 | GGCAGGCTTAAAGGCTAACCTGG |
| SEQ ID NO: 31 | GACTGGAGTTGCAGATCACGAGG |
| SEQ ID NO: 32 | CCGCGGCAGGCCCTCCGAGCGTGGTGGAGCCGTTCTGTGAGACAG CCGGGTACGAGTCGTGACGCTGGAAGGGGCAAGCGGGTGGTGGG CAGGAATGCGGTCCGCCCTGCAGCAACCGGAGGGGGAGGGAGAA GGGAGCGGAAAAGTCTCCACCGGACGCGGCCATGGCTCGGGGGG GGGGGGGCAGCGGAGGAnCGCTTCCGGCCGACGTCTCGTCGCTGA TTGGCTTnTTTTCCTCCCGCCGTGTGTGAAAACACAAATGGCGTGTT TTGGTTGGCGTAAGGCGCCTGTCAGTTAACGGCAGCCGGAGTGCG CAGCCGCCGGCAGCCTCGCTCTGCCCACTGGGTGGGGCGGGAGG TAGGTGGGGTGAGGCGAGCTGNACGTGCGGGCGCGGTCGGCCTCT GGCGGGGCGGGGGAGGGGAGGGAGGGTCAGCGAAAGTAGCTCGC GCGCGAGCGGCCGCCCACCCTCCCCTTCCTCTGGGGGAGTCGTTT TACCCGCCGCCGGCCGGGCCTCGTCGTCTGATTGGCTCTCGGGGC CCAGAAAACTGGCCCTTGCCATTGGCTCGTGTTCGTGCAAGTTGAG TCCATCCGCCGGCCAGCGGGGGCGGCGAGGAGGCGCTCCCAGGT TCCGGCCCTCCCCTCGGCCCCGCGCCGCAGAGTCTGGCCGCGCGC CCCTGCGCAACGTGGCAGGAAGCGCGCGCTGGGGGCGGGGACGG GCAGTAGGGCTGAGCGGCTGCGGGGCGGGTGCAAGCACGTTTCCG |
| | ACTTGAGTTGCCTCAAGAGGGGCGTGCTGAGCCAGACCTCCATCGC GCACTCCGGGGAGTGGAGGGAAGGAGCGAGGGCTCAGTTGGGCT GTTTTGGAGGCAGGAAGCACTTGCTCTCCCAAAGTCGCTCTGAGTT GTTATCAGTAAGGGAGCTGCAGTGGAGTAGGCGGGGAGAAGGCCG CACCCTTCTCCGGAGGGGGGAGGGGAGTGTTGCAATACCTTTCTGG GAGTTCTCTGCTGCCTCCTGGCTTCTGAGGACCGCCCTGGGCCTGG GAGAATCCCTTGCCCCCTCTTCCCCTCGTGATCTGCAACTCCAGTCT TTCTAGTGAATTCGGATCCTTAATTAAGGCCTCCGCGCCGGGTTTTG GCGCCTCCCGCGGGCGCCCCCCTCCTCACGGCGAGCGCTGCCAC GTCAGACGAAGGGCGCAGCGAGCGTCCTGATCCTTCCGCCCGGAC GCTCAGGACAGCGGCCCGCTGCTCATAAGACTCGGCCTTAGAACCC CAGTATCAGCAGAAGGACATTTTAGGACGGGACTTGGGTGACTCTA GGGCACTGGTTTTCTTTCCAGAGAGCGGAACAGGCGAGGAAAAGTA GTCCCTTCTCGGCGATTCTGCGGAGGGATCTCCGTGGGGCGGTGA ACGCCGATGATTATATAAGGACGCGCCGGGTGTGGCACAGCTAGTT CCGTCGCAGCCGGGATTTGGGTCGCGGTTCTTGTTTGTGGATCGCT GTGATCGTCACTTGGTCTAGACGCCACCATGGTGTCCAAGGGCGAG GAGGTGATCAAGGAGTTCATGCGCTTCAAGGTGCGCATGGAGGGCT CCATGAACGGCCACGAGTTCGAGATCGAGGGCGAGGGCGAGGGCC GCCCCTACGAGGGCACCCAGACCGCCAAGCTGAAGGTGACCAAGG GCGGCCCCCTGCCCTTCGCCTGGGACATCCTGTCCCCCCAGTTCAT GTACGGCTCCAAGGCCTACGTGAAGCACCCCGCCGACATCCCCGA CTACAAGAAGCTGTCCTTCCCCGAGGGCTTCAAGTGGGAGCGCGTG ATGAACTTCGAGGACGGCGGCCTGGTGACCGTGACCCAGGACTCC TCCCTGCAGGACGGCACCCTGATCTACAAGGTGAAGATGCGCGGCA CCAACTTCCCCCCCGACGGCCCCGTGATGCAGAAGAAGACCATGG GCTGGGAGGCCTCCACCGAGCGCCTGTACCCCCGCGACGGCGTGC TGAAGGGCGAGATCCACCAGGCCCTGAAGCTGAAGGACGGCGGCC ACTACCTGGTGGAGTTCAAGACCATCTACATGGCCAAGAAGCCCGT GCAGCTGCCCGGCTACTACTACGTGGACACCAAGCTGGACATCACC TCCCACAACGAGGACTACACCATCGTGGAGCAGTACGAGCGCTCCG AGGGCCGCCACCACCTGTTCCTGACCGGTGAGGGCAGAGGAAGCC TTCTAACATGCGGTGACGTGGAGGAGAATCCCGGCCCTTCCGGGAT GACCGAGTACAAGCCCACGGTGCGCCTCGCCACCCGCGACGACGT CCCCAGGGCCGTACGCACCCTCGCCGCCGCGTTCGCCGACTACCC CGCCACGCGCCACACCGTCGATCCGGACCGCCACATCGAGCGGGT CACCGAGCTGCAAGAACTCTTCCTCACGCGCGTCGGGCTCGACATC GGCAAGGTGTGGGTCGCGGACGACGGCGCCGCGGTGGCGGTCTG GACCACGCCGGAGAGCGTCGAAGCGGGGGCGGTGTTCGCCGAGAT CGGCCCGCGCATGGCCGAGTTGAGCGGTTCCCGGCTGGCCGCGCA GCAACAGATGGAAGGCCTCCTGGCGCCGCACCGGCCCAAGGAGCC CGCGTGGTTCCTGGCCACCGTCGGCGTCTCGCCCGACCACCAGGG CAAGGGTCTGGGCAGCGCCGTCGTGCTCCCCGGAGTGGAGGCGG CCGAGCGCGCCGGGGTGCCCGCCTTCCTGGAGACCTCCGCGCCCC GCAACCTCCCCTTCTACGAGCGGCTCGGCTTCACCGTCACCGCCGA CGTCGAGGTGCCCGAAGGACCGCGCACCTGGTGCATGACCCGCAA GCCCGGTGCCTGAATCTAGGTCGACCTGCAGAAGCTTGCCTCGAGC AGCGCTGCTCGAGAGATCTACGGGTGGCATCCCTGTGACCCCTCCC CAGTGCCTCTCCTGGCCCTGGAAGTTGCCACTCCAGTGCCCACCAG CCTTGTCCTAATAAAATTAAGTTGCATCATTTTGTCTgACTAGGTGTC CTTCTATAATATTATGGGGTGGAGGGGGGTGGTATGGAGCAAGGGG CAAGTTGGGAAGACAACCTGTAGGGCCTGCGGGGTCTATTGGGAAC CAAGCTGGAGTGCAGTGGCACAATCtTGGCTCACTGCAATCTCCGCC |
| | TCCTGGGTTCAAGCGATTCTCCTGCCTCAGCCTCCCGAGTTGTTGG GATTCCAGGCATGCATGACCAGGCTCAGCTAATTTTTGTTTTTTTGG TAGAGACGGGGtTTCACCATATTGGCCAGGCTGGTCTCCAACTCCTA ATCTCAGGTGATCTACCCACCTTGGCCTCCCAAATTGCTGGGATTAC AGGCGTGAACCACTGCTCCCTTCCCTGTCCTTCTGATTTTGTACcCG gGACTAGAAGATGGGCGGGAGTCTTCTGGGCAGGCTTAAAGGCTAA CCTGGTGTGTGGGCGTTGTCCTGCAGGGGAATTGAACAGGTGTAAA ATTGGAGGGACAAGACTTCCCACAGATTTTCGGTTTTGTCGGGAAGT TTTTTAATAGGGGCAAATAGGAAAATGGAGGATAGGAGTCATCTGGG GTTTATGCAGCAAAACTACAGGTATATTGCTTGTATCCGCCTCGGAG ATTTCCATGAGGAGATAAAGACATGTCACCCGAGTTTATACTCTCCT GCTTAGATCCTACTACAGTATGAAATACAGTGTnGCGAGGTAGACTA TGTAAGCAGATTTAATCATTTTAAAGAGCCCAGTACTTCATATCCATT TCTCCCGCTCCTTCTGCAGCCTTATCAAAAGGTATTTAGAACACTCA TTTTAGCCCCATTTTCATTTATTATACTGGCTTATCCAACCCCTAGAC AGAGCATTGGCATTTTCCCTTTCCTGATCTTAGAAGTCTGATGACTC ATGAAACCAGACAGATTAGTTACATACACCACAAATCGAGGCTGTAG CTGGGGCCTCAACACTGCAGTTCTTTTATAACTCCTTAGTACACTTTT TGTTGATCCTTTGCCTTGATCCTTAATTTTCAGTGTCTATCACCTCTC CCGTCAGGTGGTGTTCCACATTTGGGCCTATTCTCAGTCCAGGGAG TTTTACAACAATAGATGTATTGAGAATCCAACCTAAAGCTTAACTTTC CACTCCCATGAATGCCTCTCTCCTTTTTCTCCATTATAACTGAGCTAT nACCATTAATGGTTTCAGGTGGATGTCTCCTCCCCCAATATACCTGA TGTATCTACATATTGCCAGGCTGATATTTTAAGACATnAAAGGTATAT TTCATTATTGAGCCACATGGTATTGATTACTGCTACTAAAATTTTGTC ATTGTACACATCTGTAAAAGGTGGTTCCTTTTGGAATGCAAAGTTCA GGTGTTTGTTGTCTTTCCTGACCTAAGGTCTTGTGAGCTTGTATTTTT TCTATTTAAGCAGTGCTTTCTCTTGGACTGGCTTGACTCATGGCATT CTACACGTTATTGCTGGTCTAAATGTGATTTTGCCAAGCTTCTTCAG GACCTATAATTTTGCTTGACTTGTAGCCAAACACAAGTAAAATGATTA AGCAACAAATGTATTTGTGAAGCTTGGTTTTTAGGTTGTTGTGTTGTG TGTGCTTGTGCTCTATAATAATACTATCCAGGGGCTGGAGAGGTGG CTCGGAGTTCAAGAGCACAGACTGCTCTTCCAGAAGTCCTGAGTTC AATTCCCAGCAACCACATGGTGGCTCACAACCATCTGTAATGGGATC TGATGCCCTCTTCTGGTGTGTCTGAAGACCACAAGTGTATTCACATT AAATAAATAATCCTCCTTCTTCTTCTTTTTTTTTTTTTAAAGAGAATnCT GTCTCCAGTAGAATTACTGAAGTAATGAAATACTTTGTGTTTGTTCCA ATATGGnAGCCAATAATCAAATACTCTTnAGCACTGGAAATGTACCAA GGAACTATTTTATTTAAGTGnACTGTGGACAGAGGAGCCATAACTGC AGACTTGTGGGATACAGAAGACCAATGCAGACTTAATGTCTTTTCTC TTACACTAAGCAATAAAGAAATAAAAATTGAACTTCTAGTATCCTATTT GTTAAACTGCTAGCTTTACTAACTTTTGTGCTTCATCTATACAAAGCT GAAAGCTAAGTCTGCAGCCATTACTAAACATGAAAGCAAGTAATGAT AATTTTGGATTTCAAAAATGTAGGGCCAGAGTTTAGCCAGCCAGTGG TGGTGCTTGCCTTTATGCCTTAATCCCAGCACTCTGGAGGCAGAGA CAGGCAGATCTCTGAGTTTGAGCCCAGCCTGGTCTACACATCAAGTT CTATCTAGGATAGCCAGGAATACACACAGAAACCCTGTTGGGGAGG GGGGCTCTGAGATTTCATAAAATTATAATTGAAGCATTCCCTAATGA GCCACTATGGATGTGGCTAAATCCGTCTACCTTTCTGATGAGATTTG GGTATTATTTTTTCTGTCTCTGCTGTTGGTTGGGTCTTTTGACACTGT GGGCTTTCTTAAAGCCTCCTTCCCTGCCATGTGGTCTCTTGTTTGCT ACTAACTTCCCATGGCTTAAATGGCATGGCTTTTTGCCTTCTAAGGG CAGCTGCTGAGnTTTGCAGCCTGATTTCCAGGGTGGGGTTGGGAAA |
| | TCTTTCAAACACTAAAATTGTCCTTTAATTTTTTTTAAAAAATGGGTT ATATAATAAACCTCATAAAATAGTTATGAGGAGTGAGGTGGACTAATA TTAATGAGTCCCTCCCCTATAAAAGAGCTATTAAGGCTTTTTGTCTTA TACTAACTTTTTTTTTAAATGTGGTATCTTTAGAACCAAGGGTCTTAG AGTTTTAGTATACAGAAACTGTTGCATCGCTTAATCAGATTTTCTAGT TTCAAATCCAGAGAATCCAAATTCTTCACAGCCAAAGTCAAATTAAGA ATTTCTGACTTTAATGTTATTTGCTACTGTGAATATAAAATGATAGCTT TTCCTGAGGCAGGGTCTCACTATGTATCTCTGCCTGATCTGCAACAA GATATGTAGACTAAAGTTCTGCCTGCTTTTGTCTCCTGAATACTAAG GTTAAAATGTAGTAATACTTTTGGAACTTGCAGGTCAGATTCTTTTAT AGGGGACACACTAAGGGAGCTTGGGTGATAGTTGGTAAATGTGTTT AAGTGATGAAAACTTGAATTATTATCACCGCAACCTACTTTTTAAAAA AAAAAGCCAGGCCTGTTAGAGCATGCTAAGGGATCCCTAGGACTTG CTGAGCACACAAGAGTAGTACTTGGCAGGCTCCTGGTGAGAGCATA TTTCAAAAAACAAGGCAGACAACCAAGAAACTACAGTAAGGTTACCT GTCTTTAACCATCTGCATATACACAGGGATATTAAAATATTCCAAATA ATATTTCATTCAAGTTTTCCCCCATCAAATTGGGACATGGATTTCTCC GGTGAATAGGCAGAGTTGGAAACTAAACAAATGTTGGTTTTGTGATT TGTGAAATTGTTTTCAAGTGATAGTTAAAGCCCATGAGATACAGAAC AAAGCTGCTATTTCGAGGTCTCTTGGTTATACTCAGAAGCACTTCTTT GGGTTTCCCTGCACTATCCTGATCATGTGCTAGGCCTnCCTTAGGCT GATTGTTGTTCAAATAACTTAAGTTTCCTGTCAGGTGATGTCATATGA TTTCATATATCAAGGCAAAACATGTTATATATGTTAAACATTTGnACTT AATGTGAAAGTTAGGTCTTTGTGGGTTTTGATTTTAATTTCAAAACCT GAGCTAAATAAGTCATTTTACATGTCTTACATTTGGTGAATTGTATAT TGTGGTTTGCAGGCAAGACTCTCTGACCTAGTAACCCTCCTATAGAG CACTTTGCTGGGTCACAAGTCTAGGAGTCAAGCATTTCACCTTGAAG TTGAGACGTTTTGTTAGTGTATACTAGTTATATGTTGGAGGACATGTT TATCCAGAAGATATTCAGGACTATTTTTGACTGGGCTAAGGAATTGA TTCTGATTAGCACTGTTAGTGAGCATTGAGTGGCCTTTAGGCTTGAA TTGGAGTCACTTGTATATCTCAAATAATGCTGGCCTTTTTTnAAAAGC CCTTGTTCTTTATCACCCTGTTTTCTACATAATTTTTGTTCAAAGAAAT ACTTGTTTGGATCTCCTTTTGACAACAATAGCATGTTTTCAAGCCATA TTTTTTTTCCTTTTTTTTTTTTTTTTTGGTTTTTCGAGACAGGGTTTCTC TGTATAGCCCTGGCTGTCCTGGAACTCACTTTGTAGACCAGGCTGG CCTCGAACTCAGAAATCCGCCTGCCTCTGCCTCCTGAGTGCCGGGA TTAAAGGCGTGCACCACCACGCCTGGCTAAGTTGGATATTTTGTATA TAACTATAACCAATACTAACTCCACTGGGTGGATTTTTAATTCAGTCA GTAGTCTTAAGTGGTCTTTATTGGCCCTTATTAAAATCTACTGTTCAC TCTAACAGAGGCTGTTGGACTAGTGGnACTAAGCAACTTCCTACGGA TATACTAGCAGATAAGGGTCAGGGATAGAAACTAGTCTAGCGTTTTG TATACCTACCAGCTTATACTACCTTGTTCTGATAGAAATATTTAGGAC ATCTAGCTTATCGATCCG |
| SEQ ID NO: 33 | CCGCGGCAGGCCCTCCGAGCGTGGTGGAGCCGTTCTGTGAGACAG CCGGGTACGAGTCGTGACGCTGGAAGGGGCAAGCGGGTGGTGGG CAGGAATGCGGTCCGCCCTGCAGCAACCGGAGGGGGAGGGAGAA GGGAGCGGAAAAGTCTCCACCGGACGCGGCCATGGCTCGGGGGG GGGGGGGCAGCGGAGGAnCGCTTCCGGCCGACGTCTCGTCGCTGA TTGGCTTnTTTTCCTCCCGCCGTGTGTGAAAACACAAATGGCGTGTT TTGGTTGGCGTAAGGCGCCTGTCAGTTAACGGCAGCCGGAGTGCG CAGCCGCCGGCAGCCTCGCTCTGCCCACTGGGTGGGGCGGGAGG |
| | TAGGTGGGGTGAGGCGAGCTGNACGTGCGGGCGCGGTCGGCCTCT GGCGGGGCGGGGGAGGGGAGGGAGGGTCAGCGAAAGTAGCTCGC GCGCGAGCGGCCGCCCACCCTCCCCTTCCTCTGGGGGAGTCGTTT TACCCGCCGCCGGCCGGGCCTCGTCGTCTGATTGGCTCTCGGGGC CCAGAAAACTGGCCCTTGCCATTGGCTCGTGTTCGTGCAAGTTGAG TCCATCCGCCGGCCAGCGGGGGCGGCGAGGAGGCGCTCCCAGGT TCCGGCCCTCCCCTCGGCCCCGCGCCGCAGAGTCTGGCCGCGCGC CCCTGCGCAACGTGGCAGGAAGCGCGCGCTGGGGGCGGGGACGG GCAGTAGGGCTGAGCGGCTGCGGGGCGGGTGCAAGCACGTTTCCG ACTTGAGTTGCCTCAAGAGGGGCGTGCTGAGCCAGACCTCCATCGC GCACTCCGGGGAGTGGAGGGAAGGAGCGAGGGCTCAGTTGGGCT GTTTTGGAGGCAGGAAGCACTTGCTCTCCCAAAGTCGCTCTGAGTT GTTATCAGTAAGGGAGCTGCAGTGGAGTAGGCGGGGAGAAGGCCG CACCCTTCTCCGGAGGGGGGAGGGGAGTGTTGCAATACCTTTCTGG GAGTTCTCTGCTGCCTCCTGGCTTCTGAGGACCGCCCTGGGCCTGG GAGAATCCCTTGCCCCCTCTTCCCCTCGTGATCTGCAACTCCAGTCT TTCTAGTGAATTCGGATCCTTAATTAAGGCCTCCGCGCCGGGTTTTG GCGCCTCCCGCGGGCGCCCCCCTCCTCACGGCGAGCGCTGCCAC GTCAGACGAAGGGCGCAGCGAGCGTCCTGATCCTTCCGCCCGGAC GCTCAGGACAGCGGCCCGCTGCTCATAAGACTCGGCCTTAGAACCC CAGTATCAGCAGAAGGACATTTTAGGACGGGACTTGGGTGACTCTA GGGCACTGGTTTTCTTTCCAGAGAGCGGAACAGGCGAGGAAAAGTA GTCCCTTCTCGGCGATTCTGCGGAGGGATCTCCGTGGGGCGGTGA ACGCCGATGATTATATAAGGACGCGCCGGGTGTGGCACAGCTAGTT CCGTCGCAGCCGGGATTTGGGTCGCGGTTCTTGTTTGTGGATCGCT GTGATCGTCACTTGGTCTAGAGGATCCGGGCCGCATGGTGAGCAAG GGCGAGGAGCTGTTCACCGGGGTGGTGCCCATCCTGGTCGAGCTG GACGGCGACGTAAACGGCCACAAGTTCAGCGTGTCCGGCGAGGGC GAGGGCGATGCCACCTACGGCAAGCTGACCCTGAAGTTCATCTGCA CCACCGGCAAGCTGCCCGTGCCCTGGCCCACCCTCGTGACCACCC TGACCTGGGGCGTGCAGTGCTTCAGCCGCTACCCCGACCACATGAA GCAGCACGACTTCTTCAAGTCCGCCATGCCCGAAGGCTACGTCCAG GAGCGCACCATCTTCTTCAAGGACGACGGCAACTACAAGACCCGCG CCGAGGTGAAGTTCGAGGGCGACACCCTGGTGAACCGCATCGAGC TGAAGGGCATCGACTTCAAGGAGGACGGCAACATCCTGGGGCACAA GCTGGAGTACAACTACATCAGCCACAACGTCTATATCACCGCCGAC AAGCAGAAGAACGGCATCAAGGCCAACTTCAAGATCCGCCACAACA TCGAGGACGGCAGCGTGCAGCTCGCCGACCACTACCAGCAGAACA CCCCCATCGGCGACGGCCCCGTGCTGCTGCCCGACAACCACTACC TGAGCACCCAGTCCGCCCTGAGCAAAGACCCCAACGAGAAGCGCG ATCACATGGTCCTGCTGGAGTTCGTGACCGCCGCCGGGATCACTCT CGGCATGGACGAGCTGTACAAGGGTGGTACCGGAGGCGCCGCTGA TACTCGCATTGGTGTAACAATCTATAAGTACGACGATAACTTTATGTC TGTAGTGCGCAAGGCTATTGAGCAAGATGCGAAAGCCGCGCCAGAT GTTCAGCTGCTGATGAATGATTCTCAGAATGACCAGTCCAAGCAGAA CGATCAGATCGACGTATTGCTGGCGAAAGGGGTGAAGGCACTGGCA ATCAACCTGGTTGACCCGGCAGCTGCGGGTACGGTGATTGAGAAAG CGCGTGGGCAAAACGTGCCGGTGGTTTTCTTCAACAAAGAACCGTC TCGTAAGGCGCTGGATAGCTACGACAAAGCCTACTACGTTGGCACT GACTCCAAAGAGTCCGGCATTATTCAAGGCGATTTGATTGCTAAACA CTGGGCGGCGAATCAGGGTTGGGATCTGAACAAAGACGGTCAGATT CAGTTCGTACTGCTGAAAGGTGAACCGGGCCATCCGGATGCAGAAG CACGTACCACTTACGTGATTAAAGAATTGAACGATAAAGGCATCAAA |
| | ACTGAACAGTTACAGTTAGATACCGCAATGTGGGACACCGCTCAGG CGAAAGATAAGATGGACGCCTGGCTGTCTGGCCCGAACGCCAACAA AATCGAAGTGGTTATCGCCAACAACGATCGGATGGCAATGGGCGCG GTTGAAGCGCTGAAAGCACACAACAAGTCCAGCATTCCGGTGTTTG GCGTCGATGCGTCGCCAGAAGCGCTGGCGCTGGTGAAATCCGGTG CACTGGCGGGCACCGTACTGAACGATGCTAACAACCAGGCGAAAGC GACCTTTGATCTGGCGAAAAACCTGGCCGATGGTAAAGGTGCGGCT GATGGCACCAACTGGAAAATCGACAACAAAGTGGTCCGCGTACCTT ATGTTGGCGTAGATAAAGACAACCTGGCTGAATTCAGCAAGAAAGG CGCCGGTACCGGTGGAATGGTGAGCAAGGGCGAGGAGCTGTTCAC CGGGGTGGTGCCCATCCTGGTCGAGCTGGACGGCGACGTAAACGG CCACAAGTTCAGCGTGTCCGGCGAGGGCGAGGGCGATGCCACCTA CGGCAAGCTGACCCTGAAGTTCATCTGCACCACCGGCAAGCTGCCC GTGCCCTGGCCCACCCTCGTGACCACCTTCGGCTACGGCCTGCAGT GCTTCGCCCGCTACCCCGACCACATGAAGCAGCACGACTTCTTCAA GTCCGCCATGCCCGAAGGCTACGTCCAGGAGCGCACCATCTTCTTC AAGGACGACGGCAACTACAAGACCCGCGCCGAGGTGAAGTTCGAG GGCGACACCCTGGTGAACCGCATCGAGCTGAAGGGCATCGACTTCA AGGAGGACGGCAACATCCTGGGGCACAAGCTGGAGTACAACTACAA CAGCCACAACGTCTATATCATGGCCGACAAGCAGAAGAACGGCATC AAGGTGAACTTCAAGATCCGCCACAACATCGAGGACGGCAGCGTGC AGCTCGCCGACCACTACCAGCAGAACACCCCCATCGGCGACGGCC CCGTGCTGCTGCCCGACAACCACTACCTGAGCTACCAGTCCGCCCT GAGCAAAGACCCCAACGAGAAGCGCGATCACATGGTCCTGCTGGA GTTCGTGACCGCCGCCGGGATCACTCTCGGCATGGACGAGCTGTA CAAGGGAGGTGGCGGAAGCTCCGGTGAGGGCAGAGGAAGCCTTCT AACATGCGGTGACGTGGAGGAGAATCCCGGCCCTTCCGGGATGAC CGAGTACAAGCCCACGGTGCGCCTCGCCACCCGCGACGACGTCCC CAGGGCCGTACGCACCCTCGCCGCCGCGTTCGCCGACTACCCCGC CACGCGCCACACCGTCGATCCGGACCGCCACATCGAGCGGGTCAC CGAGCTGCAAGAACTCTTCCTCACGCGCGTCGGGCTCGACATCGGC AAGGTGTGGGTCGCGGACGACGGCGCCGCGGTGGCGGTCTGGAC CACGCCGGAGAGCGTCGAAGCGGGGGCGGTGTTCGCCGAGATCG GCCCGCGCATGGCCGAGTTGAGCGGTTCCCGGCTGGCCGCGCAGC AACAGATGGAAGGCCTCCTGGCGCCGCACCGGCCCAAGGAGCCCG CGTGGTTCCTGGCCACCGTCGGCGTCTCGCCCGACCACCAGGGCA AGGGTCTGGGCAGCGCCGTCGTGCTCCCCGGAGTGGAGGCGGCC GAGCGCGCCGGGGTGCCCGCCTTCCTGGAGACCTCCGCGCCCCG CAACCTCCCCTTCTACGAGCGGCTCGGCTTCACCGTCACCGCCGAC GTCGAGGTGCCCGAAGGACCGCGCACCTGGTGCATGACCCGCAAG CCCGGTGCCTGAATCTAGGTCGACCTGCAGAAGCTTGCCTCGAGCA GCGCTGCTCGAGAGATCTACGGGTGGCATCCCTGTGACCCCTCCCC AGTGCCTCTCCTGGCCCTGGAAGTTGCCACTCCAGTGCCCACCAGC CTTGTCCTAATAAAATTAAGTTGCATCATTTTGTCTGACTAGGTGTCC TTCTATAATATTATGGGGTGGAGGGGGGTGGTATGGAGCAAGGGGC AAGTTGGGAAGACAACCTGTAGGGCCTGCGGGGTCTATTGGGAACC AAGCTGGAGTGCAGTGGCACAATCTTGGCTCACTGCAATCTCCGCC TCCTGGGTTCAAGCGATTCTCCTGCCTCAGCCTCCCGAGTTGTTGG GATTCCAGGCATGCATGACCAGGCTCAGCTAATTTTTGTTTTTTTGG TAGAGACGGGGTTTCACCATATTGGCCAGGCTGGTCTCCAACTCCT AATCTCAGGTGATCTACCCACCTTGGCCTCCCAAATTGCTGGGATTA CAGGCGTGAACCACTGCTCCCTTCCCTGTCCTTCTGATTTTGTACCC GGGACTAGAAGATGGGCGGGAGTCTTCTGGGCAGGCTTAAAGGCT |
| | AACCTGGTGTGTGGGCGTTGTCCTGCAGGGGAATTGAACAGGTGTA AAATTGGAGGGACAAGACTTCCCACAGATTTTCGGTTTTGTCGGGAA GTTTTTTAATAGGGGCAAATAGGAAAATGGAGGATAGGAGTCATCTG GGGTTTATGCAGCAAAACTACAGGTATATTGCTTGTATCCGCCTCGG AGATTTCCATGAGGAGATAAAGACATGTCACCCGAGTTTATACTCTC CTGCTTAGATCCTACTACAGTATGAAATACAGTGTNGCGAGGTAGAC TATGTAAGCAGATTTAATCATTTTAAAGAGCCCAGTACTTCATATCCA TTTCTCCCGCTCCTTCTGCAGCCTTATCAAAAGGTATTTAGAACACTC ATTTTAGCCCCATTTTCATTTATTATACTGGCTTATCCAACCCCTAGA CAGAGCATTGGCATTTTCCCTTTCCTGATCTTAGAAGTCTGATGACT CATGAAACCAGACAGATTAGTTACATACACCACAAATCGAGGCTGTA GCTGGGGCCTCAACACTGCAGTTCTTTTATAACTCCTTAGTACACTT TTTGTTGATCCTTTGCCTTGATCCTTAATTTTCAGTGTCTATCACCTC TCCCGTCAGGTGGTGTTCCACATTTGGGCCTATTCTCAGTCCAGGG AGTTTTACAACAATAGATGTATTGAGAATCCAACCTAAAGCTTAACTT TCCACTCCCATGAATGCCTCTCTCCTTTTTCTCCATTATAACTGAGCT ATNACCATTAATGGTTTCAGGTGGATGTCTCCTCCCCCAATATACCT GATGTATCTACATATTGCCAGGCTGATATTTTAAGACATNAAAGGTAT ATTTCATTATTGAGCCACATGGTATTGATTACTGCTACTAAAATTTTG TCATTGTACACATCTGTAAAAGGTGGTTCCTTTTGGAATGCAAAGTTC AGGTGTTTGTTGTCTTTCCTGACCTAAGGTCTTGTGAGCTTGTATTTT TTCTATTTAAGCAGTGCTTTCTCTTGGACTGGCTTGACTCATGGCATT CTACACGTTATTGCTGGTCTAAATGTGATTTTGCCAAGCTTCTTCAG GACCTATAATTTTGCTTGACTTGTAGCCAAACACAAGTAAAATGATTA AGCAACAAATGTATTTGTGAAGCTTGGTTTTTAGGTTGTTGTGTTGTG TGTGCTTGTGCTCTATAATAATACTATCCAGGGGCTGGAGAGGTGG CTCGGAGTTCAAGAGCACAGACTGCTCTTCCAGAAGTCCTGAGTTC AATTCCCAGCAACCACATGGTGGCTCACAACCATCTGTAATGGGATC TGATGCCCTCTTCTGGTGTGTCTGAAGACCACAAGTGTATTCACATT AAATAAATAATCCTCCTTCTTCTTCTTTTTTTTTTTTTAAAGAGAATNC TGTCTCCAGTAGAATTACTGAAGTAATGAAATACTTTGTGTTTGTTCC AATATGGNAGCCAATAATCAAATACTCTTNAGCACTGGAAATGTACC AAGGAACTATTTTATTTAAGTGNACTGTGGACAGAGGAGCCATAACT GCAGACTTGTGGGATACAGAAGACCAATGCAGACTTAATGTCTTTTC TCTTACACTAAGCAATAAAGAAATAAAAATTGAACTTCTAGTATCCTA TTTGTTAAACTGCTAGCTTTACTAACTTTTGTGCTTCATCTATACAAA GCTGAAAGCTAAGTCTGCAGCCATTACTAAACATGAAAGCAAGTAAT GATAATTTTGGATTTCAAAAATGTAGGGCCAGAGTTTAGCCAGCCAG TGGTGGTGCTTGCCTTTATGCCTTAATCCCAGCACTCTGGAGGCAG AGACAGGCAGATCTCTGAGTTTGAGCCCAGCCTGGTCTACACATCA AGTTCTATCTAGGATAGCCAGGAATACACACAGAAACCCTGTTGGG GAGGGGGGCTCTGAGATTTCATAAAATTATAATTGAAGCATTCCCTA ATGAGCCACTATGGATGTGGCTAAATCCGTCTACCTTTCTGATGAGA TTTGGGTATTATTTTTTCTGTCTCTGCTGTTGGTTGGGTCTTTTGACA CTGTGGGCTTTCTTAAAGCCTCCTTCCCTGCCATGTGGTCTCTTGTT TGCTACTAACTTCCCATGGCTTAAATGGCATGGCTTTTTGCCTTCTAA GGGCAGCTGCTGAGNTTTGCAGCCTGATTTCCAGGGTGGGGTTGG GAAATCTTTCAAACACTAAAATTGTCCTTTAATTTTTTTTTAAAAAATG GGTTATATAATAAACCTCATAAAATAGTTATGAGGAGTGAGGTGGAC TAATATTAATGAGTCCCTCCCCTATAAAAGAGCTATTAAGGCTTTTTG TCTTATACTAACTTTTTTTTTAAATGTGGTATCTTTAGAACCAAGGGTC TTAGAGTTTTAGTATACAGAAACTGTTGCATCGCTTAATCAGATTTTC TAGTTTCAAATCCAGAGAATCCAAATTCTTCACAGCCAAAGTCAAATT |
| | AAGAATTTCTGACTTTAATGTTATTTGCTACTGTGAATATAAAATGATA GCTTTTCCTGAGGCAGGGTCTCACTATGTATCTCTGCCTGATCTGCA ACAAGATATGTAGACTAAAGTTCTGCCTGCTTTTGTCTCCTGAATACT AAGGTTAAAATGTAGTAATACTTTTGGAACTTGCAGGTCAGATTCTTT TATAGGGGACACACTAAGGGAGCTTGGGTGATAGTTGGTAAATGTG TTTAAGTGATGAAAACTTGAATTATTATCACCGCAACCTACTTTTTAA AAAAAAAAGCCAGGCCTGTTAGAGCATGCTAAGGGATCCCTAGGAC TTGCTGAGCACACAAGAGTAGTACTTGGCAGGCTCCTGGTGAGAGC ATATTTCAAAAAACAAGGCAGACAACCAAGAAACTACAGTAAGGTTA CCTGTCTTTAACCATCTGCATATACACAGGGATATTAAAATATTCCAA ATAATATTTCATTCAAGTTTTCCCCCATCAAATTGGGACATGGATTTC TCCGGTGAATAGGCAGAGTTGGAAACTAAACAAATGTTGGTTTTGTG ATTTGTGAAATTGTTTTCAAGTGATAGTTAAAGCCCATGAGATACAGA ACAAAGCTGCTATTTCGAGGTCTCTTGGTTATACTCAGAAGCACTTC TTTGGGTTTCCCTGCACTATCCTGATCATGTGCTAGGCCTNCCTTAG GCTGATTGTTGTTCAAATAACTTAAGTTTCCTGTCAGGTGATGTCATA TGATTTCATATATCAAGGCAAAACATGTTATATATGTTAAACATTTGN ACTTAATGTGAAAGTTAGGTCTTTGTGGGTTTTGATTTTAATTTCAAA ACCTGAGCTAAATAAGTCATTTTACATGTCTTACATTTGGTGAATTGT ATATTGTGGTTTGCAGGCAAGACTCTCTGACCTAGTAACCCTCCTAT AGAGCACTTTGCTGGGTCACAAGTCTAGGAGTCAAGCATTTCACCTT GAAGTTGAGACGTTTTGTTAGTGTATACTAGTTATATGTTGGAGGAC ATGTTTATCCAGAAGATATTCAGGACTATTTTTGACTGGGCTAAGGA ATTGATTCTGATTAGCACTGTTAGTGAGCATTGAGTGGCCTTTAGGC TTGAATTGGAGTCACTTGTATATCTCAAATAATGCTGGCCTTTTTTNA AAAGCCCTTGTTCTTTATCACCCTGTTTTCTACATAATTTTTGTTCAAA GAAATACTTGTTTGGATCTCCTTTTGACAACAATAGCATGTTTTCAAG CCATATTTTTTTTCCTTTTTTTTTTTTTTTTTGGTTTTTCGAGACAGGG TTTCTCTGTATAGCCCTGGCTGTCCTGGAACTCACTTTGTAGACCAG GCTGGCCTCGAACTCAGAAATCCGCCTGCCTCTGCCTCCTGAGTGC CGGGATTAAAGGCGTGCACCACCACGCCTGGCTAAGTTGGATATTT TGTATATAACTATAACCAATACTAACTCCACTGGGTGGATTTTTAATT CAGTCAGTAGTCTTAAGTGGTCTTTATTGGCCCTTATTAAAATCTACT GTTCACTCTAACAGAGGCTGTTGGACTAGTGGNACTAAGCAACTTCC TACGGATATACTAGCAGATAAGGGTCAGGGATAGAAACTAGTCTAGC GTTTTGTATACCTACCAGCTTATACTACCTTGTTCTGATAGAAATATT TAGGACATCTAGCTTATCGATCCG |
| SEQ ID NO: 34 | ATGTACAGGATGCAACTCCTGTCTTGCATTGCACTAAGTCTTGCACT TGTCACGAATTCGATATCGGCCATGGTTAGATCTGGTTGTAAGCCTT GCATATGTACAGTCCCAGAAGTATCATCTGTCTTCATCTTCCCCCCA AAGCCCAAGGATGTGCTCACCATTACTCTGACTCCTAAGGTCACGTG TGTTGTGGTAGACATCAGCAAGGATGATCCCGAGGTCCAGTTCAGC TGGTTTGTAGATGATGTGGAGGTGCACACAGCTCAGACGCAACCCC GGGAGGAGCAGTTCAACAGCACTTTCCGCTCAGTCAGTGAACTTCC CATCATGCACCAGGACTGGCTCAATGGCAAGGAGTTCAAATGCAGG GTCAACAGTGCAGCTTTCCCTGCCCCCATCGAGAAAACCATCTCCAA AACCAAAGGCAGACCGAAGGCTCCACAGGTGTACACCATTCCACCT CCCAAGGAGCAGATGGCCAAGGATAAAGTCAGTCTGACCTGCATGA TAACAGACTTCTTCCCTGAAGACATTACTGTGGAGTGGCAGTGGAAT GGGCAGCCAGCGGAGAACTACAAGAACACTCAGCCCATCATGGACA CAGATGGCTCTTACTTCGTCTACAGCAAGCTCAATGTGCAGAAGAGC AACTGGGAGGCAGGAAATACTTTCACCTGCTCTGTGTTACATGAGG |
| | GCCTGCACAACCACCATACTGAGAAGAGCCTCTCCCACTCTCCTGG TAAA |
| SEQ ID NO: 35 | ATGCGCCGCGGCCGCCTGCTGGAGATCGCCCTGGGCTTCACCGTG CTGCTGGCCTCCTACACCTCCCACGGCGCCGACGCCGCTGAATTCG AGTGCCGCTGGTACCTGGGCGGCTGCAAGGAGGACTCCGAGTGCT GCGAGCACCTGCAGTGCCACTCCTACTGGGAGTGGTGCCTGTGGG ACGGCTCCTTCTAA SIGNAL PEPTIDE-> VATX3 CODING |
| SEQ ID NO: 36 | ATGCGCCGCGGCCGCCTGCTGGAGATCGCCCTGGGCTTCACCGTG CTGCTGGCCTCCTACACCTCCCACGGCGCCGACGCCGCTGAATTCG GATCTGACTGCGCCAAGGAGGGCGAGGTGTGCTCCTGGGGCAAGA AGTGCTGCGACCTGGACAACTTCTACTGCCCCATGGAGTTCATCCC CCACTGCAAGAAGTACAAGCCCTACGTGCCCGTGACCACCAACTGC GCCAAGGAGGGCGAGGTGTGCGGCTGGGGCTCCAAGTGCTGCCAC GGCCTGGACTGCCCCCTGGCCTTCATCCCCTACTGCGAGAAGTACC GCTAA SIGNAL PEPTIDE->DKTX CODING |
| SEQ ID NO: 37 | ATGTACAGGATGCAACTCCTGTCTTGCATTGCACTAAGTCTTGCACT TGTCACGAATTCGGGATCTGAGTGCCGCTGGTACCTGGGCGGCTGC AAGGAGGACTCCGAGTGCTGCGAGCACCTGCAGTGCCACTCCTACT GGGAGTGGTGCCTGTGGGACGGCTCCTTCCGACGGAAGCGAGGG ATATCGGCCATGGTTAGATCTGGTTGTAAGCCTTGCATATGTACAGT CCCAGAAGTATCATCTGTCTTCATCTTCCCCCCAAAGCCCAAGGATG TGCTCACCATTACTCTGACTCCTAAGGTCACGTGTGTTGTGGTAGAC ATCAGCAAGGATGATCCCGAGGTCCAGTTCAGCTGGTTTGTAGATG ATGTGGAGGTGCACACAGCTCAGACGCAACCCCGGGAGGAGCAGT TCAACAGCACTTTCCGCTCAGTCAGTGAACTTCCCATCATGCACCAG GACTGGCTCAATGGCAAGGAGTTCAAATGCAGGGTCAACAGTGCAG CTTTCCCTGCCCCCATCGAGAAAACCATCTCCAAAACCAAAGGCAGA CCGAAGGCTCCACAGGTGTACACCATTCCACCTCCCAAGGAGCAGA TGGCCAAGGATAAAGTCAGTCTGACCTGCATGATAACAGACTTCTTC CCTGAAGACATTACTGTGGAGTGGCAGTGGAATGGGCAGCCAGCG GAGAACTACAAGAACACTCAGCCCATCATGGACACAGATGGCTCTTA CTTCGTCTACAGCAAGCTCAATGTGCAGAAGAGCAACTGGGAGGCA GGAAATACTTTCACCTGCTCTGTGTTACATGAGGGCCTGCACAACCA CCATACTGAGAAGAGCCTCTCCCACTCTCCTGGTAAATAA SIGNAL PEPTIDE->VATX3 CODING-> FURIN SITE-> IGG-FC |
| SEQ ID NO: 38 | ATGTACAGGATGCAACTCCTGTCTTGCATTGCACTAAGTCTTGCACT TGTCACGAATTCGGGATCTGACTGCGCCAAGGAGGGCGAGGTGTG CTCCTGGGGCAAGAAGTGCTGCGACCTGGACAACTTCTACTGCCCC ATGGAGTTCATCCCCCACTGCAAGAAGTACAAGCCCTACGTGCCCG TGACCACCAACTGCGCCAAGGAGGGCGAGGTGTGCGGCTGGGGCT CCAAGTGCTGCCACGGCCTGGACTGCCCCCTGGCCTTCATCCCCTA CTGCGAGAAGTACCGCCGGAAGCGAGGGATATCGGCCATGGTTAG ATCTGGTTGTAAGCCTTGCATATGTACAGTCCCAGAAGTATCATCTG TCTTCATCTTCCCCCCAAAGCCCAAGGATGTGCTCACCATTACTCTG ACTCCTAAGGTCACGTGTGTTGTGGTAGACATCAGCAAGGATGATC CCGAGGTCCAGTTCAGCTGGTTTGTAGATGATGTGGAGGTGCACAC AGCTCAGACGCAACCCCGGGAGGAGCAGTTCAACAGCACTTTCCGC TCAGTCAGTGAACTTCCCATCATGCACCAGGACTGGCTCAATGGCA AGGAGTTCAAATGCAGGGTCAACAGTGCAGCTTTCCCTGCCCCCAT CGAGAAAACCATCTCCAAAACCAAAGGCAGACCGAAGGCTCCACAG |
| | GTGTACACCATTCCACCTCCCAAGGAGCAGATGGCCAAGGATAAAG TCAGTCTGACCTGCATGATAACAGACTTCTTCCCTGAAGACATTACT GTGGAGTGGCAGTGGAATGGGCAGCCAGCGGAGAACTACAAGAAC ACTCAGCCCATCATGGACACAGATGGCTCTTACTTCGTCTACAGCAA GCTCAATGTGCAGAAGAGCAACTGGGAGGCAGGAAATACTTTCACC TGCTCTGTGTTACATGAGGGCCTGCACAACCACCATACTGAGAAGA GCCTCTCCCACTCTCCTGGTAAATAA SIGNAL PEPTIDE->DKTX CODING-> FURIN SITE-> IGG-FC |
| SEQ ID NO: 39 | GACAAAACTCACACATGCCCACCGTGCCCAGCACCTGAACTCCTGG GGGGACCGTCAGTCTTCCTCTTCCCCCCAAAACCCAAGGACACCCT CATGATCTCCCGGACCCCTGAGGTCACATGCGTGGTGGTGGACGTG AGCCACGAAGACCCTGAGGTCAAGTTCAACTGGTACGTGGACGGCG TGGAGGTGCATAATGCCAAGACAAAGCCGCGGGAGGAGCAGTACA ACAGCACGTACCGTGTGGTCAGCGTCCTCACCGTCCTGCACCAGGA CTGGCTGAATGGCAAGGAGTACAAGTGCAAGGTCTCCAACAAAGCC CTCCCAGCCCCCATCGAGAAAACCATCTCCAAAGCCAAAGGGCAGC CCCGAGAACCACAGGTGTACACCCTGCCCCCATCCCGGGAGGAGA TGACCAAGAACCAGGTCAGCCTGACCTGCCTGGTCAAAGGCTTCTA TCCCAGCGACATCGCCGTGGAGTGGGAGAGCAATGGGCAGCCGGA GAACAACTACAAGACCACGCCTCCCGTGCTGGACTCCGACGGCTCC TTCTTCCTCTACAGCAAGCTCACCGTGGACAAGAGCAGGTGGCAGC AGGGGAACGTCTTCTCATGCTCCGTGATGCACGAGGCTCTGCACAA CCACTACACGCAGAAGAGCCTCTCCCTGTCTCCGGGTAAA |
| SEQ ID NO: 40 | TGCTTTCTCTGACCAGCATTCTCTCCCCTGGGCCTGTGCCGCTTTCT GTCTGCAGCTTGTGGCCTGGGTCACCTCTACGGCTGGCCCAGATCC TTCCCTGCCGCCTCCTTCAGGTTCCGTCTTCCTCCACTCCCTCTTCC CCTTGCTCTCTGCTGTGTTGCTGCCCAAGGATGCTCTTTCCGGAGC ACTTCCTTCTCGGCGCTGCACCACGTGATGTCCTCTGAGCGGATCC TCCCCGTGTCTGGGTCCTCTCCGGGCATCTCTCCTCCCTCACCCAA CCCCATGCCGTCTTCACTCGCTGGGTTCCCTTTTCCTTCTCCTTCTG GGGCCTGTGCCATCTCTCGTTTCTTAGGATGGCCTTCTCCGACGGA TGTCTCCCTTGCGTCCCGCCTCCCCTTCTTGTAGGCCTGCATCATCA CCGTTTTTCTGGACAACCCCAAAGTACCCCGTCTCCCTGGCTTTAGC CACCTCTCCATCCTCTTGCTTTCTTTGCCTGGACACCCCGTTCTCCT GTGGATTCGGGTCACCTCTCACTCCTTTCATTTGGGCAGCTCCCCTA CCCCCCTTACCTCTCTAGTCTGTGCTAGCTCTTCCAGCCCCCTGTCA TGGCATCTTCCAGGGGTCCGAGAGCTCAGCTAGTCTTCTTCCTCCA ACCCGGGCCCCTATGTCCACTTCAGGACAGCATGTTTGCTGCCTCC AGGGATCCTGTGTCCCCGAGCTGGGACCACCTTATATTCCCAGGGC CGGTTAATGTGGCTCTGGTTCTGGGTACTTTTATCTGTCCCCTCCAC CCCACAGTGGGGCAAGCTTCTCGAGTTGGGGTTGCGCCTTTTCCAA GGCAGCCCTGGGTTTGCGCAGGGACGCGGCTGCTCTGGGCGTGGT TCCGGGAAACGCAGCGGCGCCGACCCTGGGTCTCGCACATTCTTCA CGTCCGTTCGCAGCGTCACCCGGATCTTCGCCGCTACCCTTGTGGG CCCCCCGGCGACGCTTCCTGCTCCGCCCCTAAGTCGGGAAGGTTC CTTGCGGTTCGCGGCGTGCCGGACGTGACAAACGGAAGCCGCACG TCTCACTAGTACCCTCGCAGACGGACAGCGCCAGGGAGCAATGGCA GCGCGCCGACCGCGATGGGCTGTGGCCAATAGCGGCTGCTCAGCA GGGCGCGCCGAGAGCAGCGGCCGGGAAGGGGCGGTGCGGGAGG CGGGGTGTGGGGCGGTAGTGTGGGCCCTGTTCCTGCCCGCGCGGT GTTCCGCATTCTGCAAGCCTCCGGAGCGCACGTCGGCAGTCGGCT CCCTCGTTGACCGAATCACCGACCTCTCTCCCCAGGGTCTAGACGC |
| | CACCATGGTGTCCAAGGGCGAGGAGGTGATCAAGGAGTTCATGCG CTTCAAGGTGCGCATGGAGGGCTCCATGAACGGCCACGAGTTCGA GATCGAGGGCGAGGGCGAGGGCCGCCCCTACGAGGGCACCCAGA CCGCCAAGCTGAAGGTGACCAAGGGCGGCCCCCTGCCCTTCGCCT GGGACATCCTGTCCCCCCAGTTCATGTACGGCTCCAAGGCCTACGT GAAGCACCCCGCCGACATCCCCGACTACAAGAAGCTGTCCTTCCCC GAGGGCTTCAAGTGGGAGCGCGTGATGAACTTCGAGGACGGCGGC CTGGTGACCGTGACCCAGGACTCCTCCCTGCAGGACGGCACCCTG ATCTACAAGGTGAAGATGCGCGGCACCAACTTCCCCCCCGACGGCC CCGTGATGCAGAAGAAGACCATGGGCTGGGAGGCCTCCACCGAGC GCCTGTACCCCCGCGACGGCGTGCTGAAGGGCGAGATCCACCAGG CCCTGAAGCTGAAGGACGGCGGCCACTACCTGGTGGAGTTCAAGA CCATCTACATGGCCAAGAAGCCCGTGCAGCTGCCCGGCTACTACTA CGTGGACACCAAGCTGGACATCACCTCCCACAACGAGGACTACACC ATCGTGGAGCAGTACGAGCGCTCCGAGGGCCGCCACCACCTGTTC CTGGGATCCGAGGGCAGAGGAAGCCTTCTAACATGCGGTGACGTG GAGGAGAATCCCGGCCCTTCCGGGATGACCGAGTACAAGCCCACG GTGCGCCTCGCCACCCGCGACGACGTCCCCAGGGCCGTACGCACC CTCGCCGCCGCGTTCGCCGACTACCCCGCCACGCGCCACACCGTC GATCCGGACCGCCACATCGAGCGGGTCACCGAGCTGCAAGAACTC TTCCTCACGCGCGTCGGGCTCGACATCGGCAAGGTGTGGGTCGCG GACGACGGCGCCGCGGTGGCGGTCTGGACCACGCCGGAGAGCGT CGAAGCGGGGGCGGTGTTCGCCGAGATCGGCCCGCGCATGGCCG AGTTGAGCGGTTCCCGGCTGGCCGCGCAGCAACAGATGGAAGGCC TCCTGGCGCCGCACCGGCCCAAGGAGCCCGCGTGGTTCCTGGCCA CCGTCGGCGTCTCGCCCGACCACCAGGGCAAGGGTCTGGGCAGCG CCGTCGTGCTCCCCGGAGTGGAGGCGGCCGAGCGCGCCGGGGTG CCCGCCTTCCTGGAGACCTCCGCGCCCCGCAACCTCCCCTTCTACG AGCGGCTCGGCTTCACCGTCACCGCCGACGTCGAGGTGCCCGAAG GACCGCGCACCTGGTGCATGACCCGCAAGCCCGGTGCCTGAATCT AGGTCGACATTCTACTTGGTACCCTGTGCCTTCTAGTTGCCAGCCAT CTGTTGTTTGCCCCTCCCCCGTGCCTTCCTTGACCCTGGAAGGTGC CACTCCCACTGTCCTTTCCTAATAAAATGAGGAAATTGCATCGCATT GTCTGAGTAGGTGTCATTCTATTCTGGGGGGTGGGGTGGGGCAGG ACAGCAAGGGGGAGGATTGGGAAGACAATAGCAGGCATGCTGGGG ATGCGGTGGGCTCTATGGAAGCTTTACTAGGGACAGGATTGGTGAC AGAAAAGCCCCATCCTTAGGCCTCCTCCTTCCTAGTCTCCTGATATT GGGTCTAACCCCCACCTCCTGTTAGGCAGATTCCTTATCTGGTGACA CACCCCCATTTCCTGGAGCCATCTCTCTCCTTGCCAGAACCTCTAAG GTTTGCTTACGATGGAGCCAGAGAGGATCCTGGGAGGGAGAGCTTG GCAGGGGGTGGGAGGGAAGGGGGGGATGCGTGACCTGCCCGGTT CTCAGTGGCCACCCTGCGCTACCCTCTCCCAGAACCTGAGCTGCTC TGACGCGGCTGTCTGGTGCGTTTCACTGATCCTGGTGCTGCAGCTT CCTTACACTTCCCAAGAGGAGAAGCAGTTTGGAAAAACAAAATCAGA ATAAGTTGGTCCTGAGTTCTAACTTTGGCTCTTCACCTTTCTAGTCCC CAATTTATATTGTTCCTCCGTGCGTCAGTTTTACCTGTGAGATAAGG CCAGTAGCCAGCCCCGTCCTGGCAGGGCTGTGGTGAGGAGGGGG GTGTCCGTGTGGAAAACTCCCTTTGTGAGAATGGTGCGTCCTAGGT GTTCACCAGGTCGTGGCCGCCTCTACTCCCTTTCTCTTTCTCCATCC TTCTTTCCTTAAAGAGTCCCCAGTGCTATCTGGGACATATTCCTCCG CCCAGAGCAGGGTCCCGCTTCCCTAAGGCCCTGCTCTGGGCTTCTG GGTTTGAGTCCTTGGCAAGCCCAGGAGAGGCGCTCAGGCTTCCCTG TCCCCCTTCCTCGTCCACCATCTCATGCCCCTGGCTCTCCTGCCCCT |
| | TCCCTACAGGGGTTCCTGGCTCTGCTCT |
| SEQ ID NO: 41 | MHSKVTIICIRFLFWFLLLCMLIGKSHTEDDIIIATKNGKVRGMNLTVFGG TVTAFLGIPYAQPPLGRLRFKKPQSLTKWSDIWNATKYANSCCQNIDQS FPGFHGSEMWNPNTDLSEDCLYLNVWIPAPKPKNATVLIWIYGGGFQT GTSSLHVYDGKFLARVERVIVVSMNYRVGALGFLALPGNPEAPGNMGL FDQQLALQWVQKNIAAFGGNPKSVTLFGESSGAASVSLHLLSPGSHSL FTRAILQSGSANAPWAVTSLYEARNRTLNLAKLTGCSRENETEIIKCLRN KDPQEILLNEAFVVPYGTALGVNFGPTVDGDFLTDMPDILLELGQFKKT QILVGVNKDEGTWFLVGGAPGFSKDNNSIITRKEFQEGLKIFFPGVSEF GKESILFHYTDWVDDQRPENYREALGDVVGDYNFICPALEFTKKFSEW GNNAFFYYFEHRSSKLPWPEWMGVMHGYEIEFVFGLPLERRDNYTKA EEILSRSIVKRWANFAKYGNPNETQNNSTSWPVFKSTEQKYLTLNTEST RIMTKLRAQQCRFWTSFFPKVLEMTGNIDEAEWEWKAGFHRWNNYM MDWKNQFNDYTSKKESCVGL |
| SEQ ID NO: 42 | ATGCACAGCAAGGTGACCATCATCTGCATCAGGTTCCTGTTCTGGTT CCTGCTGCTGTGCATGCTGATCGGCAAGAGCCACACCGAGGACGA CATCATCATCGCCACCAAGAACGGCAAGGTGAGGGGCATGAACCTG ACCGTGTTCGGCGGCACCGTGACCGCCTTCCTGGGCATCCCCTAC GCCCAGCCCCCCCTGGGCAGGCTGAGGTTCAAGAAGCCCCAGAGC CTGACCAAGTGGAGCGACATCTGGAACGCCACCAAGTACGCCAACA GCTGCTGCCAGAACATCGACCAGAGCTTCCCCGGCTTCCACGGCAG CGAGATGTGGAACCCCAACACCGACCTGAGCGAGGACTGCCTGTAC CTGAACGTGTGGATCCCCGCCCCCAAGCCCAAGAACGCCACCGTG CTGATCTGGATCTACGGCGGCGGCTTCCAGACCGGCACCAGCAGC CTGCACGTGTACGACGGCAAGTTCCTGGCCAGGGTGGAGAGGGTG ATCGTGGTGAGCATGAACTACAGGGTGGGCGCCCTGGGCTTCCTG GCCCTGCCCGGCAACCCCGAGGCCCCCGGCAACATGGGCCTGTTC GACCAGCAGCTGGCCCTGCAGTGGGTGCAGAAGAACATCGCCGCC TTCGGCGGCAACCCCAAGAGCGTGACCCTGTTCGGCGAGAGCAGC GGCGCCGCCAGCGTGAGCCTGCACCTGCTGAGCCCCGGCAGCCAC AGCCTGTTCACCAGGGCCATCCTGCAGAGCGGCAGCGCCAACGCC CCCTGGGCCGTGACCAGCCTGTACGAGGCCAGGAACAGGACCCTG AACCTGGCCAAGCTGACCGGCTGCAGCAGGGAGAACGAGACCGAG ATCATCAAGTGCCTGAGGAACAAGGACCCCCAGGAGATCCTGCTGA ACGAGGCCTTCGTGGTGCCCTACGGCACCGCCCTGGGCGTGAACT TCGGCCCCACCGTGGACGGCGACTTCCTGACCGACATGCCCGACA TCCTGCTGGAGCTGGGCCAGTTCAAGAAGACCCAGATCCTGGTGGG CGTGAACAAGGACGAGGGCACCTGGTTCCTGGTGGGCGGCGCCCC CGGCTTCAGCAAGGACAACAACAGCATCATCACCAGGAAGGAGTTC CAGGAGGGCCTGAAGATCTTCTTCCCCGGCGTGAGCGAGTTCGGC AAGGAGAGCATCCTGTTCCACTACACCGACTGGGTGGACGACCAGA GGCCCGAGAACTACAGGGAGGCCCTGGGCGACGTGGTGGGCGACT ACAACTTCATCTGCCCCGCCCTGGAGTTCACCAAGAAGTTCAGCGA GTGGGGCAACAACGCCTTCTTCTACTACTTCGAGCACAGGAGCAGC AAGCTGCCCTGGCCCGAGTGGATGGGCGTGATGCACGGCTACGAG ATCGAGTTCGTGTTCGGCCTGCCCCTGGAGAGGAGGGACAACTACA CCAAGGCCGAGGAGATCCTGAGCAGGAGCATCGTGAAGAGGTGGG CCAACTTCGCCAAGTACGGCAACCCCAACGAGACCCAGAACAACAG CACCAGCTGGCCCGTGTTCAAGAGCACCGAGCAGAAGTACCTGACC CTGAACACCGAGAGCACCAGGATCATGACCAAGCTGAGGGCCCAG CAGTGCAGGTTCTGGACCAGCTTCTTCCCCAAGGTGCTGGAGATGA |
| | CCGGCAACATCGACGAGGCCGAGTGGGAGTGGAAGGCCGGCTTCC ACAGGTGGAACAACTACATGATGGACTGGAAGAACCAGTTCAACGA CTACACCAGCAAGAAGGAGAGCTGCGTGGGCCTG |
| SEQ ID NO: 43 | MQTQHTKVTQTHFLLWILLLCMPFGKSHTEEDFIITTKTGRVRGLSMPVL GGTVTAFLGIPYAQPPLGSLRFKKPQPLNKWPDIHNATQYANSCYQNID QAFPGFQGSEMWNPNTNLSEDCLYLNVWIPVPKPKNATVMVWIYGGG FQTGTSSLPVYDGKFLARVERVIVVSMNYRVGALGFLAFPGNPDAPGN MGLFDQQLALQWVQRNIAAFGGNPKSITIFGESSGAASVSLHLLCPQSY PLFTRAILESGSANAPWAVKHPEEARNRTLTLAKFTGCSKENEMEMIKC LRSKDPQEILRNERFVLPSDSALGINFGPTVDGDFLTDMPHTLLQLGKV KKAQILVGVNKDEGTWFLVGGAPGFSKDNDSLITRKEFQEGLNMYFPG VSRLGKEAVLFYYVDWLGEQSPEVYRDALDDVIGDYNIICPALEFTKKFA ELENNAFFYFFEHRSSKLPWPEWMGVMHGYEIEFVFGLPLGRRVNYTR AEEIFSRSIMKTWANFAKYGHPNGTQGNSTMWPVFTSTEQKYLTLNTE KSKIYSKLRAPQCQFWRLFFPKVLEMTGDIDETEQEWKAGFHRWSNY MMDWQNQFNDYTSKKESCTAL |
| SEQ ID NO: 44 | ATGCAGACCCAGCACACCAAGGTGACCCAGACCCACTTCCTGCTGT GGATCCTGCTGCTGTGCATGCCCTTCGGCAAGAGCCACACCGAGGA GGACTTCATCATCACCACCAAGACCGGCAGGGTGAGGGGCCTGAG CATGCCCGTGCTGGGCGGCACCGTGACCGCCTTCCTGGGCATCCC CTACGCCCAGCCCCCCCTGGGCAGCCTGAGGTTCAAGAAGCCCCA GCCCCTGAACAAGTGGCCCGACATCCACAACGCCACCCAGTACGCC AACAGCTGCTACCAGAACATCGACCAGGCCTTCCCCGGCTTCCAGG GCAGCGAGATGTGGAACCCCAACACCAACCTGAGCGAGGACTGCC TGTACCTGAACGTGTGGATCCCCGTGCCCAAGCCCAAGAACGCCAC CGTGATGGTGTGGATCTACGGCGGCGGCTTCCAGACCGGCACCAG CAGCCTGCCCGTGTACGACGGCAAGTTCCTGGCCAGGGTGGAGAG GGTGATCGTGGTGAGCATGAACTACAGGGTGGGCGCCCTGGGCTT CCTGGCCTTCCCCGGCAACCCCGACGCCCCCGGCAACATGGGCCT GTTCGACCAGCAGCTGGCCCTGCAGTGGGTGCAGAGGAACATCGC CGCCTTCGGCGGCAACCCCAAGAGCATCACCATCTTCGGCGAGAG CAGCGGCGCCGCCAGCGTGAGCCTGCACCTGCTGTGCCCCCAGAG CTACCCCCTGTTCACCAGGGCCATCCTGGAGAGCGGCAGCGCCAA CGCCCCCTGGGCCGTGAAGCACCCCGAGGAGGCCAGGAACAGGAC CCTGACCCTGGCCAAGTTCACCGGCTGCAGCAAGGAGAACGAGAT GGAGATGATCAAGTGCCTGAGGAGCAAGGACCCCCAGGAGATCCT GAGGAACGAGAGGTTCGTGCTGCCCAGCGACAGCGCCCTGGGCAT CAACTTCGGCCCCACCGTGGACGGCGACTTCCTGACCGACATGCCC CACACCCTGCTGCAGCTGGGCAAGGTGAAGAAGGCCCAGATCCTG GTGGGCGTGAACAAGGACGAGGGCACCTGGTTCCTGGTGGGCGGC GCCCCCGGCTTCAGCAAGGACAACGACAGCCTGATCACCAGGAAG GAGTTCCAGGAGGGCCTGAACATGTACTTCCCCGGCGTGAGCAGG CTGGGCAAGGAGGCCGTGCTGTTCTACTACGTGGACTGGCTGGGC GAGCAGAGCCCCGAGGTGTACAGGGACGCCCTGGACGACGTGATC GGCGACTACAACATCATCTGCCCCGCCCTGGAGTTCACCAAGAAGT TCGCCGAGCTGGAGAACAACGCCTTCTTCTACTTCTTCGAGCACAG GAGCAGCAAGCTGCCCTGGCCCGAGTGGATGGGCGTGATGCACGG CTACGAGATCGAGTTCGTGTTCGGCCTGCCCCTGGGCAGGAGGGT GAACTACACCAGGGCCGAGGAGATCTTCAGCAGGAGCATCATGAAG ACCTGGGCCAACTTCGCCAAGTACGGCCACCCCAACGGCACCCAG GGCAACAGCACCATGTGGCCCGTGTTCACCAGCACCGAGCAGAAGT ACCTGACCCTGAACACCGAGAAGAGCAAGATCTACAGCAAGCTGAG |
| | GGCCCCCCAGTGCCAGTTCTGGAGGCTGTTCTTCCCCAAGGTGCTG GAGATGACCGGCGACATCGACGAGACCGAGCAGGAGTGGAAGGCC GGCTTCCACAGGTGGAGCAACTACATGATGGACTGGCAGAACCAGT TCAACGACTACACCAGCAAGAAGGAGAGCTGCACCGCCCTG |
| SEQ ID NO: 45 | MYRMQLLSCIALSLALVTNSHGEGTFTSDVSSYLEGQAAKEFIAWLVKG RGRSGCKPCICTVPEVSSVFIFPPKPKDVLTITLTPKVTCVVVDISKDDPE VQFSWFVDDVEVHTAQTQPREEQFNSTFRSVSELPIMHQDWLNGKEF KCRVNSAAFPAPIEKTISKTKGRPKAPQVYTIPPPKEQMAKDKVSLTCMI TDFFPEDITVEWQWNGQPAENYKNTQPIMDTDGSYFVYSKLNVQKSN WEAGNTFTCSVLHEGLHNHHTEKSLSHSPGK |
| SEQ ID NO: 46 | ATGTACAGGATGCAGCTGCTGAGCTGCATCGCCCTGAGCCTGGCCC TGGTGACCAACAGCCACGGCGAGGGCACCTTCACCAGCGACGTGA GCAGCTACCTGGAGGGCCAGGCCGCCAAGGAGTTCATCGCCTGGC TGGTGAAGGGCAGGGGCAGGAGCGGCTGCAAGCCCTGCATCTGCA CCGTGCCCGAGGTGAGCAGCGTGTTCATCTTCCCCCCCAAGCCCAA GGACGTGCTGACCATCACCCTGACCCCCAAGGTGACCTGCGTGGT GGTGGACATCAGCAAGGACGACCCCGAGGTGCAGTTCAGCTGGTT CGTGGACGACGTGGAGGTGCACACCGCCCAGACCCAGCCCAGGGA GGAGCAGTTCAACAGCACCTTCAGGAGCGTGAGCGAGCTGCCCATC ATGCACCAGGACTGGCTGAACGGCAAGGAGTTCAAGTGCAGGGTG AACAGCGCCGCCTTCCCCGCCCCCATCGAGAAGACCATCAGCAAGA CCAAGGGCAGGCCCAAGGCCCCCCAGGTGTACACCATCCCCCCC CCAAGGAGCAGATGGCCAAGGACAAGGTGAGCCTGACCTGCATGAT CACCGACTTCTTCCCCGAGGACATCACCGTGGAGTGGCAGTGGAAC GGCCAGCCCGCCGAGAACTACAAGAACACCCAGCCCATCATGGACA CCGACGGCAGCTACTTCGTGTACAGCAAGCTGAACGTGCAGAAGAG CAACTGGGAGGCCGGCAACACCTTCACCTGCAGCGTGCTGCACGA GGGCCTGCACAACCACCACACCGAGAAGAGCCTGAGCCACAGCCC CGGCAAG |
| SEQ ID NO: 47 | GCT CTA GAG CCA CCA TGC AGA CTC AGC ATA CCA AGG |
| SEQ ID NO: 48 | CGG GAT CCA CCG GTT TAG AGA GCT GTA CAA GAT TCT TTC TTG |
| SEQ ID NO: 49 | CCC AAG CTT GCC ACC ATG CAT AGC AAA GTC ACA ATC |
| SEQ ID NO: 50 | ACG CGT CGA CTT AGA GAC CCA CAC AAC TTT CTT TCT TG |
| SEQ ID NO: 51 | ATGAAGACGCTGTCACAGGCCCAGTCCAAGACCTCCTCCCAGCAGT TCTCCTTCACCGGCAACTCCTCCGCCAACGTGATCATCGGCAACCA GAAGCTGACCATCAACGACGTGGCCCGCGTGGCCCGCAACGGCAC CCTGGTGTCCCTGACCAACAACACCGACATCCTGCAGGGCATCCAG GCCTCCTGCGACTACATCAACAACGCCGTGGAGTCCGGCGAGCCC ATCTACGGCGTGACCTCCGGCTTCGGCGGCATGGCCAACGTGGCC ATCTCCCGCGAGCAGGCCTCCGAGCTGCAGACCAACCTGGTGTGG TTCCTGAAGACCGGCGCCGGCAACAAGCTGCCCCTGGCCGACGTG CGCGCCGCCATGCTGCTGCGCGCCAACTCCCACATGCGCGGCGCC TCCGGCATCCGCCTcGAGCTGATCAAGCGCATGGAGATCTTCCTGA ACGCCGGCGTGACCCCCTACGTGTACGAGTTCGGCTCCATCGGCG CCTCCGGCGACCTGGTGCCCCTGTCCTACATCACCGGCTCCCTGAT CGGCCTGGACCCCTCCTTCAAGGTGGACTTCAACGGCAAGGAGATG GACGCCCCCACCGCCCTGCGCCAGCTGAACCTGTCCCCCCTGACC CTGCTGCCCAAGGAGGGCCTGGCCATGATGAACGGCACCTCCGTG ATGACCGGCATCGCCGCCAACTGCGTGTACGACACCCAGATCCTGA CCGCCATCGCCATGGGTGTACACGCTCTGGACATCCAGGCCCTGAA CGGCACCAACCAGTCCTTCCACCCCTTCATCCACAACTCCAAGCCC CACCCCGGCCAGCTGTGGGCCGCCGACCAGATGATCTCCCTCCTC |
| | GCCAACTCCCAGCTGGTGCGCGACGAGCTGGACGGCAAGCACGAC TACCGCGACCACGAGCTGATCCAGGACCGCTACTCCCTGCGCTGCC TGCCCCAGTACCTGGGCCCCATCGTGGACGGCATCTCCCAGATCGC CAAGCAGATCGAGATCGAGATCAACTCCGTGACCGACAACCCCCTG ATCGACGTGGACAACCAGGCCTCCTACCACGGCGGCAACTTCCTGG GCCAGTACGTGGGCATGGGCATGGACCACCTGCGCTACTACATCG GCCTGCTGGCCAAGCACCTGGACGTGCAGATCGCCCTGCTGGCCT CCCCCGAGTTCTCCAACGGCCTGCCCCCCTCCCTGCTGGGCAACC GCGAGCGCAAGGTGAACATGGGCCTGAAGGGCCTGCAGATCTGCG GTAACTCGATAATGCCCCTGCTGACCTTCTACGGCAACTCCATCGCC GACCGCTTCCCCACCCACGCCGAGCAGTTCAACCAGAACATCAACT CCCAGGGCTACACCTCCGCCACCCTGGCCCGCCGCTCCGTGGACA TCTTCCAGAACTACGTGGCCATCGCCCTGATGTTCGGCGTTCAAGC TGTAGACCTGCGCACCTACAAGAAGACCGGCCACTACGACGCCCGC GCCTCCCTGTCCCCCGCCACCGAGCGCCTGTACTCCGCCGTGCGC CACGTGGTGGGCCAGAAGCCCACCTCCGACCGCCCCTACATCTGG AACGACAACGAGCAGGGCCTGGACGAGCACATCGCCCGCATCTCC GCCGACATCGCAGCAGGTGGCGTGATCGTGCAGGCCGTGCAGGAC ATCCTGCCCTCCCTGCAC |
| SEQ ID NO: 52 | ATGGGTTCGTACGGGGGGGGATCAGCAGGGGTCTGGGTTGGACCCAGC CTGCGCGTTCTCGCGGCCATAGCAACCGACGTACGGCGTTGCGCC CTCGCCGGCAGCAAGAAGCCACGGAAGTCCGCCCGGAGCAGAAAA TGCCCACGCTACTGCGGGTTTATATAGACGGTCCCCACGGATGGG GAAAACCACCACCACGCAACTGCTGGTGGCCCTGGGTTCGCGCGA CGATATCGTCTACGTACCCGAGCCGATGACTTACTGGCGGGTGCTG GGGGCTTCCGAGACAATCGCGAACATCTACACCACACAACACCGCC TTGACCAGGGTGAGATATCGGCCGGGGACGCGGCGGTGGTAATGA CAAGCGCCCAGATAACAATGGGCATGCCTTATGCCGTGACCGACGC CGTTCTGGCTCCTCATATCGGGGGGGAGGCTGGGAGCTCACATGC CCCGCCCCCGGCCCTCACCCTCATCTTCGACCGCCATCCCATCGCC GCCCTCCTGTGCTACCCGGCCGCGCGATACCTTATGGGCAGCATGA CCCCCCAGGCCGTGCTGGCGTTCGTGGCCCTCATCCCGCCGACCT TGCCCGGCACAAACATCGTGTTGGGGGCCCTTCCGGAGGACAGAC ACATCGACCGCCTGGCCAAACGCCAGCGCCCCGGCGAGCGGCTTG ACCTGGCTATGCTGGCCGCGATTCGCCGCGTTTACGGGCTGCTTGC CAATACGGTGCGGTATCTGCAGGGCGGCGGGTCGTGGCGGGAGGA TTGGGGACAGCTTTCGGGGACGGCCGTGCCGCCCCAGGGTGCCGA GCCCCAGAGCAACGCGGGCCCACGACCCCATATCGGGGACACGTT ATTTACCCTGTTTCGGGCCCCCGAGTTGCTGGCCCCCAACGGCGAC CTGTACAACGTGTTTGCCTGGGCCTTGGACGTCTTGGCCAAACGCC TCCGTCCCATGCACGTCTTTATCCTGGATTACGACCAATCGCCCGCC GGCTGCCGGGACGCCCTGCTGCAACTTACCTCCGGGATGATCCAG ACCCACGTCACCACCCCAGGCTCCATACCGACGATCTGCGACCTGG CGCGCACGTTTGCCCGGGAGATGGGGGAGGCTAAC |
| SEQ ID NO: 53 | ATGGTGACCGGCGGCATGGCCTCCAAGTGGGACCAGAAGGGCATG GACATCGCCTACGAGGAGGCCGCCCTGGGCTACAAGGAGGGCGGC GTGCCCATCGGCGGCTGCCTGATCAACAACAAGGACGGCTCCGTG CTGGGCCGCGGCCACAACATGCGCTTCCAGAAGGGCTCCGCCACC CTGCACGGCGAGATCTCCACCCTGGAGAACTGCGGCCGCCTGGAG GGCAAGGTGTACAAGGACACCACCCTGTACACCACCCTGTCCCCCT GCGACATGTGCACCGGCGCCATCATCATGTACGGCATCCCCCGCTG |
| | CGTGGTGGGCGAGAACGTGAACTTCAAGTCCAAGGGCGAGAAGTA CCTGCAGACCCGCGGCCACGAGGTGGTGGTGGTGGACGACGAGC GCTGCAAGAAGATCATGAAGCAGTTCATCGACGAGCGCCCCCAGGA CTGGTTCGAGGACATCGGCGAG |
| SEQ ID NO: 54 | ATGTCCACTGCGGTCCTGGAAAACCCAGGCTTGGGCAGGAAACTCT CTGACTTTGGACAGGAAACAAGCTATATTGAAGACAACTGCAATCAA AATGGTGCCATATCACTGATCTTCTCACTCAAAGAAGAAGTTGGTGC ATTGGCCAAAGTATTGCGCTTATTTGAGGAGAATGATGTAAACCTGA CCCACATTGAATCTAGACCTTCTCGTTTAAAGAAAGATGAGTATGAAT TTTTCACCCATTTGGATAAACGTAGCCTGCCTGCTCTGACAAACATC ATCAAGATCTTGAGGCATGACATTGGTGCCACTGTCCATGAGCTTTC ACGAGATAAGAAGAAAGACACAGTGCCCTGGTTCCCAAGAACCATT CAAGAGCTGGACAGATTTGCCAATCAGATTCTCAGCTATGGAGCGG AACTGGATGCTGACCACCCTGGTTTTAAAGATCCTGTGTACCGTGCA AGACGGAAGCAGTTTGCTGACATTGCCTACAACTACCGCCATGGGC AGCCCATCCCTCGAGTGGAATACATGGAGGAAGAAAAGAAAACATG GGGCACAGTGTTCAAGACTCTGAAGTCCTTGTATAAAACCCATGCTT GCTATGAGTACAATCACATTTTTCCACTTCTTGAAAAGTACTGTGGCT TCCATGAAGATAACATTCCCCAGCTGGAAGACGTTTCTCAATTCCTG CAGACTTGCACTGGTTTCCGCCTCCGACCTGTGGCTGGCCTGCTTT CCTCTCGGGATTTCTTGGGTGGCCTGGCCTTCCGAGTCTTCCACTG CACACAGTACATCAGACATGGATCCAAGCCCATGTATACCCCCGAA CCTGACATCTGCCATGAGCTGTTGGGACATGTGCCCTTGTTTTCAGA TCGCAGCTTTGCCCAGTTTTCCCAGGAAATTGGCCTTGCCTCTCTGG GTGCACCTGATGAATACATTGAAAAGCTCGCCACAATTTACTGGTTT ACTGTGGAGTTTGGGCTCTGCAAACAAGGAGACTCCATAAAGGCAT ATGGTGCTGGGCTCCTGTCATCCTTTGGTGAATTACAGTACTGCTTA TCAGAGAAGCCAAAGCTTCTCCCCCTGGAGCTGGAGAAGACAGCCA TCCAAAATTACACTGTCACGGAGTTCCAGCCCCTGTATTACGTGGCA GAGAGTTTTAATGATGCCAAGGAGAAAGTAAGGAACTTTGCTGCCAC AATACCTCGGCCCTTCTCAGTTCGCTACGACCCATACACCCAAAGGA TTGAGGTCTTGGACAATACCCAGCAGCTTAAGATTTTGGCTGATTCC ATTAACAGTGAAATTGGAATCCTTTGCAGTGCCCTCCAGAAAATAAA G |
| SEQ ID NO: 55 | ATGGAGAAGCCGCGGGGAGTCAGGTGCACCAATGGGTTCTCCGAG CGTGAACTACCTCGTCCTGGAGCAAGCCCACCTGCAGAGAAGTCCC GACCTCCTGAAGCAAAGGGCGCACAGCCGGCCGACGCCTGGAAGG CAGGGCGGCACCGCAGCGAGGAGGAAAACCAGGTGAACCTCCCCA AACTGGCGGCCGCTTACTCGTCCATTCTGCTCTCGCTGGGCGAGGA CCCCCAGCGGCAGGGGCTGCTCAAGACGCCCTGGAGGGCGGCCA CCGCCATGCAGTACTTCACCAAGGGATACCAGGAGACCATCTCAGA TGTCCTGAATGATGCTATATTTGATGAAGATCATGACGAGATGGTGA TTGTGAAGGACATAGATATGTTCTCCATGTGTGAGCATCACCTTGTT CCATTTGTAGGAAGGGTCCATATTGGCTATCTTCCTAACAAGCAAGT CCTTGGTCTCAGTAAACTTGCCAGGATTGTAGAAATCTACAGTAGAC GACTACAAGTTCAAGAGCGCCTCACCAAACAGATTGCGGTGGCCAT CACAGAAGCCTTGCAGCCTGCTGGCGTTGGAGTAGTGATTGAAGCG ACACACATGTGCATGGTAATGCGAGGCGTGCAGAAAATGAACAGCA AGACTGTCACTAGCACCATGCTGGGCGTGTTCCGGGAAGACCCCAA GACTCGGGAGGAGTTCCTCACACTAATCAGGAGC |
| SEQ ID NO: 56 | ATGAGCGCTGCTGGTGATCTTCGTCGTCGTGCGCGACTGTCGCGCC TCGTGTCCTTCAGCGCGAGCCACCGGCTGCACAGCCCATCTCTGAG |
| | CGATGAAGAGAACTTAAGAGTGTTTGGGAAATGCAACAATCCGAATG GCCACGGGCACAACTATAAAGTTGTGGTGACAGTCCATGGAGAGAT TGATCCTGTTACAGGAATGGTTATGAATTTGACCGACCTCAAAGAAT ACATGGAGGAGGCCATCATGAAGCCTCTTGATCACAAGAACCTGGA CCTGGATGTGCCGTACTTTGCGGATGCTGTGAGCACGACAGAAAAT GTAGCTGTCTACATCTGGGAAAGCCTCCAGAAACTTCTTCCAGTGG GAGCTCTTTATAAAGTAAAAGTGTTTGAAACCGACAACAACATCGTA GTCTATAAAGGAGAA |
| SEQ ID NO: 57 | ATGGAGGGCGGGCTGGGGCGTGCTGTGTGCTTGCTGACCGGGGCC TCCCGCGGCTTCGGCCGGACGCTGGCCCCGCTCCTGGCCTCGCTG CTGTCGCCCGGCTCCGTGCTTGTCCTTAGCGCCCGCAACGACGAG GCACTGCGCCAGCTGGAGGCCGAGCTGGGCGCCGAGCGGTCTGG CCTGCGCGTGGTGCGGGTGCCCGCCGACCTGGGCGCCGAGGCCG GCTTGCAGCAGCTGCTCGGCGCCCTGCGCGAGCTCCCCCGGCCCA AGGGGCTGCAGCGACTGCTGCTTATCAACAACGCGGGCTCTCTTGG GGATGTGTCCAAAGGCTTCGTGGACCTGAGTGACTCCACTCAAGTG AACAACTACTGGGCACTGAACTTGACCTCCATGCTCTGCCTGACTTC CAGCGTCCTGAAGGCCTTCCCGGACAGTCCTGGCCTCAACAGAACC GTGGTTAACATCTCGTCCCTCTGTGCCCTGCAACCTTTCAAAGGCTG GGCGCTGTACTGTGCAGGAAAGGCTGCTCGTGATATGCTGTTCCAG GTCCTGGCGCTGGAGGAACCTAATGTGAGGGTGCTGAACTATGCCC CAGGTCCTCTGGACACAGACATGCAGCAGTTGGCCCGGGAGACCT CCGTGGACCCAGACATGCGAAAAGGGCTGCAGGAGCTGAAGGCAA AGGGGAAGCTGGTGGATTGCAAGGTGTCAGCCCAGAAACTGCTGA GCTTACTGGAAAAGGACGAGTTCAAGTCTGGAGCCCACGTGGACTT CTATGACAAA |
| SEQ ID NO: 58 | ATGCAAATAGAGCTCTCCACCTGCTTCTTTCTGTGCCTTTTGCGATT CTGCTTTAGTGCCACCAGAAGATACTACCTGGGTGCAGTGGAACTG TCATGGGACTATATGCAAAGTGATCTCGGTGAGCTGCCTGTGGACG CAAGATTTCCTCCTAGAGTGCCAAAATCTTTTCCATTCAACACCTCAG TCGTGTACAAAAAGACTCTGTTTGTAGAATTCACGGATCACCTTTTCA ACATCGCTAAGCCAAGGCCACCCTGGATGGGTCTGCTAGGTCCTAC CATCCAGGCTGAGGTTTATGATACAGTGGTCATTACACTTAAGAACA TGGCTTCCCATCCTGTCAGTCTTCATGCTGTTGGTGTATCCTACTGG AAAGCTTCTGAGGGAGCTGAATATGATGATCAGACCAGTCAAAGGG AGAAAGAAGATGATAAAGTCTTCCCTGGTGGAAGCCATACATATGTC TGGCAGGTCCTGAAAGAGAATGGTCCAATGGCCTCTGACCCACTGT GCCTTACCTACTCATATCTTTCTCATGTGGACCTGGTAAAAGACTTG AATTCAGGCCTCATTGGAGCCCTACTAGTATGTAGAGAAGGGAGTCT GGCCAAGGAAAAGACACAGACCTTGCACAAATTTATACTACTTTTTG CTGTATTTGATGAAGGGAAAAGTTGGCACTCAGAAACAAAGAACTCC TTGATGCAGGATAGGGATGCTGCATCTGCTCGGGCCTGGCCTAAAA TGCACACAGTCAATGGTTATGTAAACAGGTCTCTGCCAGGTCTGATT GGATGCCACAGGAAATCAGTCTATTGGCATGTGATTGGAATGGGCA CCACTCCTGAAGTGCACTCAATATTCCTCGAAGGTCACACATTTCTT GTGAGGAACCATCGCCAGGCGTCCTTGGAAATCTCGCCAATAACTT TCCTTACTGCTCAAACACTCTTGATGGACCTTGGACAGTTTCTACTG TTTTGTCATATCTCTTCCCACCAACATGATGGCATGGAAGCTTATGTC AAAGTAGACAGCTGTCCAGAGGAACCCCAACTACGAATGAAAAATAA TGAAGAAGCGGAAGACTATGATGATGATCTTACTGATTCTGAAATGG ATGTGGTCAGGTTTGATGATGACAACTCTCCTTCCTTTATCCAAATTC GCTCAGTTGCCAAGAAGCATCCTAAAACTTGGGTACATTACATTGCT |
| | GCTGAAGAGGAGGACTGGGACTATGCTCCCTTAGTCCTCGCCCCCG ATGACAGAAGTTATAAAAGTCAATATTTGAACAATGGCCCTCAGCGG ATTGGTAGGAAGTACAAAAAAGTCCGATTTATGGCATACACAGATGA AACCTTTAAGACTCGTGAAGCTATTCAGCATGAATCAGGAATCTTGG GACCTTTACTTTATGGGGAAGTTGGAGACACACTGTTGATTATATTTA AGAATCAAGCAAGCAGACCATATAACATCTACCCTCACGGAATCACT GATGTCCGTCCTTTGTATTCAAGGAGATTACCAAAAGGTGTAAAACA TTTGAAGGATTTTCCAATTCTGCCAGGAGAAATATTCAAATATAAATG GACAGTGACTGTAGAAGATGGGCCAACTAAATCAGATCCTCGGTGC CTGACCCGCTATTACTCTAGTTTCGTTAATATGGAGAGAGATCTAGC TTCAGGACTCATTGGCCCTCTCCTCATCTGCTACAAAGAATCTGTAG ATCAAAGAGGAAACCAGATAATGTCAGACAAGAGGAATGTCATCCTG TTTTCTGTATTTGATGAGAACCGAAGCTGGTACCTCACAGAGAATAT ACAACGCTTTCTCCCCAATCCAGCTGGAGTGCAGCTTGAGGATCCA GAGTTCCAAGCCTCCAACATCATGCACAGCATCAATGGCTATGTTTT TGATAGTTTGCAGTTGTCAGTTTGTTTGCATGAGGTGGCATACTGGT ACATTCTAAGCATTGGAGCACAGACTGACTTCCTTTCTGTCTTCTTCT CTGGATATACCTTCAAACACAAAATGGTCTATGAAGACACACTCACC CTATTCCCATTCTCAGGAGAAACTGTCTTCATGTCGATGGAAAACCC AGGTCTATGGATTCTGGGGTGCCACAACTCAGACTTTCGGAACAGA GGCATGACCGCCTTACTGAAGGTTTCTAGTTGTGACAAGAACACTG GTGATTATTACGAGGACAGTTATGAAGATATTTCAGCATACTTGCTG AGTAAAAACAATGCCATTGAACCAAGAAGCTTCTCCCAGAATTCAAG ACACCCTAGCACTAGGCAAAAGCAATTTAATGCCACCCCACCAGTCT TGAAACGCCATCAACGGGAAATAACTCGTACTACTCTTCAGTCAGAT CAAGAGGAAATTGACTATGATGATACCATATCAGTTGAAATGAAGAA GGAAGATTTTGACATTTATGATGAGGATGAAAATCAGAGCCCCCGCA GCTTTCAAAAGAAAACACGACACTATTTTATTGCTGCAGTGGAGAGG CTCTGGGATTATGGGATGAGTAGCTCCCCACATGTTCTAAGAAACAG GGCTCAGAGTGGCAGTGTCCCTCAGTTCAAGAAAGTTGTTTTCCAG GAATTTACTGATGGCTCCTTTACTCAGCCCTTATACCGTGGAGAACT AAATGAACATTTGGGACTCCTGGGGCCATATATAAGAGCAGAAGTTG AAGATAATATCATGGTAACTTTCAGAAATCAGGCCTCTCGTCCCTATT CCTTCTATTCTAGCCTTATTTCTTATGAGGAAGATCAGAGGCAAGGA GCAGAACCTAGAAAAAACTTTGTCAAGCCTAATGAAACCAAAACTTA CTTTTTGGAAAGTGCAACATCATATGGCACCCACTAAAGATGAGTTTG ACTGCAAAGCCTGGGCTTATTTCTCTGATGTTGACCTGGAAAAAGAT GTGCACTCAGGCCTGATTGGACCCCTTCTGGTCTGCCACACTAACA CACTGAACCCTGCTCATGGGAGACAAGTGACAGTACAGGAATTTGC TCTGTTTTTCACCATCTTTGATGAGACCAAAAGCTGGTACTTCACTGA AAATATGGAAAGAAACTGCAGGGCTCCCTGCAATATCCAGATGGAA GATCCCACTTTTAAAGAGAATTATCGCTTCCATGCAATCAATGGCTA CATAATGGATACACTACCTGGCTTAGTAATGGCTCAGGATCAAAGGA TTCGATGGTATCTGCTCAGCATGGGCAGCAATGAAAACATCCATTCT ATTCATTTCAGTGGACATGTGTTCACTGTACGAAAAAAAGAGGAGTA TAAAATGGCACTGTACAATCTCTATCCAGGTGTTTTTGAGACAGTGG AAATGTTACCATCCAAAGCTGGAATTTGGCGGGTGGAATGCCTTATT GGCGAGCATCTACATGCTGGGATGAGCACACTTTTTCTGGTGTACA GCAATAAGTGTCAGACTCCCCTGGGAATGGCTTCTGGACACATTAG AGATTTTCAGATTACAGCTTCAGGACAATATGGACAGTGGGCCCCAA AGCTGGCCAGACTTCATTATTCCGGATCAATCAATGCCTGGAGCACC AAGGAGCCCTTTTCTTGGATCAAGGTGGATCTGTTGGCACCAATGAT TATTCACGGCATCAAGACCCAGGGTGCCCGTCAGAAGTTCTCCAGC |
| | CTCTACATCTCTCAGTTTATCATCATGTATAGTCTTGATGGGAAGAAG TGGCAGACTTATCGAGGAAATTCCACTGGAACCTTAATGGTCTTCTT TGGCAATGTGGATTCATCTGGGATAAAACACAATATTTTTAACCCTCC AATTATTGCTCGATACATCCGTTTGCACCCAACTCATTATAGCATTCG CAGCACTCTTCGCATGGAGTTGATGGGCTGTGATTTAAATAGTTGCA GCATGCCATTGGGAATGGAGAGTAAAGCAATATCAGATGCACAGATT ACTGCTTCATCCTACTTTACCAATATGTTTGCCACCTGGTCTCCTTCA AAAGCTCGACTTCACCTCCAAGGGAGGAGTAATGCCTGGAGACCTC AGGTGAATAATCCAAAAGAGTGGCTGCAAGTGGACTTCCAGAAGAC AATGAAAGTCACAGGAGTAACTACTCAGGGAGTAAAATCTCTGCTTA CCAGCATGTATGTGAAGGAGTTCCTCATCTCCAGCAGTCAAGATGG CCATCAGTGGACTCTCTTTTTTCAGAATGGCAAAGTAAAGGTTTTTCA GGGAAATCAAGACTCCTTCACACCTGTGGTGAACTCTCTAGACCCAC CGTTACTGACTCGCTACCTTCGAATTCACCCCCAGAGTTGGGTGCA CCAGATTGCCCTGAGGATGGAGGTTCTGGGCTGCGAGGCACAGGA CCTCTAC |
| SEQ ID NO: 59 | ATGCAAATAGAGCTCTCCACCTGCTTCTTTCTGTGCCTTTTGCGATT CTGCTTTAGTGCCACCAGAAGATACTACCTGGGTGCAGTGGAACTG TCATGGGACTATATGCAAAGTGATCTCGGTGAGCTGCCTGTGGACG CAAGATTTCCTCCTAGAGTGCCAAAATCTTTTCCATTCAACACCTCAG TCGTGTACAAAAAGACTCTGTTTGTAGAATTCACGGATCACCTTTTCA ACATCGCTAAGCCAAGGCCACCCTGGATGGGTCTGCTAGGTCCTAC CATCCAGGCTGAGGTTTATGATACAGTGGTCATTACACTTAAGAACA TGGCTTCCCATCCTGTCAGTCTTCATGCTGTTGGTGTATCCTACTGG AAAGCTTCTGAGGGAGCTGAATATGATGATCAGACCAGTCAAAGGG AGAAAGAAGATGATAAAGTCTTCCCTGGTGGAAGCCATACATATGTC TGGCAGGTCCTGAAAGAGAATGGTCCAATGGCCTCTGACCCACTGT GCCTTACCTACTCATATCTTTCTCATGTGGACCTGGTAAAAGACTTG AATTCAGGCCTCATTGGAGCCCTACTAGTATGTAGAGAAGGGAGTCT GGCCAAGGAAAAGACACAGACCTTGCACAAATTTATACTACTTTTTG CTGTATTTGATGAAGGGAAAAGTTGGCACTCAGAAACAAAGAACTCC TTGATGCAGGATAGGGATGCTGCATCTGCTCGGGCCTGGCCTAAAA TGCACACAGTCAATGGTTATGTAAACAGGTCTCTGCCAGGTCTGATT GGATGCCACAGGAAATCAGTCTATTGGCATGTGATTGGAATGGGCA CCACTCCTGAAGTGCACTCAATATTCCTCGAAGGTCACACATTTCTT GTGAGGAACCATCGCCAGGCGTCCTTGGAAATCTCGCCAATAACTT TCCTTACTGCTCAAACACTCTTGATGGACCTTGGACAGTTTCTACTG TTTTGTCATATCTCTTCCCACCAACATGATGGCATGGAAGCTTATGTC AAAGTAGACAGCTGTCCAGAGGAACCCCAACTACGAATGAAAAATAA TGAAGAAGCGGAAGACTATGATGATGATCTTACTGATTCTGAAATGG ATGTGGTCAGGTTTGATGATGACAACTCTCCTTCCTTTATCCAAATTC GCTCAGTTGCCAAGAAGCATCCTAAAACTTGGGTACATTACATTGCT GCTGAAGAGGAGGACTGGGACTATGCTCCCTTAGTCCTCGCCCCCG ATGACAGAAGTTATAAAAGTCAATATTTGAACAATGGCCCTCAGCGG ATTGGTAGGAAGTACAAAAAAGTCCGATTTATGGCATACACAGATGA AACCTTTAAGACTCGTGAAGCTATTCAGCATGAATCAGGAATCTTGG GACCTTTACTTTATGGGGAAGTTGGAGACACACTGTTGATTATATTTA AGAATCAAGCAAGCAGACCATATAACATCTACCCTCACGGAATCACT GATGTCCGTCCTTTGTATTCAAGGAGATTACCAAAAGGTGTAAAACA TTTGAAGGATTTTCCAATTCTGCCAGGAGAAATATTCAAATATAAATG GACAGTGACTGTAGAAGATGGGCCAACTAAATCAGATCCTCGGTGC CTGACCCGCTATTACTCTAGTTTCGTTAATATGGAGAGAGATCTAGC |
| | TTCAGGACTCATTGGCCCTCTCCTCATCTGCTACAAAGAATCTGTAG ATCAAAGAGGAAACCAGATAATGTCAGACAAGAGGAATGTCATCCTG TTTTCTGTATTTGATGAGAACCGAAGCTGGTACCTCACAGAGAATAT ACAACGCTTTCTCCCCAATCCAGCTGGAGTGCAGCTTGAGGATCCA GAGTTCCAAGCCTCCAACATCATGCACAGCATCAATGGCTATGTTTT TGATAGTTTGCAGTTGTCAGTTTGTTTGCATGAGGTGGCATACTGGT ACATTCTAAGCATTGGAGCACAGACTGACTTCCTTTCTGTCTTCTTCT CTGGATATACCTTCAAACACAAAATGGTCTATGAAGACACACTCACC CTATTCCCATTCTCAGGAGAAACTGTCTTCATGTCGATGGAAAACCC AGGTCTATGGATTCTGGGGTGCCACAACTCAGACTTTCGGAACAGA GGCATGACCGCCTTACTGAAGGTTTCTAGTTGTGACAAGAACACTG GTGATTATTACGAGGACAGTTATGAAGATATTTCAGCATACTTGCTG AGTAAAAACAATGCCATTGAACCAAGAAGCTTCTCCCAGAATTCAAG ACACCCTAGCACTAGGCAAAAGCAATTTAATGCCACCACAATTCCAG AAAATGACATAGAGAAGACTGACCCTTGGTTTGCACACAGAACACCT ATGCCTAAAATACAAAATGTCTCCTCTAGTGATTTGTTGATGCTCTTG CGACAGAGTCCTACTCCACATGGGCTATCCTTATCTGATCTCCAAGA AGCCAAATATGAGACTTTTTCTGATGATCCATCACCTGGAGCAATAG ACAGTAATAACAGCCTGTCTGAAATGACACACTTCAGGCCACAGCTC CATCACAGTGGGGACATGGTATTTACCCCTGAGTCAGGCCTCCAATT AAGATTAAATGAGAAACTGGGGACAACTGCAGCAACAGAGTTGAAG AAACTTGATTTCAAAGTTTCTAGTACATCAAATAATCTGATTTCAACAA TTCCATCAGACAATTTGGCAGCAGGTACTGATAATACAAGTTCCTTA GGACCCCCAAGTATGCCAGTTCATTATGATAGTCAATTAGATACCAC TCTATTTGGCAAAAAGTCATCTCCCCTTACTGAGTCTGGTGGACCTC TGAGCTTGAGTGAAGAAAATAATGATTCAAAGTTGTTAGAATCAGGT TTAATGAATAGCCAAGAAAGTTCATGGGGAAAAAATGTATCGTCAAC AGAGAGTGGTAGGTTATTTAAAGGGAAAAGAGCTCATGGACCTGCTT TGTTGACTAAAGATAATGCCTTATTCAAAGTTAGCATCTCTTTGTTAA AGACAAACAAAACTTCCAATAATTCAGCAACTAATAGAAAGACTCACA TTGATGGCCCATCATTATTAATTGAGAATAGTCCATCAGTCTGGCAA AATATATTAGAAAGTGACACTGAGTTTAAAAAAGTGACACCTTTGATT CATGACAGAATGCTTATGGACAAAAATGCTACAGCTTTGAGGCTAAA TCATATGTCAAATAAAACTACTTCATCAAAAAACATGGAAATGGTCCA ACAGAAAAAAGAGGGCCCCATTCCACCAGATGCACAAAATCCAGAT ATGTCGTTCTTTAAGATGCTATTCTTGCCAGAATCAGCAAGGTGGAT ACAAAGGACTCATGGAAAGAACTCTCTGAACTCTGGGCAAGGCCCC AGTCCAAAGCAATTAGTATCCTTAGGACCAGAAAAATCTGTGGAAGG TCAGAATTTCTTGTCTGAGAAAAACAAAGTGGTAGTAGGAAAGGGTG AATTTACAAAGGACGTAGGACTCAAAGAGATGGTTTTTCCAAGCAGC AGAAACCTATTTCTTACTAACTTGGATAATTTACATGAAAATAATACA CACAATCAAGAAAAAAAAATTCAGGAAGAAATAGAAAAGAAGGAAAC ATTAATCCAAGAGAATGTAGTTTTGCCTCAGATACATACAGTGACTG GCACTAAGAATTTCATGAAGAACCTTTTCTTACTGAGCACTAGGCAA AATGTAGAAGGTTCATATGACGGGGCATATGCTCCAGTACTTCAAGA TTTTAGGTCATTAAATGATTCAACAAATAGAACAAAGAAACACACAGC TCATTTCTCAAAAAAAGGGGAGGAAGAAAACTTGGAAGGCTTGGGA AATCAAACCAAGCAAATTGTAGAGAAATATGCATGCACCACAAGGAT ATCTCCTAATACAAGCCAGCAGAATTTTGTCACGCAACGTAGTAAGA GAGCTTTGAAACAATTCAGACTCCCACTAGAAGAAACAGAACTTGAA AAAAGGATAATTGTGGATGACACCTCAACCCAGTGGTCCAAAAACAT GAAACATTTGACCCCGAGCACCCTCACACAGATAGACTACAATGAGA AGGAGAAAGGGGCCATTACTCAGTCTCCCTTATCAGATTGCCTTACG |
| | AGGAGTCATAGCATCCCTCAAGCAAATAGATCTCCATTACCCATTGC AAAGGTATCATCATTTCCATCTATTAGACCTATATATCTGACCAGGGT CCTATTCCAAGACAACTCTTCTCATCTTCCAGCAGCATCTTATAGAAA GAAAGATTCTGGGGTCCAAGAAAGCAGTCATTTCTTACAAGGAGCCA AAAAAAATAACCTTTCTTTAGCCATTCTAACCTTGGAGATGACTGGTG ATCAAAGAGAGGTTGGCTCCCTGGGGACAAGTGCCACAAATTCAGT CACATACAAGAAAGTTGAGAACACTGTTCTCCCGAAACCAGACTTGC CCAAAACATCTGGCAAAGTTGAATTGCTTCCAAAAGTTCACATTTATC AGAAGGACCTATTCCCTACGGAAACTAGCAATGGGTCTCCTGGCCA TCTGGATCTCGTGGAAGGGAGCCTTCTTCAGGGAACAGAGGGAGC GATTAAGTGGAATGAAGCAAACAGACCTGGAAAAGTTCCCTTTCTGA GAGTAGCAACAGAAAGCTCTGCAAAGACTCCCTCCAAGCTATTGGAT CCTCTTGCTTGGGATAACCACTATGGTACTCAGATACCAAAAGAAGA GTGGAAATCCCAAGAGAAGTCACCAGAAAAAACAGCTTTTAAGAAAA AGGATACCATTTTGTCCCTGAACGCTTGTGAAAGCAATCATGCAATA GCAGCAATAAATGAGGGACAAAATAAGCCCGAAATAGAAGTCACCT GGGCAAAGCAAGGTAGGACTGAAAGGCTGTGCTCTCAAAACCCACC AGTCTTGAAACGCCATCAACGGGAAATAACTCGTACTACTCTTCAGT CAGATCAAGAGGAAATTGACTATGATGATACCATATCAGTTGAAATG AAGAAGGAAGATTTTGACATTTATGATGAGGATGAAAATCAGAGCCC CCGCAGCTTTCAAAAGAAAACACGACACTATTTTATTGCTGCAGTGG AGAGGCTCTGGGATTATGGGATGAGTAGCTCCCCACATGTTCTAAG AAACAGGGCTCAGAGTGGCAGTGTCCCTCAGTTCAAGAAAGTTGTTT TCCAGGAATTTACTGATGGCTCCTTTACTCAGCCCTTATACCGTGGA GAACTAAATGAACATTTGGGACTCCTGGGGCCATATATAAGAGCAGA AGTTGAAGATAATATCATGGTAACTTTCAGAAATCAGGCCTCTCGTC CCTATTCCTTCTATTCTAGCCTTATTTCTTATGAGGAAGATCAGAGGC AAGGAGCAGAACCTAGAAAAAACTTTGTCAAGCCTAATGAAACCAAA ACTTACTTTTGGAAAGTGCAACATCATATGGCACCCACTAAAGATGA GTTTGACTGCAAAGCCTGGGCTTATTTCTCTGATGTTGACCTGGAAA AAGATGTGCACTCAGGCCTGATTGGACCCCTTCTGGTCTGCCACAC TAACACACTGAACCCTGCTCATGGGAGACAAGTGACAGTACAGGAA TTTGCTCTGTTTTTCACCATCTTTGATGAGACCAAAAGCTGGTACTTC ACTGAAAATATGGAAAGAAACTGCAGGGCTCCCTGCAATATCCAGAT GGAAGATCCCACTTTTAAAGAGAATTATCGCTTCCATGCAATCAATG GCTACATAATGGATACACTACCTGGCTTAGTAATGGCTCAGGATCAA AGGATTCGATGGTATCTGCTCAGCATGGGCAGCAATGAAAACATCC ATTCTATTCATTTCAGTGGACATGTGTTCACTGTACGAAAAAAAGAG GAGTATAAAATGGCACTGTACAATCTCTATCCAGGTGTTTTTGAGAC AGTGGAAATGTTACCATCCAAAGCTGGAATTTGGCGGGTGGAATGC CTTATTGGCGAGCATCTACATGCTGGGATGAGCACACTTTTTCTGGT GTACAGCAATAAGTGTCAGACTCCCCTGGGAATGGCTTCTGGACAC ATTAGAGATTTTCAGATTACAGCTTCAGGACAATATGGACAGTGGGC CCCAAAGCTGGCCAGACTTCATTATTCCGGATCAATCAATGCCTGGA GCACCAAGGAGCCCTTTTCTTGGATCAAGGTGGATCTGTTGGCACC AATGATTATTCACGGCATCAAGACCCAGGGTGCCCGTCAGAAGTTCT CCAGCCTCTACATCTCTCAGTTTATCATCATGTATAGTCTTGATGGGA AGAAGTGGCAGACTTATCGAGGAAATTCCACTGGAACCTTAATGGTC TTCTTTGGCAATGTGGATTCATCTGGGATAAAACACAATATTTTTAAC CCTCCAATTATTGCTCGATACATCCGTTTGCACCCAACTCATTATAGC ATTCGCAGCACTCTTCGCATGGAGTTGATGGGCTGTGATTTAAATAG TTGCAGCATGCCATTGGGAATGGAGAGTAAAGCAATATCAGATGCA CAGATTACTGCTTCATCCTACTTTACCAATATGTTTGCCACCTGGTCT |
| | CCTTCAAAAGCTCGACTTCACCTCCAAGGGAGGAGTAATGCCTGGA GACCTCAGGTGAATAATCCAAAAGAGTGGCTGCAAGTGGACTTCCA GAAGACAATGAAAGTCACAGGAGTAACTACTCAGGGAGTAAAATCTC TGCTTACCAGCATGTATGTGAAGGAGTTCCTCATCTCCAGCAGTCAA GATGGCCATCAGTGGACTCTCTTTTTTCAGAATGGCAAAGTAAAGGT TTTTCAGGGAAATCAAGACTCCTTCACACCTGTGGTGAACTCTCTAG ACCCACCGTTACTGACTCGCTACCTTCGAATTCACCCCCAGAGTTGG GTGCACCAGATTGCCCTGAGGATGGAGGTTCTGGGCTGCGAGGCA CAGGACCTCTAC |
| SEQ ID NO: 60 | ATGAAGTGGGTAACCTTTATTTCCCTTCTTTTTCTCTTTAGCTCGGCT TATTCCAGGGGTGTGTTTCGTCGAGATGCACACAAGAGTGAGGTTG CTCATCGGTTTAAAGATTTGGGAGAAGAAAATTTCAAAGCCTTGGTG TTGATTGCCTTTGCTCAGTATCTTCAGCAGTGTCCATTTGAAGATCAT GTAAAATTAGTGAATGAAGTAACTGAATTTGCAAAAACATGTGTTGCT GATGAGTCAGCTGAAAATTGTGACAAATCACTTCATACCCTTTTTGG AGACAAATTATGCACAGTTGCAACTCTTCGTGAAACCTATGGTGAAA TGGCTGACTGCTGTGCAAAACAAGAACCTGAGAGAAATGAATGCTTC TTGCAACACAAAGATGACAACCCAAACCTCCCCCGATTGGTGAGAC CAGAGGTTGATGTGATGTGCACTGCTTTTCATGACAATGAAGAGACA TTTTTGAAAAAATACTTATATGAAATTGCCAGAAGACATCCTTACTTTT ATGCCCCGGAACTCCTTTTCTTTGCTAAAAGGTATAAAGCTGCTTTTA CAGAATGTTGCCAAGCTGCTGATAAAGCTGCCTGCCTGTTGCCAAA GCTCGATGAACTTCGGGATGAAGGGAAGGCTTCGTCTGCCAAACAG AGACTCAAGTGTGCCAGTCTCCAAAAATTTGGAGAAAGAGCTTTCAA AGCATGGGCAGTAGCTCGCCTGAGCCAGAGATTTCCCAAAGCTGAG TTTGCAGAAGTTTCCAAGTTAGTGACAGATCTTACCAAAGTCCACAC GGAATGCTGCCATGGAGATCTGCTTGAATGTGCTGATGACAGGGCG GACCTTGCCAAGTATATCTGTGAAAATCAAGATTCGATCTCCAGTAA ACTGAAGGAATGCTGTGAAAAACCTCTGTTGGAAAAATCCCACTGCA TTGCCGAAGTGGAAAATGATGAGATGCCTGCTGACTTGCCTTCATTA GCTGCTGATTTTGTTGAAAGTAAGGATGTTTGCAAAAACTATGCTGA GGCAAAGGATGTCTTCCTGGGCATGTTTTTGTATGAATATGCAAGAA GGCATCCTGATTACTCTGTCGTGCTGCTGCTGAGACTTGCCAAGAC ATATGAAACCACTCTAGAGAAGTGCTGTGCCGCTGCAGATCCTCATG AATGCTATGCCAAAGTGTTCGATGAATTTAAACCTCTTGTGGAAGAG CCTCAGAATTTAATCAAACAAAATTGTGAGCTTTTTGAGCAGCTTGGA GAGTACAAATTCCAGAATGCGCTATTAGTTCGTTACACCAAGAAAGT ACCCCAAGTGTCAACTCCAACTCTTGTAGAGGTCTCAAGAAACCTAG GAAAAGTGGGCAGCAAATGTTGTAAACATCCTGAAGCAAAAAGAATG CCCTGTGCAGAAGACTATCTATCCGTGGTCCTGAACCAGTTATGTGT GTTGCATGAGAAAACGCCAGTAAGTGACAGAGTCACCAAATGCTGC ACAGAATCCTTGGTGAACAGGCGACCATGCTTTTCAGCTCTGGAAGT CGATGAAACATACGTTCCCAAAGAGTTTAATGCTGAAACATTCACCT TCCATGCAGATATATGCACACTTTCTGAGAAGGAGAGACAAATCAAG AAACAAACTGCACTTGTTGAGCTCGTGAAACACAAGCCCAAGGCAA CAAAAGAGCAACTGAAAGCTGTTATGGATGATTTCGCAGCTTTTGTA GAGAAGTGCTGCAAGGCTGACGATAAGGAGACCTGCTTTGCCGAGG AGGGTAAAAAACTTGTTGCTGCAAGTCAAGCTGCCTTAGGCTTA |
| SEQ ID NO: 61 | GCCACCATGCAGCGCGTGAACATGATCATGGCAGAATCACCAGGCC TCATCACCATCTGCCTTTTAGGATATCTACTCAGTGCTGAATGTACA GTTTTTCTTGATCATGAAAACGCCAACAAAATTCTGAATCGGCCAAA GAGGTATAATTCAGGTAAATTGGAAGAGTTTGTTCAAGGGAACCTTG |
| | AGAGAGAATGTATGGAAGAAAAGTGTAGTTTTGAAGAAGCACGAGAA GTTTTTGAAAACACTGAAAGAACAACTGAATTTTGGAAGCAGTATGTT GATGGAGATCAGTGTGAGTCCAATCCATGTTTAAATGGCGGCAGTT GCAAGGATGACATTAATTCCTATGAATGTTGGTGTCCCTTTGGATTT GAAGGAAAGAACTGTGAATTAGATGTAACATGTAACATTAAGAATGG CAGATGCGAGCAGTTTTGTAAAAATAGTGCTGATAACAAGGTGGTTT GCTCCTGTACTGAGGGATATCGACTTGCAGAAAACCAGAAGTCCTG TGAACCAGCAGTGCCATTTCCATGTGGAAGAGTTTCTGTTTCACAAA CTTCTAAGCTCACCCGTGCTGAGACTGTTTTTCCTGATGTGGACTAT GTAAATTCTACTGAAGCTGAAACCATTTTGGATAACATCACTCAAAGC ACCCAATCATTTAATGACTTCACTCGGGTTGTTGGTGGAGAAGATGC CAAACCAGGTCAATTCCCTTGGCAGGTTGTTTTGAATGGTAAAGTTG ATGCATTCTGTGGAGGCTCTATCGTTAATGAAAAATGGATTGTAACT GCTGCCCACTGTGTTGAAACTGGTGTTAAAATTACAGTTGTCGCAGG TGAACATAATATTGAGGAGACAGAACATACAGAGCAAAAGCGAAATG TGATTCGAATTATTCCTCACCACAACTACAATGCAGCTATTAATAAGT ACAACCATGACATTGCCCTTCTGGAACTGGACGAACCCTTAGTGCTA AACAGCTACGTTACACCTATTTGCATTGCTGACAAGGAATACACGAA CATCTTCCTCAAATTTGGATCTGGCTATGTAAGTGGCTGGGGAAGAG TCTTCCACAAAGGGAGATCAGCTTTAGTTCTTCAGTACCTTAGAGTT CCACTTGTTGACCGAGCCACATGTCTTCGATCTACAAAGTTCACCAT CTATAACAACATGTTCTGTGCTGGCTTCCATGAAGGAGGTAGAGATT CATGTCAAGGAGATAGTGGGGGACCCCATGTTACTGAAGTGGAAGG GACCAGTTTCTTAACTGGAATTATTAGCTGGGGTGAAGAGTGTGCAA TGAAAGGCAAATATGGAATATATACCAAGGTATCCCGGTATGTCAAC TGGATTAAGGAAAAAACAAAGCTCACT |
| SEQ ID NO: 62 | ATGGTGGACGCTTTCCTGGGCACCTGGAAGCTAGTGGACAGCAAGA ATTTCGATGACTACATGAAGTCACTCGCTCATATACTCATAACCTTCC CCCTACCCTCAGGTGTGGGTTTTGCTACCAGGCAGGTGGCCAGCAT GACCAAGCCTACCACAATCATCGAAAAGAATGGGGACATTCTCACC CTAAAAACACACAGCACCTTCAAGAACACAGAGATCAGCTTTAAGTT GGGGGTGGAGTTCGATGAGACAACAGCAGATGACAGGAAGGTCAA GTCCATTGTGACACTGGATGGAGGGAAACTTGTTCACCTGCAGAAAT GGGACGGGCAAGAGACCACACTTGTGCGGGAGCTAATTGATGGAAA ACTCATCCTGACACTCACCCACGGCACTGCAGTTTGCACTCGCACTT ATGAGAAAGAGGCA |
| SEQ ID NO: 63 | ATGGGGCTCAGCGACGGGGAATGGCAGTTGGTGCTGAACGTCTGG GGGAAGGTGGAGGCTGACATCCCAGGCCATGGGCAGGAAGTCCTC ATCAGGCTCTTTAAGGGTCACCCAGAGACTCTGGAGAAGTTTGACAA GTTCAAGCACCTGAAGTCAGAGGACGAGATGAAGGCGTCTGAGGAC TTAAAGAAGCATGGTGCCACCGTGCTCACCGCCCTGGGTGGCATCC TTAAGAAGAAGGGGCATCATGAGGCAGAGATTAAGCCCCTGGCACA GTCGCATGCCACCAAGCACAAGATCCCCGTGAAGTACCTGGAGTTC ATCTCGGAATGCATCATCCAGGTTCTGCAGAGCAAGCATCCCGGGG ACTTTGGTGCTGATGCCCAGGGGGCCATGAACAAGGCCCTGGAGCT GTTCCGGAAGGACATGGCCTCCAACTACAAGGAGCTGGGCTTCCAG GGC |
| SEQ ID NO: 64 | ATGGAGAGGAGACGCATCACCTCCGCTGCTCGCCGCTCCTACGTCT CCTCAGGGGAGATGATGGTGGGGGGCCTGGCTCCTGGCCGCCGTC TGGGTCCTGGCACCCGCCTCTCCCTGGCTCGAATGCCCCCTCCACT CCCGACCCGGGTGGATTTCTCCCTGGCTGGGGCACTCAATGCTGG CTTCAAGGAGACCCGGGCCAGTGAGCGGGCAGAGATGATGGAGCT |
| | CAATGACCGCTTTGCCAGCTACATCGAGAAGGTTCGCTTCCTGGAA CAGCAAAACAAGGCGCTGGCTGCTGAGCTGAACCAGCTGCGGCC AAGGAGCCCACCAAGCTGGCAGACGTCTACCAGGCTGAGCTGCGA GAGCTGCGGCTGCGGCTCGATCAACTCACCGCCAACAGCGCCCGG CTGGAGGTTGAGAGGGACAATCTGGCACAGGACCTGGCCACTGTG AGGCAGAAGCTCCAGGATGAAACCAACCTGAGGCTGGAAGCCGAG AACAACCTGGCTGCCTATAGACAGGAAGCAGATGAAGCCACCCTGG CCCGTCTGGATCTGGAGAGGAAGATTGAGTCGCTGGAGGAGGAGA TCCGGTTCTTGAGGAAGATCCACGAGGAGGAGGTTCGGGAACTCCA GGAGCAGCTGGCCCGACAGCAGGTCCATGTGGAGCTTGACGTGGC CAAGCCAGACCTCACCGCAGCCCTGAAAGAGATCCGCACGCAGTAT GAGGCAATGGCGTCCAGCAACATGCATGAAGCCGAAGAGTGGTACC GCTCCAAGTTTGCAGACCTGACAGACGCTGCTGCCCGCAACGCGGA GCTGCTCCGCCAGGCCAAGCACGAAGCCAACGACTACCGGCGCCA GTTGCAGTCCTTGACCTGCGACCTGGAGTCTCTGCGCGGCACGAAC GAGTCCCTGGAGAGGCAGATGCGCGAGCAGGAGGAGCGGCACGT GCGGGAGGCGGCCAGTTATCAGGAGGCGCTGGCGCGGCTGGAGG AAGAGGGGCAGAGCCTCAAGGACGAGATGGCCCGCCACTTGCAGG AGTACCAGGACCTGCTCAATGTCAAGCTGGCCCTGGACATCGAGAT CGCCACCTACAGGAAGCTGCTAGAGGGCGAGGAGAACCGGATCAC CATTCCCGTGCAGACCTTCTCCAACCTGCAGATTCGAGGGGGCAAA AGCACCAAAGACGGGGAAAATCACAAGGTCACAAGATATCTCAAAA GCCTCACAATACGAGTTATACCAATACAGGCTCACCAGATTGTAAAT GGAACGCCGCCGGCTCGCGGT |
| SEQ ID NO: 65 | ATGTCTGAGCTGGAGAAGGCCATGGTGGCCCTCATCGACGTTTTCC ACCAATATTCTGGAAGGGAGGGAGACAAGCACAAGCTGAAGAAATC CGAACTGAAGGAGCTCATCAACAATGAGCTTTCCCATTTCTTAGAGG AAATCAAAGAGCAGGAGGTTGTGGACAAAGTCATGGAAACACTGGA CAATGATGGAGACGGCGAATGTGACTTCCAGGAATTCATGGCCTTT GTTGCCATGGTTACTACTGCCTGCCACGAGTTCTTTGAACATGAG |
| SEQ ID NO: 66 | GCCCCTGGAGGAACTGAACCCACTATCGGTCATGGGGCCGAGACTA AATGTGGCGGGTTGTCTTTAATCTGCTGCCAAGAGGAAACTCATTCA GGCAAGTTCAGCCCTTTATGAGGAATTCCCCTGTGGTCACATTCCAA TTCCTGGACCTGCTGCCACCCTCAGAACTGCATGCTCCTTCTTCAGA CTTTCTAAGAATGACTCAGGTCATTGGTGGAGTGAAGTCAAGATTTC CAACTCAGTCACCTGAAGAGATGGAGATACCATTCATGGAGCTGGA GGTCCCTGGAGATTTGGGAATTCAGATAACAAGCTAAGATAAGGAGT TTGCCTACCTCTGTCCTAGAGCGAAGCCTGAGCCTTGGGCGCGCAG CACACCACAAGTATCTGTTACTGTGTTTTGCAGAAGCTTCAGGCGGG GATATAAGCCCCACAAGGAAAGCGCTGAGCAGAGGAGGCCTCAGCT TGACCTGCGGCAGTGCAGCCCTTGGGACTTCCCTCGCCTTCCACCT CCTGCTCGTCTGCTTCACAAGCTATCGCTATGGTGTTCGTGCGCAG GCCGTGGCCCGCCTTGACCACAGTGCTTCTGGCCCTGCTCGTCTGC CTAGGGGCGCTGGTCGACGCCTACCCCATCAAACCCGAGGCTCCC GGCGAAGACGCCTCGCCGGAGGAGCTGAACCGCTACTACGCCTCC CTGCGCCACTACCTCAACCTGGTCACCCGGCAGCGGTATGGAAAA GAGACGGCCCGGACACGCTTCTTTCCAAAACGTTCTTCCCCGACGG CGAGGACCGCCCCGTCAGGTCGCGGTCGGAGGGCCCAGACCTGTG GTGAGGACCCCTGAGGCCTCCTGGGAGATCTGCCAACCACGCCCA CGTCATTTGCATACGCACTCCCGACCCCAGAAACCCGGATTCTGCC TCCCGACGGCGGCGTCTGGGCAGGGTTCGGGTGCGGCCCTCCGC CCGCGTCTCGGTGCCCCCGCCCCCTGGGCTGGAGGGCTGTGTGTG |
| | GTCCTTCCCTGGTCCCAAAATAAAGAGCAAATTCCACAGAAACGGAA AAAAAAAAAAA |
| SEQ ID NO: 67 | CGCAGCAAACACATCCGTAGAAGGCAGCGCGGCCGCCGAGAACCG CAGCGCCGCTCGCCCGCCGCCCCCACCCCGCCGCCCCGCCCGG CGAATTGCGCCCCGCGCCCCTCCCCTCGCGCCCCCGAGACAAAGA GGAGAGAAAGTTTGCGCGGCCGAGCGGGGCAGGTGAGGAGGGTG AGCCGCGCGGGAGGGGCCCGCCTCGGCCCCGGCTCAGCCCCCGC CCGCGCCCCCAGCCCGCCGCCGCGAGCAGCGCCCGGACCCCCCA GCGGCGGCCCCCGCCCGCCCAGCCCCCCGGCCCGCCATGGGCGC CGCGGCCCGCACCCTGCGGCTGGCGCTCGGCCTCCTGCTGCTGGC GACGCTGCTTCGCCCGGCCGACGCCTGCAGCTGCTCCCCGGTGCA CCCGCAACAGGCGTTTTGCAATGCAGATGTAGTGATCAGGGCCAAA GCGGTCAGTGAGAAGGAAGTGGACTCTGGAAACGACATTTATGGCA ACCCTATCAAGAGGATCCAGTATGAGATCAAGCAGATAAAGATGTTC AAAGGGCCTGAGAAGGATATAGAGTTTATCTACACGGCCCCCTCCT CGGCAGTGTGTGGGGTCTCGCTGGACGTTGGAGGAAAGAAGGAAT ATCTCATTGCAGGAAAGGCCGAGGGGGACGGCAAGATGCACATCAC CCTCTGTGACTTCATCGTGCCCTGGGACACCCTGAGCACCACCCAG AAGAAGAGCCTGAACCACAGGTACCAGATGGGCTGCGAGTGCAAGA TCACGCGCTGCCCCATGATCCCGTGCTACATCTCCTCCCCGGACGA GTGCCTCTGGATGGACTGGGTCACAGAGAAGAACATCAACGGGCAC CAGGCCAAGTTCTTCGCCTGCATCAAGAGAAGTGACGGCTCCTGTG CGTGGTACCGCGGCGCGGCGCCCCCCAAGCAGGAGTTTCTCGACA TCGAGGACCCATAAGCAGGCCTCCAACGCCCCTGTGGCCAACTGCA AAAAAAGCCTCCAAGGGTTTCGATCTGACATCCCTTCCTGGAAACAG CATGAATAAAACACTCATCCCATGGGTCCAAATTAATATGATTCTGCT CCCCCCTTCTCCTTTTAGACATGGTTGTGGGTCTGGAGGGAGACGT GGGTCCAAGGTCCTCATCCCATCCTCCCTCTGCCAGGCACTATGTG TCTGGGGCTTCGATCCTTGGGTGCAGGCAGGGCTGGGACACGCGG CTTCCCTCCCAGTCCCTGCCTTGGCACCGTCACAGATGCCAAGCAG GCAGCACTTAGGGATCTCCCAGCTGGGTTAGGGCAGGGCCTGGAA ATGTGCATTTTGCAGAAACTTTTGAGGGTCGTTGCAAGACTGTGTAG CAGGCCTACCAGGTCCCTTTCATCTTGAGAGGGACATGGCCCTTGT TTTCTGCAGCTTCCACGCCTCTGCACTCCCTGCCCCTGGCAAGTGC TCCCATCGCCCCGGTGCCCACCATGAGCTCCCAGCACCTGACTCCC CCCACATCCAAGGGCAGCCTGGAACCAGTGGCTAGTTCTTGAAGGA GCCCCATCAATCCTATTAATCCTCAGAATTCCAGTGGGAGCCTCCCT CTGAGCCTTGTAGAAATGGGAGCGAGAAACCCCAGCTGAGCTGCGT TCCAGCCTCAGCTGAGTCTTTTTGGTCTGCACCCACCCCCCCACCC CCCCCCCCCCGCCCACATGCTCCCCAGCTTGCAGGAGGAATCGGT GAGGTCCTGTCCTGAGGCTGCTGTCCGGGGCCGGTGGCTGCCCTC AAGGTCCCTTCCCTAGCTGCTGCGGTTGCCATTGCTTCTTGCCTGTT CTGGCATCAGGCACCTGGATTGAGTTGCACAGCTTTGCTTTATCCG GGCTTGTGTGCAGGGCCCGGCTGGGCTCCCCATCTGCACATCCTG AGGACAGAAAAAGCTGGGTCTTGCTGTGCCCTCCCAGGCTTAGTGT TCCCTCCCTCAAAGACTGACAGCCATCGTTCTGCACGGGGCTTTCT GCATGTGACGCCAGCTAAGCATAGTAAGAAGTCCAGCCTAGGAAGG GAAGGATTTTGGAGGTAGGTGGCTTTGGTGACACACTCACTTCTTTC TCAGCCTCCAGGACACTATGGCCTGTTTTAAGAGACATCTTATTTTTC TAAAGGTGAATTCTCAGATGATAGGTGAACCTGAGTTGCAGATATAC CAACTTCTGCTTGTATTTCTTAAATGACAAAGATTACCTAGCTAAGAA ACTTCCTAGGGAACTAGGGAACCTATGTGTTCCCTCAGTGTGGTTTC |
| | CTGAAGCCAGTGATATGGGGGTTAGGATAGGAAGAACTTTCTCGGT AATGATAAGGAGAATCTCTTGTTTCCTCCCACCTGTGTTGTAAAGATA AACTGACGATATACAGGCACATTATGTAAACATACACACGCAATGAA ACCGAAGCTTGGCGGCCTGGGCGTGGTCTTGCAAAATGCTTCCAAA GCCACCTTAGCCTGTTCTATTCAGCGGCAACCCCAAAGCACCTGTTA AGACTCCTGACCCCCAAGTGGCATGCAGCCCCCATGCCCACCGGG ACCTGGTCAGCACAGATCTTGATGACTTCCCTTTCTAGGGCAGACTG GGAGGGTATCCAGGAATCGGCCCCTGCCCCACGGGCGTTTTCATG CTGTACAGTGACCTAAAGTTGGTAAGATGTCATAATGGACCAGTCCA TGTGATTTCAGTATATACAACTCCACCAGACCCCTCCAACCCATATA ACACCCCACCCCTGTTCGCTTCCTGTATGGTGATATCATATGTAACA TTTACTCCTGTTTCTGCTGATTGTTTTTTAATGTTTTGGTTTGTTTTT GACATCAGCTGTAATCATTCCTGTGCTGTGTTTTTTATTACCCTTGGT AGGTATTAGACTTGCACTTTTTTAAAAAAAGGTTTCTGCATCGTGGAA GCATTTGACCCAGAGTGGAACGCGTGGCCTATGCAGGTGGATTCCT TCAGGTCTTTCCTTTGGTTCTTTGAGCATCTTTGCTTTCATTCGTCTC CCGTCTTTGGTTCTCCAGTTCAAATTATTGCAAAGTAAAGGATCTTTG AGTAGGTTCGGTCTGAAAGGTGTGGCCTTTATATTTGATCCACACAC GTTGGTCTTTTAACCGTGCTGAGCAGAAAACAAAACAGGTTAAGAAG AGCCGGGTGGCAGCTGACAGAGGAAGCCGCTCAAATACCTTCACAA TAAATAGTGGCAATATATATATAGTTTAAGAAGGCTCTCCATTTGGCA TCGTTTAATTTATATGTTATGTTCTAAGCACAGCTCTCTTCTCCTATTT TCATCCTGCAAGCAACTCAAAATATTTAAAATAAAGTTTACATTGTAG TTATTTTCAAATCTTTGCTTGATAAGTATTAAGAAATATTGGACTTGCT GCCGTAATTTAAAGCTCTGTTGATTTTGTTTCCGTTTGGATTTTTGGG GGAGGGGAGCACTGTGTTTATGCTGGAATATGAAGTCTGAGACCTT CCGGTGCTGGGAACACACAAGAGTTGTTGAAAGTTGACAAGCAGAC TGCGCATGTCTCTGATGCTTTGTATCATTCTTGAGCAATCGCTCGGT CCGTGGACAATAAACAGTATTATCAAAGAGAAAAAAAAAAAAAAAAA |

Having described the invention in detail and by reference to specific aspects and/or embodiments thereof, it will be apparent that modifications and variations are possible without departing from the scope of the invention defined in the appended claims. More specifically, although some aspects of the present invention may be identified herein as particularly advantageous, it is contemplated that the present invention is not limited to these particular aspects of the invention.

In particular, the present invention pertains to the following:
1. A physiologically-tailored tissue organoid, comprising:
   a plurality of genetically engineered cells comprising at least one recombinant gene encoding a reporter molecule,
   wherein the reporter molecule produces a detectable signal when associated with and/or contacting a predetermined blood factor.
2. The physiologically-tailored tissue organoid of item 1, wherein the tissue organoid comprises a stratified skin graft grown from cells taken from an individual.
3. The physiologically-tailored tissue organoid of item 2, wherein the tissue organoid comprises a cultured skin graft grown from embryonic stem cells, human induced pluripotent stem cells, epidermal stem cells, or keratinocytes.
4. The physiologically-tailored tissue organoid of item 1, wherein when the tissue organoid is biontegrated into an individual, the tissue organoid expresses the reporter molecule in proportion to a concentration of a blood factor in the individual's blood.
5. The physiologically-tailored tissue organoid of item 1, wherein the blood factor comprises a cell, an enzyme, a protein, a polypeptide, an amino acid, a polynucleotide, a nucleic acid, a sugar, a lipid, a metabolite, a synthetic chemical compound, a naturally occurring chemical compound, a mineral, a metal, a bacterium, a virus, a prion, a disease indicator, or combinations thereof.
6. The physiologically-tailored tissue organoid of item 1, wherein the physiologically-tailored tissue organoid is biointegrated into an individual.
7. The physiologically-tailored tissue organoid of item 6, wherein the individual is an animal.
8. The physiologically-tailored tissue organoid of item 1, wherein the predetermined blood factor is glucose.
9. A physiologically-tailored tissue organoid, comprising:
   a plurality of genetically engineered cells comprising at least one recombinant gene encoding a therapeutic agent,
   wherein when the therapeutic agent is administered to an individual in need thereof, the therapeutic agent improves the individual's health.
10. The physiologically-tailored tissue organoid of item 9, wherein the therapeutic agent comprises an enzyme, a protein, a clotting factor, a vitamin, a peptide, a lipid, a toxin, or a combination thereof.
11. The physiologically-tailored tissue organoid of item 10, wherein expression of the therapeutic agent is inducible by an inducer.
12. The physiologically-tailored tissue organoid of item 11, wherein the inducer comprises one or more of heat, cold, light, a protein, a hormone, a lipid, a chemical, metabolic changes, an electric potential or field, and combinations thereof.
13. The physiologically-tailored tissue organoid of item 11, wherein when expression of the therapeutic agent is induced, the therapeutic agent is released into the individual's circulation.
14. The physiologically-tailored tissue organoid of item 9, wherein the physiologically-tailored tissue organoid is biointegrated into an individual.
15. The physiologically-tailored tissue organoid of item 14, wherein the individual is an animal.
16. A physiologically-tailored tissue organoid, comprising:
   a plurality of genetically engineered cells comprising at least one recombinant gene encoding a reporter molecule and at least one recombinant gene encoding a therapeutic agent,
   wherein the therapeutic agent is expressed as a function of expression of the reporter molecule.
17. A method of monitoring a blood factor, comprising:
   biointegrating a tissue organoid according to any of items 1-8 or 16 into an individual; and
   detecting a detectable signal produced by a reporter molecule expressed by the tissue organoid in response to a blood factor,
   wherein the detectable signal is proportional to a concentration of the blood factor in the individual.
18. The method of item 17, wherein the tissue organoids are biointegrated into an individual by grafting or surgical implantation.
19. The method of item 17, wherein the detectable signal is detected by one or more of a fluorometer, a colorimeter, a bioluminescence monitoring system, and an electrical system with proper electrodes.
20. A method of treating a disease in an individual in need thereof, comprising:
   biointegrating a tissue organoid according to any of items 9 - 16 into an individual; and
   administering a therapeutic agent to the individual.
21. The method of item 20, wherein the individual is a human.
22. A physiologically-tailored tissue organoid, comprising:
   a population of genetically engineered cells comprising at least one recombinant gene encoding a therapeutic agent;
   wherein the therapeutic agent comprises at least one of mGLP1 and hBChE, and
   wherein when the therapeutic agent is administered to an individual in need thereof, the therapeutic agent improves the individual's health.
23. The physiologically-tailored tissue organoid of item 22, wherein the therapeutic agent improves the individual's health by reducing the individual's seeking and/or consumption of at least one of nicotine, alcohol, cocaine, and amphetamine.
24. A method of treating cocaine addiction in an individual in need thereof, comprising:
   contacting a tissue organoid to the individual, the tissue organoid comprising a population of genetically engineered cells comprising at least one recombinant gene encoding a therapeutic agent, wherein the therapeutic agent comprises hBChE.
25. The method of item 24, wherein the tissue organoid is transplanted into the individual.
26. The method of item 24, wherein hBChE is expressed constitutively or by induction with an inducer.
27. The method of item 24, wherein the expression of hBChE hydrolyzes cocaine in the blood of the individual.
28. The method of item 27, wherein the expression of hBChE causes decreased cocaine seeking and/or consumption by the individual.
29. A method of treating AUD in an individual in need thereof, comprising:
   contacting a tissue organoid to the individual, the tissue organoid comprising a population of genetically engineered cells comprising at least one recombinant gene encoding a therapeutic agent, wherein the therapeutic agent comprises mGLP1 or an analog thereof.
30. The method of item 29, wherein the tissue organoid is transplanted into the individual.
31. The method of item 29, wherein mGLP1 or the analog thereof is expressed constitutively or by induction with an inducer.
32. The method of item 31, wherein the expression of mGLP1 or the analog thereof causes decreased alcohol seeking and/or consumption by the individual.
33. A method of treating nicotine addiction in an individual in need thereof, comprising:
   contacting a tissue organoid to the individual, the tissue organoid comprising a population of genetically engineered cells comprising at least one recombinant gene encoding a therapeutic agent, wherein the therapeutic agent comprises mGLP1 or an analog thereof.
34. The method of item 33, wherein the tissue organoid is transplanted into the individual.
35. The method of item 33, wherein mGLP1 or the analog thereof is expressed constitutively or by induction with an inducer.
36. The method of item 35, wherein the expression of mGLP1 causes decreased nicotine seeking and consumption by the individual.
37. A method of treating amphetamine addiction in an individual in need thereof, comprising:
   contacting a tissue organoid to the individual, the tissue organoid comprising a population of genetically engineered cells comprising at least one recombinant gene encoding a therapeutic agent, wherein the therapeutic agent comprises mGLP1 or an analog thereof.
38. The method of item 37, wherein the tissue organoid is transplanted into the individual.
39. The method of item 37, wherein mGLP1 or the analog thereof is expressed constitutively or by induction with an inducer.
40. The method of item 39, wherein the expression of mGLP1 causes decreased amphetamine seeking and consumption by the individual.
41. A method of treating obesity in an individual in need thereof, comprising:
   contacting a tissue organoid to the individual, the tissue organoid comprising a population of genetically engineered cells comprising at least one recombinant gene encoding a therapeutic agent, wherein the therapeutic agent comprises peptide tyrosine tyrosine (PYY) or an analog thereof.
42. The method of item 41, wherein the tissue organoid is transplanted into the individual.
43. The method of item 41, wherein PYY or the analog thereof is expressed constitutively or by induction with an inducer.
44. The method of item 43, wherein the expression of PYY causes decreased food seeking and consumption by the individual.
45. A method of treating anti-aging effects in an individual in need thereof, comprising:
   contacting a tissue organoid to the individual, the tissue organoid comprising a population of genetically engineered cells comprising at least one recombinant gene encoding a therapeutic agent, wherein the therapeutic agent comprises TIMP2 or an analog thereof.
46. The method of item 45, wherein the tissue organoid is transplanted into the individual.
47. The method of item 45, wherein TIMP2 or the analog thereof is expressed constitutively or by induction with an inducer.
48. The method of item 47, wherein the expression of TIMP2 causes a decrease in the negative effects of aging in an individual.
49. The method item of 48, wherein the negative effects of aging include memory loss, reduced vascular response, and/or reduced immune response.

## Claims

1. A physiologically-tailored tissue organoid, comprising:
a plurality of genetically engineered cells comprising at least one recombinant gene encoding a therapeutic agent,
wherein when the therapeutic agent is administered to an individual in need thereof, the therapeutic agent improves the individual's health, preferably wherein the therapeutic agent comprises an enzyme, a protein, a clotting factor, a vitamin, a peptide, a lipid, a toxin, or a combination thereof.

2. The physiologically-tailored tissue organoid of claim 1, wherein expression of the therapeutic agent is inducible by an inducer, preferably wherein the inducer comprises one or more of heat, cold, light, a protein, a hormone, a lipid, a chemical, metabolic changes, an electric potential or field, and combinations thereof.

3. The physiologically-tailored tissue organoid of claim 2, wherein when expression of the therapeutic agent is induced, the therapeutic agent is released into the individual's circulation.

4. The physiologically-tailored tissue organoid of claim 1, wherein the physiologically-tailored tissue organoid is biointegrated into an individual, preferably wherein the individual is an animal.

5. A physiologically-tailored tissue organoid according to any of claims 1 to 4 for use in a method of treating a disease in an individual in need thereof, the method comprising:
biointegrating the tissue organoid into an individual; and
administering a therapeutic agent to the individual, preferably wherein the individual is a human.

6. A physiologically-tailored tissue organoid, comprising:
a population of genetically engineered cells comprising at least one recombinant gene encoding a therapeutic agent;
wherein the therapeutic agent comprises at least one of mGLP1 and hBChE, and
wherein when the therapeutic agent is administered to an individual in need thereof, the therapeutic agent improves the individual's health, preferably wherein the therapeutic agent improves the individual's health by reducing the individual's seeking and/or consumption of at least one of nicotine, alcohol, cocaine, and amphetamine.

7. A physiologically-tailored tissue organoid comprising a population of genetically engineered cells comprising at least one recombinant gene encoding a therapeutic agent, wherein the therapeutic agent comprises hBChE, for use in a method of treating cocaine addiction in an individual in need thereof, the method comprising:
contacting the tissue organoid to the individual, preferably wherein the tissue organoid is transplanted into the individual.

8. The tissue organoid for use of claim 7, wherein hBChE is expressed constitutively or by induction with an inducer.

9. The tissue organoid for use of claim 7, wherein the expression of hBChE hydrolyzes cocaine in the blood of the individual.

10. The tissue organoid for use of claim 9, wherein the expression of hBChE causes decreased cocaine seeking and/or consumption by the individual.

11. A physiologically-tailored tissue organoid comprising a population of genetically engineered cells comprising at least one recombinant gene encoding a therapeutic agent, wherein the therapeutic agent comprises mGLP1 or an analog thereof, for use in a method of treating AUD in an individual in need thereof, the method comprising:
contacting the tissue organoid to the individual, preferably wherein the tissue organoid is transplanted into the individual.

12. The tissue organoid for use of claim 11, wherein mGLP1 or the analog thereof is expressed constitutively or by induction with an inducer.

13. The tissue organoid for use of claim 12, wherein the expression of mGLP1 or the analog thereof causes decreased alcohol seeking and/or consumption by the individual.

14. A physiologically-tailored tissue organoid comprising a population of genetically engineered cells comprising at least one recombinant gene encoding a therapeutic agent, wherein the therapeutic agent comprises mGLP1 or an analog thereof, for use in a method of treating nicotine addiction in an individual in need thereof, the method comprising:
contacting the tissue organoid to the individual, preferably wherein the tissue organoid is transplanted into the individual.

15. The tissue organoid for use of claim 14, wherein mGLP1 or the analog thereof is expressed constitutively or by induction with an inducer.

16. The tissue organoid for use of claim 15, wherein the expression of mGLP1 causes decreased nicotine seeking and consumption by the individual.

17. A tissue organoid comprising a population of genetically engineered cells comprising at least one recombinant gene encoding a therapeutic agent, wherein the therapeutic agent comprises mGLP1 or an analog thereof, for use in a method of treating amphetamine addiction in an individual in need thereof, the method comprising:
contacting the tissue organoid to the individual, preferably wherein the tissue organoid is transplanted into the individual.

18. The tissue organoid for use of claim 17, wherein mGLP1 or the analog thereof is expressed constitutively or by induction with an inducer.

19. The tissue organoid for use of claim 18, wherein the expression of mGLP1 causes decreased amphetamine seeking and consumption by the individual.
